# EUROPEAN PATENT SPECIFICATION

(11) **EP 2 917 214 B1**
(45) Date of publication and mention of the grant of the patent: **28.08.2019**
(21) Application number: 13783956.9
(22) Date of filing: 31.10.2013
(51) Int. Cl.: A61K 31/496, A61K 45/06, A61N 5/10, A61K 31/506, C07D 471/04

(54) **SUBSTITUTED PYRIMIDINYL AND PYRIDINYL-PYRROLOPYRIDINONES, PROCESS FOR THEIR PREPARATION AND THEIR USE AS KINASE INHIBITORS**
SUBSTITUIERTE PYRIMIDINYL- UND PYRIDINYL-PYRROLOPYRIDINONE, VERFAHREN ZU IHRER HERSTELLUNG UND IHRE VERWENDUNG ALS KINASEHEMMER
PYRIMIDINYL ET PYRIDINYL-PYRROLOPYRIDINONES SUBSTITUÉS, PROCÉDÉ POUR LEUR PRÉPARATION ET LEUR UTILISATION EN TANT QU'INHIBITEURS DE KINASES

(30) Priority: 07.11.2012 EP 12191679
(43) Date of publication of application: 16.09.2015
(73) Proprietor: Nerviano Medical Sciences S.r.l., 20014 Nerviano (MI) (IT)
(72) Inventor: MENICHINCHERI, Maria, 20124 Milan (IT); ANGIOLINI, Mauro, 21026 Gavirate (VA) (IT); BERTRAND, Jay Aaron, Didcot OX11 7UZ (GB); CARUSO, Michele, 20131 Milan (IT); POLUCCI, Paolo, 22070 Cassina Rizzardi (CO) (IT); QUARTIERI, Francesca, 28041 Arona (NO) (IT); SALOM, Barbara, 20057 Vedano al Lambro (MI) (IT); SALSA, Matteo, 28043 Bellinzago Novarese (NO) (IT); ZUCCOTTO, Fabio, 20153 Milan (IT)
(86) International application number: PCT/EP2013/072793
(87) International publication number: WO 2014/072220

(56) References cited:
- WO-A1-2010/145998

## Description

The present invention relates to certain substituted pyrimidinyl- and pyridinylpyrrolopyridinone compounds, which modulate the activity of protein kinases. The compounds of this invention are therefore useful in treating diseases caused by dysregulated protein kinase activity. The present invention also provides methods for preparing these compounds and pharmaceutical compositions comprising these compounds. RET is a single-pass transmembrane receptor belonging to the tyrosine kinase superfamily (reviewed in Arighi et al., Cytokine Growth Factor Rev, 2005, 16, 441-67). The extracellular portion of the RET protein contains four calcium-dependent cadherin-like repeats involved in ligand binding and a juxtamembrane cysteine-rich region necessary for the correct folding of RET extracellular domain, while the cytoplasmic portion of the receptor includes two tyrosine kinase subdomains. RET is the signaling component of a multiprotein complex: binding of RET to the glial-derived neurotrophic factor (GDNF) family ligands (GDNF, artemin, neurturin and persephin) through ligand-specific GDNF-family receptor alpha co-receptors (GFRα1-4) induces the formation of active RET dimers and the autophosphorylation of specific tyrosine residues in the cytoplasmic domain. These phosphorylated tyrosines function as docking sites for effector/adaptor proteins such as PLC-γ, PI3K, Shc, Grb2, Src, Enigma, STAT3, which in turn activate downstream signaling pathways, including Ras/Raf/ERK, PI3K/Akt/mTOR and PLC-γ/PKC. During embryogenesis RET signaling is critical for development of the enteric nervous system and for kidney organogenesis (Schuchardt et al., Nature, 1994, 367, 380-3). In adults RET is expressed in neural crest-derived cell types, such as neuroendocrine cells (thyroid parafollicular cells and adrenal medullary cells), peripheral ganglia, urogenital tract cells and spermatogonia.

Aberrant RET expression and/or activity have been demonstrated in different human cancers.

The oncogenic role of RET was firstly described in papillary thyroid carcinoma (PTC) (Grieco et al., Cell, 1990, 60, 557-63), which arises from follicular thyroid cells and is the most common thyroid malignancy. Approximately 20-30% of PTC harbor somatic chromosomal rearrangements (translocations or inversions) linking the promoter and the 5' portions of constitutively expressed, unrelated genes to the RET tyrosine kinase domain (reviewed in Greco et al., Q. J. Nucl. Med. Mol. Imaging, 2009, 53, 440-54), therefore driving its ectopic expression in thyroid cells. To date, twelve different fusion partners have been identified, all providing a protein/protein interaction domain that induces ligand-independent RET dimerization and constitutive kinase activity. The role of RET-PTC rearrangements in the pathogenesis of PTC has been confirmed in transgenic mice (Santoro et al., Oncogene, 1996, 12, 1821-6). Recently, a 10.6 Mb pericentric inversion in chromosome 10, where ret gene maps, has been identified in about 2% of lung adenocarcinoma patients, generating different variants of the chimeric gene KIF5B-RET (Ju et al., Genome Res., 2012, 22, 436-45; Kohno et al., 2012, Nature Med., 18, 375-7; Takeuchi et al., Nature Med., 2012, 18, 378-81; Lipson et al., 2012, Nature Med., 18, 382-4). The fusion transcripts are highly expressed and all the resulting chimeric proteins contain the N-terminal portion of the coiled-coil region of KIF5B, which mediates homodimerization, and the entire RET kinase domain. None of RET positive patients harbor other known oncogenic alterations (such as EGFR or K-Ras mutation, ALK translocation), supporting the possibility that KIF5B-RET fusion could be a driver mutation of lung adenocarcinoma. The oncogenic potential of KIF5B-RET has been confirmed by transfecting the fusion gene into cultured cell lines: similarly to what observed with RET-PTC fusion proteins, KIF5B-RET is constitutively phosphorylated and induces NIH-3T3 transformation and IL-3 independent growth of BA-F3 cells.

Besides rearrangements of the RET sequence, gain of function point mutations of RET proto-oncogene are also driving oncogenic events, as shown in medullary thyroid carcinoma (MTC), which arises from parafollicular calcitonin-producing cells (reviewed in: de Groot et al., Endocrine Rev., 2006, 27, 535-60; Wells and Santoro, Clin. Cancer Res., 2009, 15, 7119-7122). Around 25% of MTC are associated with multiple endocrine neoplasia type 2 (MEN2), a group of inherited cancer syndromes affecting neuroendocrine organs caused by germline activating point mutations of RET. In MEN2 subtypes (MEN2A, MEN2B and Familial MTC/FMTC) RET gene mutations have a strong phenotype-genotype correlation defining different MTC aggressiveness and clinical manifestations of the disease. In MEN2A syndrome mutations involve one of the six cysteine residues (mainly C634) located in the cysteine-rich extracellular region, leading to ligand-independent homodimerization and constitutive RET activation. Patients develop MTC at a young age (onset at 5-25 years) and may also develop pheochromocytoma (50%) and hyperparathyroidism. MEN2B is mainly caused by M918T mutation, which is located in the kinase domain. This mutation constitutively activates RET in its monomeric state and alters substrate recognition by the kinase. MEN2B syndrome is characterized by an early onset (< 1 year) and very aggressive form of MTC, pheochromocytoma (50% of patients) and ganglioneuromas. In FMTC the only disease manifestation is MTC, usually occurring at an adult age. Many different mutations have been detected, spanning the entire RET gene. The remaining 75% of MTC cases are sporadic and about 50% of them harbor RET somatic mutations: the most frequent mutation is M918T that, as in MEN2B, is associated with the most aggressive phenotype. Somatic point mutations of RET have also been described in other tumors such as colorectal cancer (Wood et al., Science, 2007, 318, 1108-13) and small cell lung carcinoma (Jpn. J. Cancer Res., 1995, 86, 1127-30).

RET signaling components have been found to be expressed in primary breast tumors and to functionally interact with estrogen receptor-α pathway in breast tumor cell lines (Boulay et al., Cancer Res. 2008, 68, 3743-51; Plaza-Menacho et al., Oncogene, 2010, 29, 4648-57), while RET expression and activation by GDNF family ligands could play an important role in perineural invasion by different types of cancer cells (Ito et al., Surgery, 2005, 138, 788-94; Gil et al., J Natl Cancer Inst., 2010, 102, 107-18; Iwahashi et al., Cancer, 2002, 94, 167-74).

Given the relevant role of RET in human cancers, RET tyrosine kinase inhibitors could be of high therapeutic value. Pyridylpyrrole and pyrimidylpyrrole derivatives active as kinase inhibitors, particularly as Cdk2 and Cdc7 inhibitors, have been disclosed in WO2005/013986 and in WO2005/014572, in the name of Pharmacia Italia SpA, for the treatment of hyperproliferative diseases such as cancer.

Substituted pyrimidinylpyrrolopyridinone derivatives are disclosed in WO2010/145998 and are useful in the treatment of diseases associated with a disregulated protein kinase activity, particularly RAF family kinases activity, like cancer. Despite these developments, there is still need for effective agents for said diseases.

The present inventors have now discovered the compounds of formula (I) described below, which, other than having a remarkable kinase inhibitory activity, have a significantly improved solubility so that to obtain better formulation and/or pharmacokinetic/pharmacodynamic properties. These compounds are thus useful in therapy as antitumor agents.

Accordingly, an object of the present invention is to provide a substituted pyrimidinyl- and pyridinylpyrrolopyridinone compound represented by formula (I), 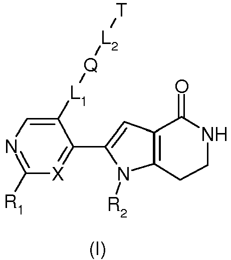 as recited in claims 1 and 4.

The present description discloses compounds of formula (I) wherein:
**X** is CH or N;
**R₁** is H or NHR₃, wherein R₃ is H, an optionally substituted group selected from straight or branched C₁-C₆ alkyl, heterocyclyl and COR', wherein R' is an optionally substituted group selected from straight or branched C₁-C₆ alkyl, C₃-C₆ carbocyclyl, aryl and heteroaryl;
**L₁** is CH₂-CH₂, CH=CH or C ≡ C;
**Q** is an optionally substituted group selected from aryl and heteroaryl;
**L₂** is C(RaRb)NRa, C(RaRb)C(RaRb)NRa, C(RaRb)NRaC(RaRb), NRaC(RaRb), NRaC(RaRb)C(RaRb), COO, C(RaRb)COO, C(RaRb)C(RaRb)COO, NRaSO₂, SO₂NRa, C(RaRb)SO₂NRa, NRaCONRa, NRaCSNRa, NRaCOO, CONRa, C(RaRb)CONRa, C(RaRb)C(RaRb)CONRa, CONRaC(RaRb), CONRaC(RaRb)C(RaRb), NRaCO, C(RaRb)NRaCO, C(RaRb)C(RaRb)NRaCO, NRaCOC(RaRb), NRaCOC(RaRb)C(RaRb), OC(RaRb)CONRa, C(RaRb)OC(RaRb), OC(RaRb)(C(RaRb))nC(RaRb), 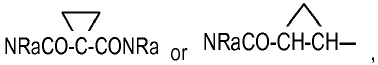 wherein
   Ra and Rb are independently H or an optionally substituted straight or branched C₁-C₆ alkyl and
   n is 0 or 1;
**T** is H or an optionally substituted group selected from straight or branched C₁-C₆ alkyl, C₃-C₆ carbocyclyl, heterocyclyl, aryl and heteroaryl;
**R₂** is H or an optionally substituted group selected from straight or branched C₁-C₆ alkyl, C₃-C₆ carbocyclyl, heterocyclyl, aryl and heteroaryl;
or pharmaceutically acceptable salts thereof,

with the proviso that the compound 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(4-trifluoromethyl-phenyl)-urea is excluded.

The present invention also provides methods of preparing the substituted pyrimidinyl- and pyridinylpyrrolopyridinone compounds, represented by formula (I), prepared through a process consisting of standard synthetic transformations. The present disclosure also relates to a method for treating diseases caused by and/or associated with dysregulated protein kinase activity, particularly RAF family, protein kinase C in different isoforms, RET, Abl, Aurora A, Aurora B, Aurora C, EphA, EphB, FLT3, KIT, LCK, LYN, EGF-R, PDGF-R, FGF-R, PAK-4, P38 alpha, TRKA, TRKB, VEGFR, more particularly RET family kinases, which comprises administering to a mammal in need thereof, more particularly a human, an effective amount of a substituted pyrimidinyl- and pyridinylpyrrolopyridinone compound represented by formula (I) as defined above.

A preferred method of the present disclosure is to treat a disease caused by and/or associated with dysregulated protein kinase activity selected from the group consisting of cancer, cell proliferative disorders, viral infections, immune-related and neurodegenerative disorders.

Another preferred method of the present disclosure is to treat specific types of cancer including but not limited to: carcinoma such as bladder, breast, colon, kidney, liver, lung, including small cell lung cancer, esophagus, gall-bladder, ovary, pancreas, stomach, cervix, thyroid, prostate, and skin, including squamous cell carcinoma; hematopoietic tumors of lymphoid lineage including leukaemia, acute lymphocitic leukaemia, acute lymphoblastic leukaemia, B-cell lymphoma, T-cell-lymphoma, Hodgkin's lymphoma, non-Hodgkin's lymphoma, hairy cell lymphoma and Burkett's lymphoma; hematopoietic tumors of myeloid lineage, including acute and chronic myelogenous leukaemias, myelodysplastic syndrome and promyelocytic leukaemia; tumors of mesenchymal origin, including fibrosarcoma and rhabdomyosarcoma; tumors of the central and peripheral nervous system, including astrocytoma neuroblastoma, glioma and schwannomas; other tumors, including melanoma, seminoma, teratocarcinoma, osteosarcoma, xeroderma pigmentosum, keratoxanthoma, thyroid cancers, such as papillary thyroid carcinoma and medullary thyroid carcinoma, and Kaposi's sarcoma.

Another preferred method of the present disclosure is to treat specific cellular proliferation disorders such as, for example, benign prostate hyperplasia, familial adenomatosis polyposis, neurofibromatosis, psoriasis, vascular smooth cell proliferation associated with atherosclerosis, pulmonary fibrosis, arthritis, glomerulonephritis and post-surgical stenosis and restenosis.

Another preferred method of the present disclosure is to treat viral infections, comprising the prevention of AIDS development in HIV-infected individuals.

Another preferred method of the present disclosure is to treat immune-related disorders including but not limited to: transplant rejection, skin disorders like psoriasis, allergies, asthma and autoimmune-mediated diseases such as rheumatoid arthritis (RA), systemic lupus erythematosus (SLE), Crohn's disease and amyotrophic lateral sclerosis. Another preferred method of the present disclosure is to treat neurodegenerative disorders including but not limited to: Alzheimer's disease, degenerative nerve diseases, encephalitis, Stroke, Parkinson's Disease, Multiple Sclerosis, Amyotrophic Lateral Sclerosis (ALS or Lou Gehrig's Disease), Huntington's Disease and Pick's Disease.

In addition, the method of the present disclosure also relates to tumor angiogenesis and metastasis inhibition as well as the treatment of organ transplant rejection and host versus graft disease.

Moreover, the method of the present disclosure further comprises subjecting the mammal in need thereof to a radiation therapy or chemotherapy regimen in combination with at least one cytostatic or cytotoxic agent.

The present invention also provides a pharmaceutical composition comprising a therapeutically effective amount of a compound of formula (I) as defined in claim 1, or of a compound of claim 4, or the pharmaceutically acceptable salts thereof, as defined above, and at least one pharmaceutically acceptable excipient, carrier and/or diluent.

The present invention further provides a pharmaceutical composition of a compound of the formula (I) as defined in claim 1, or of a compound of claim 4, further comprising one or more chemotherapeutic - e.g. cytostatic or cytotoxic - agents, antibiotic-type agents, alkylating agents, antimetabolite agents, hormonal agents, immunological agents, interferon-type agents, cyclooxygenase inhibitors (e.g. COX-2 inhibitors), matrixmetalloprotease inhibitors, telomerase inhibitors, tyrosine kinase inhibitors, anti-growth factor receptor agents, anti-HER agents, anti-EGFR agents, anti-angiogenesis agents (e.g. angiogenesis inhibitors), farnesyl transferase inhibitors, ras-raf signal transduction pathway inhibitors, cell cycle inhibitors, other cdks inhibitors, tubulin binding agents, topoisomerase I inhibitors, topoisomerase II inhibitors, and the like.

Moreover the invention provides an in vitro method for inhibiting the RET family protein activity which comprises contacting the said protein with an effective amount of a compound of formula (I) as defined in claim 1 or of a compound of claim 4.

Additionally, the invention provides a product or kit comprising a compound of formula (I) as defined in claim 1, or comprising a compound of claim 4, or the pharmaceutically acceptable salts thereof, and one or more chemotherapeutic agents, as a combined preparation for simultaneous, separate or sequential use in anticancer therapy.

In yet another aspect the invention provides a compound of formula (I) as defined in claim 1, or a compound of claim 4, or the pharmaceutically acceptable salts thereof, for use as a medicament.

Moreover the invention provides a compound of formula (I) as defined in claim 1, or a compound of claim 4, or the pharmaceutically acceptable salts thereof, for use in a method of treating cancer.

Finally, the disclosure relates to the use of a compound of formula (I) or a pharmaceutically acceptable salt thereof, as defined above, in the manufacture of a medicament with anticancer activity.

Unless otherwise specified, when referring to the compounds of formula (I) per se as well as to any pharmaceutical composition thereof or to any therapeutic treatment comprising them, the present disclosure includes all of the hydrates, solvates, complexes, metabolites, prodrugs, carriers, N-oxides and pharmaceutically acceptable salts of the compounds of this disclosure.

A metabolite of a compound of formula (I) is any compound into which this same compound of formula (I) is converted *in vivo,* for instance upon administration to a mammal in need thereof. Typically, without however representing a limiting example, upon administration of a compound of formula (I), this same derivative may be converted into a variety of compounds, for instance including more soluble derivatives like hydroxylated derivatives, which are easily excreted. Hence, depending upon the metabolic pathway thus occurring, any of these hydroxylated derivatives may be regarded as a metabolite of the compounds of formula (I).

Prodrugs are any covalently bonded compounds, which release *in vivo* the active parent drug according to formula (I).

N-oxides are compounds of formula (I) wherein nitrogen and oxigen are tethered through a dative bond.

If a stereogenic center or another form of an asymmetric center is present in a compound of the present disclosure, all forms of such isomer or isomers, including enantiomers and diastereomers, are intended to be covered herein. Compounds containing a stereogenic center may be used as a racemic mixture, an enantiomerically enriched mixture, or the racemic mixture may be separated using well-known techniques and an individual enantiomer may be used alone. In cases in which compounds have unsaturated carbon-carbon double bonds, both the cis (Z) and trans (E) isomers are within the scope of this disclosure.

In cases wherein compounds may exist in tautomeric forms, such as keto-enol tautomers, each tautomeric form is contemplated as being included within this disclosure whether existing in equilibrium or predominantly in one form. Pharmaceutically acceptable salts of the compounds of formula (I) include the salts with inorganic or organic acids. Pharmaceutically acceptable salts of the compounds of formula (I) also include the salts with inorganic or organic bases.

With the term "straight or branched C₁-C₆ alkyl", we intend any of the groups such as, for instance, methyl, ethyl, n-propyl, isopropyl, n-butyl, isobutyl, tert-butyl, sec-butyl, n-pentyl, n-hexyl, and the like.

With the term "straight or branched C₁-C₃ alkyl", we intend any of the groups such as, for instance, methyl, ethyl, n-propyl, isopropyl.

With the term "C₃-C₆ carbocyclyl" we intend, unless otherwise provided, a 3- to 6-membered all-carbon monocyclic ring, which may contain one or more double bonds but does not have a completely conjugated *π*-electron system. Examples of carbocyclyl groups, without limitation, are cyclopropane, cyclobutane, cyclopentane, cyclopentene, cyclohexane, cyclohexene and cyclohexadiene. The C₃-C₆ carbocyclyl ring can be optionally further fused or linked to aromatic and non-aromatic carbocyclic and heterocyclic rings.

With the term "heterocyclyl" we intend a 3- to 7-membered saturated or partially unsaturated carbocyclic ring where one or more carbon atoms are replaced by heteroatoms such as nitrogen, oxygen and sulfur. Non limiting examples of heterocyclyl groups are, for instance, pyrane, pyrrolidine, pyrroline, imidazoline, imidazolidine, pyrazolidine, pyrazoline, thiazoline, thiazolidine, dihydrofuran, tetrahydrofuran, 1,3-dioxolane, piperidine, piperazine, 1,4-diazepanyl, morpholine and the like. The heterocyclyl ring can be optionally further fused or linked to aromatic and non-aromatic carbocyclic and heterocyclic rings.

With the term "C₂-C₆ alkenyl" we intend an aliphatic C₂-C₈ hydrocarbon chain containing at least one carbon-carbon double bond and which can be straight or branched. Representative examples include, but are not limited to, ethenyl, 1-propenyl, 2-propenyl, 1- or 2-butenyl and the like.

With the term "C₂-C₆ alkynyl" we intend an aliphatic C₂-C₈ hydrocarbon chain containing at least one carbon-carbon triple bond and which can be straight or branched. Representative examples include, but are not limited to, ethynyl, 1-propynyl, 2-propynyl, 1- or 2-butynyl and the like.

The term "aryl" refers to a mono-, bi- or poly-carbocyclic hydrocarbon with from 1 to 4 ring systems, optionally further fused or linked to each other by single bonds, wherein at least one of the carbocyclic rings is "aromatic", wherein the term "aromatic" refers to completely conjugated *π*-electron bond system. Non limiting examples of such aryl groups are phenyl, α- or β-naphthyl, α- or β-tetrahydronaphthalenyl, biphenyl and indanyl groups. The aryl ring can be optionally further fused or linked to aromatic and non-aromatic carbocyclic and heterocyclic rings.

The term "heteroaryl" refers to aromatic heterocyclic rings, typically 5- to 7-membered heterocycles, with from 1 to 3 heteroatoms selected among nitrogen, oxygen and sulfur; the heteroaryl ring can be optionally further fused or linked to aromatic and non-aromatic carbocyclic and heterocyclic rings. Not limiting examples of such heteroaryl groups are, for instance, pyridyl, pyrazinyl, pyrimidinyl, pyridazinyl, indolyl, imidazolyl, thiazolyl, isothiazolyl, pyrrolyl, phenyl-pyrrolyl, furyl, phenyl-furyl, oxazolyl, isoxazolyl, pyrazolyl, thienyl, thiadiazolyl, isoxazolyl, isothiazolyl, oxadiazolyl, indazolyl, cinnolinyl, benzo[1,3]dioxolyl, benzo[1,4]dioxinyl, benzothiazolyl, benzothienyl, isoindolinyl, benzoimidazolyl, quinolinyl, isoquinolinyl, 1,2,3-triazolyl, 1-phenyl-1,2,3-triazolyl, 2,3-dihydroindolyl, 2,3-dihydrobenzofuranyl, 2,3-dihydrobenzothiophenyl, benzopyranyl, 2,3-dihydrobenzoxazinyl, 2,3-dihydroquinoxalinyl and the like.

According to the present disclosure and unless otherwise provided, any of the above R₂, R₃, R', Ra, Rb, T and Q groups may be optionally substituted, in any of their free positions, by one or more groups, for instance 1 to 6 groups, independently selected from: halogen, nitro, oxo groups (=O), cyano, C₁-C₆ alkyl, polyfluorinated alkyl, polyfluorinated alkoxy, C₂-C₆ alkenyl, C₂-C₆ alkynyl, hydroxyalkyl, aryl, arylalkyl, heteroaryl, heteroarylalkyl, heterocyclyl, heterocyclylalkyl, heterocyclylheterocyclyl, C₃-C₆ cycloalkyl, hydroxy, polyhydroxyalkyl, alkoxy, aryloxy, heterocyclyloxy, methylenedioxy, alkylcarbonyloxy, alkylheterocyclyl, alkylheteroaryl, arylcarbonyloxy, cycloalkenyloxy, heterocyclylcarbonyloxy, alkylideneaminooxy, carboxy, alkoxycarbonyl, aryloxycarbonyl, cycloalkyloxycarbonyl, heterocyclylalkyloxycarbonyl, amino, ureido, alkylamino, dialkylamino, arylamino, diarylamino, heterocyclylamino, formylamino, alkylcarbonylamino, arylcarbonylamino, heterocyclylcarbonylamino, aminocarbonyl, alkylaminocarbonyl, dialkylaminocarbonyl, arylaminocarbonyl, heterocyclylaminocarbonyl, alkoxycarbonylamino, hydroxyaminocarbonyl alkoxyimino, alkylsulfonylamino, arylsulfonylamino, heterocyclylsulfonylamino, formyl, alkylcarbonyl, arylcarbonyl, cycloalkylcarbonyl, heterocyclylcarbonyl, alkylsulfonyl, arylsulfonyl, aminosulfonyl, alkylaminosulfonyl, dialkylaminosulfonyl, arylaminosulfonyl, heterocyclylaminosulfonyl, arylthio, alkylthio, phosphonate and alkylphosphonate. In their turn, whenever appropriate, each of the above substituent may be further substituted by one or more of the aforementioned groups.

With the term "halogen" we intend a fluorine, chlorine, bromine or iodine atom.

With the term "polyfluorinated alkyl" or "polyfluorinated alkoxy" we intend any of the above straight or branched C₁-C₆ alkyl or alkoxy groups which are substituted by more than one fluorine atom such as, for instance, trifluoromethyl, trifluoroethyl, 1,1,1,3,3,3-hexafluoropropyl, trifluoromethoxy and the like.

With the term "hydroxyalkyl" we intend any of the above C₁-C₆ alkyl, bearing a hydroxyl group such as, for instance, hydroxymethyl, 2-hydroxyethyl, 3-hydroxypropyl and the like.

From all of the above, it is clear to the skilled person that any group which name is a composite name such as, for instance, "arylamino" has to be intended as conventionally construed by the parts from which it derives, e.g. by an amino group which is further substituted by aryl, wherein aryl is as above defined.

Likewise, any of the terms such as, for instance, alkylthio, alkylamino, dialkylamino, alkoxycarbonyl, alkoxycarbonylamino, heterocyclylcarbonyl, heterocyclylcarbonylamino, cycloalkyloxycarbonyl and the like, include groups wherein the alkyl, alkoxy, aryl, C₃-C₆ cycloalkyl and heterocyclyl moieties are as above defined.

A preferred class of compounds of formula (I) are the compounds wherein:
**R₁** is H or NHR₃, wherein R₃ is H, an optionally substituted group selected from straight or branched C₁-C₃ alkyl, heterocyclyl and COR';
**L₁** is C ≡ C;
**Q** is an optionally substituted group selected from aryl and heteroaryl, wherein the heteroaryl is monocyclic or bicyclic, and said bicyclic heteroaryl contains a nitrogen atom which the **L₂** group is attached to;
and **X, R', L₂**, **T** and **R₂** are as defined above.

Another further preferred class of compounds of formula (I) are the compounds wherein:
**Q** is an optionally substituted aryl or heteroaryl selected from: 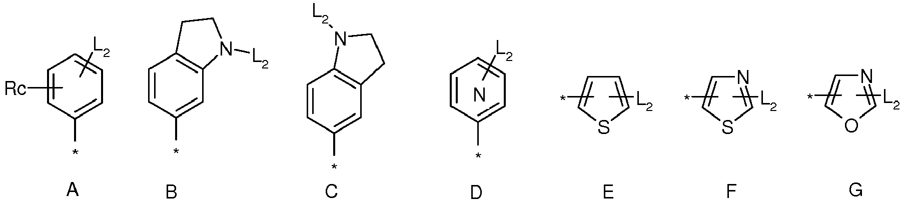
wherein **Rc** is selected from methyl and halogen, preferably fluorine;
**L₂** is CONRa, C(RaRb)CONRa, C(RaRb)C(RaRb)CONRa, CONRaC(RaRb), OC(RaRb)CONRa, SO₂NRa or C(RaRb)SO₂NRa, wherein Ra and Rb are both hydrogen;
**T** is a substituted aryl of formula J: 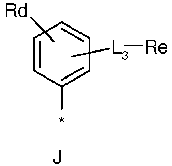 wherein
   **Rd** is halogen, optionally substituted straight or branched C₁-C₄ alkyl, C₃-C₆ cycloalkyl or trifluoromethyl;
   **L₃** is direct linkage, O, NH, NCH₃, CH₂, CH₂NH, CH₂NCH₃ or C=O;
   **Re** is
      an optionally substituted heterocyclyl or
      an optionally substituted straight or branched C₁-C₆ alkyl chain, wherein
         from 1 to 3 carbon atoms of said alkyl may be independently substituted by N or O, or
      NRfRg, wherein
         Rf and Rg are each independently hydrogen or
         an optionally substituted straight or branched C₁-C₆ alkyl chain, wherein
            from 1 to 3 carbon atoms of said alkyl may be independently substituted by N or O, or
         Rf and Rg joined together with the nitrogen atom might represent a heterocyclic ring;
   and **X, R₁**, **R₂** and **L₁** are as defined above.

Another further preferred class of compounds of formula (I) are the compounds wherein:
**Q** is an optionally substituted aryl of structure A: 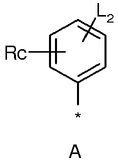
wherein **Rc** is as defined above;
**L₂** is NRaCONRa, NRaCSNRa, NRaCOO, NRaCO, NRaCOC(RaRb), NRaCOC(RaRb)C(RaRb), C(RaRb)NRaCO, NRaSO₂, 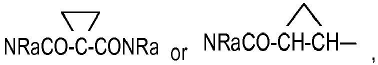 wherein Ra and Rb are both hydrogen;
and **X, R₁**, **R₂**, **L₁** and **T** are as defined above.

Another further preferred class of compounds of formula (I) are the compounds wherein:
**L₂** is C(RaRb)NRaC(RaRb), C(RaRb)OC(RaRb), and OC(RaRb)(C(RaRb))nC(RaRb), wherein
   Ra and Rb are both hydrogen and
   n is 0 or 1;
and **X, R₁**, **R₂**, **L₁**, **Q** and **T** are as defined above.

Specific, not limiting, preferred compounds (cmpds) of the present disclosure, whenever appropriate in the form of pharmaceutically acceptable salts, are the compounds listed below:
1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[4-(4-ethyl-piperazin-1-ylmethyl)-3-trifluoromethyl-phenyl]-urea (Cmpd 8),
1-{3-[4-(1-Methyl-4-oxo-4,5,6,7-tetrahydro-1H-pyrrolo[3,2-c]pyridin-2-yl)-2-(piperidin-4-ylamino)-pyrimidin-5-ylethynyl]-phenyl}-3-(4-trifluoromethyl-phenyl)-urea hydrochloride (Cmpd 18),
1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[3-(4-ethyl-piperazin-1-ylmethyl)-4-trifluoromethyl-phenyl]-urea (Cmpd 22),
1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(4trifluoromethyl-cyclohexyl)-urea (*trans* isomer) (Cmpd 27),
1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[3-(4-ethyl-piperazin-1-ylmethyl)-5-trifluoromethyl-phenyl]-urea (Cmpd 29),
1-{4-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[4-(4-ethyl-piperazin-1-ylmethyl)-3-trifluoromethyl-phenyl]-urea (Cmpd 30),
1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[3-(4-ethyl-piperazine-1-carbonyl)-5-trifluoromethyl-phenyl]-urea (Cmpd 31),
1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[3-(4-methyl-piperazin-1-yl)-phenyl]-urea (Cmpd 33),
1-{5-[2-Amino-4-(1-methyl-4-oxo-4,5,6,7-tetrahydro-1H-pyrrolo[3,2-c]pyridin-2-yl)-pyrimidin-5-ylethynyl]-2-fluoro-phenyl}-3-[4-(4-ethyl-piperazin-1-ylmethyl)-3-trifluoromethyl-phenyl]-urea (Cmpd 36),
1-[4-(4-Ethyl-piperazin-1-ylmethyl)-3-trifluoromethyl-phenyl]-3-{3-[4-(1-methyl-4-oxo-4,5,6,7-tetrahydro-1H-pyrroio[3,2-c]pyridin-2-yl)-pyridin-3-ylethynyl]-phenyl}-urea (Cmpd 38),
1-[4-(4-Ethyl-piperazin-1-ylmethyl)-3-trifluoromethyl-phenyl]-3-{3-[2-(2-hydroxy-ethylamino)-4-(1-methyl-4-oxo-4,5,6,7-tetrahydro-1H-pyrrolo[3,2-c]pyridin-2-yl)-pyrimidin-5-ylethynyl]-phenyl}-urea (Cmpd 43),
6-[2-Amino-4-(1-methyl-4-oxo-4,5,6,7-tetrahydro-1H-pyrrolo[3,2-c]pyridin-2-yl)-pyrimidin-5-ylethynyl]-2,3-dihydro-indole-1-carboxylic acid [4-(4-ethyl-piperazin-1-ylmethyl)-3-trifluoromethyl-phenyl]-amide (Cmpd 44),
5-[2-Amino-4-(1-methyl-4-oxo-4,5,6,7-tetrahydro-1H-pyrrolo[3,2-c]pyridin-2-yl)-pyrimidin-5-ylethynyl]-2,3-dihydro-indole-1-carboxylic acid [4-(4-ethyl-piperazin-1-ylmethyl)-3-trifluoromethyl-phenyl]-amide (Cmpd 45),
3-[2-Amino-4-(1-methyl-4-oxo-4,5,6,7-tetrahydro-1H-pyrrolo[3,2-c]pyridin-2-yl)-pyrimidin-5-ylethynyl]-N-[4-(4-ethyl-piperazin-1-ylmethyl)-3-trifluoromethyl-phenyl]-benzamide (Cmpd 48),
N-[4-(4-Ethyl-piperazin-1-ylmethyl)-3-trifluoromethyl-phenyl]-3-[2-methylamino-4-(1-methyl-4-oxo-4,5,6,7-tetrahydro-1H-pyrrolo[3,2-c]pyridin-2-yl)-pyrimidin-5-ylethynyl]-benzamide (Cmpd 49),
3-[2-Amino-4-(1-methyl-4-oxo-4,5,6,7-tetrahydro-1H-pyrrolo[3,2-c]pyridin-2-yl)-pyrimidin-5-ylethynyl]-N-[4-(4-ethyl-piperazin-1-ylmethyl)-3-trifluoromethyl-phenyl]-4-methyl-benzamide (Cmpd 50),
5-[2-Amino-4-(1-methyl-4-oxo-4,5,6,7-tetrahydro-1H-pyrrolo[3,2-c]pyridin-2-yl)-pyrimidin-5-ylethynyl]-thiophene-2-carboxylic acid [4-(4-ethyl-piperazin-1-ylmethyl)-3-trifluoromethyl-phenyl]-amide (Cmpd 51),
2-[2-Amino-4-(1-methyl-4-oxo-4,5,6,7-tetrahydro-1H-pyrrolo[3,2-c]pyridin-2-yl)-pyrimidin-5-ylethynyl]-thiazole-5-carboxylic acid [4-(4-ethyl-piperazin-1-ylmethyl)-3-trifluoromethyl-phenyl]-amide (Cmpd 52),
N-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-4-trifluoromethyl-benzamide (Cmpd 57),
N-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-2-(4-trifluoromethyl-phenyl)-acetamide (Cmpd 58),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenoxy}-N-phenyl-acetamide hydrochloride (Cmpd 59),
N-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(3-trifluoromethyl-phenyl)-propionamide hydrochloride (Cmpd 73),
3-[2-Amino-4-(1-methyl-4-oxo-4,5,6,7-tetrahydro-1H-pyrrolo[3,2-c]pyridin-2-yl)-pyrimidin-5-ylethynyl]-N-(3-trifluoromethyl-benzyl)-benzamide hydrochloride (Cmpd 74),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-ethyl-piperazin-1-ylmethyl)-3-trifluoromethyl-phenyl]-acetamide (Cmpd 77),
N-[4-(4-Ethyl-piperazin-1-ylmethyl)-3-trifluoromethyl-phenyl]-2-{3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-acetamide (Cmpd 78),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[3-(4-ethyl-piperazin-1-ylmethyl)-4-trifluoromethyl-phenyl]-acetamide (Cmpd 82),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-indan-5-yl-acetamide (Cmpd 100),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-benzothiazol-6-yl-acetamide (Cmpd 102),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(5-tert-butyl-2-methyl-2H-pyrazol-3-yl)-acetamide (Cmpd 105),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-quinolin-3-yl-acetamide (Cmpd 106),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3-trifluoromethyl-benzyl)-acetamide (Cmpd 111),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-ethyl-piperazin-1-ylmethyl)-3-fluoro-phenyl]-acetamide (Cmpd 120),
2-{5-[2-Amino-4-(1-methyl-4-oxo-4,5,6,7-tetrahydro-1H-pyrrolo[3,2-c]pyridin-2-yl)-pyrimidin-5-ylethynyl]-pyridin-3-yl}-N-[4-(4-ethyl-piperazin-1-ylmethyl)-3-trifluoromethyl-phenyl]-acetamide (Cmpd 122),
2-{4-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-ethyl-piperazin-1-ylmethyl)-3-trifluoromethyl-phenyl]-acetamide (Cmpd 123),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[3-(4-ethyl-piperazine-1-carbonyl)-4-trifluoromethyl-phenyl]-acetamide (Cmpd 127),
2-{5-[2-Amino-4-(1-methyl-4-oxo-4,5,6,7-tetrahydro-1H-pyrrolo[3,2-c]pyridin-2-yl)-pyrimidin-5-ylethynyl]-thiophen-2-yl}-N-[4-(4-ethyl-piperazin-1-ylmethyl)-3-trifluoromethyl-phenyl]-acetamide (Cmpd 129),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-isopropyl-phenyl)-acetamide (Cmpd 133),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-chloro-3-(4-ethyl-piperazin-1-ylmethyl)-phenyl]-acetamide (Cmpd 138),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-chloro-3-trifluoromethyl-phenyl)-acetamide (Cmpd 141),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-[1,4]diazepan-1-ylmethyl)-3-trifluoromethyl-phenyl]-acetamide (Cmpd 149),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[3-cyclopropyl-4-(4-methyl-piperazin-1-ylmethyl)-phenyl]-acetamide (Cmpd 154),
2-{3-[2-Amino-4-(1-methyl-4-oxo-4,5,6,7-tetrahydro-1H-pyrrolo[3,2-c]pyridin-2-yl)-pyrimidin-5-ylethynyl]-4-fluoro-phenyl}-N-[4-(4-ethyl-piperazin-1-ylmethyl)-3-trifluoromethyl-phenyl]-acetamide (Cmpd 155),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-propyl-piperazin-1-ylmethyl)-3-trifluoromethyl-phenyl]-acetamide (Cmpd 164),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-{[methyl-(1-methyl-piperidin-4-yl)-amino]-methyl}-3-trifluoromethyl-phenyl)-acetamide (Cmpd 166),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(1-methyl-piperidin-4-yloxy)-3-trifluoromethyl-phenyl]-acetamide (Cmpd 172),
2-{3-[2-Methylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide(Cmpd 174),
N-[4-(4-Isopropyl-piperazin-1-ylmethyl)-3-trifluoromethyl-phenyl]-2-{3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-acetamide (Cmpd 176),
2-{3-[2-Methylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-propyl-piperazin-1-ylmethyl)-3-trifluoromethyl-phenyl]-acetamide (Cmpd 179),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(3-oxo-piperazin-1-ylmethyl)-3-trifluoromethyl-phenyl]-acetamide (Cmpd 184),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-{[(2-hydroxy-ethyl)-methyl-amino]-methyl}-3-trifluoromethyl-phenyl)-acetamide (Cmpd 189),
2-{4-Fluoro-3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide (Cmpd 190),
2-(3-{2-Amino-4-[1-(2-hydroxy-ethyl)-4-oxo-4,5,6,7-tetrahydro-1H-pyrrolo[3,2-c]pyridin-2-yl]-pyrimidin-5-ylethynyl}-phenyl)-N-[4-(4-ethyl-piperazin-1-ylmethyl)-3-trifluoromethyl-phenyl]-acetamide (Cmpd 191),
2-{3-[2-Ethylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide (cmpd 193),
2-(3-{2-Amino-4-[1-(1-methyl-piperidin-4-yl)-4-oxo-4,5,6,7-tetrahydro-1H-pyrrolo[3,2-c]pyridin-2-yl]-pyrimidin-5-ylethynyl}-phenyl)-N-(4-chloro-3-trifluoromethyl-phenyl)-acetamide (Cmpd 195),
N-[4-((S)-3-Dimethylamino-pyrrolidin-1-ylmethyl)-3-trifluoromethyl-phenyl]-2-{3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-acetamide (Cmpd 198),
2-{3-[2-Acetylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide (Cmpd 200),
2-{3-[6-Amino-4-(1-methyl-4-oxo-4,5,6,7-tetrahydro-1H-pyrrolo[3,2-c]pyridin-2-yl)-pyridin-3-ylethynyl]-phenyl}-N-[4-(4-methyl-piperazin-1-ylmethyl)-3-trifluoromethyl-phenyl]-acetamide (Cmpd 201),
2-{3-[2-Isopropylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide (Cmpd 202) and
{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-carbamic acid phenyl ester (Cmpd 203).

The present invention also provides a process for the preparation of a compound of formula (I) as defined in claim 1 or for preparing a compound of claim 4, by using the reaction routes and synthetic schemes described below, employing the techniques available in the art and starting materials readily available. The preparation of certain embodiments of the present disclosure is described in the examples that follow, but those of ordinary skill in the art will recognize that the preparations described may be readily adapted to prepare other embodiments of the present disclosure. For example, the synthesis of non-examplified compounds according to the disclosure may be performed by modifications apparent to those skilled in the art, for instance by appropriately protecting interfering groups, by changing to other suitable reagents known in the art, or by making routine modifications of reaction conditions. Alternatively other reactions referred to herein or known in the art will be recognized as having adaptability for preparing other compounds of the disclosure.

The compounds of this disclosure can be prepared from readily available starting materials using the following general methods and procedures. Unless otherwise indicated, the starting materials are known compounds or may be prepared from known compounds according to well known procedures. It will be appreciated that, where typical or preferred process conditions (i.e., reaction temperatures, times, mole ratios of reactants, solvents, pressures) are given, other process conditions can also be used unless otherwise stated. Optimum reaction conditions may vary with the particular reactants or solvent used, but such conditions can be determined by one skilled in the art by routine optimization procedures. Additionally, as will be apparent to those skilled in the art, conventional protecting groups may be necessary to prevent certain functional groups from undergoing undesired reactions. Suitable protecting groups for various functional groups as well as suitable conditions for protecting and deprotecting particular functional groups are well known in the art. For example, numerous protecting groups are described in T.W.Greene and P.G.M.Wuts, Protecting Groups in Organic Synthesis, Second Edition, Wiley, New York, 1991, and references cited therein.

A compound of formula (I) can be prepared according to the general synthetic processes described hereafter in Schemes from 1 to 11.

In the following Scheme 1 the general preparation of a compound of formula (I) is shown, 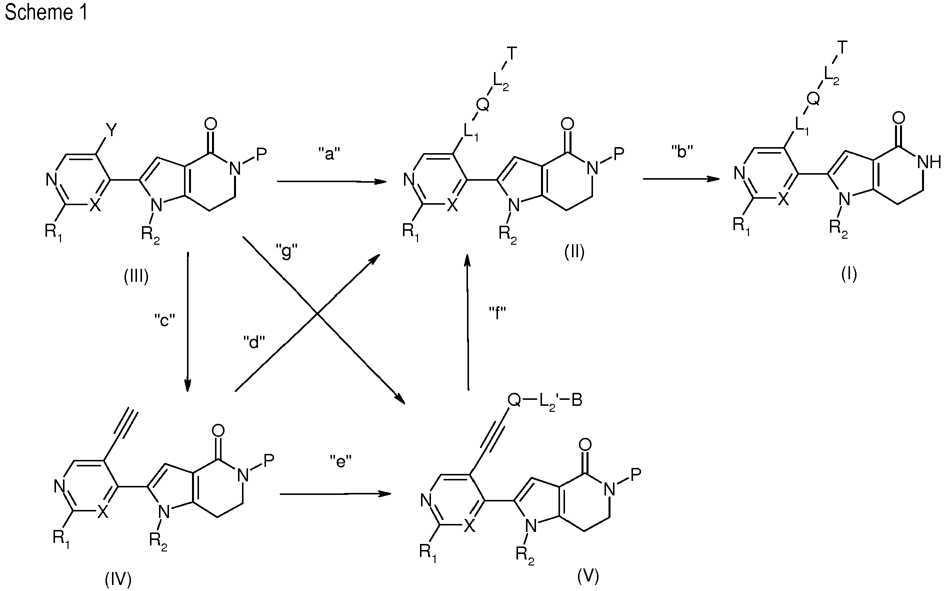 wherein P is hydrogen or a suitable protecting group such as the tert-butoxycarbonyl group, Y is a suitable halogen (e.g. Cl, Br, I), B is COOH or NHRa, L₂' is direct linkage, C(RaRb), C(RaRb)C(RaRb) or OC(RaRb) and R₁, R₂, X, L₁, Q, L₂, T, Ra and Rb are as defined above.

Compounds of formula (I) are prepared by deprotection of compounds of formula (II), when P is a suitable protecting group, or directly by compounds of formula (III), (IV) or (V), when P is hydrogen.

A compound of formula (I) is prepared according to any of the four alternative synthetic Methods A, B, C or D, summarized below.

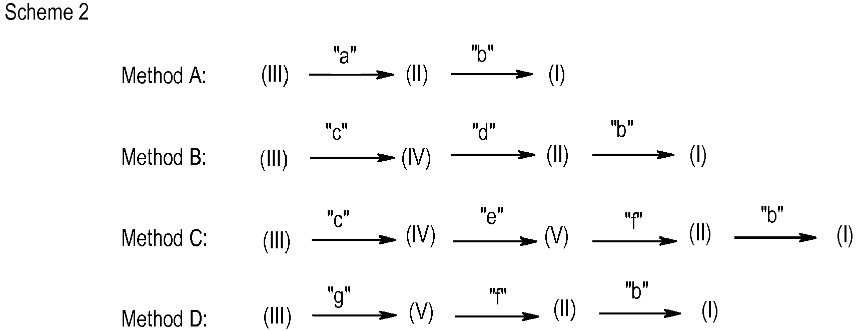

Generally, the process for preparing a compound of formula (I) as defined above comprises the following steps:
Method A:
   step a: coupling of a pyrrolopyridinone derivative of formula (III) 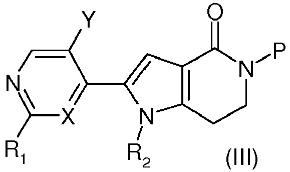 wherein X, R₁ and R₂ are as defined above, Y is halogen and P is a suitable protecting group such as the tert-butoxycarbonyl group, with an intermediate of formula (1a)-(1h) 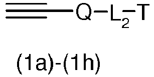 wherein Q, T and L₂ are as defined above, under Sonogashira reaction conditions;
   step b: deprotection of the resultant intermediate of formula (II) 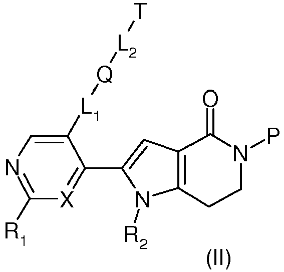 wherein X, R₁, R₂, L₁, L₂, P, Q and T are as defined above, under acid conditions to yield a compound of formula (I) as defined above;
alternatively, Method B:
   step c: coupling of a pyrrolopyridinone derivative of formula (III), as defined above, with ethynyl-trimethyl-silane under Sonogashira reaction conditions, followed by removal of the trimethylsilyl group under basic conditions;
   step d: reaction of the resultant intermediate of formula (IV) 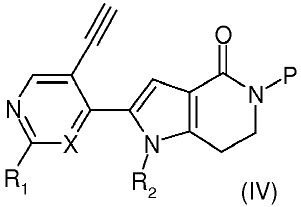 wherein X, P, R₁ and R₂ are as defined above, with an intermediate of formula (3a)-(3h) 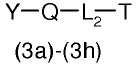 wherein Y is halogen and Q, L₂ and T are as defined above, under Sonogashira reaction conditions;
   step b: deprotection of the resultant intermediate of formula (II) as defined above;
alternatively, Method C:
   step c: coupling of a pyrrolopyridinone derivative of formula (III), as defined above, with ethynyl-trimethyl-silane under Sonogashira reaction conditions, followed by removal of the trimethylsilyl group under basic conditions;
   step e: reaction of the resultant intermediate of formula (IV) as defined above, with an intermediate of formula Y-Q-L₂'-CO-Y' wherein L₂' is direct linkage, -C(RaRb)-, -C(RaRb)C(RaRb)- or -OC(RaRb)-, Y is halogen, Y' is OH and Q, Ra and Rb are as defined above, or with an intermediate of formula Y-Q-L₂'-NH-Ra wherein Y, Q, L₂' and Ra are as defined above under Sonogashira reaction conditions;
   step f: coupling the resultant intermediate of formula (V) 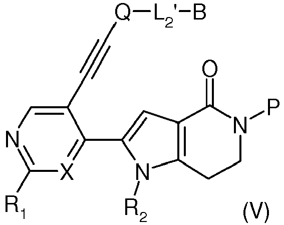 wherein B is COOH or NHRa and X, Q, Ra, R₁, R₂, L₂' and P are as defined above, with a suitable intermediate in the presence of a coupling agent to give an intermediate of formula (II) as defined above;
   step b: deprotection of the intermediate of formula (II) as defined above;
alternatively, Method D:
   step g: reaction of an intermediate of formula (III) as defined above with an intermediate of formula ≡-Q-L₂'-CO-Y' wherein L₂', Y, Y' and Q are as defined above, or with an intermediate of formula ≡-Q-L₂'-NH-Ra wherein Y, Q, L₂' and Ra are as defined above under Sonogashira reaction conditions to yield an intermediate of formula (V), as defined above;
   step f: coupling the resultant intermediate of formula (V) with a suitable intermediate in the presence of a coupling agent to give an intermediate of formula (II) as defined above;
   step b: deprotection of the intermediate of formula (II) as defined above;
   optionally converting a compound of formula (I) into a different compound of formula (I) by known chemical reactions; and/or, if desired, converting a compound of formula (I) into a pharmaceutically acceptable salt thereof or converting a salt into a free compound of formula (I).

### Method A

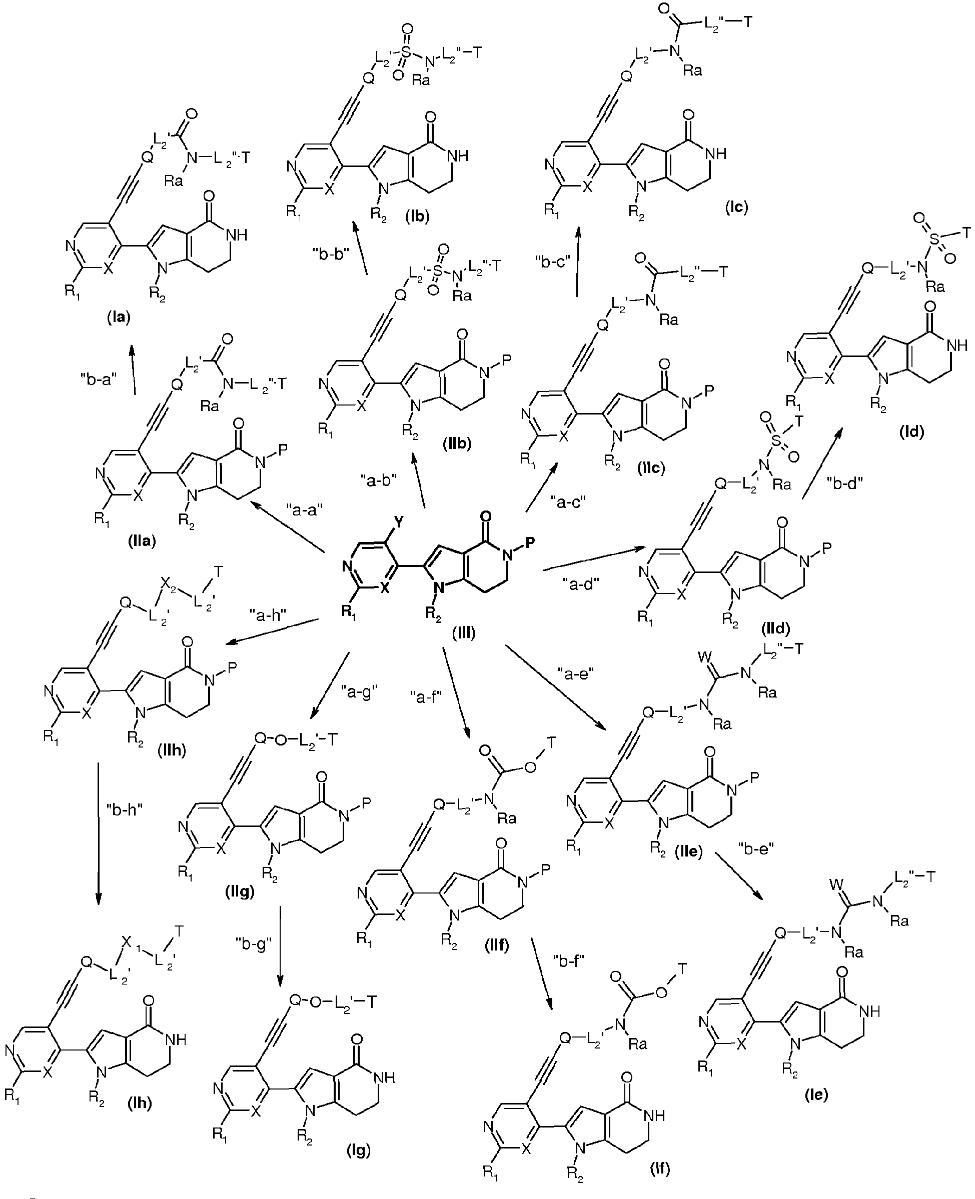

In the above Scheme, R₁, R₂, X, Q, T, P and Ra are as defined above, Y is halogen, W is O or S, X₁ is O or NH, X₂ is O or NP where P is H or a suitable protecting group preferably *tert*-butoxycarbonyl group, and the meanings of L₂' and L₂" are as follows:
for compounds of formula (Ia): L₂' is direct linkage, -C(RaRb)-, -C(RaRb)C(RaRb)- or -OC(RaRb)- and L₂" is direct linkage, -C(RaRb)- or -C(RaRb)C(RaRb)-;
for compounds of formula (Ib): L₂' is direct linkage or -C(RaRb)- and L₂" is direct linkage;
for compounds of formula (Ic): L₂' is direct linkage, -C(RaRb)- or -C(RaRb)C(RaRb)- and L₂" is direct linkage, -C(RaRb)-, -C(RaRb)C(RaRb)-, 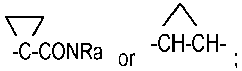
for compounds of formula (Id): L₂' is direct linkage;
for compounds of formula (Ie): L₂' is direct linkage and L₂" is direct linkage;
for compounds of formula (If): L₂' is direct linkage;
for compounds of formula (Ig): L₂' is -C(RaRb)(C(RaRb))nC(RaRb);
for compounds of formula (Ih): L₂' is -C(RaRb)-;
wherein Ra, Rb and n are as defined above.

According to steps "a-a", "a-b", "a-c", "a-d", "a-e", "a-f", "a-g" and "a-h" of Method A, the reaction can be carried out reacting an intermediate of formula (III) with an intermediate of formula (1a)-(1h) reported in the following Table 1, under Sonogashira reaction conditions to yield an intermediate of formula (IIa)-(IIh).

**Table 1**

| **Intermediate structure** | **Intermediate #** |
|---|---|
| 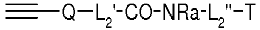 | **(1a)** |
| 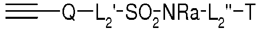 | **(1b)** |
| 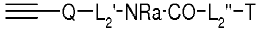 | **(1c)** |
| 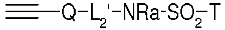 | **(1d)** |
| 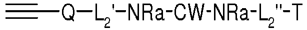 | **(1e)** |
| 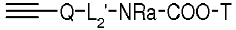 | **(1f)** |
| 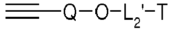 | **(1g)** |
| 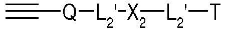 | **(1h)** |

The Sonogashira reaction can be carried out in the presence of a suitable palladium catalyst such as bis(triphenylphosphine)palladium dichloride (PdCl₂(PPh₃)₂), tetrakis(triphenylphosphine)palladium(0) (Pd(PPh₃)₄), and the like, and of a suitable copper catalyst, such as CuI. Said reaction is carried out in the presence of a suitable base, such as triethylamine, diethylamine, diisopropylamine and the like, optionally in the presence of a phosphine ligand, such as triphenylphosphine in solvents like acetonitrile, DMF, toluene, Et₂O, dioxane at temperatures ranging from -20 °C to reflux or using a microwave apparatus and for a time ranging from 30 minutes to about 48 hours. According to steps "b-a", "b-b", "b-c", "b-d", "b-e", "b-f", "b-g" and "b-h", intermediates of formula (IIa)-(IIh), wherein P is a suitable protecting group such as a *tert*-butoxycarbonyl group, can be deprotected under acid conditions for example with hydrochloric acid (HCl) in dioxane, THF, Et₂O or trifluoroacetic acid (TFA) in DCM at temperatures ranging from 0 °C to 40 °C for a time varying from 30 minutes to 24 hours to finally yield compounds of formula (Ia)-(Ih).

Intermediates of formula (1a)-(1h) are prepared as described in the following Scheme 3, steps 1 and 2: 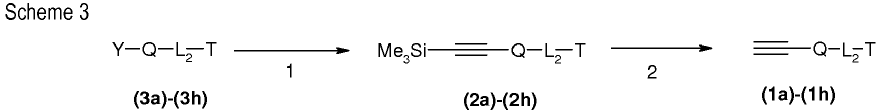 wherein:
L₂ is -L₂'-CONRa-L₂"- for intermediates (1a), (2a) and (3a);
L₂ is -L₂'-SO₂NRa-L₂"- for intermediates (1b), (2b) and (3b);
L₂ is -L₂'-NRaCO-L₂"- for intermediates (1c), (2c) and (3c);
L₂ is -L₂'-NRaSO₂- for intermediates (1d), (2d) and (3d);
L₂ is -L₂'-NRaCWNRa-L₂"- for intermediates (1e), (2e) and (3e);
L₂ is -L₂'-NRaCOO- for intermediates (1f), (2f) and (3f);
L₂ is -O-L₂'- for intermediates (1g), (2g) and (3g);
L₂ is -L₂'-X₂-L₂'- for intermediates (1h), (2h) and (3h);

According to step "1" in Scheme 3, intermediates (2a)-(2h) are obtained by reacting intermediates (3a)-(3h), wherein Y is as defined above, by reaction with ethynyl-trimethyl-silane under Sonogashira conditions as described for steps "a" of Method A.

According to step "2", from intermediates (2a)-(2h) intermediates (1a)-(1h) are obtained after removal of the trimethylsilyl group upon basic conditions with a base such as potassium carbonate, triethylamine (TEA) in solvents like MeOH, EtOH or in the presence of catalytic amount of Silver salts like triflate or nitrate in mixture of solvents like MeOH, H₂O and DCM at temperatures ranging from -20 °C to 50 °C for 1 hour to 24 hours.

A compound of formula (I) is alternatively prepared according to the Method B shown below.

### Method B

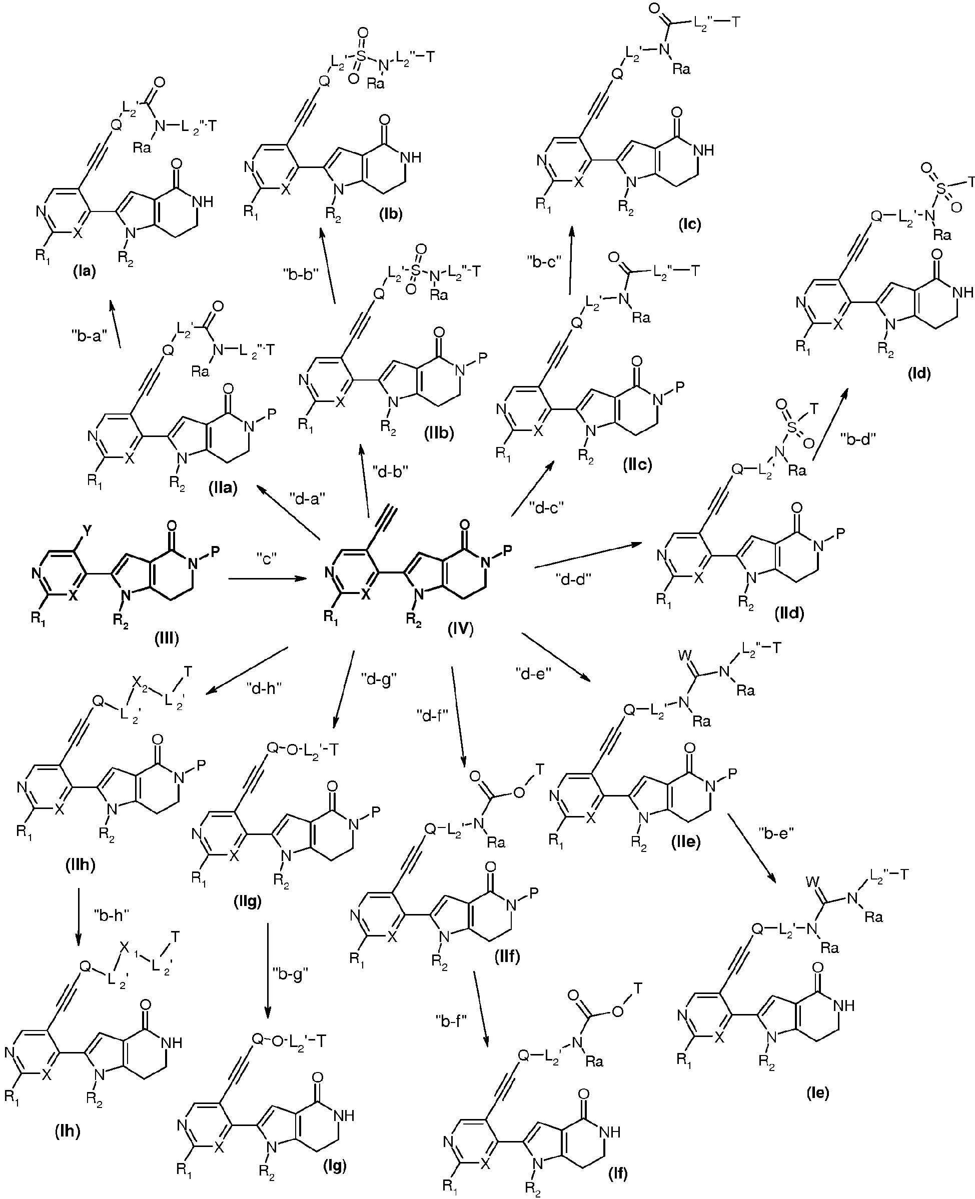

In the above Scheme R₁, R₂, X, Y, Q, T, P, Ra, W, X₁, X₂, L₂' and L₂" are as defined above.

According to step "c" of Method B, the reaction can be carried out reacting an intermediate of formula (III) with ethynyl-trimethyl-silane under Sonogashira reaction conditions as reported in step 1 of Scheme 3 followed by desilylation, as described in step 2 of Scheme 3.

According to steps "d-a", "d-b", "d-c", "d-d", "d-e", "d-f", "d-g" and "d-h" of Method B, the reaction can be carried out reacting an intermediate of formula (IV) with an intermediate of formula (3a)-(3h) to yield an intermediate of formula (IIa)-(IIh) under Sonogashira reaction conditions, as described in steps "a" of Method A.

Steps "b-a", "b-b", "b-c", "b-d", "b-e", "b-f", "b-g" and "b-h" are performed as already described for steps "b" of Method A.

Further processes of the present disclosure, alternative to Methods A and B, are reported below (Methods C and D). The following procedures are valid only for the compounds of formula (Ia), (Ic), (Id), (Ie) and (If).

### Methods C and D

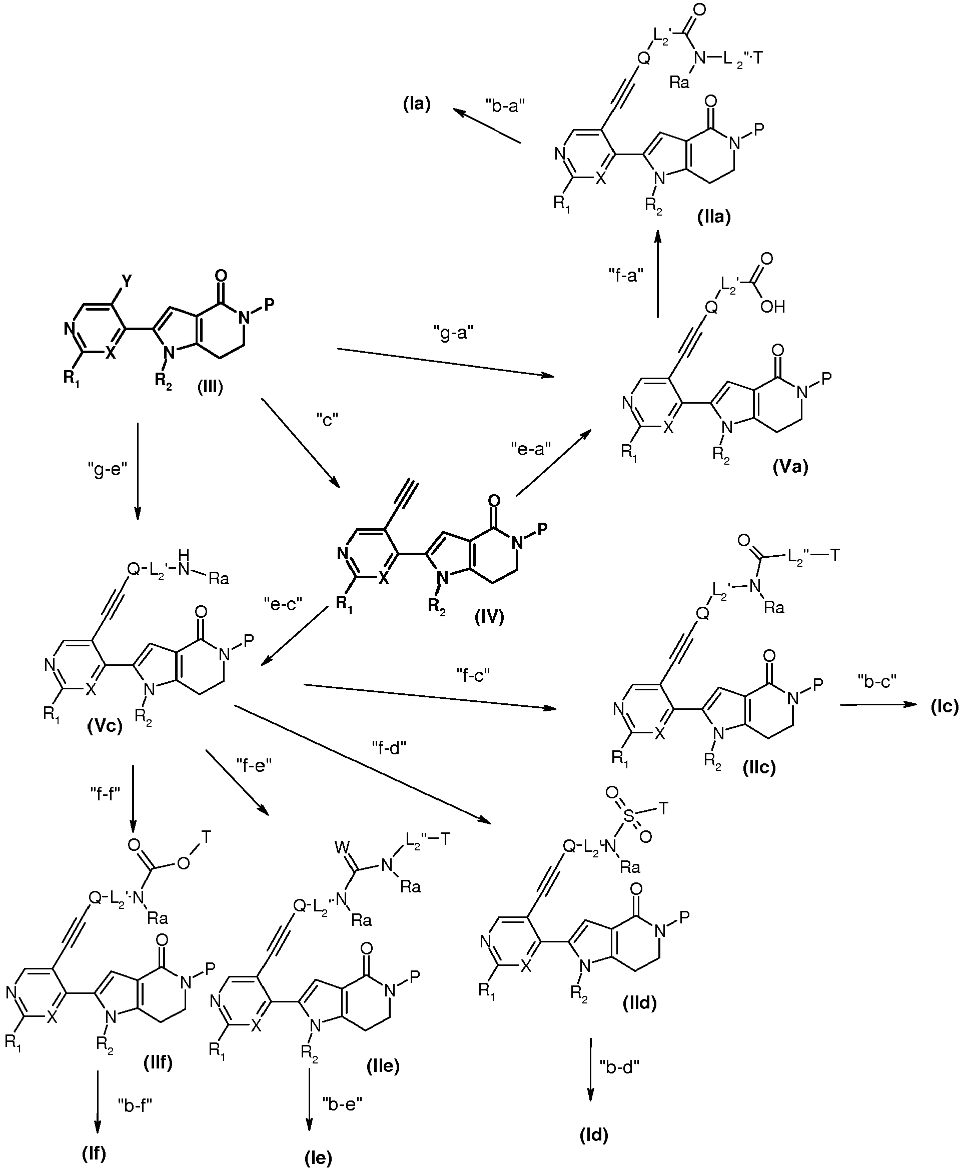

In the above Scheme R₁, R₂, X, Y, Q, T, P, Ra, W, L₂' and L₂" are as defined above.

According to step "c" of Method C, the reaction can be carried out as previously described for Method B.

According to step "e-a" of Method C, the reaction can be carried out reacting an intermediate of formula (IV) with an intermediate of formula (4) (Y-Q-L₂'-COY', depicted in the following Scheme 4) where Y' is OH, i.e. a carboxylic acid, to yield an intermediate of formula (Va) under Sonogashira reaction conditions as described for steps "a" of Method A.

According to step "e-c" of Method C, the reaction can be carried out reacting an intermediate of formula (IV) with an intermediate of formula (7) (Y-Q-L₂'-NHRa, depicted in the following Scheme 4), to yield an intermediate of formula (Vc) under Sonogashira reaction conditions as described for steps "a" of Method A.

According to step "f-a" of Method C, an intermediate of formula (Va) is reacted with an intermediate of formula (5) (RaNH-L₂"-T, depicted in the following Scheme 4) to obtain an intermediate of formula (IIa) in the presence of a coupling agent such as, for instance, 2-(1H-benzotriazol-1-yl)-1,1,3,3-tetramethyluronium tetrafluoroborate (TBTU), 1,3-dicyclohexylcarbodiimide, 1,3-diisopropylcarbodiimide, 1-(3-dimethylaminopropyl)-3-ethylcarbodiimide, N-cyclohexylcarbodiimide-N'-propyloxymethyl polystyrene or N-cyclohexylcarbodiimide-N'-methyl polystyrene, in a suitable solvent such as, for instance, dichloromethane, chloroform, tetrahydrofuran, diethyl ether, 1,4-dioxane, acetonitrile, toluene, or N,N-dimethylformamide, N,N-dimethylacetamide at a temperature ranging from about -10°C to reflux and for a suitable time, for instance from about 30 minutes to about 96 hours.

According to step "f-c" of Method C, an intermediate of formula (Vc) is reacted with an intermediate of formula (8) (Y'-CO-L₂"-T, depicted in the following Scheme 4) to obtain an intermediate of formula (IIc). Compound (8), where Y' is a halogen, preferably Cl, is reacted with intermediate (Vc) under a variety of conditions, for example in a suitable solvent such as dichloromethane, chloroform, tetrahydrofuran, diethyl ether, 1,4-dioxane, acetonitrile, toluene, or N,N-dimethylformamide or the like at a temperature ranging from about -10°C to reflux and for a suitable time, for instance from about 30 minutes to about 96 hours. The reaction is carried out in the presence of an opportune proton scavenger such as triethylamine, N,N-diisopropylethylamine or pyridine. Compound (8), where Y' is a hydroxyl group, is reacted in the presence of a coupling agent such as, for instance, 2-(1H-benzotriazol-1-yl)-1,1,3,3-tetramethyluronium tetrafluoroborate (TBTU), 1,3-dicyclohexylcarbodiimide, 1,3-diisopropylcarbodiimide, 1-(3-dimethylaminopropyl)-3-ethylcarbodiimide, N-cyclohexylcarbodiimide-N'-propyloxymethyl polystyrene or N-cyclohexylcarbodiimide-N'-methyl polystyrene, in a suitable solvent such as, for instance, dichloromethane, chloroform, tetrahydrofuran, diethyl ether, 1,4-dioxane, acetonitrile, toluene, or N,N-dimethylformamide at a temperature ranging from about -10°C to reflux and for a suitable time, for instance from about 30 minutes to about 96 hours. The said reaction is optionally carried out in the presence of a suitable catalyst, for instance 4-dimethylaminopyridine, or in the presence of a further coupling reagent such as N-hydroxybenzotriazole. Alternatively, this same reaction is also carried out, for example, through a mixed anhydride method, by using an alkyl chloroformate such as ethyl, iso-butyl, or iso-propyl chloroformate, in the presence of a tertiary base such as triethylamine, N,N-diisopropylethylamine or pyridine, in a suitable solvent such as, for instance, toluene, dichloromethane, chloroform, tetrahydrofuran, acetonitrile, diethyl ether, 1,4-dioxane, or N,N-dimethylformamide, at a temperature ranging from about -30°C to room temperature.

According to step "f-d" of Method C, an intermediate of formula (Vc) is reacted with an intermediate of formula (9) (Cl-SO₂-T, depicted in the following Scheme 4) to obtain an intermediate of formula (IId). Such a reaction is carried out in the presence of a suitable base, such as for instance, pyridine, N-methyl morpholine, diisopropyl ethylamine, in the appropriate solvent such as pyridine, dichloromethane or tetrahydrofuran, at a temperature ranging from 0°C to reflux and for a time varying from about 1 hour to about 7 days.

According to step "f-e" of Method C, an intermediate of formula (Vc) is reacted with an intermediate of formula (10) (W=C=N-L₂"-T, depicted in the following Scheme 4) to obtain an intermediate of formula (IIe). Such intermediate of formula (IIe), which can be an urea compound when W = O or a thiourea compound when W = S, can be prepared by reacting an intermediate of formula (Vc) with the appropriate isocyanate or thioisocyanate of formula (10) respectively. Such a reaction is carried out in a suitable solvent such as dichloromethane or tetrahydrofuran, normally at a temperature ranging from about -10°C to reflux and for a time varying from about 30 minutes to about 96 hours. Alternatively an urea of formula (IIe) can be prepared reacting an intermediate of formula (5) (RaNH-L₂"-T, depicted in the following Scheme 4) with triphosgene (bis(trichloromethyl) carbonate, O=C(OCCl₃)₂) followed by the addition of the intermediate of formula (Vc). This reaction can be carried out in the presence of a base like diisopropylethylamine (DIPEA), triethylamine (TEA), Na₂CO₃, in solvents like dichloromethane, chloroform, at a temperatures ranging from about -10°C to reflux and for a time varying from about 30 minutes to about 96 hours.

According to step "f-f" of Method C, an intermediate of formula (Vc) is reacted with an intermediate of formula (11) (Cl-CO-O-T, depicted in the following Scheme 4) to obtain an intermediate of formula (IIf). Such a reaction is carried out in the appropriate solvent such as tetrahydrofuran, N,N-dimethylformamide, dichloromethane, chloroform, acetonitrile, toluene or mixtures thereof, at a temperature ranging from about -10°C to reflux and for a time varying from about 30 minutes to about 96 hours. The reaction is normally carried out in the presence of an opportune proton scavenger such as triethylamine, N,N-diisopropylethylamine or pyridine.

According to step "g-a" of Method D, an intermediate of formula (III) is reacted with an intermediate of formula (27) (≡-Q-L₂'-COY', wherein Y' is OH, as depicted in the following Scheme 5) to directly yield an intermediate of formula (Va) under Sonogashira reaction conditions as described for steps "a" of Method A.

According to step "g-e" of Method D, an intermediate of formula (III) is reacted with an intermediate of formula (28) (C≡C-Q-L₂'-NHRa, as depicted in the following Scheme 5) to directly yield an intermediate of formula (Vc) under Sonogashira reaction conditions as described for steps "a" of Method A.

Steps "b-a", "b-c", "b-d", "b-e" and "b-f" are carried out as already described for Method A.

A further process to obtain a compound of formula (Ie) can also involve the **convertion** of an intermediate of formula (IIf) directly into an intermediate of formula (IIe) followed by deprotection of the *ter*t-butoxycarbonyl group, as described before. In particular an intermediate (IIf), wherein T is a *p*-NO₂Ph (para-nitrophenyl) group, can be reacted with an amine of formula (5) (NHRa-L₂"-T, as depicted in the following Scheme 4) in solvents like DCM, acetonitrile, DMSO, DMF and eventually a mixture of such solvents, in the presence of a tertiary amine such as TEA, at temperatures ranging from room temperature to reflux for a time varying from 30 minutes to 72 hours.

Compounds of formula (Ie) can be also obtained **converting** an intermediate of formula (IIe) where R₁ is NH₂ into other intermediates of formula (IIe) where R₁ is NHR₃ wherein R₃ is CHR₄R₅ and wherein R₄ and R₅ are each independently H, optionally substituted straight or branched C₁-C₆ alkyl, or R₄ and R₅ joined together represent a heterocyclyl. This process is carried out by reacting an intermediate (IIe), where R₁ is NH₂, with a carbonylic compound of formula R₄R₅CO under reductive alkylation conditions. In particular this reaction is carried out in a suitable solvent such as, for instance, methanol, N,N-dimethylformamide, dichloromethane, tetrahydrofuran, or a mixture thereof, in the presence of a suitable reducing agents such as, for instance, sodium borohydride, tetra-alkylammonium borohydride, sodium cyano borohydride, sodium triacetoxyborohydride, tetramethylammonium triacetoxy borohydride, hydrogen and a hydrogenation catalyst, and in the presence of an acid catalyst, such as, for instance, acetic acid, trifluoroacetic acid, at a temperature ranging from about 0°C to reflux and for a time varying from about 1 hour to about 96 hours. This reaction is followed by removal of the protecting group P to yield the compound (Ie). This process described for compounds of formula (Ie), is suitable to convert also compounds of formula (IIa)-(IId) and (IIf)-(IIh) into the corresponding compounds (Ia)-(Id) and (If)-(Ih).

### Preparation of the intermediates of formula (3a)-(3h)

Compounds of formula (3a)-(3h) are prepared as described in the following Scheme 4, steps i1-10, wherein Y' is halogen or hydroxyl and the other variables are as defined above.

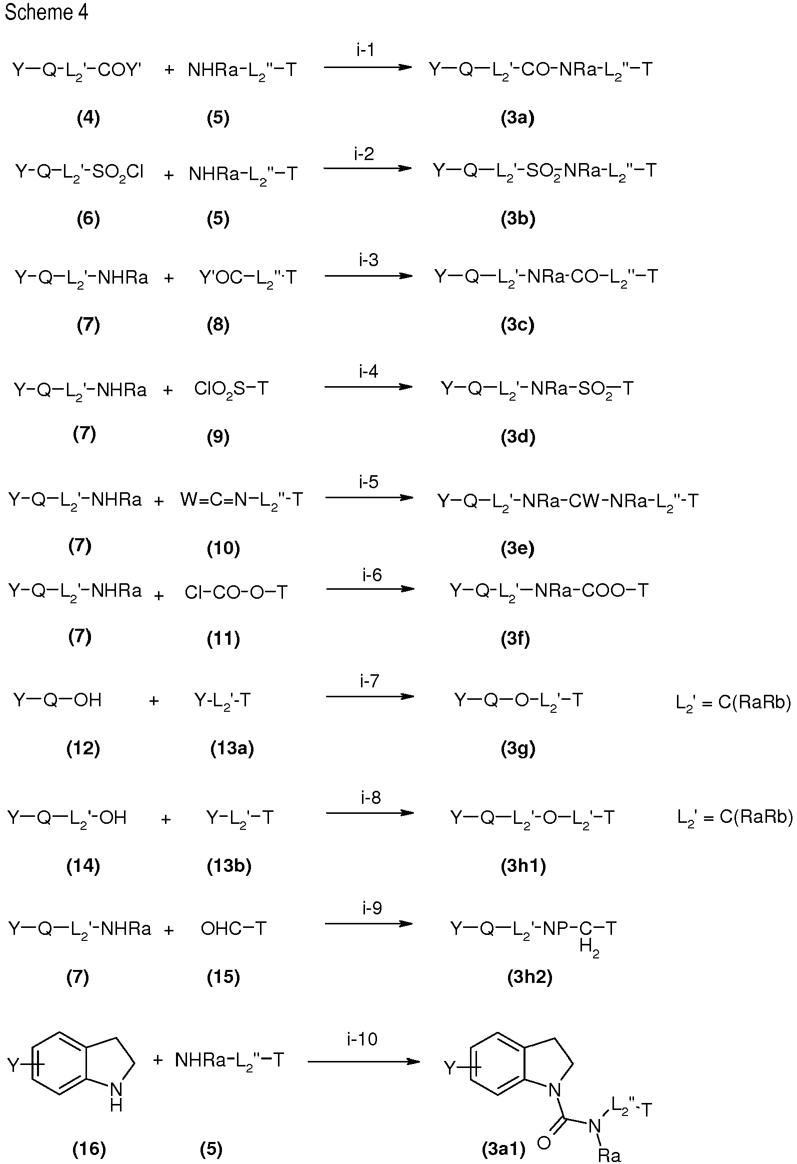

According to step i-1, an intermediate (3a) is obtained reacting an intermediate (4) with an intermediate of formula (5) under the reaction conditions reported for step "f-c" of Method C.

According to step i-2, an intermediate of formula (5) is reacted with a sulfonyl chloride of formula (6) to afford an intermediate of formula (3b). Such a reaction is carried out under the reaction conditions reported for step "f-d" of Method C.

According to step i-3, an intermediate of formula (3c) can be prepared analogously to step i-1, by reacting an intermediate of formula (7) with an intermediate of formula (8).

According to step i-4, an intermediate of formula (3d) can be prepared analogously to step i-2, by reacting an intermediate of formula (7) with a sulfonyl chloride of formula (9).

According to step i-5, an intermediate of formula (3e), which can be an urea compound when W = O or a thiourea compound when W = S, can be prepared by reacting an intermediate of formula (7) with the appropriate isocyanate or thioisocyanate of formula (10) respectively. Such a reaction is carried out under the same reaction conditions reported for step "f-e" of Method C. Alternatively an urea of formula (3e) can be prepared reacting an intermediate of formula (7) with triphosgene (bis(trichloromethyl) carbonate, O=C(OCCl₃)₂) followed by the addition of an intermediate of formula (5) or viceversa reacting an intermediate of formula (5) with triphosgene followed by the addition of an intermediate of formula (7), a well known method for the formation of ureas. This reaction can be carried out under the same reaction conditions described for step "f-e" of Method C.

According to step i-6, an intermediate of formula (3f) can be prepared by reacting a chloroformate of formula (11) with an intermediate of formula (7) under the same reaction conditions reported for step "f-f" of Method C.

According to step i-7, an intermediate of formula (3g) can be prepared by reacting an intermediate of formula (12) with an intermediate of formula (13a) under Williamson reaction conditions, a general method for the preparation of ethers, or through similar methods. Such a reaction can be carried out in the presence of a suitable base such as K₂CO₃, Na₂CO₃, KOH, NaH, pyridine and the like, eventually in the presence of NaI to allow the exchange of less reactive halide with iodide, in suitable solvents like acetone, DMF, DMSO, acetonitrile and the like, or under phase transfer catalysis conditions at temperatures ranging from 0 °C to reflux for 6 to 48 hours.

According to step i-8, an intermediate of formula (3h1) can be prepared analogously to step i-7, by reacting an intermediate of formula (14) with an intermediate of formula (13b).

According to step i-9, an intermediate of formula (3h2) can be prepared by reacting an aminic compound of formula (7) with an aldehyde of formula (15) in reductive alkylation conditions. In particular this reaction is carried out in a suitable solvent such as, for instance, methanol, N,N-dimethylformamide, dichloromethane, tetrahydrofuran, or a mixture thereof, in the presence of a suitable reducing agents such as, for instance, sodium borohydride, tetraalkylammonium borohydride, sodium cyano borohydride, sodium triacetoxyborohydride, tetramethylammonium triacetoxy borohydride, hydrogen and a hydrogenation catalyst, and in the presence of an acid catalyst, such as, for instance, acetic acid, trifluoroacetic acid, at a temperature ranging from about 0°C to reflux and for a time varying from about 1 hour to about 96 hours. In case a protecting group is needed for further synthetic steps, compound (3h2) where P is H, can be converted into compound (3h2), where P is a *tert*-butoxycarbonyl group, using di-*tert-*butyldicarbonate ((Boc)₂O, in the presence of dimethylaminopyridine (DMAP) and in solvents like DCM at temperature ranging from 0 °C to reflux and for time varying from 1 hour to 24 hours.

A particular case of the present disclosure is represented by an intermediate of formula (3a), wherein Q is one of the bicyclic ring systems B or C, L₂' is direct linkage, L₂" and Y are as defined above, i.e. the intermediate of formula (3a1).

According to step i-10 , an intermediate of formula (3a1) is prepared analogously to the method described in step i-5, reacting an intermediate of formula (5), RaNH-L₂"-T, with triphosgene (bis(trichloromethyl) carbonate, O=C(OCCl₃)₂) followed by the addition of an intermediate of formula (16).

Compounds (4)-(16) can be either commercially available or prepared according to methods well known in the literature and to the skilled in the art. For example, indolines of formula (16) can be prepared according to the procedures described in J. Am. Chem. Soc. 2002, 124, 14844-14845 and in WO2010/043000.

Specific compounds of formula (5), RaNH-L₂"-T, can be prepared according to different methods. For example compounds of formula (5) where Ra is hydrogen, L₂" is direct linkage and T is J, wherein J is as defined above, can be synthesized through several procedures depending on L₃, in some cases based on methods described in the literature (see for example Journal of Medicinal Chemistry 2010, 53, 4701-4719).

These particular cases of amines (5a)-(5e) are reported in the following Scheme 4a, wherein L₃ is direct linkage, O, NH, NCH₃, CH₂ or C=O, and Rd and Re are as defined above.

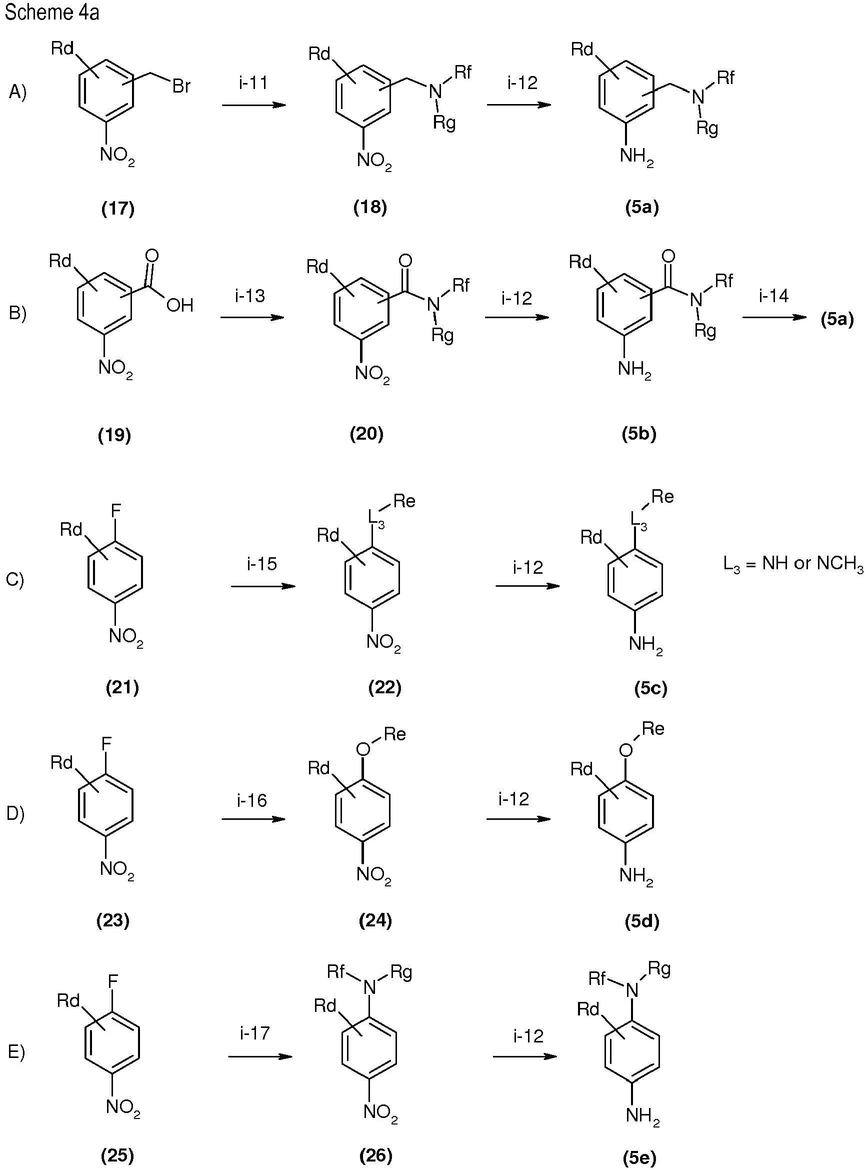

In Scheme 4a, amines (5a), wherein L₃ is CH₂ and Re is NRfRg, can be prepared according to procedure A). Amines of formula (5b), wherein L₃ is CO and Re is NRfRg are obtained through the procedure B).

From compounds (5b), described in Scheme 4a, compounds (5a) can also be obtained.

Amines of formula (5c), wherein L₃ is NH or NCH₃ and Re is as defined above, are obtained through procedure C). Amines of formula (5d), wherein L₃ is O and Re is as defined above, are obtained through procedure D).

Amines of formula (5e), wherein L₃ is direct linkage and Re is NRfRg, are obtained through procedure E).
Procedure A): According to step i-11, an intermediate of formula (17) is reacted with an intermediate RfRgNH neat or in solvents like anhydrous acetonitrile, DCM and THF at temperatures ranging from 0 °C to reflux to yield compound (18) for time ranging from 1 hour to 48 hours. According to step i-12 the compound of formula (18) is reacted with iron powder in the presence of ammonium chloride, in solvents like EtOH, MeOH and mixture with water at temperatures ranging from room temperature to reflux for a time ranging from 1 hour to 48 hours to obtain compound of formula (5a). A variety of alternative methods available in the literature and well known to the skilled in the art can be used to carry out step i-12. For example the reaction can be carried out in the presence of Pd/C catalyst and ammonium formate (HCOONH₄) in a solvent like methanol, ethanol and the like at temperatures ranging from room temperatures to reflux and for 30 minutes to 48 hours. Alternatively the reaction can be carried out reacting (18) with Stannous chloride (SnCl₂· 2H₂O) in solvents like ethanol, methanol, DCM, ethyl acetate or a mixture of them, at temperatures ranging from room temperature to reflux for 1 hour to 48 hours. In some cases further synthetic modifications of groups Rf or Rg might be necessary to achieve the final product.
Procedure B): According to step i-13 of Scheme 4a, an intermediate of formula (19) is reacted with an intermediate RfRgNH as described for step "f-c" of Method C to yield compound (20). According to step i-12, compound (20) is reacted under the same conditions already reported for step i-12 of procedure A) to yield an amine of formula (5b). According to step i-14 compound (5b) can be transformed into compound (5a) by reduction with borane tetrahydrofuran complex (BH₃·THF) in THF as the solvent at temperatures ranging from 0 °C to reflux for one hour to 72 hours.
Procedure C): According to step i-15, an intermediate of formula (21) is reacted with an intermediate ReNH₂ or ReNHCH₃, neat or in the presence of solvents like anhydrous acetonitrile, DCM and THF, at temperatures ranging from room temperature to reflux for a time ranging from 1 hour to 72 hours to yield compound (22), which is reacted as already described in step i-12 to yield compound (5c).
Procedure D): According to step i-16, an intermediate of formula (23) is reacted with an intermediate of formula Re-OH, in the presence of NaH (sodium hydride) in solvents like anhydrous THF, at temperatures ranging from -10°C to room temperature for 1 hour to 24 hours to yield compound (24), which is reacted as already described in step i-12 to yield an intermediate of formula (5d).
Procedure E): According to step i-17, an intermediate of formula (25) is reacted with an intermediate RfRgNH neat or in the presence of solvents like anhydrous acetonitrile, DCM and THF, at temperatures ranging from room temperature to reflux for a time ranging from 1 hour to 72 hours to yield compound (26), which is reacted as already described in step i-12 to yield an intermediate of formula (5e).

Alternatively specific compounds of formula (1a), (1c), (1d), (1e) and (1g) can be prepared reacting compounds of formula (27), (28) and (29) already functionalized with the ethynyl group with compounds of formula (5), (8), (9), (10) and (13a), as described in the following Scheme 5, wherein all the variables are as defined above.

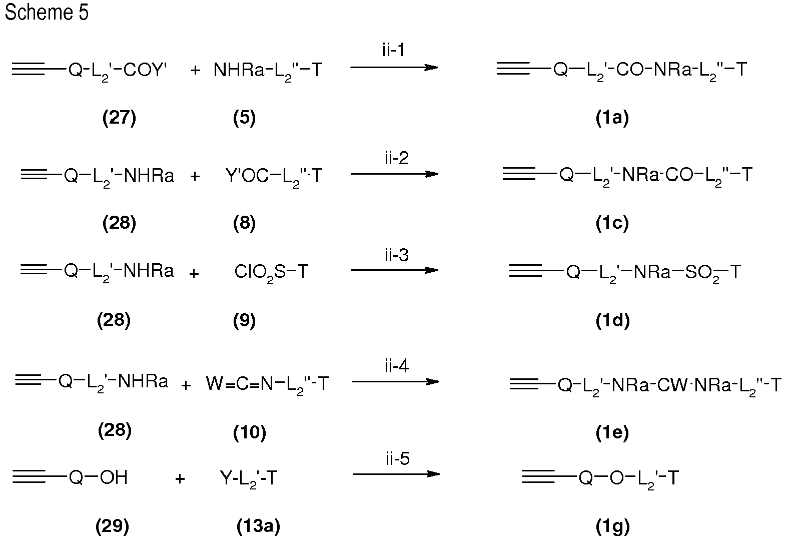

According to step ii-1, an intermediate of formula (1a) can be prepared reacting an intermediate of formula (27), wherein Y' is OH, with an intermediate of formula (5) under the same reaction conditions described for step "f-c" of Method C.

According to step ii-2, an intermediate of formula (1c) can be prepared reacting an intermediate of formula (28) with an intermediate of formula (8) under the same reaction conditions described for step "f-c" of Method C.

According to step ii-3, an intermediate of formula (1d) can be prepared reacting an intermediate of formula (28) with an intermediate of formula (9) under the same reaction conditions described for step "f-d" of Method C.

According to step ii-4, an intermediate of formula (1e) can be prepared reacting an intermediate of formula (28) with an intermediate of formula (10) under the same reaction conditions described for step "f-e" of Method C.

According to step ii-5, an intermediate of formula (1g) can be prepared reacting an intermediate of formula (29) with an intermediate of formula (13a) under the same reaction conditions described for step i-7 of Scheme 4.

Specific compounds of formula (27), (28) and (29) are either commercially available or easily prepared according to methods well known to the skilled in the art.

### Preparation of intermediates of formula (III) and (IV)

The present disclosure further discloses the processes for the preparation of the intermediates of formula (III) useful for the synthesis of different intermediates of formula (II), which are prepared according to the following Schemes 6 to 11 reported below.

In particular, compounds of formula (III) where X is N, Y is I or Br, R₁ is NH₂ and P and R₂ are as defined above, can be prepared from an intermediate of formula (VI) upon halogenation according to the methods reported in the literature (WO2010/145998), as described in the following Scheme 6.

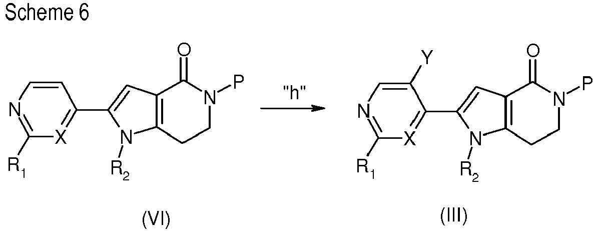

According to step "h" an intermediate of formula (VI) is reacted with Silver trifluoroacetate and iodine thus obtaining an intermediate of formula (III) wherein Y is I, provided that R₁ is NH₂ or NHR₃, wherein R₃ is as defined above but not COR'. Alternatively compound (VI) can be reacted with NBS, in solvents like DMF thus obtaining an intermediate of formula (III) wherein Y is Br, provided that R₁ is NH₂ or NHR₃, wherein R₃ is as defined above but not COR'. Compounds of formula (III) can be also obtained according to a number of different methods, depending on the specific structural moieties of the compounds. For example, compounds of formula (IIIa), wherein Y is I or Br, R₃ is H or an optionally substituted straight or branched C₁-C₆ alkyl or a heterocyclyl, and R₂ and P are as described above, can be prepared according to the procedure reported in the following Scheme 7.

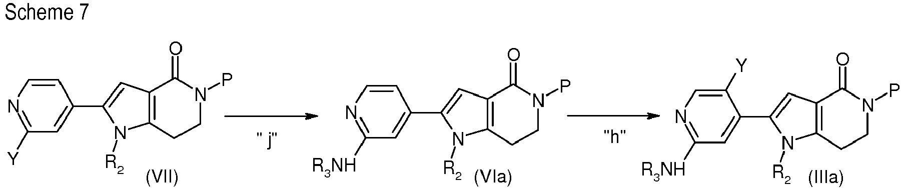

According to step "j" of Scheme 7, an intermediate of formula (VII), wherein Y is Cl and R₂ and P are as defined above, prepared as described in WO2004/058762 and in Journal of Medicinal Chemistry 2007, 50, 2647-54, is reacted with *tert*-butyl carbamate (*t*-BuCOONH₂) (when R₃ and P are H in (VIa) after acidic treatment according to step "b" of Method A) or with an amine R₃NH₂ (when R₃ is a straight or branched optionally substituted C₁-C₆ alkyl or a heterocyclyl in (VIa)) in the presence of a Palladium catalyst (Pd(AcO)₂, PdCl₂, Pd₂dba₃) and a suitable ligand (PPh₃, 9,9-dimethyl-4,5-bis(diphenylphosphino)xanthene (Xantphos), *rac*-2,2'-bis(diphenylphosphino)-1,1'-binaphthyl (rac-Binap)) using a base such as Cs₂CO₃, LiHMDS, NaHMDS in solvents like DMF, toluene, dioxane, acetonitrile and the like at temperatures ranging from room temperature to reflux, for a time period ranging from 1 hour to 48 hours, to yield an intermediate of formula (VIa). Intermediate (VII) can also be reacted with an amine R₃NH₂ (wherein R₃ is H or a straight or branched optionally substituted C₁-C₆ alkyl or a heterocyclyl) in solvents like methanol, ethanol, N,N-dimethylformamide, dimethoxyethane at temperatures ranging from room temperature to reflux or under microwave irradiation for times ranging from 1 hour to 48 hours.

According to step "h", an intermediate of formula (VIa) is reacted under the same reaction conditions previously described in Scheme 6 to finally yield an intermediate of formula (IIIa).

Compounds of formula (III) can be also prepared, such that depicted in the following Scheme 8. This synthetic approach involves a Palladium catalyzed cross-coupling reaction between an intermediate of formula (VIII), wherein R₁ and X are as defined above, and M is a suitable reacting group, such as for example a boronic acid B(OH)₂, a boronic ester B(OAlk)₂, or Sn(Alk)₃, wherein Alk is C₁-C₆ alkyl, and an intermediate of formula (IX), wherein Y is Br or I and R₂ and P are as defined above.

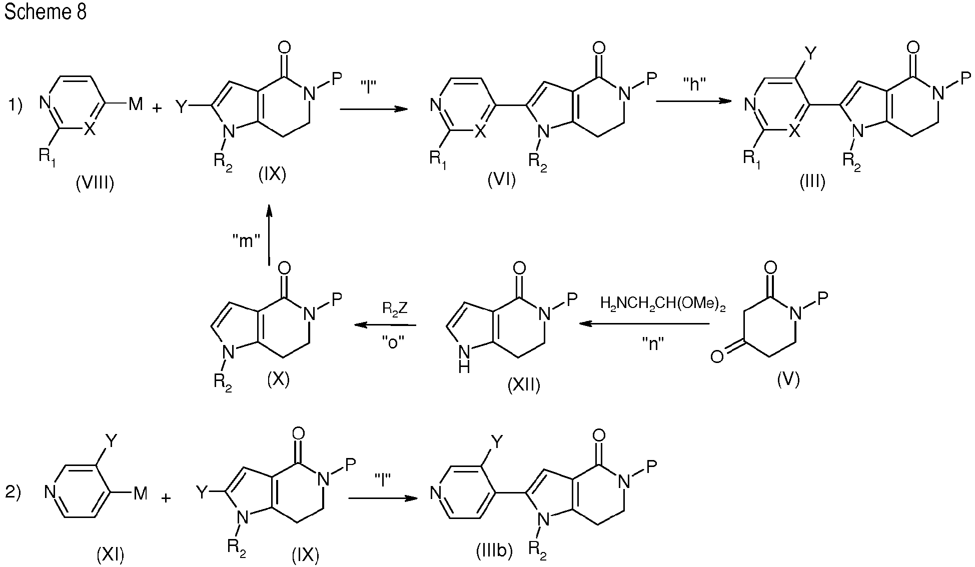

Accordingly, a process 1) in Scheme 8 of the present disclosure comprises the following steps:
Step "I": cross-coupling reaction of compounds of formula (VIII) with compounds of formula (IX). These cross-coupling reactions can be performed under standard conditions as for Suzuki coupling using a Pd-based catalyst (Pd(dppf)₂Cl₂, PdCl₂(PPh₃)₂, Pd(PPh₃)₄) with a suitable base such as Sodium Carbonate (Na₂CO₃), Caesium Carbonate (Cs₂CO₃), potassium phosphate (K₃PO₄) in suitable organic solvents such as dioxane, DMF, toluene and the like at temperatures ranging from room temperature to reflux, for a time period ranging from 1 hour to 48 hours.

Step "h": reacting the intermediate of formula (VI) under the same conditions described for step "h" in Scheme 6, to yield an intermediate of formula (III), provided that R₁ is NHR₃ and R₃ is as defined above but not COR'.

Compounds of formula (VIII) might be commercially available or prepared according to methods known to the skilled in the art.

The intermediate of formula (IX) can be prepared according to the following procedure:
Step "m": reacting an intermediate of formula (X) wherein P and R₂ are as defined above by standard halogenation with NBS or NIS in suitable organic solvents such as THF-Methanol mixture, DMF from -20°C to 40 °C for a time ranging from 30 minutes to 48 hours.

The intermediate of formula (X) can be prepared according to the following steps:
Step "n": reacting an intermediate of formula (V) wherein P is H or *tert*-butoxycarbonyl group, with 2,2-dimethoxyethylamine in solvents like toluene, N,N-dimethylformamide, N,N-dimethylacetamide, N-methylpirrolidone at temperatures ranging from room temperature to reflux and for a time ranging from 1 hour to 72 hours, to yield an intermediate that is further treated under acidic condition (HCl, TFA) in a variaty of solvents such as DCM, diethylether, dioxane at temperatures varying from 0 °C to reflux for a time period ranging from 1 hour to 48 hours to yield intermediate of formula (XII) where P is H. Compound (XII) can be protected again when needed, for example with di-*tert*-butyl dicarbonate (Boc₂O), providing compound (X) where P is *tert*-butoxycarbonyl group.

Step "o": reacting the intermediate of formula (XII) with an alkylating agent R₂Z wherein R₂ is as defined above but not hydrogen and Z is halogen, tosyl, methylsulphonyl, trifluoromethylsulphonyl, or another suitable leaving group, in the presence of a base such as TEA, DIPEA, Caesium carbonate (Cs₂CO₃), Potassium Carbonate (K₂CO₃), Sodium Hydride (NaH), NaOH, DBU, LiHMDS and the like, in solvents like 1,2-dimethoxyethane, methanol, ethanol, isopropanol, acetonitrile, N,N-dimethylformamide, N,N-dimethylacetamide, dimethylsulfoxide, and THF, at temperatures ranging from 0 °C to reflux for a time ranging from 30 minutes to 72 hours.

The process 2) in Scheme 8 of the present disclosure reports the synthesis of a particular compound of formula (IIIb) obtained by reacting an intermediate (XI), wherein M is a pinacolboronic ester group and Y is Br, with an intermediate of formula (IX) wherein Y is Br and P and R₂ are as defined above.

Accordingly, the process 2) in Scheme 8 comprises the following step:
Step "I": reacting an iintermediate of formula (XI) with an intermediate of formula (IX) under the same conditions as previously described for step "I" via cross-coupling reaction.

Intermediate (X) is useful also to obtain other compounds of formula (III). In the following Scheme 9 is shown the process for the preparation of compounds of formula (IIIc) wherein P, R₁, R₂ and Y are as defined above.

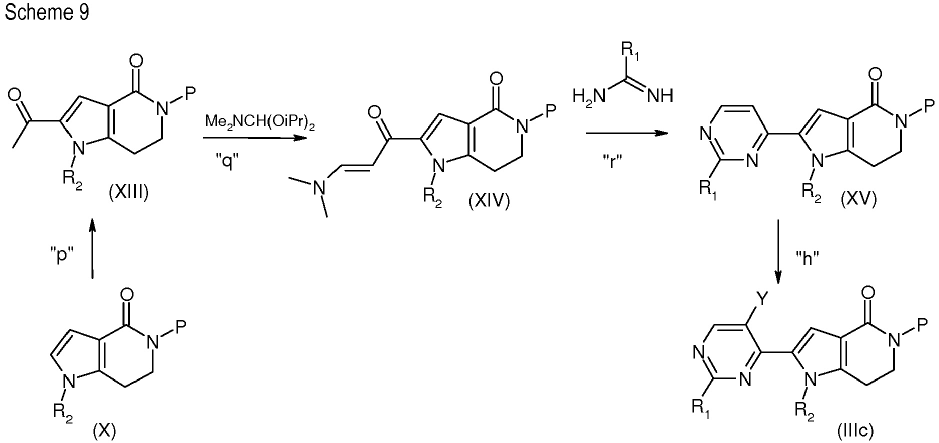

Accordingly, a further process of the present disclosure comprises the following steps:
Step "p": reacting an intermediate of formula (X) wherein P is H or *tert*-butoxycarbonyl group, with acetyl chloride or acetic anhydride in solvents like dichloromethane, dichloroethane, chlorobenzene, THF, dioxane in the presence of a catalyst such as aluminium trichloride (AlCl₃), titanium tetrachloride (TiCl₄), boron trifluoride diethylether complex (BF₃·Et₂O), zinc dichloride (ZnCl₂) at temperatures ranging from 0 °C to reflux for a time ranging from 30 minutes to 72 hours.

Step "q": reacting an intermediate of formula (XIII) wherein P is H or *tert*-butoxycarbonyl group, with dimethylformamide diisopropylacetal, dimethylformamide dimethylacetal, dimethylformamide diethylacetal, dimethylformamide ditert-butylacetal in solvents like N,N-dimethylformamide, N,N-dimethylacetamide, N-methylpirrolidone, dimethylsulfoxide at temperatures ranging from 0 °C to reflux for a time ranging from 30 minutes to 72 hours.

Step "r": reacting an intermediate of formula (XIV) wherein P is H or *tert*-butoxycarbonyl group, with a suitable guanidine wherein R₁ is NH₂ or R₃NH and R₃ is optionally substituted straight or branched C₁-C₆ alkyl or heterocyclyl, in solvents like methanol, ethanol, N,N-dimethylformamide, N,N-dimethylacetamide, N-methylpirrolidone, dimethylsulfoxide in the presence of a base such as sodium methylate (MeONa), sodium ethylate (EtONa), sodium tert-butoxide (*t*BuONa), potassium acetate (AcONa), at temperatures ranging from 0 °C to reflux for a time ranging from 30 minutes to 72 hours.

Step "h": halogenation of the resultant intermediate of formula (XV), under the same conditions described for step "h" in Scheme 6, to yield the compound of formula (IIIc), provided that R₁ is NHR₃ and R₃ is H, a straight or branched optionally substituted C₁-C₆ alkyl, or a heterocyclyl.

The Scheme 10 below shows an alternative process for the preparation of compounds of formula (IIId) wherein P is hydrogen, R₃ is a straight or branched optionally substituted C₁-C₆ alkyl, R₂, X and Y are as defined above.

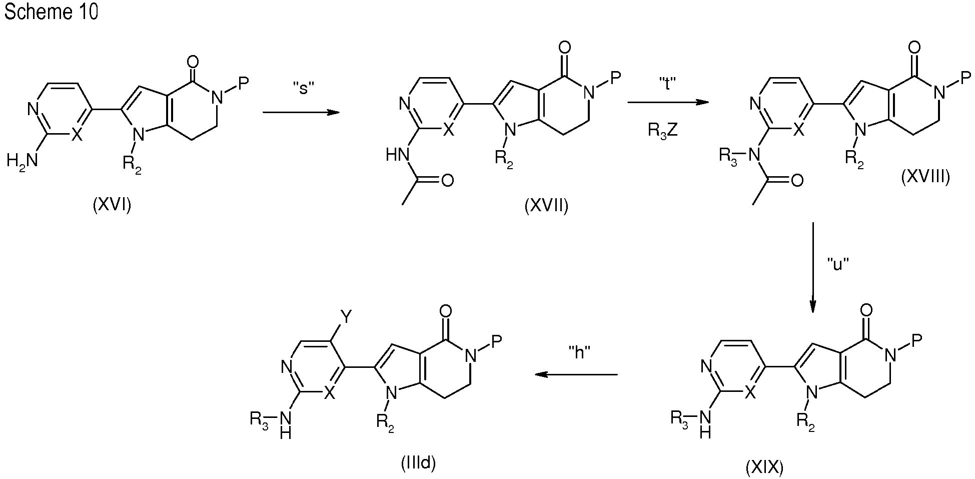

Accordingly, a further process of the present disclosure comprises the following steps:
Step "s": reacting an intermediate of formula (XVI), wherein P is a suitable protecting group (for example a *tert-*butoxycarbonyl group) and R₂ and X are as defined above, with acetic anhydride or acetyl chloride in solvents like dichloromethane, THF, in the presence of a base such as triethylamine (TEA), diisopropylethylamine (DIPEA) and a catalytic amount of an acyl transfer like pyridine, dimethylaminopyridine (DMAP) at temperatures ranging from room to reflux, for a time ranging from 1 hour to 72 hours to yield an intermediate of formula (XVII);

Step "t": alkylation of the resultant compound of formula (XVII) with an alkylating agent R₃Z wherein Z is as defined above and R₃ is a straight or branched optionally substituted C₁-C₆ alkyl, according to the method described for step "o" in scheme 8, followed by removal of the protecting group P (for example when it is a *tert*-butoxycarbonyl group) under acidic conditions according to steps "b" of Method A, to achieve the intermediate of formula (XVIII), wherein P is H;

Step "u": reacting compound of formula (XVIII), wherein P is H, under basic conditions using potassium carbonate (K₂CO₃), triethylamine (TEA) in solvents like methanol, ethanol and the like, at temperatures ranging from 0 °C to reflux for a time period ranging from 30' to 24 hours, thus obtaining the intermediate of formula (XIX);

Step "h": halogenation of the intermediate of formula (XIX), under the same conditions described for step "h" in Scheme 6, providing compounds of formula (IIId) wherein P is H and R₂, R₃, X and Y are as defined above.

The present disclosure further discloses the processes for the preparation of the intermediates of formula (IVa) and (IVb), useful for the synthesis of compounds of formula (I), wherein R₁ is NHR₃, wherein R₃ is a straight or branched optionally substituted C₁-C₆ alkyl, a heterocyclyl, or R'CO and the other variables are as defined above. In the intermediate (IVa) R' is preferentially a methyl group.

The preparation of the intermediates of formula (IVa) and (IVb) is reported in the following Scheme 11.

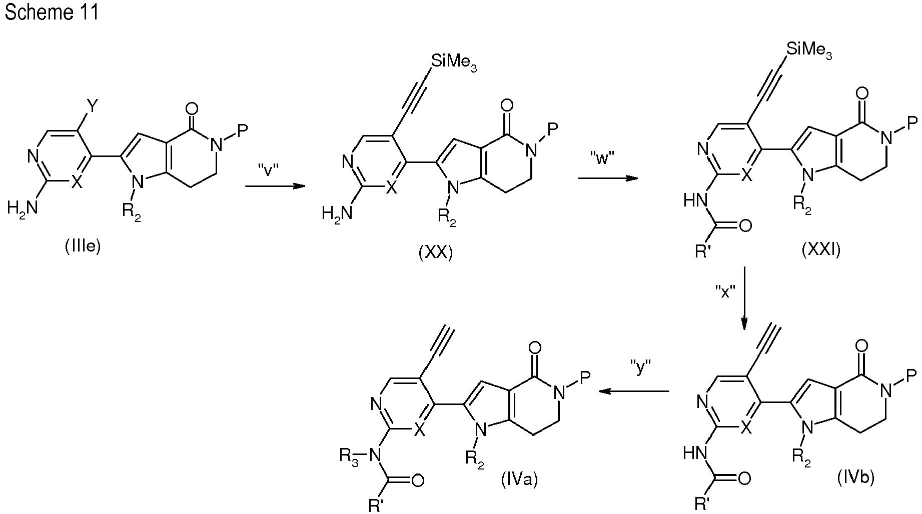

Accordingly, a further process of the present disclosure comprises the following steps:
Step "v": reaction of an intermediate of formula (IIIe), wherein P is a *tert*-butoxycarbonyl protecting group and Y and R₂ are as defined above, with trimethylsilylacetilene under Sonogashira reaction conditions as described for steps "a" of Method A;
Step "w": reacting an intermediate of formula (XX) with acid anhydride (R'CO)₂O or acyl chloride R'COCI in solvents like dichloromethane, THF, in the presence of a base such as triethylamine (TEA), diisopropylethylamine (DIPEA) and a catalytic amount of an acyl transfer like pyridine, dimethylaminopyridine (DMAP) at temperatures ranging from room temperature to reflux, for a time ranging from 1 hour to 72 hours to yield an intermediate of formula (XXI);
Step "x": compound of formula (XXI) is reacted with a base such as triethylamine (TEA), potassium carbonate (K₂CO₃), Caesium carbonate (Cs₂CO₃) in solvents like methanol, ethanol and the like at temperatures ranging from room temperature to 40 °C, for a time ranging from 1 hour to 48 hours to obtain compound (IVb);
Step "y": reaction with an alkylating agent R₃Z wherein Z is as defined above and R₃ is a straight or branched optionally substituted C₁-C₆ alkyl under the same reaction conditions described for step "o" in Scheme 8, providing the intermediate of formula (IVa).

An intermediate of formula (IVa) can be further transformed into a compound of formula (I) reacting it with intermediates of formula (3a)-(3h) under the same reaction conditions described for steps "d" of Method B, followed by removal of the *tert*-butoxycarbonyl protecting group P under the same acidic conditions previously reported and finally removing the acyl group R'CO, where R' is preferentially a methyl group, under the same basic conditions previously described for step "u" in Scheme 10.

An intermediate of formula (IVb) can be further transformed into a compound of formula (I) reacting it with intermediates of formula (3a)-(3h) under the same reaction conditions described for steps "d" of Method B, followed by removal of the *tert*-butoxycarbonyl protecting group P under the same acidic conditions previously reported.

### PHARMACOLOGY

### In vitro Cell Proliferation assay

To evaluate the antiproliferative activity of a compound of formula (I) the following human cell lines were used: A2780 ovarian and MCF-7 breast carcinoma; TT and MZ-CRC-1 medullary thyroid carcinoma, harboring a mutated RET-C634W and RET-M918T receptor respectively, LC-2/ad Non Small Cell Lung Carcinoma, expressing the CCDC6-RET fusion gene product. Exponentially growing cells were seeded and incubated at 37°C in a humidified 5% CO2 atmosphere using appropriate medium supplemented with 10% Fetal Bovine Serum. 24 hours following cell plating, scalar doses of the compounds dissolved in 0.1% DMSO were added to the medium and cells were exposed to drugs for either 72 hours (MCF-7 and A2780) or 144 hours (TT, MZ-CRC-1 and LC-2/ad), according to their different proliferation rate. At the end of treatment, cell proliferation was determined by an intracellular ATP monitoring system (CellTiterGlo - Promega), following manufacturer's instructions, and using an Envision instrument (PerkinElmer) as reader. Data obtained from compound versus vehicle treated cells were compared using Assay Explorer (Symyx Technologies Inc) software. IC50 values were calculated using sigmoidal interpolation curve fitting.

### High-Throughput Solubility assay

A nominal 200 µM suspensions/solutions of a compound of formula (I) in aqueous potassium phosphate buffer at pH = 7 and aqueous citric acid buffer at pH = 3 were prepared on a "Multiscreen-HTS", 0.2 µm filter plate (Millipore, Billerica, MA). These solutions were stirred for 10 min and stored for 24 h at room temperature to pre-saturate the membrane filter and to reach a "pseudo-thermodynamic solubility". Then the plate was put on a 500 µL 96 multiwell plate containing 200 µL of acetonitrile in each well and centrifuged at 2000 rpm for 5 minutes. The so obtained solutions were then analyzed simultaneously by HPLC-UV with standard solutions for quantification.

In the following Table A the antiproliferative activity of representative compounds of formula (I) on two medullary thyroid carcinoma cell lines expressing the aforementioned mutated forms of RET (TT and MZ-CRC-1) is reported. As control, two unrelated non RET-dependent cell lines (A2780 and MCF7) were tested. All these compounds show remarkable activity on RET-driven cellular models with respect to the unrelated ones. In addition, most of the compouds displays superior activity compared to the Reference compound, which corresponds to compound of Example 11 of the patent application WO2010/145998 cited above and is the disclaimed compound in the present formula (I).

In Table A solubility data of representative compounds of formula (I) at neutral, physiological pH (pH 7) are also reported. These data are useful to evaluate the improvement of the compounds of the present invention with respect to the reference compound. In fact they are endowed with a largely improved solubility, thus allowing to evaluate them *in vivo* both after i.v. (intravenous) and after os (oral) administration. The lack of solubility of the reference compound instead makes it hardly suitable for *in vivo* studies. Furthermore, the improved solubility allows obtaining compounds with better formulation and/or pharmaocokinetic/pharmacodynamic properties.

**Table A**

| Cmpd # | Solubility (µM) @ pH 7 | A2780 IC₅₀ (µM) | MCF7 IC₅₀ (µM) | TT IC₅₀ (µM) (144h) | MZ-CRC-1 IC₅₀ (µM) (144h) | LC-2/ad IC₅₀ (µM) (144h) |
|---|---|---|---|---|---|---|
| Reference cmpd (Example 11 of WO2010/145998) | <1 | 2.746 | 4.543 | 0.041 | 0.317 | |
| 8 | 6.5 | 1.761 | 4.783 | 0.002 | 0.074 | 0.038 |
| 22 | 5.8 | 1.148 | 2.806 | 0.007 | | |
| 30 | 1.7 | 1.002 | 2.189 | 0.008 | | |
| 29 | 7.4 | 2.229 | 9.162 | 0.085 | | |
| 38 | 10.8 | 1.950 | 5.038 | 0.038 | | |
| 44 | 4.7 | 1.718 | 3.483 | 0.069 | 0.231 | |
| 48 | 26.9 | 0.085 | 1.836 | 0.0005 | 0.004 | 0.004 |
| 77 | 23 | 2.817 | 6.653 | 0.002 | 0.023 | 0.003 |
| 78 | 1.4 | 2.223 | 2.310 | 0.007 | 0.026 | 0.014 |
| 105 | 15.3 | 2.738 | > 10 | 0.025 | | |
| 122 | 9.4 | 0.704 | 2.687 | 0.001 | | |
| 123 | 25.1 | 3.898 | > 10 | 0.060 | | |
| 129 | 26.4 | 0.105 | 8.182 | 0.002 | | |
| 149 | 30.4 | 1.906 | 3.725 | 0.013 | | |
| 150 | 40.2 | 1.927 | 5.637 | 0.006 | | |
| 164 | 8.9 | 2.022 | 5.623 | 0.002 | | |
| 174 | 3.5 | 1.147 | | 0.005 | 0.015 | 0.010 |
| 176 | 7.6 | 3.833 | | 0.014 | 0.023 | 0.020 |
| 179 | 1.2 | 3.243 | | 0.017 | 0.038 | |
| 190 | 2.9 | 2.310 | | 0.008 | 0.039 | 0.051 |
| 193 | < 1 | 0.910 | | 0.016 | 0.039 | 0.015 |

From all of the above, the novel compounds of formula (I) of the present invention appear to be particularly advantageous in the therapy of diseases caused by dysregulated protein kinase activity, such as cancer.

The compounds of the present invention can be administered either as single agents or, alternatively, in combination with known anticancer treatments, such as radiation therapy or chemotherapy regimen, in combination with, for example, antihormonal agents, such as antiestrogens, antiandrogens and aromatase inhibitors, topoisomerase I inhibitors, topoisomerase II inhibitors, agents that target microtubules, platin-based agents, alkylating agents, DNA damaging or intercalating agents, antineoplastic antimetabolites, other kinase inhibitors, other anti-angiogenic agents, inhibitors of kinesins, therapeutic monoclonal antibodies, inhibitors of mTOR, histone deacetylase inhibitors, farnesyl transferase inhibitors and inhibitors of hypoxic response.

If formulated as a fixed dose, such combination products employ the compounds of this invention within the dosage range described below and the other pharmaceutically active agent within the approved dosage range.

Compounds of formula (I) may be used sequentially with known anticancer agents when a combination formulation is inappropriate.

The compounds of formula (I) of the present invention, suitable for administration to a mammal, e.g., to humans, can be administered by the usual routes and the dosage level depends upon the age, weight and conditions of the patient and administration route.

For example, a suitable dosage adopted for oral administration of a compound of formula (I) may range from about 10 to about 1g per dose, from 1 to 5 times daily. The compounds of the invention can be administered in a variety of dosage forms, e.g. orally, in the form tablets, capsules, sugar or film coated tablets, liquid solutions or suspensions; rectally in the form suppositories; parenterally, e.g. intramuscularly, or through intravenous and/or intrathecal and/or intraspinal injection or infusion.

The present invention also includes pharmaceutical compositions comprising a compound of formula (I) or a pharmaceutically acceptable salt thereof in association with a pharmaceutically acceptable excipient, which may be a carrier or a diluent.

The pharmaceutical compositions containing the compounds of the invention are usually prepared following conventional methods and are administered in a suitable pharmaceutical form.

For example, the solid oral forms may contain, together with the active compound, diluents, e.g. lactose, dextrose saccharose, sucrose, cellulose, corn starch or potato starch; lubricants, e.g. silica, talc, stearic acid, magnesium or calcium stearate, and/or polyethylene glycols; binding agents, e.g. starches, arabic gum, gelatine methylcellulose, carboxymethylcellulose or polyvinyl pyrrolidone; disintegrating agents, e.g. starch, alginic acid, alginates or sodium starch glycolate; effervescing mixtures; dyestuffs; sweeteners; wetting agents such as lecithin, polysorbates, laurylsulphates; and, in general, non-toxic and pharmacologically inactive substances used in pharmaceutical formulations. These pharmaceutical preparations may be manufactured in known manner, for example, by means of mixing, granulating, tabletting, sugar-coating, or film-coating processes.

The liquid dispersions for oral administration may be, e.g. syrups, emulsions and suspensions.

As an example the syrups may contain, as a carrier, saccharose or saccharose with glycerine and/or mannitol and sorbitol.

The suspensions and the emulsions may contain, as examples of carriers, natural gum, agar, sodium alginate, pectin, methylcellulose, carboxymethylcellulose, or polyvinyl alcohol.

The suspension or solutions for intramuscular injections may contain, together with the active compound, a pharmaceutically acceptable carrier, e.g. sterile water, olive oil, ethyl oleate, glycols, e.g. propylene glycol and, if desired, a suitable amount of lidocaine hydrochloride.

The solutions for intravenous injections or infusions may contain, as a carrier, sterile water or preferably they may be in the form of sterile, aqueous, isotonic, saline solutions or they may contain propylene glycol as a carrier.

The suppositories may contain, together with the active compound, a pharmaceutically acceptable carrier, e.g. cocoa butter, polyethylene glycol, a polyoxyethylene sorbitan fatty acid ester surfactant or lecithin.

### EXPERIMENTAL SECTION

For a reference to any specific compound of the invention, optionally in the form of a pharmaceutically acceptable salt, see the experimental section and claims.

The short forms and abbreviations used herein have the following meaning:

| | |
|---|---|
| g (grams) | mg (milligrams) |
| mL (milliliters) | mM (millimolar) |
| µM (micromolar) | mmol (millimoles) |
| h (hours) | MHz (Mega-Hertz) |
| mm (millimetres) | Hz (Hertz) |
| M (molar) | min (minutes) |
| mol (moles) | TLC (thin layer chromatography) |
| NaHCO₃ (sodium bicarbonate) | Na₂SO₄ (sodium sulfate) |
| K₃PO₄ (potassium phosphate tribasic) | Cs₂CO₃ (cesium carbonate) |
| NH₄Cl (ammonium chloride) | HOBT (1-hydroxybenzotriazole) |
| K₂CO₃ (potassium carbonate) | MeI (methyl iodide) |
| SnCl₂·2H₂O (tin(II) chloride dihydrate) | TBAF (Tetrabutylammonium fluoride) |
| PdCl₂(PPh₃)₂(Bis(triphenylphosphine)palladium(II) dichloride | |
| EDAC (N-(3-dimethylaminopropyl)-N'-ethylcarbodiimide hydroc hloride) | |
| Pd(PPh₃)₄ (tetrakis(triphenylphosphine)palladium(0)) | NBS (N-Bromosuccinimide) |
| PdCl₂(dppf)₂ (1,1'-Bis(diphenylphosphino)ferrocene-palladium(I) II)dichloride dichloromethane complex) | |
| Pd(OAc)₂ (palladium acetate) | DMA (dimethylacetamide) |
| Xantphos (4,5-Bis(diphenylphosphino)-9,9-dimethylxanthene) | |
| NaBH(OAc)₃ (sodium triacetoxyborohydride) | |
| NaCNBH₃ (sodium cyanoborohydride) | TMSCH₂N₂ (trimethylsilyldiazomethane) |
| MTBE (methyl *tert*-butyl ether) | AlCl₃ (aluminium chloride) |
| *t*BuONa (sodium *tert*-butoxide) | Na₂S₂O₅ (sodium metabisulfite) |
| NH₃ (33% in water ammonium hydroxide solution) | Fe (iron powder) |
| NaI (sodium iodide) | DMAP (dimethylaminopyridine) |
| r.t. (room temperature) | TEA (triethylamine) |
| HCOONH₄ (ammonium formate) | Na₂CO₃ (sodium carbonate) |
| EtOAc (ethyl acetate) | MeCN (acetonitrile) |
| TFA (trifluoroacetic acid) | DMF (*N,N*-dimethyl formamide) |
| DIPEA (*N,N*-diisopropyl-*N*-ethylamine) | DCM (dichloromethane) |
| THF (tetrahydrofuran) | Hex (hexane) |
| EtOH (ethanol) | NaOH (sodium hydroxide solution) |
| HCl(hydrochloric acid solution) | CuI (copper(I) iodide) |
| MeOH (Methanol) | DMSO (dimethylsulfoxide) |
| Et₂O (ethyl ether) | PTSA (*p*-toluenesulfonic acid monohydrate) |
| TIPS (triisopropylsilyl) | bs (broad singlet) |
| TBDMSCI (dimethyl-*tert*-butylsilyl chloride) | AcOH (glacial acetic acid) |
| BOC (*tert*-butyloxycarbonyl) | Ac₂O (acetic anhydride) |
| NaH = sodium hydride, 60% in mineral oil | ESI = electrospray ionization |
| TBTU ((2-(1H-benzotriazol-1-yl)-1,1,3,3-tetramethyluronium tetrafluoroborate) | |
| RP-HPLC (reverse phase high performance liquid chromatography) | |

With the aim to better illustrate the present invention, without posing any limitation to it, the following examples are now given.

As used herein the symbols and conventions used in the processes, schemes and examples are consistent with those used in the contemporary scientific literature, for example, the *Journal of the American Chemical Society* or the *Journal of Biological Chemistry.*

Unless otherwise noted, all materials were obtained from commercial suppliers, of the best grade and used without further purification. Anhydrous solvent such as DMF, THF, DCM and toluene were obtained from the Aldrich Chemical Company. All reactions involving air- or moisture-sensitive compounds were performed under nitrogen or argon atmosphere.

### General purification and analytical methods

Flash Chromatography was performed on silica gel (Merck grade 9395, 60A).

Electrospray (ESI) mass spectra were obtained on a Thermo Finnigan LCQ Deca XP ion trap. HPLC-UV-MS analyses, used to assess compound purity, were carried out combining the ion trap MS instrument with HPLC system Surveyor (Thermo Finnigan) equipped with an autosampler and a diode array detector (UV detection 215-400 nm). Instrument control, data acquisition and processing were performed by using Xcalibur 1.4 SR1 software (Thermo Finnigan). HPLC chromatography was run at room temperature, and 1 ml/min flow rate, using a Phenomenex Gemini NX C18 column (4.6x 50mm; 3 µm). Mobile phase A was ammonium acetate 5 mM buffer (pH 5.5 with acetic acid)/ acetonitrile 95:5, and mobile phase B was ammonium acetate 5 mM buffer (pH 5.5 with acetic acid)/ acetonitrile 5:95; the gradient was from 0 to 100% B in 7 minutes then hold 100% B for 2 minutes before requilibration.

Exact mass data ESI(+) were obtained on a Waters Q-Tof Ultima directly connected with micro HPLC 1100 Agilent as previously described [M.Colombo, F. Riccardi-Sirtori, V. Rizzo, Rapid Commun. Mass Sectrom. 2004, 18, 511-517].

Mass are given as m/z ratio.

When necessary, compounds were purified by preparative HPLC on a Waters Symmetry C18 (19 x 50 mm, 5 um) column or on a Waters X Terra RP 18 (30 x 150 mm, 5 µm) column using a Waters preparative HPLC 600 equipped with a 996 Waters PDA detector and a Waters ZQ single quadrupole mass spectrometer, electron spray ionization, positive mode. Mobile phase A was water/0.1% trifluoroacetic acid and mobile phase B was acetonitrile. Gradient from 10 to 90% B in 8 min, hold 90% B 2 min. Flow rate 20 mL/min. In alternative, mobile phase A was water/0.05% NH₃, and mobile phase B was acetonitrile. Gradient from 10 to 100% B in 8 min, hold 100% B 2 min. Flow rate 20 mL/min.

1H-NMR spectrometry was performed on a Mercury VX 600 operating at 600 MHz equipped with a 5 mm double resonance probe [1H (15N-31P) ID_PFG Varian].

### Preparation 1

### 1-Cyclopropylmethyl-4-(4-nitro-2-trifluoromethyl-benzyl)-piperazine

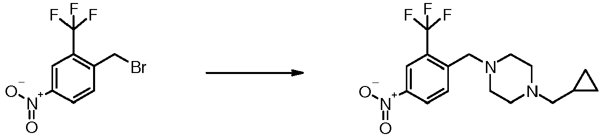

To solution of 1-bromomethyl-4-nitro-2-trifluoromethyl-benzene (0.25 g, 0.88 mmol) in anhydrous MeCN (5 mL), 1-cyclopropylmethyl-piperazine (0.37 g, 2.64 mmol) was added. The reaction was stirred at r.t. for 1 h, then a saturated solution of NaHCO₃ was added and the mixture was extracted with EtOAc (2 x 10 mL). The organic phase was washed with brine, dried with anhydrous Na₂SO₄ and evaporated under vacuum to obtain the title compound (0.29 g, 96%) as yellow oil.
¹H NMR (600 MHz, DMSO-*d*₆) δ ppm 0.00 - 0.09 (m, 2 H) 0.39 - 0.49 (m, 2 H) 0.80 (d, *J*=8.06 Hz, 1 H) 3.65 - 3.79 (m, 2 H) 8.09 (d, *J*=8.79 Hz, 1 H) 8.41 (d, *J*=2.38 Hz, 1 H) 8.51 (dd, *J*=8.61, 2.38 Hz, 1 H).

According to this same methodology, but employing suitable substituted derivatives, the following intermediates were prepared:

### 1-Methyl-4-(4-nitro-2-trifluoromethyl-benzyl)-piperazine

(ESI) *m*/*z* 304 [(M + H)⁺]. HRMS (ESI) calculated for C₁₃H₁₇F₃N₃O₂⁺ [(M + H)⁺] 304.2802, found 304.2803.

### 1-Ethyl-4-(4-nitro-2-trifluoromethyl-benzyl)-piperazine

(ESI) *m*/*z* 318 [(M + H)⁺]. HRMS (ESI) calculated for C₁₄H₁₉F₃N₃O₂⁺ [(M + H)⁺] 318.3068, found 318.3070.

### 1-(4-Nitro-2-trifluoromethyl-benzyl)-4-propyl-piperazine

¹H NMR (600 MHz, DMSO-*d*₆) δ ppm 0.82 - 0.87 (m, 3 H) 1.42 (t, *J*=7.40 Hz, 2 H) 2.19 - 2.25 (m, 2 H) 2.30 - 2.48 (m, 7 H) 3.72 (s, 2 H) 8.09 (d, *J*=8.61 Hz, 1 H) 8.41 (d, *J*=2.20 Hz, 1 H) 8.51 (dd, *J*=8.61, 2.20 Hz, 1 H).

### 1-Isopropyl-4-(4-nitro-2-trifluoromethyl-benzyl)-piperazine

¹H NMR (600 MHz, DMSO-*d*₆) δ ppm 0.96 (d, *J*=6.41 Hz, 6 H) 2.59 - 2.67 (m, 1 H) 3.71 (s, 2 H) 8.09 (d, *J*=8.61 Hz, 1 H) 8.41 (d, *J*=2.38 Hz, 1 H) 8.51 (dd, *J*=8.61, 2.20 Hz, 1 H).

### 1-(4-Nitro-2-trifluoromethyl-benzyl)-piperidine

¹H NMR (600 MHz, DMSO-*d*₆) δ ppm 1.42 (d, *J*=5.31 Hz, 2 H) 1.53 (quin, *J*=5.59 Hz, 4 H) 2.34 - 2.40 (m, 4 H) 3.68 (s, 2 H) 8.10 (d, *J*=8.42 Hz, 1 H) 8.40 (d, *J*=2.38 Hz, 1 H) 8.51 (dd, *J*=8.61, 2.20 Hz, 1 H).

### 4-(4-Nitro-2-trifluoromethyl-benzyl)-morpholine

¹H NMR (600 MHz, DMSO-*d*₆) δ ppm 2.40 - 2.46 (m, 4 H) 3.57 - 3.64 (m, 4 H) 3.74 (s, 2 H) 8.12 (d, *J*=8.61 Hz, 1 H) 8.42 (d, *J*=2.20 Hz, 1 H) 8.51 (dd, *J*=8.52, 2.29 Hz, 1 H).

### Dimethyl-[(S)-1-(4-nitro-2-trifluoromethyl-benzyl)-pyrrolidin-3-yl]-amine

¹H NMR (600 MHz, DMSO-*d*₆) δ ppm 1.60 - 1.71 (m, 1 H) 1.84 - 1.94 (m, 1 H) 2.09 (s, 6 H) 2.40 (dd, *J*=8.97, 6.78 Hz, 1 H) 2.53 - 2.57 (m, 1 H) 2.59 - 2.65 (m, 1 H) 2.66 - 2.70 (m, 1 H) 2.74 - 2.80 (m, 1 H) 3.78 - 3.90 (m, 2 H) 8.05 (d, *J*=8.61 Hz, 1 H) 8.40 (d, *J*=2.20 Hz, 1 H) 8.51 (dd, *J*=8.61, 2.38 Hz, 1 H).

### Dimethyl-[(R)-1-(4-nitro-2-trifluoromethyl-benzyl)-pyrrolidin-3-yl]-amine

¹H NMR (600 MHz, DMSO-*d*₆) δ ppm 1.60 - 1.71 (m, 1 H) 1.84 - 1.94 (m, 1 H) 2.09 (s, 6 H) 2.40 (dd, *J*=8.97, 6.78 Hz, 1 H) 2.53 - 2.57 (m, 1 H) 2.59 - 2.65 (m, 1 H) 2.66 - 2.70 (m, 1 H) 2.74 - 2.80 (m, 1 H) 3.78 - 3.90 (m, 2 H) 8.05 (d, *J*=8.61 Hz, 1 H) 8.40 (d, *J*=2.20 Hz, 1 H) 8.51 (dd, *J*=8.61, 2.38 Hz, 1 H).

### 1-Methyl-4-(4-nitro-2-trifluoromethyl-benzyl)-[1,4]diazepane

¹H NMR (600 MHz, DMSO-*d*₆) δ ppm 2.26 (s, 3 H) 2.53 - 2.55 (m, 2 H) 2.56 - 2.60 (m, 2 H) 2.64 - 2.71 (m, 4 H) 3.88 (s, 2 H) 8.14 (d, *J*=8.61 Hz, 1 H) 8.40 (d, *J*=2.01 Hz, 1 H) 8.52 (dd, *J*=8.52, 2.29 Hz, 1 H).

### 2-[4-(4-Nitro-2-trifluoromethyl-benzyl)-piperazin-1-yl]-ethanol

¹H NMR (600 MHz, DMSO-*d*₆) δ ppm 2.38 (t, *J*=6.32 Hz, 3 H) 2.43 (br. s., 6 H) 3.48 (q, *J*=6.23 Hz, 2 H) 3.72 (s, 2 H) 4.36 (t, *J*=5.31 Hz, 1 H) 8.09 (d, *J*=8.61 Hz, 1 H) 8.41 (d, *J*=2.20 Hz, 1 H) 8.51 (dd, *J*=8.61, 2.20 Hz, 1 H).

### 1-(4-Nitro-2-trifluoromethyl-benzyl)-piperazine

¹H NMR (600 MHz, DMSO-*d*₆) δ ppm 2.35 (br. s., 4 H) 2.73 (t, *J*=4.67 Hz, 5 H) 3.69 (s, 2 H) 8.11 (d, *J*=8.43 Hz, 1 H) 8.41 (d, *J*=2.20 Hz, 1 H) 8.51 (dd, *J*=8.70, 2.11 Hz, 1 H).

### 1-[1-(4-Nitro-2-trifluoromethyl-benzyl)-azetidin-3-yl]-pyrrolidine

¹H NMR (600 MHz, DMSO-*d*₆) δ ppm 1.68 (br. s., 4 H) 2.26 - 2.48 (m, 4 H) 2.98 - 3.05 (m, 2 H) 3.11 (br. s., 1 H) 3.44 (t, *J*=6.96 Hz, 2 H) 3.86 (s, 2 H) 7.99 (d, *J*=8.61 Hz, 1 H) 8.39 (d, *J*=2.38 Hz, 1 H) 8.49 (dd, *J*=8.52, 2.29 Hz, 1 H).

### Methyl-(1-methyl-piperidin-4-yl)-(4-nitro-2-trifluoromethyl-benzyl)-amine

¹H NMR (600 MHz, DMSO-*d*₆) δ ppm 1.50 (qd, *J*=12.00, 3.94 Hz, 2 H) 1.68 (d, *J*=11.90 Hz, 2 H) 1.78 - 1.90 (m, 2 H) 2.12 (s, 2 H) 2.13 (s, 3 H) 2.33 - 2.39 (m, 1 H) 2.79 (d, *J*=10.62 Hz, 2 H) 3.79 (s, 2 H) 8.10 (d, *J*=8.43 Hz, 1 H) 8.37 (d, *J*=2.20 Hz, 1 H) 8.48 (dd, *J*=8.61, 2.20 Hz, 1 H).

### Dimethyl-[1-(4-nitro-2-trifluoromethyl-benzyl)-piperidin-4-yl]-amine

¹H NMR (600 MHz, DMSO-*d*₆) δ ppm 1.42 (qd, *J*=11.90, 3.66 Hz, 2 H) 1.73 (d, *J*=12.82 Hz, 2 H) 1.95 - 2.13 (m, 3 H) 2.18 (s, 6 H) 2.80 (d, *J*=11.72 Hz, 2 H) 3.70 (s, 2 H) 8.10 (d, *J*=8.61 Hz, 1 H) 8.40 (d, *J*=2.20 Hz, 1 H) 8.51 (dd, *J*=8.61, 2.38 Hz, 1 H).

### (2R,5S)-1,2,5-Trimethyl-4-(4-nitro-2-trifluoromethyl-benzyl)-piperazine

¹H NMR (600 MHz, DMSO-*d*₆) δ ppm 0.86 (d, *J*=6.23 Hz, 3 H) 0.95 - 1.00 (m, 3 H) 1.86 - 1.95 (m, 2 H) 1.98 (d, *J*=6.23 Hz, 1 H) 2.40 - 2.47 (m, 1 H) 2.70 (dd, *J*=11.26, 2.66 Hz, 1 H) 4.15 (d, *J*=15.93 Hz, 1 H) 8.16 (d, *J*=8.61 Hz, 1 H) 8.40 (d, *J*=2.38 Hz, 1 H) 8.51 (dd, *J*=8.61, 2.20 Hz, 1 H).

### 1,2,6-Trimethyl-4-(4-nitro-2-trifluoromethyl-benzyl)-piperazine

¹H NMR (600 MHz, DMSO-*d*₆) δ ppm 0.92 - 0.98 (m, 6 H) 1.89 (t, *J*=10.62 Hz, 2 H) 2.18 (d, *J*=6.23 Hz, 2 H) 2.60 - 2.66 (m, 2 H) 3.66 (s, 2 H) 8.09 (d, *J*=8.61 Hz, 1 H) 8.41 (d, *J*=2.20 Hz, 1 H) 8.51 (dd, *J*=8.52, 2.29 Hz, 1 H).

### 1-Cyclopropyl-4-(4-nitro-2-trifluoromethyl-benzyl)-piperazine

¹H NMR (600 MHz, DMSO-*d*₆) δ ppm 0.27 - 0.30 (m, 2 H) 0.36 - 0.43 (m, 2 H) 1.61 (dt, *J*=6.73, 3.14 Hz, 1 H) 2.39 (d, *J*=1.65 Hz, 4 H) 2.54 - 2.60 (m, 3 H) 3.71 (s, 2 H) 8.10 (d, *J*=8.61 Hz, 1 H) 8.41 (d, *J*=2.38 Hz, 1 H) 8.52 (dd, *J*=8.61, 2.20 Hz, 1 H).

### 4-(4-Nitro-2-trifluoromethyl-benzyl)-piperazine-1-carboxylic acid tert-butyl ester

¹H NMR (600 MHz, DMSO-*d*₆) δ ppm 1.40 (s, 9 H) 2.38 (t, *J*=4.95 Hz, 4 H) 3.35 (br. s., 3 H) 3.76 (s, 2 H) 8.11 (d, *J*=8.61 Hz, 1 H) 8.42 (d, *J*=2.20 Hz, 1 H) 8.51 (dd, *J*=8.61, 2.20 Hz, 1 H).

### 4-(4-Nitro-2-trifluoromethyl-benzyl)-piperazin-2-one

¹H NMR (600 MHz, DMSO-*d*₆) δ ppm 2.58 - 2.63 (m, 2 H) 3.02 (s, 2 H) 3.16 - 3.21 (m, 2 H) 3.83 (s, 2 H) 7.80 (br. s., 1 H) 8.10 (d, *J*=8.61 Hz, 1 H) 8.42 (d, *J*=2.20 Hz, 1 H) 8.51 (dd, *J*=8.52, 2.29 Hz, 1 H).

### 1-(4-Nitro-2-trifluoromethyl-benzyl)-piperidin-3-ol

¹H NMR (600 MHz, DMSO-*d*₆) δ ppm 1.06 - 1.16 (m, 1 H) 1.40 - 1.51 (m, 1 H) 1.65 (dt, *J*=13.23, 3.37 Hz, 1 H) 1.75 - 1.83 (m, 1 H) 1.86 (t, *J*=9.80 Hz, 1 H) 1.97 - 2.03 (m, 1 H) 2.58 - 2.64 (m, 1 H) 2.74 (dd, *J*=10.35, 3.57 Hz, 1 H) 3.51 (td, *J*=9.39, 4.67 Hz, 1 H) 3.65 - 3.78 (m, 2 H) 4.63 (d, *J*=4.95 Hz, 1 H) 8.10 (d, *J*=8.43 Hz, 1 H) 8.41 (d, *J*=2.20 Hz, 1 H) 8.51 (dd, *J*=8.61, 2.38 Hz, 1 H).

### 1-(4-Nitro-2-trifluoromethyl-benzyl)-piperidin-4-ol

¹H NMR (600 MHz, DMSO-*d*₆) δ ppm 1.39 - 1.48 (m, 2 H) 1.69 - 1.76 (m, 2 H) 2.15 (t, *J*=9.62 Hz, 2 H) 2.62 - 2.70 (m, 2 H) 3.49 (dt, *J*=8.20, 4.24 Hz, 1 H) 3.70 (s, 2 H) 4.58 (d, *J*=4.03 Hz, 1 H) 8.10 (d, *J*=8.43 Hz, 1 H) 8.40 (d, *J*=2.20 Hz, 1 H) 8.51 (dd, *J*=8.52, 2.29 Hz, 1 H).

### 2-[Methyl-(4-nitro-2-trifluoromethyl-benzyl)-amino]-ethanol

¹H NMR (600 MHz, DMSO-*d*₆) δ ppm 2.22 (s, 3 H) 2.51 - 2.53 (m, 2 H) 3.54 (q, *J*=6.04 Hz, 2 H) 3.79 (s, 2 H) 4.48 (t, *J*=5.31 Hz, 1 H) 8.19 (d, *J*=8.61 Hz, 1 H) 8.40 (d, *J*=2.20 Hz, 1 H) 8.50 (dd, *J*=8.52, 2.29 Hz, 1 H).

### 3-[Methyl-(4-nitro-2-trifluoromethyl-benzyl)-amino]-propane-1,2-diol

¹H NMR (600 MHz, DMSO-*d*₆) δ ppm 2.27 - 2.42 (m, 1 H) 2.45 (dd, *J*=11.72, 7.14 Hz, 1 H) 2.58 - 2.65 (m, 1 H) 3.52 - 3.60 (m, 1 H) 3.99 (br. s., 2 H) 4.48 (t, *J*=5.49 Hz, 1 H) 4.61 (d, *J*=4.95 Hz, 1 H) 8.13 (d, *J*=8.61 Hz, 1 H) 8.40 (d, *J*=2.20 Hz, 1 H) 8.51 (dd, *J*=8.70, 2.29 Hz, 1 H).

### 1-Ethyl-4-(2-fluoro-4-nitro-benzyl)-piperazine

(ESI) *m*/*z* 268 [(M + H)⁺]. HRMS (ESI) calculated for C₁₃H₁₉FN₃O₂⁺ [(M + H)⁺] 268.2993, found 268.2991.

### 1-Ethyl-4-(2-nitro-4-trifluoromethyl-benzyl)-piperazine

(ESI) *m*/*z* 318 [(M + H)⁺]. HRMS (ESI) calculated for C₁₄H₁₉F₃N₃O₂⁺ [(M + H)⁺] 318.3068, found 318.3066.

### 1-(2-Chloro-5-nitro-benzyl)-4-ethyl-piperazine

(ESI) *m*/*z* 284 [(M + H)⁺]. HRMS (ESI) calculated for C₁₃H₁₉ClN₃O₂⁺ [(M + H)⁺] 284.7539, found 284.7540.

### [2-(4-Nitro-2-trifluoromethyl-benzylamino)-ethyl]-carbamic acid tert-butyl ester

¹H NMR (600 MHz, DMSO-*d*₆) δ ppm 1.32 - 1.40 (m, 9 H) 2.53 - 2.59 (m, 2 H) 3.04 (q, *J*=5.92 Hz, 2 H) 3.96 (s, 2 H) 6.76 (br. s., 1 H) 8.12 (d, *J*=8.61 Hz, 1 H) 8.39 (d, *J*=2.01 Hz, 1 H) 8.50 (dd, *J*=8.42, 2.20 Hz, 1 H).

### 1-(2-Bromo-4-nitro-benzyl)-4-methyl-piperazine

¹H NMR (600 MHz, DMSO-*d*₆) δ ppm 2.16 (s, 3 H) 3.62 (s, 2 H) 7.75 (d, *J*=8.61 Hz, 1 H) 8.24 (dd, *J*=8.43, 2.38 Hz, 1 H) 8.40 (d, *J*=2.38 Hz, 1 H).

### Preparation 2

### 1-(2-Cyclopropyl-4-nitro-benzyl)-4-methyl-piperazine

A solution of 1-(2-bromo-4-nitro-benzyl)-4-methyl-piperazine (0.22 g, 0.7 mmol), cyclopropaneboronic acid (0.18 g, 2.1 mmol), K₃PO₄ (0.67 g, 3.15 mmol), palladium acetate (0.016 g, 0.14 mmol) and tricyclohexylphosphine (0.04 g, 0.14 mmol) in toluene (3 mL) and water (0.6 mL) was degassed with argon and then heated at reflux for 5 h. The mixture was cooled at r.t., poured in water (15 mL) and extracted with EtOAc (2 x 15 mL). The organic phase was washed with brine, dried with anhydrous Na₂SO₄ and evaporated under vacuum to afford a crude that was purified by flash column chromatography (DCM-MeOH 90:10). The title compound (0.15 g, 76%) was obtained as yellow oil.
¹H NMR (600 MHz, DMSO-*d*₆) δ ppm 0.69 - 0.75 (m, 2 H) 0.98 - 1.05 (m, 2 H) 2.16 (s, 3 H) 2.19 - 2.25 (m, 1 H) 2.44 - 2.52 (m, 8 H) 3.71 (s, 2 H) 7.58 (d, *J*=8.43 Hz, 1 H) 7.72 (d, *J*=2.56 Hz, 1 H) 8.01 (dd, *J*=8.43, 2.38 Hz, 1 H).

### Preparation 3

### 4-Methyl-1-(4-nitro-2-trifluoromethyl-benzyl)-piperazin-2-one

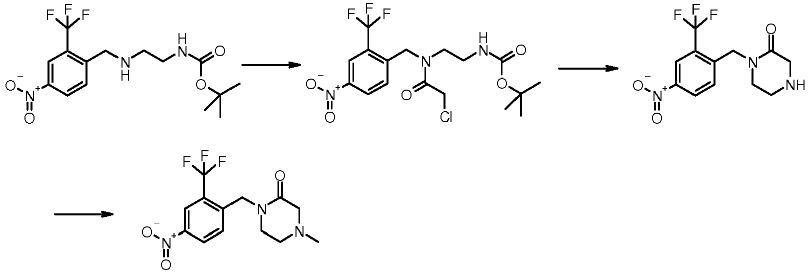

### Step 1. {2-[(2-Chloro-acetyl)-(4-nitro-2-trifluoromethyl-benzyl)-amino]-ethyl}-carbamic acid tert-butyl ester

To a solution of [2-(4-nitro-2-trifluoromethyl-benzylamino)-ethyl]-carbamic acid tert-butyl ester (0.4 g, 1.1 mmol) and TEA (0.23 mL, 1.67 mmol) in anhydrous THF (5 mL), cooled at 0-5 °C, chloro-acetyl chloride (0.1 mL, 1.25 mmol) was added. The reaction was stirred at 0-5 °C for 1 h, then a saturated solution of NaHCO₃ was added and the mixture was extracted with EtOAc (2 x 15 mL). The organic phase was washed with brine, dried with anhydrous Na₂SO₄ and evaporated under vacuum to give a crude that was purified by flash column chromatography (DCM-MeOH 96:4). The title compound (0.32 g, 67%) was obtained as yellow oil.
¹H NMR (600 MHz, DMSO-*d*₆) δ ppm 1.34 - 1.37 (m, 9 H) 1.94 - 1.94 (m, 0 H) 3.16 (q, *J*=6.04 Hz, 2 H) 3.45 (t, *J*=5.86 Hz, 2 H) 4.57 (s, 2 H) 4.76 (s, 2 H) 7.05 (t, *J*=5.68 Hz, 1 H) 7.59 (d, *J*=8.06 Hz, 1 H) 8.42 - 8.49 (m, 2 H).

### Step 2. 1-(4-Nitro-2-trifluoromethyl-benzyl)-piperazin-2-one

To a solution of {2-[(2-chloro-acetyl)-(4-nitro-2-trifluoromethyl-benzyl)-amino]-ethyl}-carbamic acid tert-butyl ester (0.32 g, 0.73 mmol) in DCM (3 mL), TFA (0.5 mL) was added and the reaction was stirred at r.t. overnight. The suspension was evaporated under vacuum, MeCN (3 mL) and Cs₂CO₃ (0.27 g, 0.82 mmol) were added and the mixture was stirred at 50 °C for 1 h. The reaction was then poured in water (20 mL) and extracted with EtOAc (2 x 15 mL), the organic phase was washed with brine, dried with anhydrous Na₂SO₄ and evaporated under vacuum. The crude was purified by flash column chromatography (DCM/MeOH 90/10) to give the title compound (0.08 g, 32%) as white solid.
¹H NMR (600 MHz, DMSO-*d*₆) δ ppm 2.86 (d, *J*=14.10 Hz, 1 H) 2.96 (t, *J*=5.40 Hz, 2 H) 3.24 - 3.28 (m, 2 H) 3.37 (s, 2 H) 4.76 (s, 2 H) 7.66 (d, *J*=8.61 Hz, 1 H) 8.45 (d, *J*=2.20 Hz, 1 H) 8.48 (dd, *J*=8.61, 2.20 Hz, 1 H).

### Step 3. 4-Methyl-1-(4-nitro-2-trifluoromethyl-benzyl)-piperazin-2-one

To a solution of 1-(4-nitro-2-trifluoromethyl-benzyl)-piperazin-2-one (0.07 g, 0.23 mmol) in MeOH (2 mL) 37% formaldehyde (0.1 mL, 1.2 mmol) and AcOH (0.05 mL, 0.94 mmol) were added. The mixture was stirred at r.t. for 15 min., then NaCNBH₃ (0.03 g, 0.47 mmol) was added. The reaction was stirred at r.t. for 1 h, then poured in a saturated solution of NaHCO₃ and extracted with EtOAc (2 x15 mL). The organic phase was washed with brine, dried with anhydrous Na₂SO₄ and evaporated under vacuum to obtain the title compound (0.07 g, 95%) as white solid.
¹H NMR (600 MHz, DMSO-*d*₆) δ ppm 2.27 (s, 3 H) 2.67 (t, *J*=5.49 Hz, 2 H) 3.10 (s, 2 H) 4.78 (s, 2 H) 7.57 (d, *J*=8.61 Hz, 1 H) 8.45 (d, *J*=2.38 Hz, 1 H) 8.51 (dd, *J*=8.61, 2.20 Hz, 1 H).

### Preparation 4

### (1-Methyl-piperidin-4-yl)-(4-nitro-2-trifluoromethyl-phenyl)-amine.

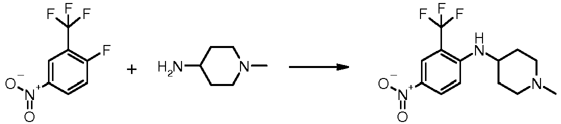

A mixture of 1-fluoro-4-nitro-2-trifluoromethyl-benzene (0.3 g, 1.43 mmol) and 1-methyl-piperidin-4-ylamine (0.73 mL, 5.72 mmol) in THF (7 mL) was stirred at r.t. for 4 h. The reaction was poured in a saturated solution of NaHCO₃ and extracted with EtOAc (2 x15 mL). The organic phase was washed with brine, dried with anhydrous Na₂SO₄ and evaporated under vacuum to obtain the title compound (0.41 g, 95%) as yellow solid.
¹H NMR (600 MHz, DMSO-*d*₆) δ ppm 1.63 - 1.75 (m, 2 H) 1.82 (d, *J*=11.17 Hz, 2 H) 2.01 - 2.07 (m, 2 H) 2.17 (s, 3 H) 2.73 (d, *J*=11.54 Hz, 2 H) 3.52 - 3.64 (m, 1 H) 6.05 (d, *J*=7.88 Hz, 1 H) 7.09 (d, *J*=9.16 Hz, 1 H) 8.20 - 8.25 (m, 2 H).

According to this same methodology, but employing suitable substituted derivatives, the following intermediates were prepared:

### Methyl-(1-methyl-piperidin-4-yl)-(4-nitro-2-trifluoromethyl-phenyl)-amine.

¹H NMR (600 MHz, DMSO-*d*₆) δ ppm 1.60 - 1.66 (m, 2 H) 1.72 (qd, *J*=11.90, 3.66 Hz, 2 H) 1.87 (t, *J*=10.99 Hz, 2 H) 2.14 (s, 2 H) 2.76 - 2.83 (m, 5 H) 3.23 (tt, *J*=11.17, 3.66 Hz, 1 H) 7.53 (d, *J*=9.16 Hz, 1 H) 8.33 (dd, *J*=9.25, 2.84 Hz, 1 H) 8.38 (d, *J*=2.75 Hz, 1 H).

### 1-Ethyl-4-(4-nitro-2-trifluoromethyl-phenyl)-piperazine.

¹H NMR (600 MHz, DMSO-*d*₆) δ ppm 1.03 (t, *J*=7.23 Hz, 3 H) 2.39 (q, *J*=7.14 Hz, 2 H) 3.11 - 3.17 (m, 4 H) 7.54 (d, *J*=8.97 Hz, 1 H) 8.36 - 8.42 (m, 2 H).

### Preparation 5

### 1-Methyl-4-(4-nitro-2-trifluoromethyl-phenoxy)-piperidine

To a solution of 1-methyl-piperidin-4-ol (0.4 g, 3.47 mmol) in anhydrous THF (10 mL), cooled at 0-5 °C, NaH (0.14 g, 3.47 mmol) was added. The mixture was stirred at 0-5 °C for 30 min, then 1-fluoro-4-nitro-2-trifluoromethyl-benzene (0.33 mL, 2.31 mmol) was added. The reaction was stirred at r.t. for 1 h, then poured in water and extracted with EtOAc (2 x 20 mL). The organic phase was washed with brine, dried with Na₂SO₄ and evaporated under vacuum to obtain the title compound (0.65 g, 93%) as yellow oil.
¹H NMR (600 MHz, DMSO-*d*₆) δ ppm 1.67 - 1.79 (m, 2 H) 1.95 (td, *J*=8.43, 4.03 Hz, 2 H) 2.18 (s, 3 H) 2.27 - 2.37 (m, 2 H) 2.47 (br. s., 2 H) 4.87 (br. s., 1 H) 7.57 (d, *J*=9.34 Hz, 1 H) 8.38 (d, *J*=2.75 Hz, 1 H) 8.47 (dd, *J*=9.34, 2.93 Hz, 1 H).

### Preparation 6

### 3-(Isopropyl-dimethyl-silanyloxy)-1-(4-nitro-2-trifluoromethyl-benzyl)-piperidine

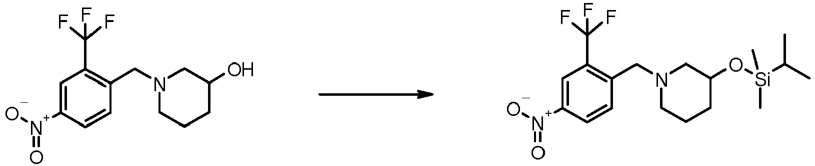

A mixture of 1-(4-nitro-2-trifluoromethyl-benzyl)-piperidin-3-ol (0.17 g, 0.55 mmol), imidazole (0.09 g, 1.37 mmol) and TBDMSCl (0.1 g, 0.66 mmol) in DMF (5 mL) was stirred at r.t. for 6 h. The reaction was poured in a saturated solution of NaHCO₃ and extracted with Et₂O (2 x 20 mL). The organic phase was washed with brine, dried with anhydrous Na₂SO₄ and evaporated under vacuum to give the title compound (0.19 g, 83%) as white solid.
¹H NMR (600 MHz, DMSO-*d*₆) δ ppm 0.00 (s, 3 H) 0.01 - 0.03 (m, 3 H) 0.83 (s, 9 H) 1.13 - 1.25 (m, 1 H) 1.41 - 1.50 (m, 1 H) 1.66 (dt, *J*=13.55, 3.48 Hz, 1 H) 1.78 - 1.84 (m, 1 H) 1.98 (t, *J*=9.71 Hz, 1 H) 2.01 - 2.06 (m, 1 H) 2.56 - 2.62 (m, 1 H) 2.75 (d, *J*=6.78 Hz, 1 H) 3.68 - 3.80 (m, 2 H) 8.09 (d, *J*=8.61 Hz, 1 H) 8.41 (d, *J*=2.38 Hz, 1 H) 8.49 (dd, *J*=8.61, 2.38 Hz, 1 H).

According to this same methodology, but employing suitable substituted derivatives, the following intermediates were prepared:

### 4-(Isopropyl-dimethyl-silanyloxy)-1-(4-nitro-2-trifluoromethyl-benzyl)-piperidine

¹H NMR (600 MHz, DMSO-*d*₆) δ ppm 0.02 - 0.05 (m, 6 H) 0.86 (s, 9 H) 1.43 - 1.53 (m, 2 H) 1.68 - 1.79 (m, 2 H) 2.24 (t, *J*=8.97 Hz, 2 H) 2.56 - 2.66 (m, 2 H) 3.70 (s, 2 H) 3.76 (m, *J*=3.85 Hz, 1 H) 8.10 (d, *J*=8.42 Hz, 1 H) 8.40 (d, *J*=2.20 Hz, 1 H) 8.50 (dd, *J*=8.52, 2.29 Hz, 1 H).

### [2-(Isopropyl-dimethyl-silanyloxy)-ethyl]-methyl-(4-nitro-2-trifluoromethyl-benzyl)-amine

¹H NMR (600 MHz, DMSO-*d*₆) δ ppm 0.01 - 0.05 (m, 6 H) 0.81 - 0.88 (m, 10 H) 2.24 (s, 3 H) 2.57 - 2.61 (m, 2 H) 3.73 (t, *J*=5.77 Hz, 2 H) 3.85 (s, 2 H) 8.16 (d, *J*=8.61 Hz, 1 H) 8.40 (d, *J*=2.20 Hz, 1 H) 8.49 (dd, *J*=8.61, 2.20 Hz, 1 H).

### Preparation 7

### (2,2-Dimethyl-[1,3]dioxolan-4-ylmethyl)-methyl-(4-nitro-2-trifluoromethyl-benzyl)-amine

To a solution of 3-[methyl-(4-nitro-2-trifluoromethyl-benzyl)-amino]-propane-1,2-diol (0.23 g, 0.74 mmol) in DCM (5 mL), 2-methoxypropene (0.21 mL, 2.22 mmol) and PTSA (0.16 g, 0.81 mmol) were added. The mixture was stirred at r.t. for 2 h, then poured in a saturated solution of NaHCO₃ and extracted with DCM (2 x 10 mL). The organic phase was washed with brine, dried with anhydrous Na₂SO₄ and evaporated under vacuum to obtain the title compound (0.18 g, 70%) as yellow oil.
¹H NMR (600 MHz, DMSO-*d*₆) δ ppm 1.26 (s, 6 H) 2.24 (s, 3 H) 2.58 (d, *J*=5.86 Hz, 2 H) 3.55 (dd, *J*=7.88, 6.96 Hz, 1 H) 3.77 - 3.87 (m, 2 H) 4.03 (dd, *J*=8.06, 6.41 Hz, 1 H) 4.26 (t, *J*=6.23 Hz, 1 H) 8.13 (d, *J*=8.79 Hz, 1 H) 8.41 (d, *J*=2.20 Hz, 1 H) 8.52 (dd, *J*=8.61, 2.20 Hz, 1 H).

### Preparation 8

### Acetic acid 2-[4-(4-nitro-2-trifluoromethyl-benzyl)-piperazin-1-yl]-ethyl ester

To a solution of 2-[4-(4-nitro-2-trifluoromethyl-benzyl)-piperazin-1-yl]-ethanol (0.30 g, 0.895 mmol) and TEA (0.18 mL, 1.34 mmol) in anhydrous THF (5 mL), cooled at 0-5 °C, acetyl chloride (0.1 mL, 1.34 mmol) was added. The reaction was stirred at 0-5 °C for 1h, then was poured in a saturated solution of NaHCO₃ and extracted with EtOAc (2 x 15 mL). The organic phase was washed with brine, dried with anhydrous Na₂SO₄ and evaporated under vacuum to afford the title compound (0.31 g, 92%) as yellow oil.
¹H NMR (600 MHz, DMSO-*d*₆) δ ppm 2.00 (s, 3 H) 2.53 - 2.56 (m, 2 H) 3.72 (s, 2 H) 4.09 (t, *J*=5.95 Hz, 2 H) 8.08 (d, *J*=8.43 Hz, 1 H) 8.41 (d, *J*=2.01 Hz, 1 H) 8.51 (dd, *J*=8.61, 2.20 Hz, 1 H).

### Preparation 9

### 4-(4-Cyclopropylmethyl-piperazin-1-ylmethyl)-3-trifluoromethyl-phenylamine

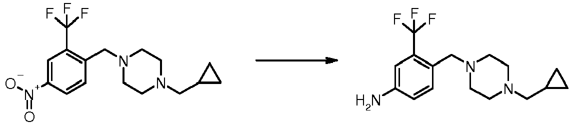

To a solution of 1-cyclopropylmethyl-4-(4-nitro-2-trifluoromethyl-benzyl)-piperazine (0.33 g, 0.954 mmol) in a mixture of EtOH (10 mL) and water (3 mL), Fe (0.27 g, 4.77 mmol) and NH₄Cl (0.51 g, 9.54 mmol) were added. The reaction was heated at 80 °C for 5 h, then was cooled at room temperature and filtered through celite washing with EtOH. The solution was evaporated under vacuum, the residue dissolved with EtOAc (20 mL) and washed with a saturated solution of NaHCO₃. The organic phase was washed with brine, dried with anhydrous Na₂SO₄ and evaporated under vacuum to give the title compound (0.28 g, 94%) as yellow solid.
¹H NMR (600 MHz, DMSO-*d*₆) δ ppm -0.01 - 0.07 (m, 2 H) 0.39 - 0.47 (m, 2 H) 0.72 - 0.84 (m, 1 H) 2.14 (d, *J*=6.59 Hz, 2 H) 3.38 (s, 1 H) 5.41 (s, 1 H) 6.75 (dd, *J*=8.42, 2.01 Hz, 1 H) 6.85 (d, *J*=2.20 Hz, 1 H) 7.29 (d, *J*=8.24 Hz, 1 H).

According to this same methodology, but employing suitable substituted derivatives, the following intermediates were prepared:

### 4-(4-Methyl-piperazin-1-ylmethyl)-3-trifluoromethyl-phenylamine

¹H NMR (600 MHz, DMSO-*d*₆) δ ppm 2.14 (s, 4 H) 2.17 - 2.41 (m, 9 H) 3.38 (s, 2 H) 5.41 (s, 2 H) 6.74 (dd, *J*=8.33, 2.11 Hz, 1 H) 6.85 (d, *J*=2.38 Hz, 1 H) 7.28 (d, *J*=8.43 Hz, 1 H).

### 4-(4-Ethyl-piperazin-1-ylmethyl)-3-trifluoromethyl-phenylamine

¹H NMR (401 MHz, DMSO-*d*₆) δ ppm 0.97 (t, *J*=7.20 Hz, 3 H) 2.20 - 2.45 (m, 8 H) 3.38 (s, 2 H) 5.41 (s, 2 H) 6.75 (dd, *J*=8.24, 2.26 Hz, 1 H) 6.85 (d, *J*=2.32 Hz, 1 H) 7.28 (d, J=8.42 Hz, 1 H).

### 4-(4-Isopropyl-piperazin-1-ylmethyl)-3-trifluoromethyl-phenylamine

¹H NMR (600 MHz, DMSO-*d*₆) δ ppm 0.90 - 0.99 (m, 6 H) 2.26 - 2.47 (m, 7 H) 2.56 - 2.61 (m, 1 H) 3.37 (s, 2 H) 5.41 (s, 2 H) 6.75 (dd, *J*=8.43, 2.20 Hz, 1 H) 6.83 - 6.86 (m, 1 H) 7.29 (d, *J*=8.24 Hz, 1 H).

### 4-(4-Propyl-piperazin-1-ylmethyl)-3-trifluoromethyl-phenylamine

¹H NMR (600 MHz, DMSO-*d*₆) δ ppm 0.83 (t, *J*=7.42 Hz, 3 H) 1.40 (sxt, *J*=7.36 Hz, 2 H) 2.16 - 2.23 (m, 2 H) 2.26 - 2.47 (m, 7 H) 3.38 (s, 2 H) 5.41 (s, 2 H) 6.74 (dd, *J*=8.33, 2.11 Hz, 1 H) 6.85 (d, *J*=2.38 Hz, 1 H) 7.28 (d, *J*=8.24 Hz, 1 H).

### 4-Piperidin-1-ylmethyl-3-trifluoromethyl-phenylamine

¹H NMR (600 MHz, DMSO-*d*₆) δ ppm 1.38 (d, *J*=4.95 Hz, 2 H) 1.47 (quin, *J*=5.54 Hz, 5 H) 2.28 (br. s., 4 H) 5.39 (s, 2 H) 6.75 (dd, *J*=8.61, 2.20 Hz, 1 H) 6.81 - 6.85 (m, 1 H) 7.30 (d, *J*=8.42 Hz, 1 H).

### 4-Morpholin-4-ylmethyl-3-trifluoromethyl-phenylamine

¹H NMR (600 MHz, DMSO-*d*₆) δ ppm 2.31 (br. s., 4 H) 3.39 (s, 2 H) 3.55 (t, *J*=4.49 Hz, 4 H) 5.44 (s, 2 H) 6.75 (dd, *J*=8.24, 2.01 Hz, 1 H) 6.86 (d, *J*=2.20 Hz, 1 H) 7.30 (d, *J*=8.42 Hz, 1 H).

### [(S)-1-(4-Amino-2-trifluoromethyl-benzyl)-pyrrolidin-3-yl]-dimethyl-amine

¹H NMR (600 MHz, DMSO-*d*₆) δ ppm 1.58 (ddt, *J*=12.50, 8.65, 6.18, 6.18 Hz, 1 H) 1.78 - 1.86 (m, 1 H) 2.04 - 2.07 (m, 6 H) 2.26 (dd, *J*=8.79, 6.59 Hz, 1 H) 2.39 - 2.44 (m, 1 H) 2.56 - 2.60 (m, 1 H) 2.65 - 2.73 (m, 1 H) 3.43 - 3.55 (m, 2 H) 5.40 (s, 2 H) 6.74 (dd, *J*=8.24, 1.83 Hz, 1 H) 6.84 (d, *J*=2.38 Hz, 1 H) 7.28 (d, *J*=8.42 Hz, 1 H).

### [(R)-1-(4-Amino-2-trifluoromethyl-benzyl)-pyrrolidin-3-yl]-dimethyl-amine

¹H NMR (600 MHz, DMSO-*d*₆) δ ppm 1.58 (ddt, *J*=12.50, 8.65, 6.18, 6.18 Hz, 1 H) 1.78 - 1.86 (m, 1 H) 2.04 - 2.07 (m, 6 H) 2.26 (dd, *J*=8.79, 6.59 Hz, 1 H) 2.39 - 2.44 (m, 1 H) 2.56 - 2.60 (m, 1 H) 2.65 - 2.73 (m, 1 H) 3.43 - 3.55 (m, 2 H) 5.40 (s, 2 H) 6.74 (dd, *J*=8.24, 1.83 Hz, 1 H) 6.84 (d, *J*=2.38 Hz, 1 H) 7.28 (d, *J*=8.42 Hz, 1 H).

### 4-(4-Methyl-[1,4]diazepan-1-ylmethyl)-3-trifluoromethyl-phenylamine

¹H NMR (600 MHz, DMSO-*d*₆) δ ppm 1.68 (quin, *J*=5.95 Hz, 2 H) 2.23 (s, 4 H) 3.51 (s, 2 H) 5.39 (s, 2 H) 6.75 (dd, *J*=8.24, 2.01 Hz, 1 H) 6.84 (d, *J*=2.38 Hz, 1 H) 7.34 (d, *J*=8.43 Hz, 1 H).

### 4-(3-Pyrrolidin-1-yl-azetidin-1-ylmethyl)-3-trifluoromethyl-phenylamine

(ESI) *m*/*z* 300 [(M + H)⁺]. HRMS (ESI) calculated for C₁₅H₂₁N₃F₃⁺ [(M + H)⁺] 300.1682, found 300.1678.

### (4-Amino-2-trifluoromethyl-benzyl)-methyl-(1-methyl-piperidin-4-yl)-amine

¹H NMR (600 MHz, DMSO-*d*₆) δ ppm 1.49 (qd, *J*=12.03, 3.85 Hz, 3 H) 1.64 (d, *J*=11.90 Hz, 2 H) 1.76 - 1.83 (m, 3 H) 2.05 (s, 3 H) 2.09 - 2.12 (m, 4 H) 2.20 - 2.25 (m, 2 H) 2.26 - 2.35 (m, 1 H) 2.78 (d, *J*=11.36 Hz, 2 H) 3.48 (s, 2 H) 5.38 (s, 2 H) 6.72 - 6.78 (m, 1 H) 6.82 - 6.85 (m, 1 H) 7.32 (d, *J*=8.42 Hz, 1 H).

### [1-(4-Amino-2-trifluoromethyl-benzyl)-piperidin-4-yl]-dimethyl-amine

¹H NMR (600 MHz, DMSO-*d*₆) δ ppm 1.33 (qd, *J*=11.87, 3.75 Hz, 2 H) 1.68 (d, *J*=12.64 Hz, 2 H) 1.88 (t, *J*=10.81 Hz, 2 H) 1.97 - 2.05 (m, 1 H) 2.14 (s, 6 H) 2.77 (d, *J*=11.72 Hz, 2 H) 5.40 (s, 2 H) 6.73 - 6.77 (m, 1 H) 6.84 (d, *J*=2.38 Hz, 1 H) 7.30 (d, *J*=8.24 Hz, 1 H).

### 3-Trifluoromethyl-4-((2S,5R)-2,4,5-trimethyl-piperazin-1-ylmethyl)-phenylamine

¹H NMR (600 MHz, DMSO-*d*₆) δ ppm 0.83 (d, *J*=6.23 Hz, 3 H) 1.00 (d, *J*=6.04 Hz, 3 H) 1.67 (t, *J*=10.71 Hz, 1 H) 1.82 - 1.90 (m, 2 H) 2.10 (s, 3 H) 2.32 (dt, *J*=6.50, 3.34 Hz, 1 H) 2.42 (dd, *J*=11.17, 2.56 Hz, 1 H) 2.64 (dd, *J*=11.17, 2.56 Hz, 1 H) 2.90 (d, *J*=13.92 Hz, 1 H) 3.95 (d, *J*=14.10 Hz, 1 H) 5.39 (s, 2 H) 6.75 (dd, *J*=8.33, 1.92 Hz, 1 H) 6.84 (d, *J*=2.20 Hz, 1 H) 7.33 (d, *J*=8.24 Hz, 1 H).

### 3-Trifluoromethyl-4-(3,4,5-trimethyl-piperazin-1-ylmethyl)-phenylamine

¹H NMR (600 MHz, DMSO-*d*₆) δ ppm 0.93 (d, *J*=6.04 Hz, 6 H) 1.73 (t, *J*=10.71 Hz, 2 H) 2.05 - 2.10 (m, 1 H) 2.12 (s, 3 H) 2.57 - 2.61 (m, 2 H) 5.42 (s, 2 H) 6.74 (dd, *J*=8.33, 2.11 Hz, 1 H) 6.85 (d, *J*=2.38 Hz, 1 H) 7.28 (d, *J*=8.24 Hz, 1 H).

### 4-(4-Amino-2-trifluoromethyl-benzyl)-piperazine-1-carboxylic acid tert-butyl ester

¹H NMR (600 MHz, DMSO-*d*₆) δ ppm 1.35 - 1.40 (m, 9 H) 2.22 - 2.30 (m, 4 H) 3.20 - 3.31 (m, 4 H) 3.38 - 3.43 (m, 2 H) 5.44 (s, 2 H) 6.75 (dd, *J*=8.24, 2.01 Hz, 1 H) 6.86 (d, *J*=2.38 Hz, 1 H) 7.30 (d, *J*=8.24 Hz, 1 H).

### 4-(4-Amino-2-trifluoromethyl-benzyl)-piperazin-2-one

¹H NMR (600 MHz, DMSO-*d*₆) δ ppm 2.87 (s, 2 H) 3.09 - 3.15 (m, 2 H) 3.46 (s, 2 H) 5.48 (s, 2 H) 6.76 (dd, *J*=8.33, 2.11 Hz, 1 H) 6.87 (d, *J*=2.38 Hz, 1 H) 7.29 (d, *J*=8.42 Hz, 1 H) 7.71 (br. s., 1 H).

### 4-[3-(Isopropyl-dimethyl-silanyloxy)-piperidin-1-ylmethyl]-3-trifluoromethyl-phenylamine

¹H NMR (600 MHz, DMSO-*d*₆) δ ppm -0.04 - 0.00 (m, 6 H) 0.79 - 0.83 (m, 9 H) 1.36 - 1.47 (m, 1 H) 1.57 - 1.66 (m, 1 H) 1.72 - 1.82 (m, 2 H) 1.88 (t, *J*=10.44 Hz, 1 H) 2.58 (d, *J*=11.17 Hz, 1 H) 2.70 (d, *J*=7.51 Hz, 1 H) 3.36 - 3.42 (m, 2 H) 3.62 (dq, *J*=9.25, 4.49 Hz, 1 H) 5.40 (s, 2 H) 6.74 (dd, *J*=8.33, 2.11 Hz, 1 H) 6.85 (d, *J*=2.20 Hz, 1 H) 7.30 (d, *J*=8.43 Hz, 1 H).

### 4-[4-(Isopropyl-dimethyl-silanyloxy)-piperidin-1-ylmethyl]-3-trifluoromethyl-phenylamine

¹H NMR (600 MHz, DMSO-*d*₆) δ ppm 0.84 - 0.86 (m, 11 H) 1.38 - 1.46 (m, 3 H) 1.68 (d, *J*=11.54 Hz, 3 H) 2.04 - 2.14 (m, 3 H) 2.55 (d, *J*=12.64 Hz, 3 H) 3.70 (br. s., 1 H) 5.40 (s, 2 H) 6.75 (dd, *J*=8.33, 1.92 Hz, 1 H) 6.84 (d, *J*=2.38 Hz, 1 H) 7.30 (d, *J*=8.24 Hz, 1 H).

### 4-({[2-(Isopropyl-dimethyl-silanyloxy)-ethyl]-methyl-amino}-methyl)-3-trifluoromethyl-phenylamine

¹H NMR (600 MHz, DMSO-*d*₆) δ ppm 0.01 (s, 8 H) 0.84 (s, 10 H) 2.13 - 2.17 (m, 3 H) 2.43 - 2.46 (m, 2 H) 3.44 - 3.47 (m, 2 H) 3.65 - 3.67 (m, 2 H) 5.38 - 5.42 (m, 2 H) 6.71 - 6.77 (m, 1 H) 6.82 - 6.85 (m, 1 H) 7.32 - 7.37 (m, 1 H).

### 4-{[(2,2-Dimethyl-[1,3]dioxolan-4-ylmethyl)-methyl-amino]-methyl}-3-trifluoromethyl-phenylamine

¹H NMR (600 MHz, DMSO-*d*₆) δ ppm 1.26 (s, 6 H) 2.24 (s, 3 H) 2.58 (d, *J*=5.86 Hz, 2 H) 3.55 (dd, *J*=7.88, 6.96 Hz, 1 H) 3.77 - 3.87 (m, 2 H) 4.03 (dd, *J*=8.06, 6.41 Hz, 1 H) 4.26 (t, *J*=6.23 Hz, 1 H), 6.68 - 6.75 (m, 1 H) 6.80 - 6.82 (m, 1 H) 7.30 - 7.35 (m, 1 H).

### Acetic acid 2-[4-(4-amino-2-trifluoromethyl-benzyl)-piperazin-1-yl]-ethyl ester

¹H NMR (600 MHz, DMSO-*d*₆) δ ppm 2.00 (s, 3 H) 2.53 - 2.56 (m, 2 H) 3.72 (s, 2 H) 4.09 (t, *J*=5.95 Hz, 2 H) 6.60 - 6.70 (m, 1 H) 6.83 - 6.88 (m, 1 H) 7.40 - 7.55 (m, 1 H).

### 1-(4-Amino-2-trifluoromethyl-benzyl)-4-methyl-piperazin-2-one

¹H NMR (600 MHz, DMSO-*d*₆) δ ppm 2.22 (s, 3 H) 2.56 (t, *J*=5.49 Hz, 2 H) 3.02 (s, 2 H) 3.10 (t, *J*=5.59 Hz, 2 H) 4.50 (s, 2 H) 5.51 (s, 2 H) 6.76 (dd, *J*=8.24, 2.01 Hz, 1 H) 6.90 (d, *J*=2.20 Hz, 1 H) 6.95 (d, *J*=8.24 Hz, 1 H).

### 4-(4-Cyclopropyl-piperazin-1-ylmethyl)-3-trifluoromethyl-phenylamine

¹H NMR (600 MHz, DMSO-*d*₆) δ ppm 0.25 - 0.28 (m, 2 H) 0.35 - 0.39 (m, 2 H) 1.57 (tt, *J*=6.62, 3.46 Hz, 1 H) 2.09 - 2.40 (m, 5 H) 5.42 (s, 2 H) 6.75 (dd, *J*=8.15, 2.11 Hz, 1 H) 6.85 (d, *J*=2.38 Hz, 1 H) 7.29 (d, *J*=8.42 Hz, 1 H).

### N¹-(1-Methyl-piperidin-4-yl)-2-(trifluoromethyl)benzene-1,4-diamine

¹H NMR (600 MHz, DMSO-*d*₆) δ ppm 1.31 - 1.43 (m, 2 H) 1.84 (d, *J*=11.54 Hz, 2 H) 2.01 (t, *J*=10.90 Hz, 2 H) 2.15 (s, 3 H) 2.60 - 2.69 (m, 2 H) 3.14 - 3.25 (m, 1 H) 3.69 (d, *J*=8.06 Hz, 1 H) 4.70 (s, 2 H) 6.70 - 6.75 (m, 3 H).

### N¹-Methyl-N¹-(1-methyl-piperidin-4-yl)-2-trifluoromethyl-benzene-1,4-diamine

¹H NMR (600 MHz, DMSO-*d*₆) δ ppm 1.31 (qd, J=12.03, 3.66 Hz, 3 H) 1.61 (br. s., 3 H) 1.76 (t, J=11.26 Hz, 2 H) 2.10 (s, 3 H) 2.46 (s, 3 H) 2.55 - 2.60 (m, 1 H) 2.67 - 2.74 (m, 3 H) 5.30 (s, 2 H) 6.76 (dd, J=8.43, 2.56 Hz, 1 H) 6.79 (d, J=2.56 Hz, 1 H) 7.18 (d, J=8.61 Hz, 1 H).

### 4-(4-Ethyl-piperazin-1-yl)-3-trifluoromethyl-phenylamine

¹H NMR (600 MHz, DMSO-*d*₆) δ ppm 1.00 (t, *J*=7.23 Hz, 3 H) 2.34 (q, *J*=7.14 Hz, 3 H) 2.37 - 2.48 (m, 4 H) 2.72 (t, *J*=4.40 Hz, 5 H) 5.31 (s, 2 H) 6.76 (dd, *J*=8.52, 2.47 Hz, 1 H) 6.80 (d, *J*=2.56 Hz, 1 H) 7.23 (d, *J*=8.61 Hz, 1 H).

### 4-(1-Methyl-piperidin-4-yloxy)-3-trifluoromethyl-phenylamine

¹H NMR (600 MHz, DMSO-*d*₆) δ ppm 1.52 - 1.66 (m, 2 H) 1.79 - 1.88 (m, 2 H) 2.10 - 2.19 (m, 5 H) 2.51 - 2.57 (m, 3 H) 4.27 (br. s., 1 H) 5.00 (s, 2 H) 6.75 (dd, *J*=8.79, 2.75 Hz, 1 H) 6.80 (s, 1 H) 6.97 (d, *J*=8.79 Hz, 1 H).

### 4-(4-Ethyl-piperazin-1-ylmethyl)-3-fluoro-phenylamine

¹H NMR (600 MHz, DMSO-*d*₆) δ ppm 0.96 (t, *J*=7.14 Hz, 3 H) 5.27 (s, 2 H) 6.27 (dd, *J*=12.73, 1.92 Hz, 1 H) 6.32 (dd, *J*=8.06, 2.01 Hz, 1 H) 6.93 (t, *J*=8.43 Hz, 1 H).

### 2-(4-Ethyl-piperazin-1-ylmethyl)-5-trifluoromethyl-phenylamine

¹H NMR (600 MHz, DMSO-*d*₆) δ ppm 0.97 (t, *J*=7.23 Hz, 3 H) 2.09 - 2.48 (m, 11 H) 3.42 (s, 2 H) 5.70 (s, 2 H) 6.77 (d, *J*=7.69 Hz, 1 H) 6.91 (d, *J*=1.28 Hz, 1 H) 7.14 (d, *J*=7.69 Hz, 1 H).

### 4-Chloro-3-(4-ethyl-piperazin-1-ylmethyl)-phenylamine

¹H NMR (600 MHz, DMSO-*d*₆) δ ppm 0.98 (t, *J*=7.23 Hz, 3 H) 2.30 (q, *J*=7.14 Hz, 3 H) 2.33 - 2.48 (m, 6 H) 3.38 (s, 2 H) 5.16 (s, 2 H) 6.43 (dd, *J*=8.52, 2.84 Hz, 1 H) 6.69 (d, *J*=2.75 Hz, 1 H) 6.98 (d, *J*=8.43 Hz, 1 H).

### 3-Bromo-4-(4-methyl-piperazin-1-ylmethyl)-phenylamine

(ESI) *m*/*z* 285 [(M + H)⁺]. HRMS (ESI) calculated for C₁₂H₁₉BrN₃⁺ [(M + H)⁺] 285.1954, found 285.1955.

### 3-Cyclopropyl-4-(4-methyl-piperazin-1-ylmethyl)-phenylamine

¹H NMR (600 MHz, DMSO-*d*₆) δ ppm 0.46 - 0.57 (m, 2 H) 0.81 - 0.86 (m, 2 H) 2.13 (s, 3 H) 2.15 - 2.23 (m, 1 H) 2.44 - 2.52 (m, 8 H) 4.65 (s, 2 H) 6.14 (d, *J*=8.43 Hz, 1 H) 6.74 (dd, *J*=8.43, 2.38 Hz, 1 H) 6.80 (d, *J*=2.56 Hz, 1 H).

### Preparation 10

### 4-Amino-N-(2-dimethylamino-ethyl)-benzamide

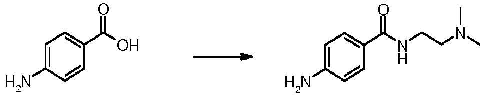

A mixture of 4-amino-benzoic acid (0.30 g, 2.19 mmol), N¹,N¹-dimethyl-ethane-1,2-diamine (0.29 mL, 2.63 mmol), TEA (0.58 mL, 4.38 mmol), EDAC (0.63 g, 3.29 mmol) and HOBT (0.45 g, 3.29 mmol) in anhydrous DMF (5 mL) was stirred at r.t. overnight. The reaction was poured in 2 N NaOH and extracted with EtOAc (4 x 15 mL). The organic phase was washed with brine, dried with Na₂SO₄ and evaporated under vacuum. The crude was purified by flash column chromatography (DCM-MeOH-NH₃ 90:10:1) to afford the title intermediate (0.18 g, 40%) as white solid.
¹H NMR (600 MHz, DMSO-*d*₆) δ ppm 2.15 (s, 6 H) 2.34 (t, *J*=6.96 Hz, 2 H) 5.55 (s, 2 H) 6.49 - 6.54 (m, 2 H) 7.53 (d, *J*=8.61 Hz, 2 H) 7.84 (t, *J*=5.40 Hz, 1 H).

According to this same methodology, but employing suitable substituted derivatives, the following intermediates were prepared:

### (5-Amino-2-bromo-phenyl)-(4-ethyl-piperazin-1-yl)-methanone

¹H NMR (600 MHz, DMSO-*d*₆) δ ppm 0.99 (t, *J*=7.14 Hz, 4 H) 2.17 - 2.46 (m, 8 H) 3.13 (t, *J*=6.04 Hz, 2 H) 3.50 - 3.67 (m, 2 H) 5.43 (s, 2 H) 6.42 (d, *J*=2.56 Hz, 1 H) 6.51 (dd, *J*=8.61, 2.75 Hz, 1 H) 7.20 (d, *J*=8.79 Hz, 1 H).

### (4-Amino-phenyl)-(4-ethyl-piperazin-1-yl)-methanone

¹H NMR (600 MHz, DMSO-*d*₆) δ ppm 0.92 - 1.08 (m, 3 H) 2.20 - 2.41 (m, 6 H) 3.47 (br. s., 4 H) 5.47 (s, 2 H) 6.49 - 6.56 (m, 2 H) 7.10 (d, *J*=8.61 Hz, 2 H).

### (4-Amino-2-trifluoromethyl-phenyl)-(4-ethyl-piperazin-1-yl)-methanone

¹H NMR (600 MHz, DMSO-*d*₆) δ ppm 0.98 (t, *J*=7.14 Hz, 3 H) 2.11 - 2.43 (m, 6 H) 3.00 - 3.20 (m, 2 H) 3.56 (d, *J*=17.77 Hz, 2 H) 5.76 (s, 2 H) 6.78 (dd, *J*=8.33, 1.92 Hz, 1 H) 6.88 (d, *J*=2.20 Hz, 1 H) 7.00 (d, *J*=8.24 Hz, 1 H).

### Preparation 11

### 3-(4-Ethyl-piperazin-1-ylmethyl)-4-trifluoromethyl-phenylamine

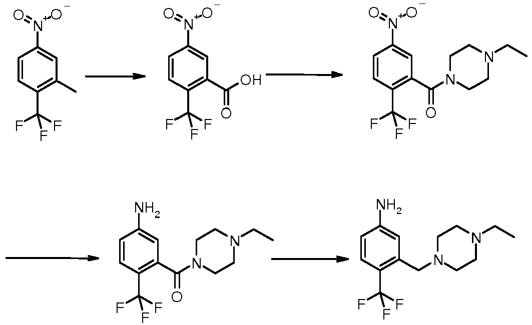

### Step 1. 5-Nitro-2-trifluoromethyl-benzoic acid

(According to the procedure described in J. Med. Chem. 1975, 18, 177). To a solution of 2-methyl-4-nitro-1-trifluoromethyl-benzene (0.50 g, 2.44 mmol) in AcOH (3.3 mL) a mixture of CrO₃ (0.61 g, 6.1 mmol) in AcOH (2.4 mL) and H₂SO₄ (0.5 mL) in water (1.5 mL) was added drop wise and heated at reflux for 1 h. After cooling to r.t., the mixture was poured in water and ice (40 mL) and treated with 2N NaOH (20 mL). The aqueous layer was extracted with toluene, taken to acidic pH with conc. HCl and extracted with DCM (4 x 15 mL). The organic layer was dried over anhydrous Na₂SO₄, filtered and concentrated under vacuum yielding the title product (0.26 g, 45%) that was used without further purification.
¹H NMR (600 MHz, DMSO-*d*₆) δ ppm 8.16 (d, J=8.61 Hz, 1 H) 8.47 - 8.52 (m, 1 H) 8.54 (d, J=2.01 Hz, 1 H) 14.24 (bs, 1 H).

### Step 2. (4-Ethyl-piperazin-1-yl)-(5-nitro-2-trifluoromethyl-phenyl)-methanone

The mixture of 5-nitro-2-trifluoromethyl-benzoic acid (0.24 g, 1.01 mmol), TBTU (0.39 g, 1.21 mmol), 1-ethyl-piperazine (0.26 mL, 2.02 mmol) and DIPEA (0.86 mL, 5.04 mmol) was let under stirring at r.t. for 2 h. After dilution with EtOAc, the organic layer was washed with a saturated solution of NaHCO₃, water and brine, dried over anhydrous Na₂SO₄, filtered and concentrated under vacuum. The product was purified by flash column chromatography (EtOAc/MeOH 95/5) and isolated as yellow oil (0.30 g, 90%).
¹H NMR (401 MHz, DMSO-*d*₆) δ ppm 0.99 (t, J=7.14 Hz, 3 H) 2.10 - 2.20 (m, 1 H) 2.25 - 2.41 (m, 1 H) 2.53 - 2.58 (m, 1 H) 3.01 - 3.12 (m, 1 H) 3.14 - 3.24 (m, 1 H) 3.49 - 3.62 (m, 1 H) 3.72 (ddd, J=13.03, 6.26, 3.42 Hz, 1 H) 8.13 (d, J=8.67 Hz, 1 H) 8.32 (d, J=2.32 Hz, 1 H) 8.42 (ddd, J=8.67, 2.32, 0.85 Hz, 1 H).

According to this same methodology, but employing suitable substituted derivatives, the following intermediates were prepared:

### (3-Amino-5-trifluoromethyl-phenyl)-(4-ethyl-piperazin-1-yl)-methanone

¹H NMR (401 MHz, DMSO-*d*₆) δ ppm 1.00 (t, J=7.20 Hz, 3 H) 2.24 - 2.45 (m, 6 H) 3.35 - 3.69 (m, 4 H) 5.78 (s, 2 H) 6.69 (s, 1 H) 6.76 (s, 1 H) 6.89 (s, 1 H).

### (4-Amino-2-chloro-phenyl)-(4-ethyl-piperazin-1-yl)-methanone

¹H NMR (600 MHz, DMSO-*d*₆) δ ppm 0.99 (t, J=7.14 Hz, 3 H) 2.13 - 2.43 (m, 6 H) 3.15 (br. s., 2 H) 3.57 (br. s., 2 H) 5.59 (s, 2 H) 6.51 (dd, J=8.24, 2.20 Hz, 1 H) 6.60 (d, J=2.20 Hz, 1 H) 6.93 (d, J=8.24 Hz, 1 H).

### (5-Amino-2-chloro-phenyl)-(4-ethyl-piperazin-1-yl)-methanone

¹H NMR (600 MHz, DMSO-*d*₆) δ ppm 1.00 (br. s., 3 H) 2.12 - 2.47 (m, 6 H) 3.02 - 3.21 (m, 2 H) 3.43 - 3.75 (m, 2 H) 5.41 (br. s., 2 H) 6.43 (br. s., 1 H) 6.58 (dd, J=8.70, 2.11 Hz, 1 H) 7.08 (d, J=8.79 Hz, 1 H).

### (4-Ethyl-piperazin-1-yl)-(2-nitro-4-trifluoromethyl-phenyl)-methanone

¹H NMR (600 MHz, DMSO-*d*₆) δ ppm 1.00 (t, J=7.14 Hz, 3 H) 2.30 (br. s., 2 H) 2.36 (q, J=7.20 Hz, 2 H) 2.46 (br. s., 2 H) 3.15 - 3.23 (m, 2 H) 3.52 - 3.77 (m, 2 H) 7.80 (d, J=7.88 Hz, 1 H) 8.24 (dd, J=7.97, 1.19 Hz, 1 H) 8.50 (s, 1 H).

### Step 3. (5-Amino-2-trifluoromethyl-phenyl)-(4-ethyl-piperazin-1-yl)-methanone

A suspension of (4-ethyl-piperazin-1-yl)-(5-nitro-2-trifluoromethyl-phenyl)-methanone (0.30 g, 0.90 mmol), HCOONH₄ (0.28 g, 4.49 mmol) and 5% Pd/C (30 mg) in MeOH (20 mL) was refluxed for 3 h. After cooling at r.t., the mixture was filtered over a pad of celite rinsing thoroughly with MeOH. The removal of the solvent afforded a residue that was dissolved in DCM and washed with water. The organic layer was dried over anhydrous Na₂SO₄, filtered and concentrated under vacuum affording the product as yellow oil (0.28 g, 84%).
¹H NMR (401 MHz, DMSO-*d*₆) δ ppm 0.99 (t, J=7.20 Hz, 3 H) 2.12 - 2.43 (m, 6 H) 2.96 - 3.20 (m, 2 H) 3.45 - 3.70 (m, 2 H) 5.97 (s, 2 H) 6.41 (d, J=2.20 Hz, 1 H) 6.64 (dd, J=8.67, 1.59 Hz, 1 H) 7.35 (d, J=8.67 Hz, 1 H).

### Step 4. 3-(4-Ethyl-piperazin-1-ylmethyl)-4-trifluoromethyl-phenylamine

A solution of (5-amino-2-trifluoromethyl-phenyl)-(4-ethyl-piperazin-1-yl)-methanone (0.22 g, 0.73 mmol) in dry THF (5 mL) was treated drop wise with 1 M BH₃·THF in THF (3.60 mL, 3.63 mmol) and let under stirring at r.t. overnight. The reaction was quenched with a solution of conc. HCl/water (1:1), let under stirring overnight and then taken to neutral pH by a portion wise addition of solid Na₂CO₃. The mixture was extracted with EtOAc (3 x 15 mL) and the organic layer was then washed with water and brine, dried over anhydrous Na₂SO₄, filtered and concentrated to reduce volume. The product was isolated by flash column chromatography (DCM-MeOH 98:2) as white solid (160 mg, 76%).
¹H NMR (401 MHz, DMSO-*d*₆) δ ppm 0.99 (t, J=7.20 Hz, 3 H) 2.28 - 2.35 (m, 2 H) 2.36 - 2.46 (m, 8 H) 3.43 (s, 2 H) 5.74 (s, 2 H) 6.48 (dd, J=8.42, 2.32 Hz, 1 H) 6.91 (d, J=2.20 Hz, 1 H) 7.27 (d, J=8.54 Hz, 1 H).

According to this same methodology, but employing suitable substituted derivatives, the following intermediates were prepared:

### 3-(4-Ethyl-piperazin-1-ylmethyl)-5-trifluoromethyl-phenylamine

¹H NMR (600 MHz, DMSO-*d*₆) δ ppm 0.97 (t, J=7.14 Hz, 3 H) 2.29 (q, J=7.20 Hz, 3 H) 2.30 - 2.46 (m, 5 H) 3.35 (s, 2 H) 5.51 (s, 2 H) 6.68 (s, 1 H) 6.71 (s, 1 H) 6.76 (s, 1 H).

### 3-Chloro-4-(4-ethyl-piperazin-1-ylmethyl)-phenylamine

¹H NMR (600 MHz, DMSO-*d*₆) δ ppm 0.97 (t, J=7.23 Hz, 3 H) 1.94 - 2.48 (m, 10 H) 3.35 (s, 2 H) 5.26 (s, 2 H) 6.47 (dd, J=8.24, 2.38 Hz, 1 H) 6.58 (d, J=2.20 Hz, 1 H) 7.02 (d, J=8.24 Hz, 1 H).

### (2-Amino-4-trifluoromethyl-phenyl)-(4-ethyl-piperazin-1-yl)-methanone

(4-Ethyl-piperazin-1-yl)-(2-nitro-4-trifluoromethyl-phenyl)-methanone (0.44 g, 1.33 mmol) in a mixture of DCM (5 mL) and EtOAc (12.5 mL) was treated with SnCl₂·2H₂O (1.50 g, 6.64 mmol) at r.t. overnight. The solvent was removed under vacuum, the residue was suspended in DCM, neutralized with a saturated solution of NaHCO₃ and extracted with DCM (2 x 15 mL). The organic layer was dried over anhydrous Na₂SO₄, filtered and concentrated under vacuum. The product was purified by flash column chromatography (DCM-MeOH 95:5) and isolated as yellow oil (0.19 g, 47%).
¹H NMR (600 MHz, DMSO-*d*₆) δ ppm 0.94 - 1.03 (m, 4 H) 2.38 (dd, J=3.66, 1.65 Hz, 9 H) 3.39 - 3.81 (m, 4 H) 5.56 (s, 2 H) 6.83 (d, J=7.88 Hz, 1 H) 7.02 (s, 1 H) 7.16 (d, J=7.69 Hz, 1 H).

### Preparation 12

### 5-Ethynyl-2-fluoro-phenylamine

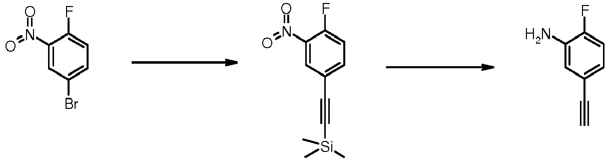

### Step 1. (4-Fluoro-3-nitro-phenylethynyl)-trimethyl-silane

To a solution of 4-bromo-1-fluoro-2-nitro-benzene (0.50 g, 2.27 mmol), CuI (10% mol, 43 mg, 0.23 mmol) and PdCl₂(PPh₃)₂ (10% mol, 0.16 g, 0.23 mmol) in degassed THF (12 mL) kept under argon in a microwave vial, trimethylsilylacetilene (0.97 mL, 6.82 mmol) and TEA (3 mL) were added. The resulting mixture was degassed three times back filling with argon each time and then heated at 120 °C for 30 min under microwave irradiation. The solvent was removed under vacuum, the residue was dissolved in DCM and washed with NH₃, water and brine. The organic layer was dried over anhydrous Na₂SO₄, filtered and concentrated under vacuum. The crude product was used without further purification in the subsequent step.
¹H NMR (401 MHz, DMSO-*d*₆) δ ppm 0.24 - 0.27 (m, 9 H) 7.61 (dd, J=11.17, 8.73 Hz, 1 H) 7.88 (ddd, J=8.70, 4.36, 2.20 Hz, 1 H) 8.17 (dd, J=7.14, 2.01 Hz, 1 H).

### Step 2. 5-Ethynyl-2-fluoro-phenylamine

(4-Fluoro-3-nitro-phenylethynyl)-trimethyl-silane (2.27 mmol from the previous step) was dissolved in EtOH (30 mL) and treated with SnCl₂·2H₂O (3.10 g, 13.60 mmol) at r.t. for 5 h. The mixture was concentrated under vacuum, dissolved with EtOAc and washed with 2 N NaOH and water. The organic layer was dried over anhydrous Na₂SO₄, filtered and concentrated under vacuum yielding a residue that was treated with TBAF·H₂O (0.76 g, 2.72 mmol) in THF (30 mL) and was let under stirring at r.t. for 30 min. The mixture was diluted with water and extracted with DCM (3 x 20 mL). The organic layer was dried over Na₂SO₄, filtered and concentrated. The product was purified by flash column chromatography (DCM/Hex 60/40) and isolated as solid (0.17 g, 55% over three steps).
¹H NMR (401 MHz, DMSO-*d*₆) δ ppm 3.97 (s, 1 H) 5.28 (s, 2 H) 6.61 (ddd, J=8.27, 4.42, 2.07 Hz, 1 H) 6.85 (dd, J=8.67, 2.08 Hz, 1 H) 6.97 (dd, J=11.53, 8.24 Hz, 1 H).

### Preparation 13

### (2,4-Dimethoxy-benzyl)-(4-trifluoromethyl-cyclohexyl)-amine (cis and trans isomers)

A mixture of 4-trifluoromethyl-cyclohexanone (60 mg, 0.36 mmol) and 2,4-dimethoxy-benzylamine (78.5 mg, 0.47 mmol) in MeOH (0.5 mL) and AcOH (two drops) was let under stirring at r.t. for 1 h, then NaCNBH₃ (11 mg, 0.18 mmol) was added. After 2 h the solvent was removed, the crude dissolved with DCM and washed with water (2 x 10 mL). The organic layer was dried over anhydrous Na₂SO₄ and taken to dryness under vacuum. The purification by column chromatography (DCM/MeOH/ 7N NH₃ in MeOH 98/1/1) afforded (2,4-dimethoxy-benzyl)-(4-trifluoromethyl-cyclohexyl)-amine as *cis* (20 mg) and *trans* (40 mg) isomers.

### (2,4-Dimethoxy-benzyl)-(4-trifluoromethyl-cyclohexyl)-amine (cis) isomer

¹H NMR (500 MHz, CHLOROFORM-d) δ ppm 1.50 - 1.59 (m, 2 H) 1.65 - 1.85 (m, 4 H) 1.92 (d, J=12.02 Hz, 2 H) 2.01 - 2.11 (m, 1 H) 2.92 (br. s., 1 H) 3.78 (s, 2 H) 3.81 (s, 3 H) 3.84 (s, 3 H) 6.33 - 6.56 (m, 2 H) 7.17 - 7.28 (m, 1 H).

### (2,4-Dimethoxy-benzyl)-(4-trifluoromethyl-cyclohexyl)-amine (trans) isomer

¹H NMR (500 MHz, CHLOROFORM-d) δ ppm 1.15 - 1.38 (m, 4 H) 1.92 - 2.03 (m, 3 H) 2.07 (d, J=12.02 Hz, 2 H) 2.47 (t, J=3.51 Hz, 3 H) 3.78 (s, 2 H) 3.80 (s, 3 H) 3.83 (s, 3 H) 6.42 - 6.47 (m, 2 H) 7.15 (d, J=8.01 Hz, 1 H).

### (S)-1-Phenyl-2-pyrrolidin-1-yl-ethylamine dihydrochloride

Synthesized according to the procedure described in EP 2 070 928.

### (S)-2-Morpholin-4-yl-1-phenyl-ethylamine dihydrochloride

Synthesized according to the procedure described in WO 2004/104007.

### Preparation 14

### 3-Bromo-N-[4-(4-ethyl-piperazin-1-ylmethyl)-3-trifluoromethyl-phenyl]-5-fluoro-benzamide

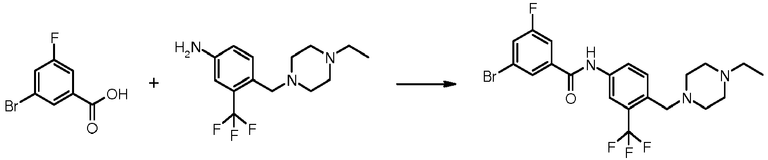

A mixture of (4-Trifluoromethyl-phenyl)-acetic acid (0.14 g, 0.63 mmol), 4-(4-ethyl-piperazin-1-ylmethyl)-3-trifluoromethyl-phenylamine (0.15 g, 0.52 mmol), TBTU (0.20 g, 0.63 mmol) and DIPEA (0.11 mL, 0.63 mmol) in anhydrous DMF (3 mL) was stirred at r.t. overnight. The reaction was poured in a saturated solution of NaHCO₃ and extracted with EtOAc (2 x 15 mL), the organic phase was washed with brine, dried with anhydrous Na₂SO₄ and evaporated under vacuum. The crude was purified by flash column chromatography (DCM/MeOH 97/3) to obtain the title compound (0.25 g, 80%) as white solid.
¹H NMR (600 MHz, DMSO-*d*₆) δ ppm 0.99 (br. s., 3 H) 2.17 - 2.48 (m, 8 H) 3.58 (s, 2 H) 7.73 (d, *J*=8.42 Hz, 1 H) 7.81 (dd, *J*=9.25, 1.37 Hz, 1 H) 7.85 (dt, *J*=7.97, 2.06 Hz, 1 H) 8.00 - 8.05 (m, 2 H) 8.17 (d, *J*=2.02 Hz, 1 H) 10.61 (s, 1 H).

According to this same methodology, but employing suitable substituted derivatives, the following intermediates were prepared:

### N-[4-(4-Ethyl-piperazin-1-ylmethyl)-3-trifluoromethyl-phenyl]-3-iodo-benzamide

(ESI) *m*/*z* 518 [(M + H)⁺]. HRMS (ESI) calculated for C₂₁H₂₄F₃IN₃O⁺ [(M + H)⁺] 518.3265, found 518.3278.

### N-[4-(4-Ethyl-piperazin-1-ylmethyl)-3-trifluoromethyl-phenyl]-3-iodo-4-methyl-benzamide

¹H NMR (600 MHz, DMSO-*d*₆) δ ppm 0.98 (t, *J*=7.14 Hz, 3 H) 2.29 - 2.35 (m, 3 H) 2.36 - 2.43 (m, 5 H) 2.45 (s, 3 H) 3.56 (s, 2 H) 7.47 - 7.52 (m, 1 H) 7.71 (d, *J*=8.54 Hz, 1 H) 7.91 (dd, *J*=7.93, 1.83 Hz, 1 H) 8.03 (dd, *J*=8.48, 2.01 Hz, 1 H) 8.17 (d, *J*=2.20 Hz, 1 H) 8.42 (d, *J*=1.83 Hz, 1 H) 10.47 (s, 1 H).

### 5-Bromo-thiophene-2-carboxylic acid [4-(4-ethyl-piperazin-1-ylmethyl)-3-trifluoromethyl-phenyl]-amide

¹H NMR (600 MHz, DMSO-*d*₆) δ ppm 0.98 (t, *J*=7.14 Hz, 3 H) 2.29 - 2.31 (m, 2 H) 2.39 (d, *J*=1.47 Hz, 7 H) 3.56 (s, 2 H) 7.39 (d, *J*=4.03 Hz, 1 H) 7.71 (d, *J*=8.43 Hz, 1 H) 7.86 (d, *J*=4.03 Hz, 1 H) 7.95 (dd, *J*=8.61, 1.65 Hz, 1 H) 8.10 (d, *J*=2.01 Hz, 1 H) 10.52 (s, 1 H).

### 2-Bromo-thiazole-5-carboxylic acid [4-(4-ethyl-piperazin-1-ylmethyl)-3-trifluoromethyl-phenyl]-amide

¹H NMR (600 MHz, DMSO-*d*₆) δ ppm 0.98 (t, *J*=7.23 Hz, 3 H) 2.30 (q, *J*=7.27 Hz, 3 H) 2.33 - 2.47 (m, 6 H) 3.56 (s, 2 H) 7.73 (d, *J*=8.61 Hz, 1 H) 7.91 - 7.97 (m, 1 H) 8.08 (d, *J*=2.01 Hz, 1 H) 8.45 (s, 1 H) 10.73 (s, 1 H).

### 5-Bromo-N-[4-(4-ethyl-piperazin-1-ylmethyl)-3-trifluoromethyl-phenyl]-nicotinamide

¹H NMR (600 MHz, DMSO-*d*₆) δ ppm 0.98 (t, *J*=7.14 Hz, 3 H) 2.31 (q, *J*=7.14 Hz, 3 H) 2.39 (dd, *J*=3.57, 1.74 Hz, 6 H) 3.57 (s, 2 H) 7.74 (d, *J*=8.61 Hz, 1 H) 8.00 (dd, *J*=8.42, 1.83 Hz, 1 H) 8.17 (d, *J*=2.01 Hz, 1 H) 8.55 - 8.56 (m, 1 H) 8.93 (d, *J*=2.01 Hz, 1 H) 9.07 (d, *J*=1.65 Hz, 1 H) 10.73 (s, 1 H).

### 3-Bromo-N-[4-(4-ethyl-piperazin-1-ylmethyl)-3-trifluoromethyl-phenyl]-2-fluoro-benzamide

(ESI) *m*/*z* 489 [(M + H)⁺]. HRMS (ESI) calculated for C₂₁H₂₃BrF₄N₃O⁺ [(M + H)⁺] 489.3165, found 489.3166.

### 2-(3-Ethynyl-phenoxy)-N-phenyl-acetamide

¹H NMR (600 MHz, DMSO-*d*₆) δ ppm 4.18 (s, 1 H) 4.72 (s, 2 H) 7.02 - 7.12 (m, 4 H) 7.28 - 7.36 (m, 3 H) 7.60 - 7.66 (m, 2 H) 10.05 (s, 1 H).

### N-(3-Ethynyl-phenyl)-3-phenyl-propionamide

¹H NMR (600 MHz, DMSO-*d*₆) δ ppm 2.63 (t, *J*=7.75 Hz, 2 H) 2.87 - 2.95 (m, 2 H) 4.15 (s, 1 H) 7.13 (dt, *J*=7.66, 1.24 Hz, 1 H) 7.16 - 7.21 (m, 1 H) 7.22 - 7.33 (m, 5 H) 7.53 (dd, *J*=8.24, 1.04 Hz, 1 H) 7.75 - 7.79 (m, 1 H) 9.99 (s, 1 H).

### (1S,2S)-2-Phenyl-cyclopropanecarboxylic acid (3-ethynyl-phenyl)-amide

¹H NMR (600 MHz, DMSO-*d*₆) δ ppm 1.36 - 1.53 (m, 2 H) 2.01 - 2.09 (m, 1 H) 2.34 - 2.42 (m, 1 H) 4.15 (s, 1 H) 7.14 (dt, *J*=7.69, 1.22 Hz, 1 H) 7.17 - 7.23 (m, 3 H) 7.27 - 7.35 (m, 3 H) 7.53 - 7.57 (m, 1 H) 7.79 (t, *J*=1.71 Hz, 1 H) 10.32 (s, 1 H).

### N-(3-lodo-benzyl)-benzamide

¹H NMR (600 MHz, DMSO-*d*₆) δ ppm 4.44 (d, *J*=5.86 Hz, 2 H) 7.14 (t, *J*=7.69 Hz, 1 H) 7.34 (d, *J*=7.33 Hz, 1 H) 7.45 - 7.50 (m, 2 H) 7.53 - 7.57 (m, 1 H) 7.61 (d, *J*=7.88 Hz, 1 H) 7.69 (s, 1 H) 7.86 - 7.90 (m, 2 H) 9.05 (t, *J*=5.59 Hz, 1 H).

### 3-(3-lodo-phenyl)-N-phenyl-propionamide

¹H NMR (600 MHz, DMSO-*d*₆) δ ppm 2.61 (t, *J*=7.69 Hz, 2 H) 2.84 - 2.89 (m, 2 H) 7.02 (s, 1 H) 7.09 (s, 1 H) 7.25 - 7.31 (m, 3 H) 7.55 (d, *J*=8.06 Hz, 3 H) 7.65 (s, 1 H) 9.87 (s, 1 H).

### N-(3-Ethynyl-phenyl)-2-(3-trifluoromethyl-phenyl)-acetamide

¹H NMR (600 MHz, DMSO-*d*₆) δ ppm 3.79 (s, 2 H) 4.15 (s, 1 H) 7.15 (d, *J*=7.51 Hz, 1 H) 7.32 (t, *J*=7.88 Hz, 1 H) 7.52 - 7.66 (m, 4 H) 7.69 (s, 1 H) 7.78 (s, 1 H) 10.32 (s, 1 H).

### N-(3-Ethynyl-phenyl)-3-(4-trifluoromethyl-phenyl)-propionamide

¹H NMR (600 MHz, DMSO-*d*₆) δ ppm 2.65 - 2.70 (m, 2 H) 3.00 (t, *J*=7.51 Hz, 2 H) 4.14 (s, 1 H) 7.13 (d, *J*=7.69 Hz, 1 H) 7.30 (t, *J*=7.97 Hz, 1 H) 7.47 - 7.49 (m, 2 H) 7.52 (d, *J*=9.16 Hz, 1 H) 7.64 (d, *J*=8.06 Hz, 2 H) 7.76 (s, 1 H) 10.03 (s, 1 H).

### N-(3-Ethynyl-phenyl)-3-(3-trifluoromethyl-phenyl)-propionamide

¹H NMR (600 MHz, DMSO-*d*₆) δ ppm 2.67 (t, *J*=7.69 Hz, 2 H) 3.01 (t, *J*=7.60 Hz, 2 H) 4.14 (s, 1 H) 7.13 (d, *J*=7.51 Hz, 1 H) 7.30 (t, *J*=7.88 Hz, 1 H) 7.49 - 7.59 (m, 4 H) 7.61 (s, 1 H) 7.75 (s, 1 H) 10.01 (s, 1 H).

### 3-Ethynyl-N-(3-trifluoromethyl-benzyl)-benzamide

¹H NMR (600 MHz, DMSO-*d*₆) δ ppm 4.27 (s, 1 H) 4.56 (d, *J*=5.86 Hz, 2 H) 7.51 (t, *J*=7.88 Hz, 1 H) 7.56 - 7.59 (m, 1 H) 7.60 - 7.69 (m, 4 H) 7.91 (dt, *J*=7.97, 1.42 Hz, 1 H) 7.99 (t, *J*=1.37 Hz, 1 H) 9.22 (t, *J*=5.77 Hz, 1 H).

### N-[4-(4-Ethyl-piperazin-1-ylmethyl)-3-trifluoromethyl-phenyl]-2-(3-iodo-phenyl)-acetamide

¹H NMR (600 MHz, DMSO-*d*₆) δ ppm 0.97 (t, *J*=7.14 Hz, 3 H) 2.27 - 2.32 (m, 2 H) 2.32 - 2.46 (m, 4 H) 3.52 (s, 2 H) 3.64 (s, 2 H) 7.14 (t, *J*=7.78 Hz, 1 H) 7.34 (d, *J*=7.69 Hz, 1 H) 7.64 (dd, *J*=16.48, 8.24 Hz, 2 H) 7.72 (s, 1 H) 7.76 (d, *J*=8.43 Hz, 1 H) 8.03 (d, *J*=1.83 Hz, 1 H) 10.44 (s, 1 H).

### N-[4-(4-Ethyl-piperazin-1-ylmethyl)-3-trifluoromethyl-phenyl]-2-(4-iodo-phenyl)-acetamide

¹H NMR (600 MHz, DMSO-*d*₆) δ ppm 0.97 (t, *J*=7.14 Hz, 3 H) 2.18 - 2.46 (m, 10 H) 3.52 (s, 2 H) 3.62 (s, 2 H) 7.14 (d, *J*=8.43 Hz, 2 H) 7.65 (d, *J*=8.61 Hz, 1 H) 7.66 - 7.70 (m, 2 H) 7.75 (dd, *J*=8.52, 1.92 Hz, 1 H) 8.02 (d, *J*=2.02 Hz, 1 H) 10.44 (s, 1 H).

### N-[3-(4-Ethyl-piperazin-1-ylmethyl)-4-trifluoromethyl-phenyl]-2-(3-iodo-phenyl)-acetamide

¹H NMR (600 MHz, DMSO-*d*₆) δ ppm 0.99 (t, *J*=7.14 Hz, 3 H) 2.23 - 2.48 (m, 9 H) 3.55 (s, 2 H) 3.66 (s, 2 H) 7.15 (t, *J*=7.78 Hz, 1 H) 7.48 (d, *J*=7.69 Hz, 1 H) 7.61 (dd, *J*=16.48, 8.24 Hz, 2 H) 7.70 (s, 1 H) 7.78 (d, *J*=8.43 Hz, 1 H) 8.10 (d, *J*=1.83 Hz, 1 H) 10.49 (s, 1 H).

### N-[4-(4-Ethyl-piperazin-1-ylmethyl)-3-fluoro-phenyl]-2-(3-iodo-phenyl)-acetamide

¹H NMR (600 MHz, DMSO-*d*₆) δ ppm 0.96 (t, *J*=7.14 Hz, 3 H) 2.11 - 2.47 (m, 9 H) 3.43 (s, 2 H) 3.62 (s, 2 H) 7.14 (t, *J*=7.69 Hz, 1 H) 7.22 - 7.26 (m, 1 H) 7.27 - 7.31 (m, 1 H) 7.34 (d, *J*=7.88 Hz, 1 H) 7.54 (dd, *J*=12.45, 1.47 Hz, 1 H) 7.62 (d, *J*=7.88 Hz, 1 H) 7.71 (s, 1 H) 10.33 (s, 1 H).

### N-(2-Dimethylamino-ethyl)-4-[2-(3-iodo-phenyl)-acetylamino]-benzamide

¹H NMR (600 MHz, DMSO-*d*₆) δ ppm 2.17 (s, 6 H) 2.35 - 2.43 (m, 2 H) 3.65 (s, 2 H) 7.14 (t, *J*=7.69 Hz, 1 H) 7.35 (d, *J*=7.69 Hz, 1 H) 7.62 (s, 1 H) 7.64 (d, *J*=8.79 Hz, 2 H) 7.78 (d, *J*=8.79 Hz, 2 H) 8.23 (t, *J*=5.59 Hz, 1 H) 10.36 (s, 1 H).

### 2-(5-Bromo-pyridin-3-yl)-N-[4-(4-ethyl-piperazin-1-ylmethyl)-3-trifluoromethyl-phenyl]-acetamide

¹H NMR (600 MHz, DMSO-*d*₆) δ ppm 0.97 (t, *J*=7.14 Hz, 3 H) 2.30 (q, *J*=7.14 Hz, 3 H) 2.33 - 2.46 (m, 6 H) 3.53 (s, 2 H) 3.76 (s, 2 H) 7.66 (d, *J*=8.43 Hz, 1 H) 7.75 (dd, *J*=8.52, 1.74 Hz, 1 H) 7.99 - 8.05 (m, 2 H) 8.50 (d, *J*=1.65 Hz, 1 H) 8.61 (d, *J*=2.20 Hz, 1 H) 10.51 (s, 1 H).

### N-[2-(4-Ethyl-piperazin-1-ylmethyl)-5-trifluoromethyl-phenyl]-2-(3-iodo-phenyl)-acetamide

¹H NMR (600 MHz, DMSO-*d*₆) δ ppm 0.98 (t, *J*=7.14 Hz, 3 H) 1.95 - 2.46 (m, 9 H) 3.61 (s, 2 H) 3.72 (s, 2 H) 7.17 (t, *J*=7.78 Hz, 1 H) 7.38 (t, *J*=8.70 Hz, 2 H) 7.45 (d, *J*=7.69 Hz, 1 H) 7.66 (d, *J*=7.88 Hz, 1 H) 7.74 (s, 1 H) 8.40 (s, 1 H) 10.84 (br. s., 1 H).

### N-[4-Chloro-3-(4-ethyl-piperazine-1-carbonyl)-phenyl]-2-(3-iodo-phenyl)-acetamide

¹H NMR (600 MHz, DMSO-*d*₆) δ ppm 0.99 (t, *J*=7.14 Hz, 3 H) 2.33 - 2.36 (m, 3 H) 3.12 (t, *J*=5.04 Hz, 2 H) 3.54 - 3.70 (m, 4 H) 7.14 (t, *J*=7.69 Hz, 1 H) 7.34 (d, *J*=7.88 Hz, 1 H) 7.44 (d, *J*=8.79 Hz, 1 H) 7.56 (dd, *J*=8.79, 2.56 Hz, 1 H) 7.61 - 7.64 (m, 2 H) 7.71 (s, 1 H) 10.40 (s, 1 H).

### 2-(4-Bromo-thiophen-2-yl)-N-[4-(4-ethyl-piperazin-1-ylmethyl)-3-trifluoromethyl-phenyl]-acetamide

¹H NMR (600 MHz, DMSO-*d*₆) δ ppm 0.97 (t, *J*=7.14 Hz, 3 H) 2.16 - 2.43 (m, 9 H) 3.53 (s, 2 H) 3.91 (s, 2 H) 7.00 (d, *J*=1.28 Hz, 1 H) 7.53 (d, *J*=1.47 Hz, 1 H) 7.67 (d, *J*=8.43 Hz, 1 H) 7.75 (dd, *J*=8.61, 1.83 Hz, 1 H) 8.02 (d, *J*=1.83 Hz, 1 H) 10.50 (s, 1 H).

### 2-(2-Bromo-pyridin-4-yl)-N-[4-(4-ethyl-piperazin-1-ylmethyl)-3-trifluoromethyl-phenyl]-acetamide

¹H NMR (600 MHz, DMSO-*d*₆) δ ppm 0.97 (t, *J*=7.14 Hz, 3 H) 2.17 - 2.45 (m, 10 H) 3.53 (s, 2 H) 3.76 (s, 2 H) 7.40 (dd, *J*=5.04, 1.19 Hz, 1 H) 7.62 (s, 1 H) 7.67 (d, *J*=8.43 Hz, 1 H) 7.75 (dd, *J*=8.52, 1.74 Hz, 1 H) 8.02 (d, *J*=2.01 Hz, 1 H) 8.33 (d, *J*=5.13 Hz, 1 H) 10.52 (s, 1 H).

### 2-(5-Bromo-thiophen-2-yl)-N-[4-(4-ethyl-piperazin-1-ylmethyl)-3-trifluoromethyl-phenyl]-acetamide

¹H NMR (600 MHz, DMSO-*d*₆) δ ppm 0.97 (t, *J*=7.14 Hz, 3 H) 2.30 (q, *J*=7.14 Hz, 2 H) 2.33 - 2.45 (m, 8 H) 3.53 (s, 1 H) 3.89 (s, 1 H) 6.83 (d, *J*=3.85 Hz, 1 H) 7.07 (d, *J*=3.66 Hz, 1 H) 7.67 (d, *J*=8.61 Hz, 1 H) 7.75 (dd, *J*=8.52, 1.74 Hz, 1 H) 8.02 (d, *J*=2.01 Hz, 1 H) 10.50 (s, 1 H).

### N-[4-Chloro-3-(4-ethyl-piperazin-1-ylmethyl)-phenyl]-2-(3-iodo-phenyl)-acetamide

¹H NMR (600 MHz, DMSO-*d*₆) δ ppm 0.98 (t, *J*=7.14 Hz, 3 H) 2.18 - 2.49 (m, 10 H) 3.46 - 3.51 (m, 2 H) 3.62 (s, 2 H) 7.14 (t, *J*=7.78 Hz, 1 H) 7.28 - 7.36 (m, 2 H) 7.58 (dd, *J*=8.52, 2.11 Hz, 1 H) 7.62 (d, *J*=7.69 Hz, 1 H) 7.67 (br. s., 1 H) 7.71 (s, 1 H) 10.27 (s, 1 H).

### N-[4-Bromo-3-(4-ethyl-piperazine-1-carbonyl)-phenyl]-2-(3-iodo-phenyl)-acetamide

¹H NMR (600 MHz, DMSO-*d*₆) δ ppm 0.96 - 1.02 (m, 3 H) 2.19 - 2.47 (m, 6 H) 3.11 (t, *J*=5.04 Hz, 2 H) 3.53 - 3.70 (m, 4 H) 7.14 (t, *J*=7.78 Hz, 1 H) 7.34 (d, *J*=7.69 Hz, 1 H) 7.47 - 7.50 (m, 1 H) 7.57 - 7.61 (m, 2 H) 7.62 (d, *J*=7.88 Hz, 1 H) 7.71 (s, 1 H) 10.39 (s, 1 H).

### 2-(3-lodo-phenyl)-N-[4-(4-methyl-[1,4]diazepan-1-ylmethyl)-3-trifluoromethyl-phenyl]-acetamide

¹H NMR (600 MHz, DMSO-*d*₆) δ ppm 1.71 (quin, *J*=5.95 Hz, 2 H) 2.25 (s, 3 H) 2.55 - 2.65 (m, 7 H) 3.64 (s, 2 H) 3.66 - 3.68 (m, 2 H) 7.14 (t, *J*=7.78 Hz, 1 H) 7.35 (d, *J*=7.88 Hz, 1 H) 7.63 (d, *J*=7.88 Hz, 1 H) 7.68 - 7.74 (m, 2 H) 7.75 - 7.78 (m, 1 H) 8.02 (d, *J*=1.83 Hz, 1 H) 10.44 (s, 1 H).

### N-[4-((S)-3-Dimethylamino-pyrrolidin-1-ylmethyl)-3-trifluoromethyl-phenyl]-2-(3-iodo-phenyl)-acetamide

¹H NMR (600 MHz, DMSO-*d*₆) δ ppm 1.56 - 1.67 (m, 1 H) 1.84 (dt, *J*=8.38, 5.24 Hz, 1 H) 2.05 - 2.09 (m, 6 H) 2.31 (dd, *J*=8.70, 6.69 Hz, 1 H) 2.46 (td, *J*=8.75, 5.77 Hz, 1 H) 2.53 - 2.58 (m, 1 H) 2.60 - 2.63 (m, 1 H) 2.69 - 2.75 (m, 1 H) 3.58 - 3.63 (m, 1 H) 3.64 (s, 2 H) 3.65 - 3.68 (m, 1 H) 7.14 (t, *J*=7.78 Hz, 1 H) 7.35 (d, *J*=7.88 Hz, 1 H) 7.59 - 7.66 (m, 2 H) 7.72 (s, 1 H) 7.76 (dd, *J*=8.61, 1.83 Hz, 1 H) 8.02 (d, *J*=2.01 Hz, 1 H) 10.44 (s, 1 H).

### N-[4-(4-Ethyl-piperazine-1-carbonyl)-phenyl]-2-(3-iodo-phenyl)-acetamide

¹H NMR (600 MHz, DMSO-*d*₆) δ ppm 0.99 (t, *J*=7.14 Hz, 3 H) 2.18 - 2.46 (m, 6 H) 3.34 - 3.62 (m, 4 H) 3.64 (s, 2 H) 7.14 (t, *J*=7.78 Hz, 1 H) 7.30 - 7.37 (m, 3 H) 7.59 - 7.66 (m, 3 H) 7.73 (s, 1 H) 10.33 (s, 1 H).

### N-[3-Cyclopropyl-4-(4-ethyl-piperazin-1-ylmethyl)-phenyl]-2-(3-iodo-phenyl)-acetamide

¹H NMR (600 MHz, DMSO-*d*₆) δ ppm 0.49 - 0.56 (m, 2 H) 0.86 - 0.94 (m, 2 H) 2.12 - 2.19 (m, 4 H) 2.19 - 2.47 (m, 8 H) 3.51 (s, 2 H) 3.56 - 3.59 (m, 2 H) 7.07 - 7.20 (m, 3 H) 7.30 - 7.37 (m, 2 H) 7.61 (d, *J*=8.06 Hz, 1 H) 7.70 (s, 1 H) 10.03 (s, 1 H).

### 2-(3-Bromo-4-fluoro-phenyl)-N-[4-(4-ethyl-piperazin-1-ylmethyl)-3-trifluoromethyl-phenyl]-acetamide

¹H NMR (600 MHz, DMSO-*d*₆) δ ppm 0.97 (t, *J*=7.14 Hz, 3 H) 2.22 - 2.48 (m, 10 H) 3.52 (s, 2 H) 3.69 (s, 2 H) 7.28 - 7.39 (m, 2 H) 7.63 - 7.68 (m, 2 H) 7.74 - 7.77 (m, 1 H) 8.03 (d, *J*=1.83 Hz, 1 H) 10.44 (s, 1 H).

### 2-(3-Bromo-2-fluoro-phenyl)-N-[4-(4-ethyl-piperazin-1-ylmethyl)-3-trifluoromethyl-phenyl]-acetamide

¹H NMR (600 MHz, DMSO-*d*₆) δ ppm 0.97 (t, *J*=7.14 Hz, 3 H) 2.20 - 2.47 (m, 9 H) 3.53 (s, 2 H) 3.81 (s, 2 H) 7.14 (t, *J*=7.78 Hz, 1 H) 7.38 - 7.42 (m, 1 H) 7.60 - 7.63 (m, 1 H) 7.66 (d, *J*=8.42 Hz, 1 H) 7.75 (dd, *J*=8.52, 1.74 Hz, 1 H) 8.04 (d, *J*=1.83 Hz, 1 H) 10.52 (s, 1 H).

### N-[4-((R)-3-Dimethylamino-pyrrolidin-1-ylmethyl)-3-trifluoromethyl-phenyl]-2-(3-iodo-phenyl)-acetamide

¹H NMR (600 MHz, DMSO-*d*₆) δ ppm 1.56 - 1.70 (m, 1 H) 1.81 - 1.91 (m, 1 H) 2.08 (s, 6 H) 2.28 - 2.35 (m, 1 H) 2.44 - 2.47 (m, 1 H) 2.53 - 2.58 (m, 1 H) 2.60 - 2.64 (m, 1 H) 2.73 (br. s., 1 H) 3.57 - 3.69 (m, 2 H) 3.64 (s, 2 H) 7.14 (t, *J*=7.69 Hz, 1 H) 7.35 (d, *J*=7.88 Hz, 1 H) 7.61 - 7.66 (m, 2 H) 7.72 (s, 1 H) 7.76 (dd, *J*=8.43, 1.83 Hz, 1 H) 8.02 (d, *J*=2.01 Hz, 1 H) 10.44 (s, 1 H).

### 2-(3-lodo-phenyl)-N-[4-(4-isopropyl-piperazin-1-ylmethyl)-3-trifluoromethyl-phenyl]-acetamide

¹H NMR (600 MHz, DMSO-*d*₆) δ ppm 0.95 (d, *J*=6.23 Hz, 6 H) 2.25 - 2.48 (m, 8 H) 2.56 - 2.62 (m, 1 H) 3.51 (s, 2 H) 3.64 (s, 2 H) 7.14 (t, *J*=7.78 Hz, 1 H) 7.35 (d, *J*=7.88 Hz, 1 H) 7.63 (d, *J*=7.88 Hz, 1 H) 7.66 (d, *J*=8.61 Hz, 1 H) 7.72 (s, 1 H) 7.74 - 7.78 (m, 1 H) 8.03 (d, *J*=2.01 Hz, 1 H) 10.44 (s, 1 H).

### 2-(3-lodo-phenyl)-N-(4-piperidin-1-ylmethyl-3-trifluoromethyl-phenyl)-acetamide

¹H NMR (600 MHz, DMSO-*d*₆) δ ppm 1.39 (br. s., 2 H) 1.49 (quin, *J*=5.49 Hz, 4 H) 2.31 (br. s., 4 H) 3.48 (s, 2 H) 3.64 (s, 2 H) 7.14 (t, *J*=7.69 Hz, 1 H) 7.35 (d, *J*=7.69 Hz, 1 H) 7.63 (d, *J*=7.88 Hz, 1 H) 7.67 (d, *J*=8.61 Hz, 1 H) 7.72 (s, 1 H) 7.75 - 7.77 (m, 1 H) 8.02 (d, *J*=2.01 Hz, 1 H) 10.43 (s, 1 H).

### 2-(3-Iodo-phenyl)-N-(4-morpholin-4-ylmethyl-3-trifluoromethyl-phenyl)-acetamide

¹H NMR (600 MHz, DMSO-*d*₆) δ ppm 2.35 (br. s., 4 H) 3.54 (s, 2 H) 3.57 (t, *J*=4.40 Hz, 4 H) 3.64 (s, 2 H) 7.14 (t, *J*=7.78 Hz, 1 H) 7.34 (d, *J*=7.88 Hz, 1 H) 7.63 (d, *J*=8.43 Hz, 1 H) 7.68 (d, *J*=8.61 Hz, 1 H) 7.72 (s, 1 H) 7.77 (dd, *J*=8.52, 1.74 Hz, 1 H) 8.04 (d, *J*=2.01 Hz, 1 H) 10.45 (s, 1 H).

### 2-(3-lodo-phenyl)-N-[4-(4-methyl-piperazin-1-ylmethyl)-3-trifluoromethyl-phenyl]-acetamide

¹H NMR (600 MHz, DMSO-*d*₆) δ ppm 2.15 (s, 3 H) 2.19 - 2.47 (m, 8 H) 3.52 (s, 2 H) 3.64 (s, 2 H) 7.14 (t, *J*=7.78 Hz, 1 H) 7.34 (d, *J*=7.69 Hz, 1 H) 7.64 (dd, *J*=13.55, 8.24 Hz, 1 H) 7.72 (s, 1 H) 7.76 (dd, *J*=8.42, 1.47 Hz, 1 H) 8.03 (d, *J*=1.83 Hz, 1 H) 10.44 (s, 1 H).

### 2-(3-lodo-phenyl)-N-[4-(3-pyrrolidin-1-yl-azetidin-1-ylmethyl)-3-trifluoromethyl-phenyl]-acetamide

¹H NMR (600 MHz, DMSO-*d*₆) δ ppm 1.67 (br. s., 4 H) 2.31 - 2.38 (m, 4 H) 2.92 (t, *J*=6.23 Hz, 2 H) 3.04 (br. s., 1 H) 3.36 (t, *J*=6.87 Hz, 2 H) 3.64 (s, 2 H) 3.65 (s, 2 H) 7.14 (t, *J*=7.78 Hz, 1 H) 7.34 (d, *J*=7.51 Hz, 1 H) 7.58 (d, *J*=8.61 Hz, 1 H) 7.63 (d, *J*=7.69 Hz, 1 H) 7.72 (s, 1 H) 7.74 - 7.77 (m, 1 H) 8.01 (d, *J*=2.01 Hz, 1 H) 10.43 (s, 1 H).

### 2-(3-lodo-phenyl)-N-[4-(4-propyl-piperazin-1-ylmethyl)-3-trifluoromethyl-phenyl]-acetamide

¹H NMR (600 MHz, DMSO-*d*₆) δ ppm 0.83 (t, *J*=7.42 Hz, 3 H) 1.37 - 1.46 (m, 2 H) 2.21 (t, *J*=7.14 Hz, 2 H) 2.26 - 2.47 (m, 7 H) 3.52 (s, 2 H) 3.64 (s, 2 H) 7.14 (t, *J*=7.78 Hz, 1 H) 7.34 (d, *J*=7.51 Hz, 1 H) 7.63 (d, *J*=7.88 Hz, 1 H) 7.66 (s, 1 H) 7.72 (s, 1 H) 7.76 (dd, *J*=8.43, 1.83 Hz, 1 H) 8.03 (d, *J*=2.01 Hz, 1 H) 10.44 (s, 1 H).

### N-[4-(4-Ethyl-piperazine-1-carbonyl)-3-trifluoromethyl-phenyl]-2-(3-iodo-phenyl)-acetamide

¹H NMR (600 MHz, DMSO-*d*₆) δ ppm 7.15 (t, *J*=7.78 Hz, 1 H) 7.36 (d, *J*=7.51 Hz, 1 H) 7.42 - 7.57 (m, 1 H) 7.64 (d, *J*=7.88 Hz, 1 H) 7.74 (s, 1 H) 7.88 (d, *J*=2.38 Hz, 1 H) 8.13 (br. s., 1 H) 10.37 (br. s., 1 H) 10.73 (br. s., 1 H).

### 2-(3-Iodo-phenyl)-N-(4-{[methyl-(1-methyl-piperidin-4-yl)-amino]-methyl}-3-trifluoromethyl-phenyl)-acetamide

¹H NMR (600 MHz, DMSO-*d*₆) δ ppm 1.48 (qd, *J*=11.91, 3.48 Hz, 2 H) 1.64 (d, *J*=11.90 Hz, 2 H) 1.81 (br. s., 2 H) 2.06 (s, 3 H) 2.11 (s, 3 H) 2.77 (d, *J*=10.44 Hz, 2 H) 3.59 - 3.60 (m, 2 H) 3.60 - 3.62 (m, 2 H) 7.11 (t, *J*=7.69 Hz, 1 H) 7.32 (d, *J*=7.69 Hz, 1 H) 7.60 (d, *J*=7.88 Hz, 1 H) 7.63 - 7.68 (m, 1 H) 7.69 (s, 1 H) 7.71 - 7.73 (m, 1 H) 7.99 (d, *J*=2.01 Hz, 1 H) 10.40 (s, 1 H).

### N-[4-(4-Dimethylamino-piperidin-1-ylmethyl)-3-trifluoromethyl-phenyl]-2-(3-iodo-phenyl)-acetamide

¹H NMR (600 MHz, DMSO-*d*₆) δ ppm 1.32 - 1.42 (m, 2 H) 1.69 (d, *J*=11.72 Hz, 2 H) 1.95 (t, *J*=10.81 Hz, 2 H) 2.00 - 2.10 (m, 1 H) 2.16 (s, 7 H) 2.78 (d, *J*=11.72 Hz, 2 H) 3.50 (s, 2 H) 3.64 (s, 2 H) 7.14 (t, *J*=7.78 Hz, 1 H) 7.35 (d, *J*=7.69 Hz, 1 H) 7.63 (d, *J*=7.88 Hz, 1 H) 7.67 (d, *J*=8.43 Hz, 1 H) 7.72 (s, 1 H) 7.76 (dd, *J*=8.43, 1.83 Hz, 1 H) 8.03 (d, *J*=2.01 Hz, 1 H) 10.44 (s, 1 H).

### N-[3-Bromo-4-(4-ethyl-piperazin-1-ylmethyl)-phenyl]-2-(3-iodo-phenyl)-acetamide

(ESI) *m*/*z* 543 [(M + H)⁺]. HRMS (ESI) calculated for C₂₁H₂₆BrIN₃O⁺ [(M + H)⁺] 543.2512, found 543.2519.

### Acetic acid 2-(4-{4-[2-(3-iodo-phenyl)-acetylamino]-2-trifluoromethyl-benzyl}-piperazin-1-yl)-ethyl ester

¹H NMR (600 MHz, DMSO-*d*₆) δ ppm 1.99 (s, 3 H) 2.27 - 2.47 (m, 8 H) 2.51 - 2.53 (m, 2 H) 3.52 (s, 2 H) 3.64 (s, 2 H) 4.08 (t, *J*=5.95 Hz, 2 H) 7.14 (t, *J*=7.69 Hz, 1 H) 7.34 (d, *J*=7.69 Hz, 1 H) 7.63 (d, *J*=7.88 Hz, 1 H) 7.65 (d, *J*=8.61 Hz, 1 H) 7.72 (s, 1 H) 7.76 (dd, *J*=8.61, 1.83 Hz, 1 H) 8.03 (d, *J*=2.01 Hz, 1 H) 10.44 (s, 1 H).

### N-[4-(4-Cyclopropylmethyl-piperazin-1-ylmethyl)-3-trifluoromethyl-phenyl]-2-(3-iodo-phenyl)-acetamide

¹H NMR (600 MHz, DMSO-*d*₆) δ ppm 0.06 (d, *J*=3.30 Hz, 2 H) 0.45 (br. s., 2 H) 0.81 (br. s., 1 H) 2.17 (d, *J*=5.86 Hz, 2 H) 2.26 - 2.48 (m, 7 H) 3.53 (br. s., 2 H) 3.64 (s, 2 H) 7.12 - 7.16 (m, 1 H) 7.34 (d, *J*=7.33 Hz, 1 H) 7.64 (dd, *J*=16.94, 8.15 Hz, 2 H) 7.72 (s, 1 H) 7.76 (d, *J*=8.61 Hz, 1 H) 8.03 (d, *J*=1.83 Hz, 1 H) 10.45 (s, 1 H).

### 2-(3-Iodo-phenyl)-N-[4-(1-methyl-piperidin-4-yloxy)-3-trifluoromethyl-phenyl]-acetamide

¹H NMR (600 MHz, DMSO-*d*₆) δ ppm 1.58 - 1.71 (m, 2 H) 1.88 (td, *J*=8.01, 3.57 Hz, 2 H) 2.15 (s, 3 H) 2.22 (br. s., 2 H) 3.60 (s, 2 H) 4.53 (br. s., 1 H) 7.14 (t, *J*=7.78 Hz, 1 H) 7.26 (d, *J*=9.16 Hz, 1 H) 7.34 (d, *J*=7.69 Hz, 1 H) 7.62 (d, *J*=7.88 Hz, 1 H) 7.69 - 7.72 (m, 2 H) 7.91 (d, *J*=2.56 Hz, 1 H) 10.26 (s, 1 H).

### N-[4-(4-Ethyl-piperazin-1-yl)-3-trifluoromethyl-phenyl]-2-(3-iodo-phenyl)-acetamide

¹H NMR (600 MHz, DMSO-*d*₆) δ ppm 1.02 (t, *J*=6.87 Hz, 3 H) 2.29 - 2.48 (m, 4 H) 2.82 (br. s., 4 H) 3.63 (s, 2 H) 7.14 (t, *J*=7.78 Hz, 1 H) 7.34 (d, *J*=7.69 Hz, 1 H) 7.52 (d, *J*=8.79 Hz, 1 H) 7.62 (d, *J*=7.88 Hz, 1 H) 7.71 (s, 1 H) 7.77 (d, *J*=8.42 Hz, 1 H) 7.98 (d, *J*=2.38 Hz, 1 H) 10.39 (s, 1 H).

### N-[4-(4-Cyclopropyl-piperazin-1-ylmethyl)-3-trifluoromethyl-phenyl]-2-(3-iodo-phenyl)-acetamide

¹H NMR (600 MHz, DMSO-*d*₆) δ ppm 0.25 - 0.28 (m, 2 H) 0.34 - 0.41 (m, 2 H) 1.55 - 1.63 (m, 1 H) 2.32 (br. s., 4 H) 2.69 (s, 2 H) 3.51 (s, 2 H) 3.64 (s, 2 H) 7.14 (t, *J*=7.78 Hz, 1 H) 7.35 (d, *J*=8.24 Hz, 1 H) 7.63 (d, *J*=7.88 Hz, 1 H) 7.66 (d, *J*=8.42 Hz, 1 H) 7.72 (s, 1 H) 7.76 (dd, *J*=8.33, 1.74 Hz, 1 H) 8.03 (d, *J*=2.01 Hz, 1 H) 10.44 (s, 1 H).

### 2-(3-Iodo-phenyl)-N-[4-(1-methyl-piperidin-4-ylamino)-3-trifluoromethyl-phenyl]-acetamide

¹H NMR (600 MHz, DMSO-*d*₆) δ ppm 1.43 - 1.53 (m, 2 H) 1.86 (d, *J*=10.99 Hz, 2 H) 2.00 - 2.07 (m, 2 H) 2.16 (s, 3 H) 2.65 (d, *J*=10.44 Hz, 2 H) 3.34 - 3.38 (m, 1 H) 3.56 (s, 2 H) 4.37 (d, *J*=7.69 Hz, 1 H) 6.89 (d, *J*=9.16 Hz, 1 H) 7.13 (t, *J*=7.78 Hz, 1 H) 7.33 (d, *J*=7.69 Hz, 1 H) 7.54 (dd, *J*=9.07, 2.29 Hz, 1 H) 7.62 (d, *J*=7.88 Hz, 1 H) 7.70 (s, 1 H) 7.78 (d, *J*=2.38 Hz, 1 H) 10.06 (s, 1 H).

### 2-(3-lodo-phenyl)-N-{4-[methyl-(1-methyl-piperidin-4-yl)-amino]-3-trifluoromethyl-phenyl}-acetamide

¹H NMR (600 MHz, DMSO-*d*₆) δ ppm 1.36 (qd, *J*=11.97, 3.48 Hz, 2 H) 1.62 (d, *J*=12.27 Hz, 2 H) 1.77 (t, *J*=11.54 Hz, 2 H) 2.08 - 2.12 (m, 3 H) 2.68 - 2.74 (m, 3 H) 3.62 (s, 2 H) 7.14 (t, *J*=7.78 Hz, 1 H) 7.34 (d, *J*=7.51 Hz, 1 H) 7.51 (d, *J*=8.79 Hz, 1 H) 7.62 (d, *J*=8.06 Hz, 1 H) 7.72 (s, 1 H) 7.77 (dd, *J*=8.70, 2.29 Hz, 1 H) 7.96 (d, *J*=2.38 Hz, 1 H) 10.39 (s, 1 H).

### N-(4-Chloro-3-trifluoromethyl-phenyl)-2-(3-iodo-phenyl)-acetamide

¹H NMR (600 MHz, DMSO-*d*₆) δ ppm 3.66 (s, 2 H) 7.14 (t, *J*=7.69 Hz, 1 H) 7.34 (d, *J*=8.06 Hz, 1 H) 7.63 (d, *J*=8.06 Hz, 1 H) 7.66 (d, *J*=8.79 Hz, 1 H) 7.72 (s, 1 H) 7.83 (dd, *J*=8.79, 2.38 Hz, 1 H) 8.18 (d, *J*=2.38 Hz, 1 H) 10.60 (s, 1 H).

### N-(5-Bromo-pyridin-3-yl)-2-(3-iodo-phenyl)-acetamide

¹H NMR (600 MHz, DMSO-*d*₆) δ ppm 3.69 (s, 2 H) 7.15 (t, *J*=7.78 Hz, 1 H) 7.34 (d, *J*=8.06 Hz, 1 H) 7.63 (d, *J*=8.06 Hz, 1 H) 7.72 (s, 1 H) 8.36 - 8.37 (m, 1 H) 8.39 (d, *J*=2.01 Hz, 1 H) 8.65 (d, *J*=2.01 Hz, 1 H) 10.57 (s, 1 H)

### 2-(3-lodo-phenyl)-N-[3-trifluoromethyl-4-((2S,5R)-2,4,5-trimethyl-piperazin-1-ylmethyl)-phenyl]-acetamide

¹H NMR (600 MHz, DMSO-*d*₆) δ ppm 0.84 (d, *J*=6.04 Hz, 3 H) 0.99 (d, *J*=6.23 Hz, 3 H) 1.76 (t, *J*=10.71 Hz, 1 H) 1.85 - 1.97 (m, 2 H) 2.11 (s, 3 H) 2.32 - 2.45 (m, 2 H) 2.66 (d, *J*=10.62 Hz, 1 H) 3.09 (d, *J*=14.84 Hz, 1 H) 3.64 (s, 2 H) 4.04 (d, *J*=14.47 Hz, 1 H) 7.14 (t, *J*=7.78 Hz, 1 H) 7.35 (d, *J*=7.69 Hz, 1 H) 7.63 (d, *J*=7.88 Hz, 1 H) 7.68 - 7.81 (m, 3 H) 8.03 (d, *J*=1.47 Hz, 1 H) 10.43 (s, 1 H).

### 2-(3-lodo-phenyl)-N-[3-trifluoromethyl-4-(3,4,5-trimethyl-piperazin-1-ylmethyl)-phenyl]-acetamide

¹H NMR (600 MHz, DMSO-*d*₆) δ ppm 0.94 (br. s., 6 H) 1.80 (br. s., 2 H) 2.02 - 2.25 (m, 5 H) 2.56 - 2.65 (m, 2 H) 3.43 - 3.50 (m, 2 H) 3.64 (s, 2 H) 7.14 (t, *J*=7.69 Hz, 1 H) 7.35 (d, *J*=8.06 Hz, 1 H) 7.63 (d, *J*=7.88 Hz, 1 H) 7.66 (s, 1 H) 7.72 (s, 1 H) 7.77 (d, *J*=7.88 Hz, 1 H) 8.03 (d, *J*=1.83 Hz, 1 H) 10.45 (s, 1 H).

### 4-{4-[2-(3-lodo-phenyl)-acetylamino]-2-trifluoromethyl-benzyl}-piperazine-1-carboxylic acid tert-butyl ester

¹H NMR (600 MHz, DMSO-*d*₆) δ ppm 1.39 (s, 10 H) 2.31 (t, *J*=4.85 Hz, 4 H) 3.55 (s, 2 H) 3.64 (s, 2 H) 7.14 (t, *J*=7.78 Hz, 1 H) 7.35 (d, *J*=7.69 Hz, 1 H) 7.63 (d, *J*=7.88 Hz, 1 H) 7.67 (d, *J*=8.61 Hz, 1 H) 7.72 (s, 1 H) 7.77 (dd, *J*=8.61, 1.83 Hz, 1 H) 8.03 (d, *J*=2.02 Hz, 1 H) 10.45 (s, 1 H).

### 2-(3-lodo-phenyl)-N-[4-(3-oxo-piperazin-1-ylmethyl)-3-trifluoromethyl-phenyl]-acetamide

¹H NMR (600 MHz, DMSO-*d*₆) δ ppm 2.52 - 2.55 (m, 2 H) 2.94 (s, 2 H) 3.14 (t, *J*=4.30 Hz, 2 H) 3.61 (s, 2 H) 3.64 (s, 2 H) 7.14 (t, *J*=7.78 Hz, 1 H) 7.35 (d, *J*=7.69 Hz, 1 H) 7.63 (d, *J*=8.06 Hz, 1 H) 7.67 (d, *J*=8.42 Hz, 1 H) 7.72 (s, 1 H) 7.74 (s, 1 H) 7.78 (dd, *J*=8.43, 1.65 Hz, 1 H) 8.05 (d, *J*=1.83 Hz, 1 H) 10.47 (s, 1 H).

### N-{4-[3-(tert-Butyl-dimethyl-silanyloxy)-piperidin-1-ylmethyl]-3-trifluoromethyl-phenyl}-2-(3-iodo-phenyl)-acetamide

¹H NMR (600 MHz, DMSO-*d*₆) δ ppm -0.03 (s, 3 H) -0.01 - 0.00 (m, 3 H) 0.82 (s, 9 H) 1.13 - 1.22 (m, 1 H) 1.43 (d, *J*=11.72 Hz, 1 H) 1.61 - 1.67 (m, 1 H) 1.79 (dd, *J*=11.91, 3.66 Hz, 1 H) 1.86 (t, *J*=9.71 Hz, 1 H) 1.96 (br. s., 1 H) 2.58 (d, *J*=11.17 Hz, 1 H) 2.69 (d, *J*=6.96 Hz, 1 H) 3.54 (s, 2 H) 3.64 (s, 2 H) 3.66 (d, *J*=4.58 Hz, 1 H) 7.12 - 7.16 (m, 1 H) 7.32 - 7.37 (m, 1 H) 7.61 - 7.65 (m, 1 H) 7.65 - 7.68 (m, 1 H) 7.71 - 7.73 (m, 1 H) 7.74 - 7.76 (m, 1 H) 8.03 (d, J=2.02 Hz, 1 H) 10.44 (s, 1 H).

### N-Cyclopropyl-2-(3-iodo-phenyl)-acetamide

¹H NMR (600 MHz, DMSO-*d*₆) δ ppm 0.36 - 0.40 (m, 2 H) 0.58 - 0.63 (m, 2 H) 2.58 - 2.62 (m, 1 H) 7.10 (t, *J*=7.78 Hz, 1 H) 7.24 (d, *J*=8.24 Hz, 1 H) 7.58 (d, *J*=7.88 Hz, 1 H) 7.62 (s, 1 H) 8.13 (br. s., 1 H).

### 2-(3-lodo-phenyl)-N-[4-(4-methyl-2-oxo-piperazin-1-ylmethyl)-3-trifluoromethyl-phenyl]-acetamide

¹H NMR (600 MHz, DMSO-*d*₆) δ ppm 2.24 (s, 3 H) 2.58 - 2.63 (m, 2 H) 3.06 (s, 2 H) 3.18 (t, *J*=5.40 Hz, 2 H) 3.65 (s, 2 H) 4.62 (s, 2 H) 7.14 (t, *J*=7.78 Hz, 1 H) 7.24 (d, *J*=8.61 Hz, 1 H) 7.34 (d, *J*=7.69 Hz, 1 H) 7.63 (d, *J*=7.88 Hz, 1 H) 7.72 (s, 1 H) 7.74 - 7.77 (m, 1 H) 8.10 (d, *J*=1.83 Hz, 1 H) 10.49 (s, 1 H).

### N-{4-[4-(tert-Butyl-dimethyl-silanyloxy)-piperidin-1-ylmethyl]-3-trifluoromethyl-phenyl}-2-(3-iodo-phenyl)-acetamide

¹H NMR (600 MHz, DMSO-*d*₆) δ ppm 0.01 - 0.04 (m, 6 H) 0.83 - 0.89 (m, 9 H) 1.40 - 1.48 (m, 2 H) 1.70 (d, *J*=10.99 Hz, 2 H) 2.15 (t, *J*=9.07 Hz, 2 H) 2.55 - 2.61 (m, 2 H) 3.50 (s, 2 H) 3.64 (s, 2 H) 3.72 (br. s., 1 H) 7.14 (t, *J*=7.78 Hz, 1 H) 7.35 (d, *J*=7.69 Hz, 1 H) 7.63 (d, *J*=8.06 Hz, 1 H) 7.67 (d, *J*=8.43 Hz, 1 H) 7.72 (s, 1 H) 7.76 (d, *J*=8.61 Hz, 1 H) 8.02 (d, *J*=1.65 Hz, 1 H) 10.44 (s, 1 H).

### N-[4-({[2-(tert-Butyl-dimethyl-silanyloxy)-ethyl]-methyl-amino}-methyl)-3-trifluoromethyl-phenyl]-2-(3-iodophenyl)-acetamide

(ESI) *m*/*z* 607 [(M + H)⁺]. HRMS (ESI) calculated for C₂₅H₃₅F₃IN₂O₂Si⁺ [(M + H)⁺] 607.5348, found 607.5344.

### 2-(3-Bromo-4-fluoro-phenyl)-N-[4-(4-methyl-piperazin-1-ylmethyl)-3-trifluoromethyl-phenyl]-acetamide

¹H NMR (600 MHz, DMSO-*d*₆) δ ppm 2.15 (s, 3 H) 2.19 - 2.47 (m, 7 H) 3.52 (s, 2 H) 3.69 (s, 2 H) 7.25 - 7.40 (m, 2 H) 7.62 - 7.67 (m, 2 H) 7.76 (dd, *J*=8.42, 1.65 Hz, 1 H) 8.03 (d, *J*=1.83 Hz, 1 H) 10.44 (s, 1 H).

### N-(4-{[(2,2-Dimethyl-[1,3]dioxolan-4-ylmethyl)-methyl-amino]-methyl}-3-trifluoromethyl-phenyl)-2-(3-iodo-phenyl)-acetamide

(ESI) *m*/*z* 563 [(M + H)⁺]. HRMS (ESI) calculated for C₂₃H₂₇F₃IN₂O₃⁺ [(M + H)⁺] 563.3638, found 563.3640.

### 2-(3-Bromo-4-fluoro-phenyl)-N-[4-(4-isopropyl-piperazin-1-ylmethyl)-3-trifluoromethyl-phenyl]-acetamide

¹H NMR (600 MHz, DMSO-*d*₆) δ ppm 0.96 (br. s., 6 H) 2.26 - 2.48 (m, 7 H) 3.52 (br. s., 2 H) 3.69 (s, 2 H) 7.31 - 7.35 (m, 1 H) 7.35 - 7.39 (m, 1 H) 7.64 - 7.69 (m, 2 H) 7.76 (d, *J*=8.61 Hz, 1 H) 8.03 (d, *J*=2.01 Hz, 1 H) 10.45 (s, 1 H).

### 2-(5-Bromo-thiophen-2-yl)-N-[4-(4-methyl-piperazin-1-ylmethyl)-3-trifluoromethyl-phenyl]-acetamide

¹H NMR (600 MHz, DMSO-*d*₆) δ ppm 2.17 (br. s., 3 H) 2.38 (d, *J*=1.83 Hz, 8 H) 3.53 (s, 2 H) 3.89 (s, 2 H) 6.83 (d, *J*=3.66 Hz, 1 H) 7.07 (d, *J*=3.66 Hz, 1 H) 7.66 (d, *J*=8.61 Hz, 1 H) 7.76 (d, *J*=8.42 Hz, 1 H) 8.02 (d, *J*=1.65 Hz, 1 H) 10.50 (s, 1 H).

### 2-(3-Bromo-4-fluoro-phenyl)-N-[4-(4-propyl-piperazin-1-ylmethyl)-3-trifluoromethyl-phenyl]-acetamide

(ESI) *m*/*z* 517 [(M + H)⁺]. HRMS (ESI) calculated for C₂₃H₂₇BrF₄N₃O⁺ [(M + H)⁺] 517.3697, found 517.3695.

### 3-lodo-4-methyl-N-[4-(4-methyl-piperazin-1-ylmethyl)-3-trifluoromethyl-phenyl]-benzamide.

¹H NMR (600 MHz, DMSO-*d*₆) d ppm 2.16 (s, 3 H) 2.23 - 2.43 (m, 8 H) 2.45 (s, 3 H) 3.56 (s, 2 H) 7.45 - 7.55 (m, 1 H) 7.70 (d, *J*=8.79 Hz, 1 H) 7.91 (dd, *J*=7.87, 1.77 Hz, 1 H) 8.03 (dd, *J*=8.61, 2.01 Hz, 1 H) 8.17 (d, *J*=1.95 Hz, 1 H) 8.42 (d, *J*=1.83 Hz, 1 H) 10.47 (s, 1 H).

### Preparation 44

### 3-Bromo-N-[4-(4-ethyl-piperazin-1-ylmethyl)-3-trifluoromethyl-phenyl]-benzenesulfonamide

To a solution of 4-(4-ethyl-piperazin-1-ylmethyl)-3-trifluoromethyl-phenylamine (0.10 g, 0.35 mmol), and TEA (0.10 mL, 0.70 mmol) in anhydrous THF (5 mL), 3-bromo-benzenesulfonyl chloride (0.10 g, 0.38 mmol) was added. The mixture was heated at 60 °C for 16 h, then was cooled at r.t., poured in a saturated solution of NaHCO₃ (15 mL) and extracted with DCM (2 x 10 mL). The organic phase was washed with brine, dried with anhydrous Na₂SO₄ and evaporated under vacuum. The crude was purified by flash column chromatography (DCM-MeOH 90:10) to obtain the title compound (0.04 g, 23%) as brown oil.
(ESI) *m*/*z* 507 [(M + H)⁺]. HRMS (ESI) calculated for C₂₀H₂₄BrF₃N₃O₂S⁺ [(M + H)⁺] 507.3797, found 507.3790.

### Preparation 15

### 3-Bromo-N-cyclopropyl-benzenesulfonamide

A mixture of cyclopropylamine (0.12 mL, 1.76 mmol) and 3-bromo-benzenesulfonyl chloride (0.15 g, 0.59 mmol) in anhydrous THF (5 mL) was stirred at r.t. for 2 h. The reaction was poured in a saturated solution of NaHCO₃ (15 mL) and extracted with DCM (2 x 10 mL), the organic phase was washed with brine, dried with anhydrous Na₂SO₄ and evaporated under vacuum. The title product (0.10 g, 62%) was obtained as brown oil.
¹H NMR (600 MHz, DMSO-*d*₆) δ ppm 0.33 - 0.41 (m, 2 H) 0.48 - 0.53 (m, 2 H) 2.13 (tt, *J*=6.91, 3.53 Hz, 1 H) 7.59 (t, *J*=7.78 Hz, 1 H) 7.78 - 7.83 (m, 1 H) 7.87 - 7.90 (m, 1 H) 7.93 (t, *J*=1.74 Hz, 1 H) 8.03 (br. s., 1 H).

### Preparation 16

### 1-Ethynyl-3-phenethyloxy-benzene

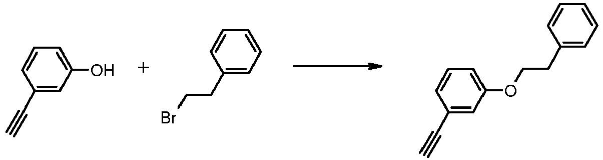

A mixture of 3-ethynyl-phenol (0.20 g, 1.73 mmol), (2-bromo-ethyl)-benzene (0.24 mL, 1.73 mmol), Cs₂CO₃ (1.13 g, 3.46 mmol) and NaI (0.26 g, 1.73 mmol) in acetone (5 mL) was heated at reflux for 12 h. The reaction was cooled at r.t., poured in water (15 mL) and extracted with EtOAc (2 x 15 mL). The organic phase was washed with brine, dried with anhydrous Na₂SO₄ and evaporated under vacuum. The crude was purified by flash column chromatography (Hex-EtOAc 95:5) to obtain the title product (0.08 g, 21%) as colorless oil.
¹H NMR (401 MHz, DMSO-*d*₆) δ ppm 3.02 (t, *J*=6.84 Hz, 2 H) 4.14 (s, 1 H) 4.21 (t, *J*=6.84 Hz, 2 H) 6.86 - 7.06 (m, 3 H) 7.15 - 7.38 (m, 6 H).

According to this same methodology, but employing suitable substituted derivatives, the following intermediate was prepared:

### 1-Ethynyl-3-(3-phenyl-propoxy)-benzene

¹H NMR (600 MHz, DMSO-*d*₆) δ ppm 1.97 - 2.04 (m, 2 H) 2.71 - 2.75 (m, 2 H) 3.97 (t, *J*=6.32 Hz, 2 H) 4.15 (s, 1 H) 6.96 - 7.00 (m, 2 H) 7.04 (d, *J*=7.51 Hz, 1 H) 7.16 - 7.20 (m, 1 H) 7.21 - 7.24 (m, 2 H) 7.26 - 7.32 (m, 3 H).

### Preparation 17

### 1-Benzyloxymethyl-3-iodo-benzene

To a solution of (3-iodo-phenyl)-methanol (0.23 mL, 2.14 mmol) in anhydrous DMF (3 mL), cooled at 0-5 °C, NaH (0.17 g, 4.28 mmol) was added. The mixture was stirred at 0-5 °C for 30 min and then benzyl chloride (0.31 mL, 2.57 mmol) was added. The reaction was stirred at r.t. for 5 h, then was poured in water (15 mL) and extracted with EtOAc (2 x 15 mL). The organic phase was washed with brine, dried with anhydrous Na₂SO₄ and evaporated under vacuum. The crude was purified by flash column chromatography (Hex/EtOAc 95/5) to obtain the title product (0.35 g, 51%) as colorless oil.
¹H NMR (600 MHz, DMSO-*d*₆) δ ppm 4.50 (s, 2 H) 4.53 (s, 2 H) 7.17 (t, *J*=7.78 Hz, 1 H) 7.28 - 7.32 (m, 1 H) 7.33 - 7.39 (m, 5 H) 7.66 (d, *J*=7.88 Hz, 1 H) 7.72 (s, 1 H).

### Preparation 18

### Benzyl-(3-iodo-benzyl)-amine

To a mixture of 3-iodo-benzylamine (0.30 g, 1.29 mmol) and AcOH (0.008 mL, 0.13 mmol) in DCM (5 mL), a solution of benzaldehyde (0.13 mL, 1.29 mmol) in DCM (3 mL) was added drop wise. The reaction was stirred at r.t. for 2 h, then tetramethylammonium triacetoxyborohydride (0.68 g, 2.58 mmol) was added. After 2 h at r.t., the solution was washed with a saturated solution of NaHCO₃ (10 mL), water (10 mL) and brine. The organic phase was dried with anhydrous Na₂SO₄ and evaporated under vacuum to give a crude that was purified by flash column chromatography (DCM-MeOH 97:3). The title product (0.22 g, 53%) was obtained as colorless oil.
¹H NMR (600 MHz, DMSO-*d*₆) δ ppm 2.62 - 2.74 (m, 1 H) 3.64 (d, *J*=10.26 Hz, 5 H) 7.12 (t, *J*=7.69 Hz, 1 H) 7.20 - 7.25 (m, 1 H) 7.29 - 7.37 (m, 6 H) 7.58 (d, *J*=8.06 Hz, 1 H) 7.73 (s, 1 H).

### Preparation 19

### Benzyl-(3-iodo-benzyl)-carbamic acid tert-butyl ester

To a solution of benzyl-(3-iodo-benzyl)-amine (0.21 g, 0.67 mmol) in anhydrous DCM (5 mL), di-*tert*-butyl dicarbonate (0.16 g, 0.73 mmol) and DMAP (0.004 g, 0.03 mmol) were added. The mixture was stirred at r.t. for 1 h, then the solution was evaporated under vacuum and the crude purified by flash column chromatography (DCM) to give the title product (0.23 g, 81%) as colorless oil.
¹H NMR (600 MHz, DMSO-*d*₆) δ ppm 1.38 (s, 9 H) 4.21 - 4.48 (m, 4 H) 7.11 - 7.15 (m, 1 H) 7.22 (br. s., 2 H) 7.25 - 7.28 (m, 1 H) 7.31 - 7.37 (m, 2 H) 7.55 (br. s., 1 H) 7.62 (d, *J*=7.88 Hz, 1 H).

### Preparation 20

### 1-Phenylcarbamoyl-cyclopropanecarboxylic acid

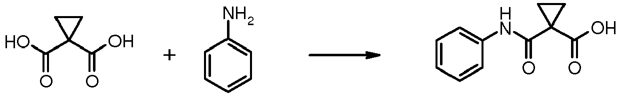

To a mixture of cyclopropane-1,1-dicarboxylic acid (0.36 g, 2.77 mmol) and TEA (0.39 mL, 2.77 mmol) in anhydrous THF (5 mL), cooled at 0-5 °C, a solution of thionyl chloride (0.20 mL, 2.77 mmol) in anhydrous THF (2 mL) was added drop wise in 15 min. A solution of aniline (0.28 mL, 3.05 mmol) in anhydrous THF (4 mL) was added drop wise in 1 h. The reaction was stirred, at 0-5 °C a further 1 h, then was diluted with EtOAc (15 mL) and extracted with 2 N NaOH (2 x 15 mL). The aqueous phase was acidified to pH 1-2 with 2 N HCl and extracted with EtOAc (2 x 15 mL). The organic phase was washed with brine, dried with anhydrous Na₂SO₄ and evaporated under vacuum to give the title product (0.21 g, 37% yield) as white solid.
¹H NMR (600 MHz, DMSO-*d*₆) δ ppm 1.42 (s, 4 H) 6.97 - 7.11 (m, 1 H) 7.22 - 7.35 (m, 2 H) 7.59 (dd, *J*=8.54, 0.98 Hz, 2 H) 10.57 (s, 1 H) 12.35 - 13.48 (m, 1 H).

According to this same methodology, but employing suitable substituted derivatives, the following intermediate was prepared:

### 1-(4-Trifluoromethyl-phenylcarbamoyl)-cyclopropanecarboxylic acid

¹H NMR (600 MHz, DMSO-*d*₆) δ ppm 1.43 (s, 4 H) 7.67 (d, *J*=8.61 Hz, 2 H) 7.82 (d, *J*=8.42 Hz, 2 H) 10.84 (s, 1 H) 13.07 (s, 1 H).

### Preparation 21

### Cyclopropane-1,1-dicarboxylic acid (3-ethynyl-phenyl)-amide phenylamide

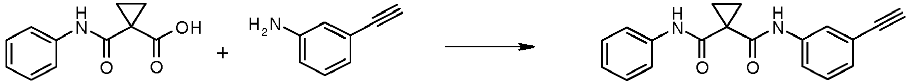

To a solution of 1-phenylcarbamoyl-cyclopropanecarboxylic acid (0.20 g, 0.95 mmol) in anhydrous THF, cooled at 0-5 °C, oxalyl chloride (0.09 mL, 1.00 mmol) and DMF (1 drop) were added. The mixture was stirred at r.t. for 1 h, then evaporated under vacuum. The crude acyl chloride was dissolved in anhydrous THF (3 mL) and added drop wise to a solution of 3-ethynyl-phenylamine (0.13 g, 1.14 mmol) and 2,6-lutidine (0.44 mL, 3.80 mmol) in anhydrous THF (5 mL), cooled at 0-5 °C. The reaction was then stirred at r.t. for 1 h, poured in 2 N HCl (15 mL) and extracted with EtOAc (2 x 15 mL). The organic phase was washed with 2 N HCl (10 mL), a saturated solution of NaHCO₃ (10 mL), brine (10 mL), dried with anhydrous Na₂SO₄ and evaporated under vacuum. The title product (0.11 g, 37%) was obtained as white solid.
¹H NMR (600 MHz, DMSO-*d*₆) δ ppm 1.46 (s, 4 H) 4.15 (s, 1 H) 7.02 - 7.09 (m, 1 H) 7.17 (dt, *J*=7.63, 1.19 Hz, 1 H) 7.26 - 7.34 (m, 3 H) 7.59 - 7.63 (m, 3 H) 7.81 (t, *J*=1.77 Hz, 1 H) 9.98 (s, 1 H) 10.07 (s, 1 H).

According to this same methodology, but employing suitable substituted derivatives, the following intermediate was prepared:

### Cyclopropane-1,1-dicarboxylic acid (3-ethynyl-phenyl)-amide (4-trifluoromethyl-phenyl)-amide

¹H NMR (600 MHz, DMSO-*d*₆) δ ppm 1.45 (s, 4 H) 4.14 (s, 1 H) 7.02 - 7.09 (m, 1 H) 7.17 (dt, *J*=7.63, 1.19 Hz, 1 H) 7.27 - 7.33 (m, 2 H) 7.67 (d, *J*=8.61 Hz, 2 H) 7.82 (d, *J*=8.42 Hz, 2 H) 10.02 (s, 1 H) 10.37 (s, 1 H).

### Preparation 22

### N-(4-Chloro-3-trifluoromethyl-phenyl)-2-(3-trimethylsilanylethynyl-phenyl)-acetamide

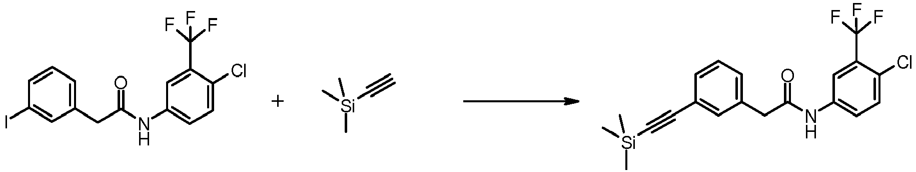

A solution of N-(4-chloro-3-trifluoromethyl-phenyl)-2-(3-iodo-phenyl)-acetamide (0.26 g, 0.59 mmol), ethynyltrimethylsilane (0.12 g, 1.24 mmol), CuI (10% mol, 11 mg, 0.059 mmol), PdCl₂(PPh₃)₂ (10% mol, 41 mg, 0.059 mmol) and TEA (0.81 mL, 5.90 mmol) in MeCN (5 mL) was degassed with argon and stirred at r.t. for 2 h. The reaction was poured in water (15 mL) and extracted with EtOAc (2 x 15 mL). The organic phase was washed with brine, dried with anhydrous Na₂SO₄ and evaporated under vacuum to give a crude that was purified by flash column chromatography (Hex-EtOAc 85:15). The title product (0.23 g, 93%) was obtained as colorless oil.
¹H NMR (600 MHz, DMSO-*d*₆) δ ppm 0.18 - 0.27 (m, 9 H) 3.68 (s, 2 H) 7.28 - 7.37 (m, 3 H) 7.43 (s, 1 H) 7.66 (d, *J*=8.79 Hz, 1 H) 7.83 (dd, *J*=8.79, 2.38 Hz, 1 H) 8.18 (d, *J*=2.56 Hz, 1 H) 10.59 (s, 1 H).

According to this same methodology, but employing suitable substituted derivatives, the following intermediates were prepared:

### N-[4-(4-Ethyl-piperazin-1-ylmethyl)-3-trifluoromethyl-phenyl]-3-trimethylsilanylethynyl-benzamide

(ESI) *m*/*z* 488 [(M + H)⁺]. HRMS (ESI) calculated for C₂₆H₃₃F₃N₃OSi⁺ [(M + H)⁺] 488.6325, found 488.6329.

### N-(3-Trimethylsilanylethynyl-benzyl)-benzamide

¹H NMR (600 MHz, DMSO-*d*₆) δ ppm 0.20 - 0.23 (m, 9 H) 4.46 (d, *J*=6.04 Hz, 2 H) 7.30 - 7.37 (m, 3 H) 7.40 (s, 1 H) 7.45 - 7.50 (m, 2 H) 7.52 - 7.57 (m, 1 H) 7.87 - 7.91 (m, 2 H) 9.04 (t, *J*=5.86 Hz, 1 H).

### N-Phenyl-3-(3-trimethylsilanylethynyl-phenyl)-propionamide

¹H NMR (600 MHz, DMSO-*d*₆) δ ppm 0.21 - 0.23 (m, 9 H) 2.62 (t, *J*=7.60 Hz, 2 H) 2.89 (t, *J*=7.69 Hz, 2 H) 7.02 (t, *J*=7.42 Hz, 1 H) 7.21 - 7.29 (m, 5 H) 7.35 (s, 1 H) 7.55 (d, *J*=7.69 Hz, 2 H) 9.86 (s, 1 H).

### (3-Benzyloxymethyl-phenylethynyl)-trimethyl-silane

¹H NMR (600 MHz, DMSO-*d*₆) δ ppm 4.50 - 4.55 (m, 4 H) 7.27 - 7.32 (m, 1 H) 7.33 - 7.41 (m, 7 H) 7.43 (s, 1 H).

### Benzyl-(3-trimethylsilanylethynyl-benzyl)-carbamic acid tert-butyl ester

¹H NMR (600 MHz, DMSO-*d*₆) δ ppm 1.38 (s, 9 H) 4.35 (d, *J*=16.67 Hz, 4 H) 7.16 - 7.38 (m, 9 H).

### Preparation 23

### N-(4-Chloro-3-trifluoromethyl-phenyl)-2-(3-ethynyl-phenyl)-acetamide

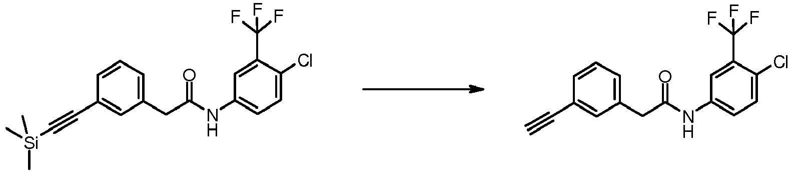

To a solution of N-(4-chloro-3-trifluoromethyl-phenyl)-2-(3-trimethylsilanylethynyl-phenyl)-acetamide (0.20 g, 0.49 mmol) in MeOH (10 mL), K₂CO₃ (0.07 g, 0.54 mmol) was added. The reaction was degassed with argon and stirred at r.t. for 2 h. The mixture was poured in water (30 mL) and extracted with EtOAc (2 x 15 mL). The organic phase was washed with brine, dried with anhydrous Na₂SO₄ and evaporated under vacuum to give the title product (0.15 g, 91%) as colorless oil.
¹H NMR (600 MHz, DMSO-*d*₆) δ ppm 3.69 (s, 2 H) 4.17 (s, 1 H) 7.31 - 7.40 (m, 3 H) 7.44 (s, 1 H) 7.66 (d, *J*=8.79 Hz, 1 H) 7.83 (dd, *J*=8.79, 2.38 Hz, 1 H) 8.18 (d, *J*=2.38 Hz, 1 H) 10.61 (s, 1 H).

According to this same methodology, but employing suitable substituted derivatives, the following itermediates were prepared:

### N-[4-(4-Ethyl-piperazin-1-ylmethyl)-3-trifluoromethyl-phenyl]-3-ethynyl-benzamide

(ESI) *m*/*z* 416 [(M + H)⁺]. HRMS (ESI) calculated for C₂₃H₂₅F₃N₃O⁺ [(M + H)⁺] 416.4514, found 416.4515.

### N-(3-Ethynyl-benzyl)-benzamide

¹H NMR (600 MHz, DMSO-*d*₆) δ ppm 4.15 (s, 1 H) 4.48 (d, *J*=6.04 Hz, 2 H) 7.31 - 7.36 (m, 3 H) 7.39 (s, 1 H) 7.45 - 7.51 (m, 2 H) 7.51 - 7.58 (m, 1 H) 7.88 - 7.91 (m, 2 H) 9.06 (t, *J*=5.86 Hz, 1 H).

### 3-(3-Ethynyl-phenyl)-N-phenyl-propionamide

¹H NMR (600 MHz, DMSO-*d*₆) δ ppm 2.59 - 2.65 (m, 2 H) 2.90 (t, *J*=7.60 Hz, 2 H) 4.13 (s, 1 H) 7.02 (t, *J*=7.42 Hz, 1 H) 7.24 - 7.32 (m, 5 H) 7.37 (s, 1 H) 7.55 (d, *J*=7.69 Hz, 2 H) 9.87 (s, 1 H).

### 1-Benzyloxymethyl-3-ethynyl-benzene

¹H NMR (600 MHz, DMSO-*d*₆) δ ppm 4.17 (s, 1 H) 4.54 (d, *J*=2.93 Hz, 4 H) 7.27 - 7.33 (m, 1 H) 7.34 - 7.42 (m, 7 H) 7.45 (s, 1 H).

### Benzyl-(3-ethynyl-benzyl)-carbamic acid tert-butyl ester

¹H NMR (600 MHz, DMSO-*d*₆) δ ppm 1.38 (br. s., 9 H) 4.16 (s, 1 H) 4.37 (br. s., 3 H) 7.15 - 7.31 (m, 4 H) 7.31 - 7.39 (m, 3 H) 7.41 (t, *J*=7.69 Hz, 1 H) 7.51 (d, *J*=7.88 Hz, 1 H).

### Preparation 24

### 6-Iodo-2,3-dihydro-indole-1-carboxylicacid [4-(4-ethyl-piperazin-1-ylmethyl)-3-trifluoromethyl-phenyl]-amide

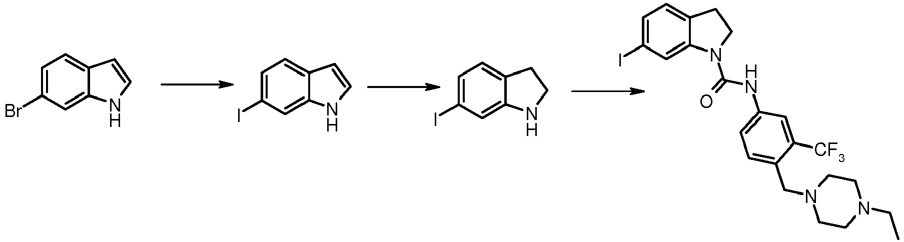

### Step 1. 6-Iodo-1 H-indole

(According to the procedure described in J. Am. Chem. Soc. 2002, 124, 14844-14845). 6-Bromo-1H-indole (0.65 g, 3.34 mmol), NaI (2.0 g, 13.34 mmol) and CuI (63.5 mg, 0.33 mmol) were charged in a Shlenk tube and degassed three times back filling with argon each time. Dioxane (6.5 mL) and (1*S*,2*S*)-*N*,*N'*-dimethyl-cyclohexane-1,2-diamine (0.11 mL, 0.67 mmol) were then added and the mixture was heated at 110 °C for 24 h. The reaction was quenched with NH₃, poured in water and extracted with DCM (3 x 20 mL). The organic layer was dried over anhydrous Na₂SO₄, filtered and concentrated to reduce volume. The product was purified by flash column chromatography (Hex/EtOAc 98/2) and isolated as white solid (0.53 g, 65%).
¹H NMR (600 MHz, DMSO-*d*₆) δ ppm 6.43 (t, J=2.01 Hz, 1 H) 7.25 (dd, J=8.24, 1.47 Hz, 1 H) 7.31 (t, J=2.75 Hz, 1 H) 7.38 (d, J=8.24 Hz, 1 H) 7.75 (s, 1 H) 11.15 (br. s., 1 H).

### Step 2. 6-Iodo-2,3-dihydro-1H-indole

(According to the procedure described in WO2010/043000) A solution of 6-iodo-1H-indole (0.52 g, 2.14 mmol) in AcOH (6.5 mL) was treated with NaBH₃CN (0.62 g, 9.84 mmol) at r.t. for 1 h. The solvent was removed under reduced pressure and the residue was dissolved in Et₂O and washed with 1 N NaOH, water and brine. The organic layer was dried over Na₂SO₄, filtered and concentrated to reduce volume. A flash column chromatography (Hex/EtOAc 95/5) afforded the title product as white solid (0.40 g, 76%).
¹H NMR (600 MHz, DMSO-*d*₆) δ ppm 2.85 (t, J=8.61 Hz, 2 H) 3.40 (t, J=8.43 Hz, 2 H) 5.68 (br. s., 1 H) 6.74 - 6.84 (m, 3 H).

According to this same methodology, but employing suitable substituted derivative, the following intermediate was prepared:

### 5-Iodo-2,3-dihydro-1H-indole

(ESI) *m*/*z* 245 [(M + H)⁺]. HRMS (ESI): calculated for C₈H₉IN [(M + H)⁺] 245.9774; found 245.9798.

### Step 3. 6-lodo-2,3-dihydro-indole-1-carboxylic acid [4-(4-ethyl-piperazin-1-ylmethyl)-3-trifluoromethylphenyl]-amide

To a suspension of triphosgene (0.16 g, 0.53 mmol) and Na₂CO₃ (0.67 g, 6.28 mmol) in DCM (10 mL) kept at 0 °C under argon, 4-(4-ethyl-piperazin-1-ylmethyl)-3-trifluoromethyl-phenylamine (0.45 g, 1.57 mmol) was added. The reaction was monitored by HPLC (following the formation of 4-ethyl-piperazine-1-carboxylic acid [4-(4-ethyl-piperazin-1-ylmethyl)-3-trifluoromethyl-phenyl]-amide by treating a sample of the reaction mixture with N-ethylpiperazine). After 1 h, 6-Iodo-2,3-dihydro-1H-indole (0.39 g, 1.61 mmol) was added and the reaction was let under stirring 1 h at r.t. The mixture was diluted with DCM (10 mL), washed with water (3 x 10 mL), dried over anhydrous Na₂SO₄ and concentrated under vacuum. Purification by flash column chromatography (DCM/MeOH 95/5) afforded the product as yellow foam (0.68 g, 77%).
¹H NMR (600 MHz, DMSO-*d*₆) δ ppm 1.00 (br. s., 3 H) 2.12 - 2.48 (m, 10 H) 3.15 (t, J=8.61 Hz, 2 H) 3.55 (br. s., 2 H) 4.13 (t, J=8.70 Hz, 2 H) 7.03 (d, J=7.88 Hz, 1 H) 7.26 (dd, J=7.69, 1.65 Hz, 1 H) 7.64 (d, J=8.61 Hz, 1 H) 7.83 (d, J=8.43 Hz, 1 H) 7.97 (d, J=2.01 Hz, 1 H) 8.24 (d, J=1.28 Hz, 1 H) 8.84 (s, 1 H).

According to this same methodology, but employing suitable substituted derivatives, the following intermediates were prepared:

### 5-Iodo-2,3-dihydro-indole-1-carboxylic acid [4-(4-ethyl-piperazin-1-ylmethyl)-3-trifluoromethyl-phenyl]-amide

¹H NMR (600 MHz, DMSO-*d*₆) δ ppm 0.98 (t, J=7.14 Hz, 3 H) 2.30 (q, J=7.14 Hz, 3 H) 2.32 - 2.48 (m, 8 H) 3.18 (t, J=8.70 Hz, 2 H) 3.53 (s, 2 H) 4.13 (t, J=8.61 Hz, 2 H) 7.45 (dd, J=8.42, 1.83 Hz, 1 H) 7.53 (s, 1 H) 7.63 (d, J=8.43 Hz, 1 H) 7.69 (d, J=8.61 Hz, 1 H) 7.79 - 7.84 (m, 1 H) 7.95 - 7.99 (m, 1 H) 8.82 (s, 1 H).

### Preparation 25

### 2-(2-Amino-pyrimidin-4-yl)-4-oxo-1,4,6,7-tetrahydro-pyrrolo[3,2-c]pyridine-5-carboxylic acid tert-butyl ester

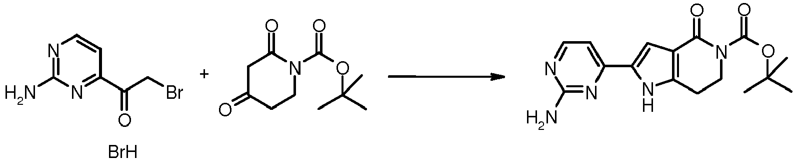

According to the procedure described in WO2004/058762 and in Journal of Medicinal Chemistry 2007, 50, 2647-54. According to this same methodology, but employing suitable substituted derivatives, the following intermediates were prepared:

### 2-(2-Amino-pyrimidin-4-yl)-4-oxo-1-(2-pyrrolidin-1-yl-ethyl)-1,4,6,7-tetrahydro-pyrrolo[3,2-c]pyridine-5-carboxylic acid tert-butyl ester hydrobromide

¹H NMR (600 MHz, DMSO-*d*₆) δ ppm 1.47 (s, 9 H) 1.87 (br. s., 2 H) 2.04 (br. s., 2 H) 3.06 (t, *J*=6.04 Hz, 4 H) 3.54 (d, *J*=6.96 Hz, 2 H) 3.62 (br. s., 2 H) 4.02 (t, *J*=6.23 Hz, 2 H) 4.79 (t, *J*=7.23 Hz, 2 H) 6.68 (br. s., 2 H) 7.00 (d, *J*=5.13 Hz, 1 H) 7.25 (s, 1 H) 8.20 (d, *J=*5.31 Hz, 1 H) 9.71 (br. s., 1 H).

### 2-(2-Amino-pyrimidin-4-yl)-1-(2-dimethylamino-ethyl)-4-oxo-1,4,6,7-tetrahydro-pyrrolo[3,2-c]pyridine-5-carboxylic acid tert-butyl ester hydrobromide

¹H NMR (600 MHz, DMSO-*d*₆) δ ppm 1.47 (s, 9 H) 2.86 (br. s., 6 H) 3.06 (t, *J*=6.32 Hz, 2 H) 3.39 - 3.47 (m, 2 H) 4.01 (t, *J*=6.32 Hz, 2 H) 4.78 - 4.90 (m, 2 H) 6.73 (br. s., 2 H) 6.99 (d, *J*=5.13 Hz, 1 H) 7.24 (s, 1 H) 8.19 (d, *J*=5.31 Hz, 1 H) 10.20 (br. s., 1 H).

### 2-(2-Amino-pyrimidin-4-yl)-1-(1-methyl-piperidin-4-yl)-1,5,6,7-tetrahydro-pyrrolo[3,2-c]pyridin-4-one dihydrochloride

¹H NMR (400 MHz, DMSO-*d*₆) δ ppm 2.08 - 2.16 (m, 2 H) 2.53 - 2.62 (m, 2 H) 2.79 (d, *J*=4.63 Hz, 3 H) 3.16 (t, *J*=6.46 Hz, 2 H) 3.24 - 3.45 (m, 6 H overlapped by water signal) 5.77 (br. s., 1 H) 7.27 (br. s., 1 H) 7.41 (br. s., 1 H) 7.46 (br. s., 1 H) 8.16 (d, *J*=6.46 Hz, 1 H) 10.59 (br. s., 1 H).

### 2-(3-Bromo-pyridin-4-yl)-4-oxo-1,4,6,7-tetrahydro-pyrrolo[3,2-c]pyridine-5-carboxylic acid tert-butyl ester

(ESI) *m*/*z* 392 [(M + H)⁺]. HRMS (ESI) calculated for C₁₇H₁₉BrN₃O₃ [(M + H)⁺] 392.0604; found 392.0621.

### Preparation 26

### 2-Bromo-1-(3-bromo-pyridin-4-yl)-ethanone

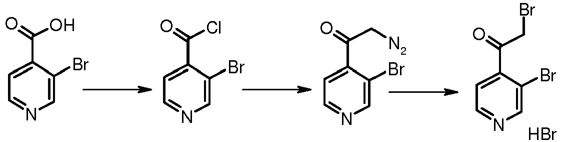

3-Bromo-isonicotinic acid (1.0 g, 4.95 mmol) was suspended in thionyl chloride (10 mL) and refluxed for 1 h when the solubilization was complete (detection of methyl ester by HPLC-MS for treatment of a small sample with methanol). The solvent was removed by stripping with toluene for three times. The acyl chloride was then suspended in dry THF (18 mL) and added drop wise to a 2 M solution of TMSCH₂N₂ in hexane (6.18 mL, 12.37 mmol) at 0 °C in 15 min. The mixture was let under stirring at 0 °C for 3.5 h, then 48% HBr (4 mL) was carefully added (gas evolution) and the reaction was let at r.t. for 1.5 h. The solvent was reduced to small volume and the precipitate was filtered and rinsed with cold water affording the title product as hydrobromide (1.34 g, 75% over three steps).
(ESI) *m*/*z* 277 [(M + H)⁺]. HRMS (ESI) calculated for C₇H₆Br₂NO [(M + H)⁺] 277.8811; found 277.8830.

### Preparation 45

### 2-(2-Amino-pyrimidin-4-yl)-1-methyl-4-oxo-1,4,6,7-tetrahydro-pyrrolo[3,2-c]pyridine-5-carboxylic acid tert-butyl ester

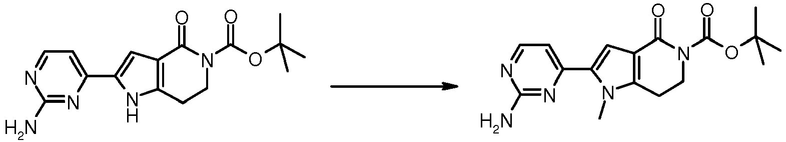

The synthetic procedure has already been described in WO2010/145998.

According to this same methodology, but employing suitable substituted derivatives, the following intermediates were prepared:

### 2-(2-Amino-pyrimidin-4-yl)-1-(2-tert-butoxycarbonylamino-ethyl)-4-oxo-1,4,6,7-tetrahydro-pyrrolo[3,2-c]pyridine-5-carboxylic acid tert-butyl ester

(ESI) *m*/*z* 473 [(M + H)⁺]. HRMS (ESI) calculated for C₂₃H₃₃N₅O₅ [(M + H)⁺] 473.2507; found 473.2521.

### 2-(2-Amino-pyrimidin-4-yl)-4-oxo-1-[2-(tetrahydro-pyran-2-yloxy)-ethyl]-1,4,6,7-tetrahydro-pyrrolo[3,2-c]pyridine-5-carboxylic acid tert-butyl ester

¹H NMR (600 MHz, DMSO-*d*₆) δ ppm 1.27 - 1.64 (m, 6 H) 1.47 (s, 9 H) 3.01 (t, J=6.32 Hz, 2 H) 3.37 - 3.43 (m, 1 H) 3.61 - 3.68 (m, 1 H) 3.82 - 3.88 (m, 1 H) 3.93 - 3.99 (m, 2 H) 4.46 (t, *J*=3.11 Hz, 1 H) 4.75 (t, *J*=4.85 Hz, 2 H) 6.55 (s, 2 H) 6.94 (d, *J*=5.31 Hz, 1 H) 7.18 (s, 1 H) 8.14 (d, *J*=5.31 Hz, 1 H).

### Preparation 27

### 2-(2-Amino-5-iodo-pyrimidin-4-yl)-4-oxo-1-[2-(tetrahydro-pyran-2-yloxy)-ethyl]-1,4,6,7-tetrahydro-pyrrolo[3,2-c]pyridine-5-carboxylic acid tert-butyl

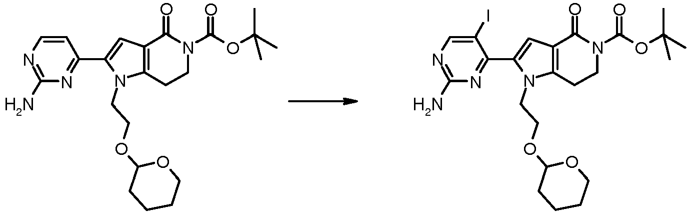

According to the procedure described in WO2010/145998.

To a solution of 2-(2-amino-pyrimidin-4-yl)-4-oxo-1-[2-(tetrahydro-pyran-2-yloxy)-ethyl]-1,4,6,7-tetrahydro-pyrrolo[3,2-c]pyridine-5-carboxylic acid tert-butyl ester (0.48 g, 1.05 mmol) in THF (35 mL), cooled at 0-5 °C, iodine (0.53 g, 2.10 mmol) and silver trifluoroacetate (0.46 g, 2.10 mmol) were added. The reaction was stirred at r.t. overnight, then filtered through celite washing with EtOAc. The organic phase was evaporated under vacuum, the crude was dissolved with DMF (10 mL) and precipitated with water (100 mL). The resulting solid was dried at 45 °C, under vacuum, to obtain the title product (0.36 g, 60%) as yellow powder.
¹H NMR (600 MHz, DMSO-*d*₆) δ ppm 1.24 - 1.45 (m, 6 H) 1.45 - 1.49 (m, 10 H) 3.01 (t, *J*=6.41 Hz, 2 H) 3.40 - 3.51 (m, 2 H) 3.64 (ddd, *J*=10.62, 6.50, 3.94 Hz, 1 H) 3.98 (dq, *J*=13.60, 6.70 Hz, 2 H) 4.26 - 4.43 (m, 2 H) 4.52 (ddd, *J*=14.65, 6.69, 3.94 Hz, 1 H) 6.85 (br. s., 2 H) 7.03 (s, 1 H) 8.57 (s, 1 H).

According to this same methodology, but employing suitable substituted derivatives, the following intermediates were prepared:

### 2-(2-Amino-5-iodo-pyrimidin-4-yl)-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.47 (s, 9 H) 2.97 (t, *J*=6.32 Hz, 2 H) 3.61 (s, 3 H) 3.98 (t, *J*=6.32 Hz, 2 H) 6.86 (s, 2 H) 7.01 (s, 1 H) 8.56 (s, 1 H).

### 2-(2-Amino-5-iodo-pyrimidin-4-yl)-1-(2-tert-butoxycarbonylamino-ethyl)-4-oxo-1,4,6,7-tetrahydro-pyrrolo[3,2-c]pyridine-5-carboxylic acid tert-butyl ester

(ESI) *m*/*z* 599 [(M + H)⁺]. HRMS (ESI) calculated for C₂₃H₃₂IN₆O₅ [(M + H)⁺] 599.1473; found 599.1460.

### 2-(2-Amino-5-iodo-pyrimidin-4-yl)-4-oxo-1,4,6,7-tetrahydro-pyrrolo[3,2-c]pyridine-5-carboxylic acid tert-butyl ester

¹H NMR (401 MHz, DMSO-*d*₆) δ ppm 1.47 (s, 9 H) 2.94 (t, *J*=6.35 Hz, 2 H) 3.95 (t, *J*=6.23 Hz, 2 H) 6.57 (br. s., 2 H) 7.69 (d, *J*=2.44 Hz, 1 H) 8.51 (s, 1 H) 11.88 (br. s., 1 H).

### 2-(2-Amino-5-iodo-pyrimidin-4-yl)-1-(1-methyl-piperidin-4-yl)-1,5,6,7-tetrahydro-pyrrolo[3,2-c]pyridin-4-one

(ESI) *m*/*z* 453 [(M + H)⁺]. HRMS (ESI) calculated for C₁₇H₂₂IN₆O [(M + H)⁺] 453.0894; found 453.0905.

### Preparation 28

### 2-[2-(Acetyl-methyl-amino)-5-ethynyl-pyrimidin-4-yl]-1-methyl-4-oxo-1,4,6,7-tetrahydro-pyrrolo[3,2-c]pyridine-5-carboxylic acid tert-butyl ester

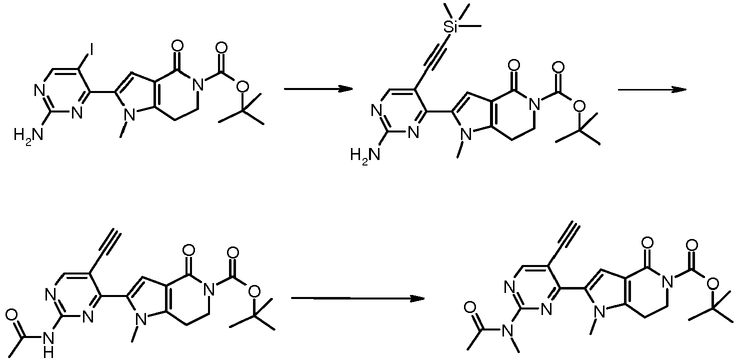

### Step 1. 2-(2-Amino-5-trimethylsilanylethynyl-pyrimidin-4-yl)-1-methyl-4-oxo-1,4,6,7-tetrahydro-pyrrolo[3,2-c]pyridine-5-carboxylic acid tert-butyl ester

A solution of 2-(2-Amino-5-iodo-pyrimidin-4-yl)-1-methyl-4-oxo-1,4,6,7-tetrahydro-pyrrolo[3,2-c]pyridine-5-carboxylic acid tert-butyl ester (2.00 g, 4.26 mmol), ethynyltrimethylsilane (1.35 mL, 8.52 mmol), CuI (5% mol, 0.04 g, 0.21 mmol), PdCl₂(PPh₃)₂ (5% mol, 0.15 g, 0.21 mmol) and TEA (5.80 mL, 42.60 mmol) in DMF (50 mL) was degassed with argon and stirred at r.t. for 4 h. The solvent was evaporated under vacuum, the crude was dissolved in EtOAc (250 mL), washed with NH₃, and brine. The organic phase was dried with anhydrous Na₂SO₄ and evaporated under vacuum to give a crude that was purified by flash column chromatography (DCM-MeOH 97:3) to give the title product (1.78 g, 95%) as brownish solid.
¹H NMR (600 MHz, DMSO-*d*₆) δ ppm 0.19 - 0.22 (m, 9 H) 1.47 (s, 9 H) 2.97 (t, *J*=6.32 Hz, 2 H) 3.82 (s, 3 H) 3.98 (t, *J*=6.23 Hz, 2 H) 7.10 (s, 2 H) 7.55 (s, 1 H) 8.34 (s, 1 H).

According to this same methodology, but employing suitable substituted derivatives, the following intermediate was prepared:

### 2-(2-Amino-5-trimethylsilanylethynyl-pyrimidin-4-yl)-4-oxo-1-[2-(tetrahydro-pyran-2-yloxy)-ethyl]-1,4,6,7-tetrahydro-pyrrolo[3,2-c]pyridine-5-carboxylic acid tert-butyl ester

¹H NMR (600 MHz, DMSO-*d*₆) δ ppm 0.21 (s, 8 H) 1.27 - 1.42 (m, 4 H) 1.46 (s, 11 H) 3.05 (br. s., 2 H) 3.19 - 3.29 (m, 2 H) 3.55 - 3.62 (m, 1 H) 3.73 (ddd, *J*=10.85, 7.10, 3.66 Hz, 1 H) 3.89 - 4.05 (m, 2 H) 4.40 - 4.46 (m, 1 H) 4.63 (ddd, *J*=14.38, 7.23, 3.66 Hz, 1 H) 4.70 - 4.79 (m, 1 H) 7.07 (s, 2 H) 7.65 (s, 1 H) 8.34 (s, 1 H).

### Step 2. 2-(2-Acetylamino-5-ethynyl-pyrimidin-4-yl)-1-methyl-4-oxo-1,4,6,7-tetrahydro-pyrrolo[3,2-c]pyridine-5-carboxylic acid tert-butyl ester

A mixture of 2-(2-amino-5-trimethylsilanylethynyl-pyrimidin-4-yl)-1-methyl-4-oxo-1,4,6,7-tetrahydro-pyrrolo[3,2-c]pyridine-5-carboxylic acid tert-butyl ester (0.50 g, 1.14 mmol) and Ac₂O (10 mL) was stirred at 80 °C for 6 h (the suspension turned to a yellow solution). The solvent was then removed under reduced pressure. EtOH was added and the solvents were evaporated. This process was repeated 3 times. The crude was dissolved in MeOH (10 mL), TEA (1.55 mL, 11.40 mmol) was added and the reaction was stirred at r.t. for 24 h. The suspension was then poured in water (100 mL), the solid precipitated was filtered, washed with water and dried at 45 °C under vacuum to give the title product (0.30 g, 64%) as white solid. ¹H NMR (600 MHz, DMSO-*d*₆) δ ppm 1.47 (s, 9 H) 2.17 (s, 3 H) 3.00 (t, *J*=6.32 Hz, 2 H) 3.94 (s, 3 H) 3.99 (t, *J*=6.32 Hz, 2 H) 4.73 (s, 1 H) 7.65 (s, 1 H) 8.74 (s, 1 H) 10.77 (s, 1 H).

### Step 3. 2-[2-(Acetyl-methyl-amino)-5-ethynyl-pyrimidin-4-yl]-1-methyl-4-oxo-1,4,6,7-tetrahydro-pyrrolo[3,2-c]pyridine-5-carboxylic acid tert-butyl ester

To a suspension of 2-(2-acetylamino-5-ethynyl-pyrimidin-4-yl)-1-methyl-4-oxo-1,4,6,7-tetrahydro-pyrrolo[3,2-c]pyridine-5-carboxylic acid tert-butyl ester (0.16 g, 0.39 mmol) in anhydrous DMF (5 mL), Cs₂CO₃ (0.26 g, 0.78 mmol) and Mel (0.05 mL, 0.78 mmol) were added. The mixture was stirred at r.t. for 3 h, then poured in water (50 mL); the solid precipitated was filtered, washed with water and dried at 45 °C under vacuum. The title product (0.13 g, 78%) was obtained as white solid.
¹H NMR (600 MHz, DMSO-*d*₆) δ ppm 1.42 - 1.51 (m, 9 H) 2.35 - 2.43 (m, 3 H) 3.01 (t, *J*=6.23 Hz, 2 H) 3.36 - 3.41 (m, 3 H) 3.79 - 3.86 (m, 3 H) 4.00 (t, *J*=6.32 Hz, 2 H) 4.80 (s, 1 H) 7.61 (s, 1 H) 8.84 (s, 1 H).

By analogous procedure, using EtI instead of Mel the following itermediate was obtained:

### 2-[2-(Acetyl-ethyl-amino)-5-ethynyl-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 438 [(M + H)⁺]. HRMS (ESI) calculated for C₂₃H₂₈N₅O₄⁺ [(M + H)⁺] 438.4916, found 438.4920.

### Preparation 29

### 2-(2-Amino-5-ethynyl-pyrimidin-4-yl)-1-methyl-4-oxo-1,4,6,7-tetrahydro-pyrrolo[3,2-c]pyridine-5-carboxylic acid tert-butyl ester

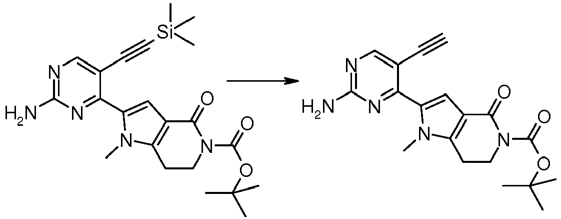

2-(2-Amino-5-trimethylsilanylethynyl-pyrimidin-4-yl)-1-methyl-4-oxo-1,4,6,7-tetrahydro-pyrrolo[3,2-c]pyridine-5-carboxylic acid tert-butyl ester (2.04 g, 4.64 mmol) was dissolved in degassed MeOH (75 mL) and treated with K₂CO₃ (0.64 g, 4.64 mmol), under argon, at r.t. for 6 h. The reaction was quenched with 2N HCl (4.64 mL) and the solvent was removed under reduced pressure. The residue was suspended in water and the solid was filtered affording the title product (1.56 g, 91%).
¹H NMR (500 MHz, DMSO-*d*₆) δ ppm 1.47 (s, 6 H) 2.97 (t, *J*=6.33 Hz, 2 H) 3.78 - 3.82 (m, 3 H) 3.98 (t, *J*=6.25 Hz, 2 H) 4.42 (s, 1 H) 7.11 (s, 2 H) 7.42 (s, 1 H) 8.38 (s, 1 H).

According to this same methodology, but employing suitable substituted derivatives, the following intermediate was prepared:

### 2-(2-Amino-5-ethynyl-pyrimidin-4-yl)-4-oxo-1-[2-(tetrahydro-pyran-2-yloxy)-ethyl]-1,4,6,7-tetrahydro-pyrrolo [3,2-c]pyridine-5-carboxylic acid tert-butyl ester

(ESI) *m*/*z* 482 [(M + H)⁺]. HRMS (ESI) calculated for C₂₅H₃₂N₅O₅⁺ [(M + H)⁺] 482.5441, found 482.5440.

### Preparation 30

### 2-(5-lodo-2-methylamino-pyrimidin-4-yl)-1-methyl-1,5,6,7-tetrahydro-pyrrolo[3,2-c]pyridin-4-one

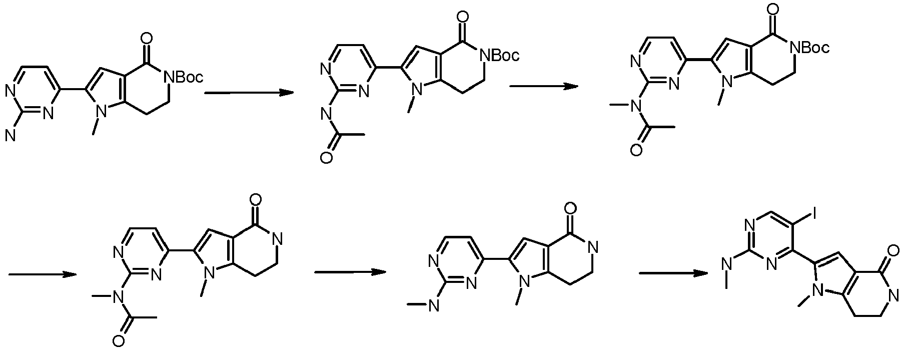

### Step 1. 2-(2-Acetylamino-pyrimidin-4-yl)-1-methyl-4-oxo-1,4,6,7-tetrahydro-pyrrolo[3,2-c]pyridine-5-carboxylic acid tert-butyl ester

2-(2-Amino-pyrimidin-4-yl)-1-methyl-4-oxo-1,4,6,7-tetrahydro-pyrrolo[3,2-c]pyridine-5-carboxylic acid tert-butyl ester (0.46 g, 1.34 mmol) was suspended in Ac₂O (8 mL) and heated at 80 °C. The suspension turned to a yellow solution during 1 h when the reaction reached completion. The solvent was removed under reduced pressure stripping with EtOH for three times affording the product as white solid, which was used in the following step without further purification.
¹H NMR (500 MHz, DMSO-*d*₆) δ ppm 1.47 (s, 9 H) 2.16 (s, 3 H) 2.97 (t, *J*=6.31 Hz, 2 H) 3.93 - 4.00 (m, 2 H) 4.04 (s, 3 H) 7.34 (s, 1 H) 7.51 (d, *J*=5.49 Hz, 1 H) 8.51 (d, *J*=5.49 Hz, 1 H) 10.56 (s, 1 H).

### Step 2. 2-[2-(Acetyl-methyl-amino)-pyrimidin-4-yl]-1-methyl-4-oxo-1,4,6,7-tetrahydro-pyrrolo[3,2-c]pyridine-5-carboxylic acid tert-butyl ester

2-(2-Acetylamino-pyrimidin-4-yl)-1-methyl-4-oxo-1,4,6,7-tetrahydro-pyrrolo[3,2-c]pyridine-5-carboxylic acid tert-butyl ester deriving from the previous step was dissolved in dry DMF (9 mL) and treated with Cs₂CO₃ (0.52 mg, 1.60 mmol) and Mel (0.14 mL, 2.22 mmol) at r.t. overnight. The mixture was then poured in water and let under stirring for 30 min. The resulting white solid was filtered and dried under vacuum (0.41 g, 78% over two steps).
¹H NMR (500 MHz, DMSO-*d*₆) δ ppm 1.47 (s, 9 H) 2.33 - 2.37 (m, 3 H) 2.99 (t, *J*=6.31 Hz, 2 H) 3.38 (s, 3 H) 3.93 (s, 3 H) 3.96 - 4.02 (m, 2 H) 7.40 (s, 1 H) 7.65 (d, *J*=5.22 Hz, 1 H) 8.63 (d, *J*=5.22 Hz, 1 H).

According to this same methodology, but employing suitable substituted derivatives, the following intermediates were prepared:

### 2-(2-{Acetyl-[2-(tetrahydro-pyran-2-yloxy)-ethyl]-amino}-pyrimidin-4-yl)-1-methyl-4-oxo-1,4,6,7-tetrahydro-pyrrolo[3,2-c]pyridine-5-carboxylic acid tert-butyl ester

(ESI) *m*/*z* 514 [(M + H)⁺]. HRMS (ESI) calculated for C₂₆H₃₆N₅O₆ [(M + H)⁺] 514.2660; found 514.2679.

### Step 3. N-Methyl-N-[4-(1-methyl-4-oxo-4,5,6,7-tetrahydro-1H-pyrrolo[3,2-c]pyridin-2-yl)-pyrimidin-2-yl]-acetamide

A solution of 2-[2-(acetyl-methyl-amino)-pyrimidin-4-yl]-1-methyl-4-oxo-1,4,6,7-tetrahydro-pyrrolo[3,2-c]pyridine-5-carboxylic acid tert-butyl ester (0.41 g, 1.03 mmol) and TFA (2 mL) in DCM (10 mL) was let under stirring at r.t. for 1h. The solvent was evaporated under reduced pressure yielding the title product as yellow solid.
(ESI) *m*/*z* 300 [(M + H)⁺]. HRMS (ESI) calculated for C₁₅H₁₈N₅ [(M + H)⁺] 300.1455; found 300.1432.

According to this same methodology, but employing suitable substituted derivative, the following intermediate was prepared:

### N-(2-Hydroxy-ethyl)-N-[4-(1-methyl-4-oxo-4,5,6,7-tetrahydro-1H-pyrrolo[3,2-c]pyridin-2-yl)-pyrimidin-2-yl]-acetamide

(ESI) *m*/*z* 330 [(M + H)⁺]. HRMS (ESI) calculated for C₁₆H₂₀N₅O₃ [(M + H)⁺] 330.1561; found 330.1580.

### Step 4. 1-Methyl-2-(2-methylamino-pyrimidin-4-yl)-1,5,6,7-tetrahydro-pyrrolo[3,2-c]pyridin-4-one.

N-Methyl-N-[4-(1-methyl-4-oxo-4,5,6,7-tetrahydro-1H-pyrrolo[3,2-c]pyridin-2-yl)-pyrimidin-2-yl]-acetamide deriving from the previous step was refluxed in MeOH (10 mL) with K₂CO₃ (0.40 g, 2.89 mmol) for 1 h. The solvent was evaporated and the residue was suspended in water. The resulting solid was filtered giving the title product (0.25 g, 94% over two steps).
¹H NMR (600 MHz, DMSO-*d*₆) δ ppm 2.83 (d, *J*=4.95 Hz, 3 H) 2.85 (t, *J*=6.87 Hz, 2 H) 3.42 (td, *J*=6.87, 2.38 Hz, 2 H) 3.96 (br. s, 3 H) 6.87 (d, *J*=5.31 Hz, 1 H) 6.97 (d, *J*=4.21 Hz, 1 H) 7.01 (s, 1 H) 7.08 (br. s., 1 H) 8.16 (d, *J*=4.40 Hz, 1 H).

According to this same methodology, but employing suitable substituted derivative, the following intermediate was prepared:

### 2-[2-(2-Hydroxy-ethylamino)-pyrimidin-4-yl]-1-methyl-1,5,6,7-tetrahydro-pyrrolo[3,2-c]pyridin-4-one

(ESI) *m*/*z* 288 [(M + H)⁺]. HRMS (ESI) calculated for C₁₄H₁₈N₅O₂ [(M + H)⁺] 288.1455; found 288.1455.

### Step 5. 2-(5-lodo-2-methylamino-pyrimidin-4-yl)-1-methyl-1,5,6,7-tetrahydro-pyrrolo[3,2-c]pyridin-4-one.

According to the experimental procedure described in WO2010/145998.
¹H NMR (600 MHz, DMSO-*d*₆) δ ppm 2.79 (d, *J*=4.76 Hz, 3 H) 2.87 (t, *J*=6.78 Hz, 2 H) 3.43 (td, *J*=6.82, 2.29 Hz, 3 H) 3.66 (br. s., 3 H) 7.00 (br. s., 1 H) 7.09 (br. s., 1 H) 7.26 (br. s., 1 H) 8.49 - 8.65 (m, 1 H).

According to this same methodology, but employing suitable substituted derivative, the following intermediate was prepared:

### 2-[2-(2-Hydroxy-ethylamino)-5-iodo-pyrimidin-4-yl]-1-methyl-1,5,6,7-tetrahydro-pyrrolo[3,2-c]pyridin-4-one

¹H NMR (600 MHz, DMSO-*d*₆) δ ppm 2.87 (t, *J*=6.78 Hz, 2 H) 3.33 (2 H overlapped by water signal) 3.43 (td, *J*=6.87, 2.20 Hz, 2 H) 3.50 (t, *J*=6.32 Hz, 2 H) 3.64 (br. s, 3 H) 4.67 (br. s., 1 H) 6.99 (br. s., 1 H) 7.09 (br. s., 1 H) 7.23 (t, *J*=5.68 Hz, 1 H) 8.56 (br. s., 1 H).

### Preparation 31

### 1-Methyl-2-(2-methylamino-pyrimidin-4-yl)-1,5,6,7-tetrahydro-pyrrolo[3,2-c]pyridin-4-one

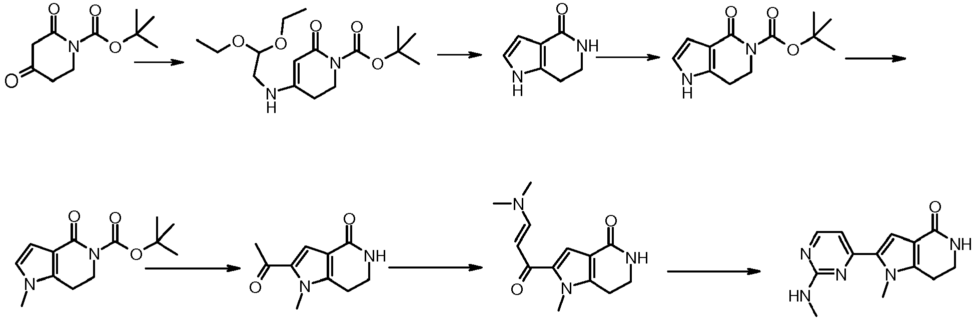

### Step 1. 4-(2,2-Diethoxy-ethylamino)-6-oxo-3,6-dihydro-2H-pyridine-1-carboxylic acid tert-butyl ester

In a four-necked round bottom flask 4-oxo-1,4,6,7-tetrahydro-pyrrolo[3,2-c]pyridine-5-carboxylic acid tert-butyl ester (50.6 g, 0.24 mol) was suspended in toluene (750 mL). Amino acetaldehyde diethyl acetal (34.5 mL, 0.24 mol) was added dropwise under stirring at r.t. during 15 min when the suspension turned to solution. The mixture was heated at 70 °C for 1 h. After cooling to r.t. the solvent was removed under reduced pressure affording the title product as yellow oil (82.8 g, quantitative yield, 4.8 g of residual of toluene).
¹H NMR (401 MHz, DMSO-*d*₆) δ ppm 1.13 (t, *J*=7.02 Hz, 6 H) 1.42 (s, 9 H) 2.37 - 2.44 (m, 2 H) 3.06 (t, *J*=5.43 Hz, 1 H) 3.50 (dq, *J*=9.58, 7.02 Hz, 2 H) 3.57 - 3.68 (m, 4 H) 4.50 (s, 1 H) 4.53 - 4.62 (m, 1 H) 6.95 (br. s., 1 H).

### Step 2. 1,5,6,7-Tetrahydro-pyrrolo[3,2-c]pyridin-4-one trifluoroacetate

A solution of 4-(2,2-diethoxy-ethylamino)-6-oxo-3,6-dihydro-2H-pyridine-1-carboxylic acid tert-butyl ester from the previous step (82.8 g, 0.24 theoretical mol) in DCM (150 mL) was added drop wise in 40 min to TFA (500 mL) kept in an ice-cold bath in a four-necked round bottom flask under stirring. When the addition was complete, the mixture was let under stirring at r.t. for 1 h. The solvents were removed under reduced pressure affording a yellow oil that was treated with MTBE (1 L) under stirring for 2 h. The precipitate was filtered and eliminated. After concentration of the filtrate, the resulting solid was treated with MTBE (5 vol) and cyclohexane (3 vol) and filtered yielding a first aliquot of the title product as yellow solid (18.7 g). The mother liquor was concentrated to small volume affording, after filtration, a further aliquot of product that was rinsed with cyclohexane and dried (7.22 g, 43% total yield).
¹H NMR (600 MHz, DMSO-*d*₆) δ ppm 2.72 (t, *J*=6.87 Hz, 2 H) 3.33 (m, 2 H overlapped by water signal) 6.19 (t, *J*=2.56 Hz, 1 H) 6.64 (t, *J*=2.56 Hz, 1 H) 6.81 (br. s., 1 H) 11.07 (br. s., 1 H).

### Step 3. 4-Oxo-1,4,6,7-tetrahydro-pyrrolo[3,2-c]pyridine-5-carboxylic acid tert-butyl ester

1,5,6,7-Tetrahydro-pyrrolo[3,2-c]pyridin-4-one trifluoroacetate (28.25 g, 0.11 mol) was suspended in MeCN and treated with Boc₂O (86.3 g, 0.40 mol), TEA (63 mL, 0.45 mol) and DMAP (10% mol, 1.38 g, 0.011 mol). The mixture was let under stirring at r.t. for 22 h (formation of the bis-Boc derivative detected by HPLC-MS). After removal of the solvent, the residue was dissolved in DCM and washed with 5% solution of citric acid. The aqueous layer was extracted with DCM (2 x 50 mL). The organic phase was dried over anhydrous Na₂SO₄, filtered and concentrated to yield the bis-Boc intermediate as brown solid that was suspended in MeOH (445 mL), treated with NH₃ (53 mL) and heated at 75 °C for 7 h. The solvent was removed under vacuum, the residue was dissolved in DCM (50 mL) and treated with MTBE (160 mL) affording the title product as brownish solid after filtration (13.36 g, 50%).
¹H NMR (600 MHz, DMSO-*d*₆) δ ppm 1.45 (s, 9 H) 2.83 (t, *J*=6.32 Hz, 2 H) 3.91 (t, *J*=6.23 Hz, 2 H) 6.31 (t, *J*=2.11 Hz, 1 H) 6.64 - 6.81 (m, 1 H) 11.33 (br. s., 1 H).

### Step 4. 1-Methyl-4-oxo-1,4,6,7-tetrahydro-pyrrolo[3,2-c]pyridine-5-carboxylic acid tert-butyl ester

To a solution of 4-oxo-1,4,6,7-tetrahydro-pyrrolo[3,2-c]pyridine-5-carboxylic acid tert-butyl ester (1 g, 4.23 mmol) in dry DMF (10 mL) Cs₂CO₃ (1.79 g, 5.50 mmol) and Mel (0.40 mL, 6.35 mmol) were added. The mixture was let under stirring at r.t. for 1 h, then it was diluted with EtOAc and washed with water (2 x 20 mL) and brine. The organic phase was dried over anhydrous Na₂SO₄, filtered and taken to dryness under vacuum affording the title product as light yellow solid (0.96 g, 91%).
¹H NMR (600 MHz, DMSO-*d*₆) δ ppm 1.45 (s, 9 H) 2.83 (t, *J*=6.32 Hz, 2 H) 3.54 (s, 3 H) 3.92 (t, *J*=6.32 Hz, 2 H) 6.32 (d, *J*=3.11 Hz, 1 H) 6.75 (d, *J*=2.93 Hz, 1 H).

### Step 5. 2-Acetyl-1-methyl-1,5,6,7-tetrahydro-pyrrolo[3,2-c]pyridin-4-one

The solution of 1-methyl-4-oxo-1,4,6,7-tetrahydro-pyrrolo[3,2-c]pyridine-5-carboxylic acid tert-butyl ester (0.94 g, 3.77 mmol) in dry DCM (18 mL) was kept at 0 °C and AlCl₃ (1.50 g, 11.30 mmol) was added portionwise under a vigorous stirring. After 20 min, a solution of acetyl chloride (0.40 mL, 5.65 mmol) in dry DCM (1.5 mL) was dropped into the suspension, the mixture was warmed to r.t. and let under stirring for 2 h. The reaction was quenched by pouring into 2 N HCl (20 mL) at 0°C, keeping temperature below 15 °C. The aqueous layer was extracted with DCM (4 x 10 mL), the organic phase was washed with brine, dried over anhydrous Na₂SO₄, filtered and concentrated under reduced pressure. The residue was treated with Et₂O and filtered affording the title product as light orange solid (0.36 g, 50%).
¹H NMR (600 MHz, DMSO-*d*₆) δ ppm 2.38 (s, 3 H) 2.83 (t, *J*=6.87 Hz, 2 H) 3.41 (td, *J*=6.87, 2.56 Hz, 2 H) 3.77 (s, 3 H) 7.23 (br. s., 1 H) 7.29 (s, 1 H).

### Step 6. 2-((E)-3-Dimethylamino-acryloyl)-1-methyl-1,5,6,7-tetrahydro-pyrrolo[3,2-c]pyridin-4-one

The mixture of 2-acetyl-1-methyl-1,5,6,7-tetrahydro-pyrrolo[3,2-c]pyridin-4-one (0.35 g, 1.84 mmol) and dimethylformamide diisopropylacetal (1.15 mL, 5.50 mmol) in DMF (5 mL) was heated at 100 °C for 7 h. The solvent was removed under reduced pressure giving the product as red solid that was used in the subsequent step without further purification.
¹H NMR (600 MHz, DMSO-*d*₆) δ ppm 2.80 (t, *J*=6.87 Hz, 2 H) 2.86 (br. s., 3 H) 3.06 (br. s., 3 H) 3.39 (td, *J*=6.87, 2.38 Hz, 3 H) 3.81 (s, 3 H) 5.66 (d, *J*=12.45 Hz, 1 H) 7.06 (br. s., 1 H) 7.07 (s, 1 H) 7.49 - 7.52 (m, 1 H).

### Step 7. 1-Methyl-2-(2-methylamino-pyrimidin-4-yl)-1,5,6,7-tetrahydro-pyrrolo[3,2-c]pyridin-4-one

The mixture of 2-((*E*)-3-dimethylamino-acryloyl)-1-methyl-1,5,6,7-tetrahydro-pyrrolo[3,2-c]pyridin-4-one (1.84 theoretical mmol) from the previous step, *N*-methylguanidine hydrochloride (0.40 g, 3.67 mmol) and *t*BuONa (0.35 g, 3.67 mmol) in DMF (5 mL) was heated at 100 °C for 4 h. After cooling the mixture was poured into cold water and let under stirring. The white solid was filtered affording a first aliquot of product (0.10 g). The mother liquor was concentrated to reduced volume and a second aliquot of product was recovered by filtration (0.07 g, 36% total yield).
¹H NMR (600 MHz, DMSO-*d*₆) δ ppm 2.83 (d, *J*=4.95 Hz, 3 H) 2.85 (t, *J*=6.87 Hz, 2 H) 3.42 (td, *J*=6.87, 2.38 Hz, 2 H) 3.92 - 4.01 (m, 3 H) 6.87 (d, *J*=5.31 Hz, 1 H) 6.97 (d, *J*=4.21 Hz, 1 H) 7.01 (s, 1 H) 7.08 (br. s., 1 H) 8.16 (d, *J*=4.40 Hz, 1 H).

### Example 1

### 1-[4-(4-Ethyl-piperazin-1-ylmethyl)-3-trifluoromethyl-phenyl]-3-{3-[4-(1-methyl-4-oxo-4,5,6,7-tetrahydro-1H-pyrrolo[3,2-c]pyridin-2-yl)-pyridin-3-ylethynyl]-phenyl}-urea (cmpd 38)

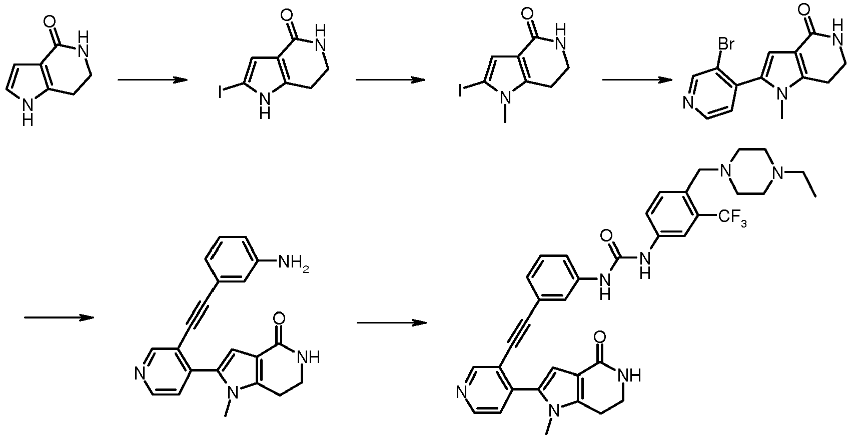

### Step 1. 2-lodo-1,5,6,7-tetrahydro-pyrrolo[3,2-c]pyridin-4-one

To a solution of 1,5,6,7-tetrahydro-pyrrolo[3,2-c]pyridin-4-one (0.12 g, 0.85 mmol) in dry THF (5 mL) and MeOH (1.5 mL), kept at -70 °C, *N*-iodosuccinimide (0.19 g, 0.84 mmol) was added. After 1.5 h the reaction was quenched with a saturated solution of Na₂S₂O₅, let to reach r.t. and the solvent was removed under reduced pressure. The residue was suspended in water and the white solid was filtered and rinsed with water (0.16 g), while the filtrate was extracted with DCM (3 x 10 mL). The organic layer was dried over anhydrous Na₂SO₄, filtered and concentrated affording a second aliquot of product (32 mg, 89% total yield).
¹H NMR (600 MHz, DMSO-*d*₆) δ ppm 2.72 (t, *J*=6.87 Hz, 2 H) 3.30 - 3.32 (m, 2 H) 6.34 (s, 1 H) 6.90 (br. s., 1 H) 11.61 (br. s., 1 H).

### Step 2. 2-lodo-1-methyl-1,5,6,7-tetrahydro-pyrrolo[3,2-c]pyridin-4-one

2-lodo-1,5,6,7-tetrahydro-pyrrolo[3,2-c]pyridin-4-one (0.2 g, 0.75 mmol) was disolved in dry DMF (3.5 mL) and treated with Cs₂CO₃ (0.44 g, 1.35 mmol) and Mel (0.12 mL, 1.87 mmol). The mixture was let under stirring at r.t. for 6 h. After dilution with EtOAc, the organic layer was washed with water (2 x 10 mL) and brine, then it was dried over anhydrous Na₂SO₄, filtered and concentrated under reduced pressure. A purification by flash column chromatography afforded the product as yellow solid (0.18 g, 87%).
¹H NMR (600 MHz, DMSO-*d*₆) δ ppm 2.80 (t, *J*=6.87 Hz, 2 H) 3.35 (m, 2H under water signal) 3.46 (s, 3 H) 6.48 (s, 1 H) 6.94 (br. s., 1 H).

### Step 3. 2-(3-Bromo-pyridin-4-yl)-1-methyl-1,5,6,7-tetrahydro-pyrrolo[3,2-c]pyridin-4-one

(Ref. Tetrahedron 2003, 59, 10043-10049). To a solution of 2-iodo-1-methyl-1,5,6,7-tetrahydro-pyrrolo[3,2-c]pyridin-4-one (0.30 g, 1.09 mmol) and 3-bromo-4-(4,4,5,5-tetramethyl-[1,3,2]dioxaborolan-2-yl)-pyridine (0.34 g, 1.20 mmol) in dry DMF (30 mL), a 2 N solution of K₃PO₄ (1.09 mL, 2.19 mmol) and Pd(PPh₃)₄ (51 mg, 0.044 mmol) were added. The resulting mixture was degassed three times back filling with argon each time then it was heated at 85 °C for 4 h. After cooling to r.t., the mixture was diluted with EtOAc and treated with water. The resulting emulsion was filtered over a pad of celite rinsing with EtOAc and DCM. The solution was concentrated under reduced pressure. The residue was suspended in water, stirred for 15 min and the precipitate was filtered affording a first aliquot of the product as white solid (0.15 g). The filtrate was evaporated giving a residue that was purified by flash column chromatography (DCM/MeOH 97/3) yielding a second aliquot of product (71 mg, 66% total yield).
¹H NMR (600 MHz, DMSO-*d*₆) δ ppm 2.85 (t, *J*=6.96 Hz, 2 H) 3.40 (s, 3 H) 3.44 (td, *J*=6.96, 2.56 Hz, 2 H) 6.47 (s, 1 H) 7.05 (br. s., 1 H) 7.46 (d, *J*=4.95 Hz, 1 H) 8.59 (d, *J*=4.94 Hz, 1 H) 8.86 (s, 1 H).

### Step 4. 2-[3-(3-Amino-phenylethynyl)-pyridin-4-yl]-1-methyl-1,5,6,7-tetrahydro-pyrrolo[3,2-c]pyridin-4-one

To a solution of 2-(3-bromo-pyridin-4-yl)-1-methyl-1,5,6,7-tetrahydro-pyrrolo[3,2-c]pyridin-4-one (0.21 g, 0.69 mmol), CuI (19.2 mg, 0.1 mmol), 3-ethynyl-phenylamine (0.24 g, 2.07 mmol) and TEA (1.16 mL, 8.37 mmol) in degassed dry DMF (13 mL), PdCl₂(PPh₃)₂ (72.6 mg, 0.1 mmol) was added. The resulting mixture was degassed three times back filling with argon each time then it was heated at 50 °C for 8 h. After cooling, the mixture was diluted with EtOAc and washed with water (2 x 10 mL). The organic layer was dried over anhydrous Na₂SO₄, filtered and concentrated under reduced pressure. The residue was purified by flash column chromatography (DCM/MeOH 90/10) affording the title compound as yellowish solid (0.11 g, 46%).
¹H NMR (600 MHz, DMSO-*d*₆) δ ppm 2.89 (t, J=6.78 Hz, 2 H) 3.46 (td, J=6.69, 2.20 Hz, 2 H) 3.54 (s, 3 H) 5.22 (s, 2 H) 6.53 - 6.63 (m, 2 H) 6.67 (s, 1 H) 6.74 (s, 1 H) 7.03 (t, J=7.78 Hz, 1 H) 7.09 (br. s., 1 H) 7.46 (d, J=5.13 Hz, 1 H) 8.56 (d, J=4.95 Hz, 1 H) 8.77 (s, 1 H).

**Step 5. 1-[4-(4-Ethyl-piperazin-1-ylmethyl)-3-trifluoromethyl-phenyl]-3-{3-[4-(1-methyl-4-oxo-4,5,6,7-tetrahydro -1H-pyrrolo[3,2-c]pyridin-2-yl)-pyridin-3-ylethynyl]-phenyl}-urea**

Procedure with triphosgene as already described.
¹H NMR (600 MHz, DMSO-*d*₆) δ ppm 0.98 (t, J=7.23 Hz, 3 H) 2.30 (q, J=7.20 Hz, 2 H) 2.38 (br. s., 8 H) 2.92 (t, J=6.87 Hz, 2 H) 3.45 (td, J=6.91, 2.66 Hz, 2 H) 3.50 (s, 2 H) 3.56 (s, 3 H) 6.72 (s, 1 H) 7.07 (d, J=7.14 Hz, 2 H) 7.33 (t, J=7.78 Hz, 1 H) 7.39 (d, J=9.16 Hz, 1 H) 7.47 - 7.50 (m, 1 H) 7.56 - 7.59 (m, 1 H) 7.61 - 7.65 (m, 1 H) 7.71 (s, 1 H) 7.97 (d, J=1.83 Hz, 1 H) 8.59 (d, J=5.13 Hz, 1 H) 8.83 (s, 1 H) 8.97 (br. s., 1 H) 9.13 (br. s., 1 H).

### Example 2

### 2-{3-[6-Amino-4-(1-methyl-4-oxo-4,5,6,7-tetrahydro-1H-pyrrolo[3,2-c]pyridin-2-yl)-pyridin-3-ylethynyl]-phenyl}-N-[4-(4-methyl-piperazin-1-ylmethyl)-3-trifluoromethyl-phenyl]-acetamide (cmpd 201)

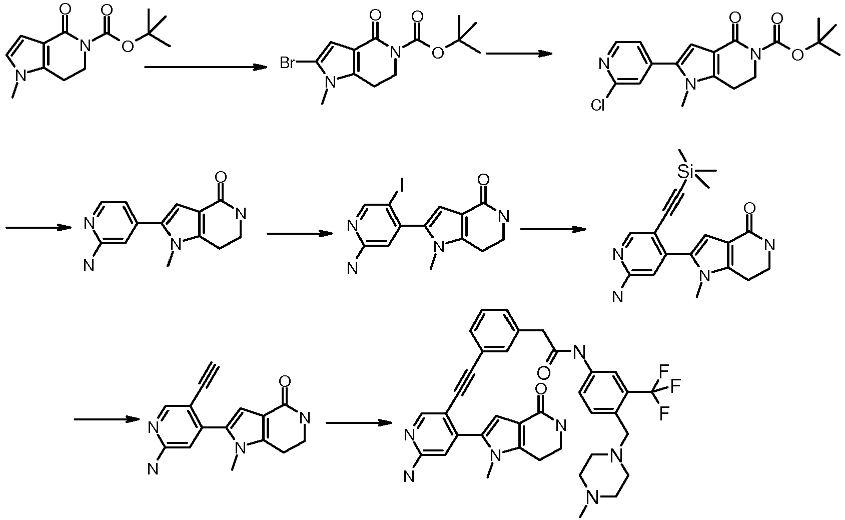

### Step 1. 2-Bromo-1-methyl-4-oxo-1,4,6,7-tetrahydro-pyrrolo[3,2-c]pyridine-5-carboxylic acid tert-butyl ester

To a solution of 1-methyl-4-oxo-1,4,6,7-tetrahydro-pyrrolo[3,2-c]pyridine-5-carboxylic acid tert-butyl ester (4.32 g, 17.29 mmol) in dry THF (108 mL) and MeOH (32 mL), kept at -70 °C, a first portion of NBS (1.54 g, 8.65 mmol) was added. After 45 min, a second portion of NBS (1.54 g, 8.65 mmol) was added and the mixture let under stirring at -70 °C for further 30 min. The temperature was allowed to rise up to r.t. and kept at this temperature for additional 2.5 h. The reaction was quenched with a 10% solution of Na₂S₂O₅ (50 mL). After removal of the solvent under reduced pressure, the residue was dissolved with EtOAc and washed with water and brine. The organic layer was dried over anhydrous Na₂SO₄, filtered and evaporated. The product was purified by flash column chromatography (DCM/MeOH 98/2) and isolated as white solid (4.40 g, 77%).
¹H NMR (600 MHz, DMSO-*d₆*) δ ppm 1.45 (s, 9 H) 2.88 (t, J=6.41 Hz, 2 H) 3.48 (s, 3 H) 3.93 (t, J=6.32 Hz, 2 H) 6.49 (s, 1 H).

### Step 2. 2-(2-Chloro-pyridin-4-yl)-1-methyl-4-oxo-1,4,6,7-tetrahydro-pyrrolo[3,2-c]pyridine-5-carboxylic acid tert-butyl ester

2-Bromo-1-methyl-4-oxo-1,4,6,7-tetrahydro-pyrrolo[3,2-c]pyridine-5-carboxylic acid tert-butyl ester (1.03 g, 3.13 mmol), 2-chloro-4-pyridineboronic acid (0.98 g, 6.26 mmol) and PdCl₂(dppf)₂ (0.26 g, 0.31 mmol) were charged in a Schlenk tube under argon. Degassed dioxane (52 mL) and a 2 M solution of Na₂CO₃ (4.69 mL) were added and the resulting mixture was degassed three times back filling with argon each time. The reaction was heated at 85 °C for 4.5 h. After cooling, it was filtered over a pad of celite rinsing thoroughly with EtOAc. The filtrate was washed with a saturated solution of NaHCO₃, water and brine. The organic layer was dried over anhydrous Na₂SO₄, filtered and evaporated. Purification by flash column chromatography (Hex/EtOAc 50/50) afforded the title product as white solid (0.64 g, 56%).
¹H NMR (600 MHz, DMSO-*d₆*) δ ppm 1.47 (s, 9 H) 2.97 (t, J=6.32 Hz, 2 H) 3.65 (s, 3 H) 3.98 (t, J=6.32 Hz, 2 H) 6.88 (s, 1 H) 7.55 (dd, J=5.31, 1.47 Hz, 1 H) 7.62 - 7.64 (m, 1 H) 8.41 (d, J=5.31 Hz, 1 H).

### Step 3. 2-(2-Amino-pyridin-4-yl)-1-methyl-1,5,6,7-tetrahydro-pyrrolo[3,2-c]pyridin-4-one

In a Schlenk tube 2-(2-chloro-pyridin-4-yl)-1-methyl-4-oxo-1,4,6,7-tetrahydro-pyrrolo[3,2-c]pyridine-5-carboxylic acid tert-butyl ester (0.50 g, 1.38 mmol) and carbamic acid tert-butyl ester (0.81 g, 6.91 mmol) were charged with degassed dioxane (15 mL) under argon. Pd(OAc)₂ (30 mg, 0.14 mmol), Xantphos (0.12 g, 0.14 mmol) and Cs₂CO₃ (0.90 g, 2.76 mmol) were added. The resulting mixture was degassed three times back filling with argon each time and heated at 120 °C for 2 h. After cooling, the solvent was removed under reduced pressure and the residue was treated with TFA (10 mL) in DCM (15 mL) overnight. After removal of the solvents to dryness, the product was purified by flash column chromatography (EtOAc/MeOH 80/20) and isolated as white solid (86 mg, 25%).
¹H NMR (600 MHz, DMSO-*d₆*) δ ppm 2.83 (t, J=6.87 Hz, 2 H) 3.42 (td, J=6.91, 2.47 Hz, 2 H) 3.57 (s, 3 H) 5.92 (s, 2 H) 6.42 (s, 1 H) 6.49 (s, 1 H) 6.57 (dd, J=5.31, 1.47 Hz, 1 H) 7.00 (br. s., 1 H) 7.91 (d, J=5.31 Hz, 1 H).

### Step 4. 2-(2-Amino-5-iodo-pyridin-4-yl)-1-methyl-1,5,6,7-tetrahydro-pyrrolo[3,2-c]pyridin-4-one

As described in WO2010/145998 1H NMR (600 MHz, DMSO-*d₆*) δ ppm 2.83 (t, J=6.98 Hz, 1 H) 3.33 (s, 3 H overlapped by water signal) 3.42 (dd, J=6.96, 2.56 Hz, 2 H) 6.23 (s, 1 H) 6.25 (br. s, 2 H) 6.46 (s, 1 H) 6.99 (br. s., 1 H) 8.25 (s, 1 H).

### Step 5. 2-(2-Amino-5-trimethylsilanylethynyl-pyridin-4-yl)-1-methyl-1,5,6,7-tetrahydro-pyrrolo[3,2-c]pyridin-4-one

As described for the synthesis of 2-(2-amino-5-trimethylsilanylethynyl-pyrimidin-4-yl)-1-methyl-4-oxo-1,4,6,7-tetrahydro-pyrrolo[3,2-c]pyridine-5-carboxylic acid tert-butyl ester.
(ESI) *m*/*z* 339 [(M + H)⁺]. HRMS (ESI) calculated for C₁₈H₂₃N₄OSi [(M + H)⁺] 339.1636; found 339.1620.

### Step 6. 2-(2-Amino-5-ethynyl-pyridin-4-yl)-1-methyl-1,5,6,7-tetrahydro-pyrrolo[3,2-c]pyridin-4-one

As described for the synthesis of 2-(2-amino-5-ethynyl-pyrimidin-4-yl)-1-methyl-4-oxo-1,4,6,7-tetrahydro-pyrrolo[3,2-c]pyridine-5-carboxylic acid tert-butyl ester.
(ESI) *m*/*z* 267 [(M + H)⁺]. HRMS (ESI) calculated for C₁₅H₁₅N₄O[(M + H)⁺] 267.1240; found 267.1257.

### Step 7. 2-{3-[6-Amino-4-(1-methyl-4-oxo-4,5,6,7-tetrahydro-1H-pyrrolo[3,2-c]pyridin-2-yl)-pyridin-3-ylethynyl]-phenyl}-N-[4-(4-methyl-piperazin-1-ylmethyl)-3-trifluoromethyl-phenyl]-acetamide

A solution of 2-(2-Amino-5-ethynyl-pyridin-4-yl)-1-methyl-1,5,6,7-tetrahydro-pyrrolo[3,2-c]pyridin-4-one (0.10 g, 0.38 mmol), 2-(3-iodo-phenyl)-N-[4-(4-propyl-piperazin-1-ylmethyl)-3-trifluoromethyl-phenyl]-acetamide (0.19 g, 0.36 mmol), CuI (10% mol, 7 mg, 0.038 mmol), PdCl₂(PPh₃)₂ (10% mol, 30 mg, 0.038 mmol) and TEA (0.52 mL, 3.80 mmol) in DMF (3 mL) was degassed with argon and stirred at r.t. for 4 h. The reaction was poured in water (15 mL) and extracted with EtOAc (2 x 15 mL). The organic phase was washed with brine, dried with anhydrous Na₂SO₄ and evaporated under vacuum to give a crude that was purified by flash column chromatography (DCM/MeOH/NH₃ 90/10/0.3). The title compound (0.12 g, 51%) was obtained as yellowish solid.
(ESI) *m*/*z* 656 [(M + H)⁺]. HRMS (ESI) calculated for C₃₆H₃₇F₃N₇O₂ [(M + H)⁺] 656.2956; found 656.2959.

### Preparation 32

### 1-[4-(4-Ethyl-piperazin-1-ylmethyl)-3-trifluoromethyl-phenyl]-3-(3-ethynyl-phenyl)-urea

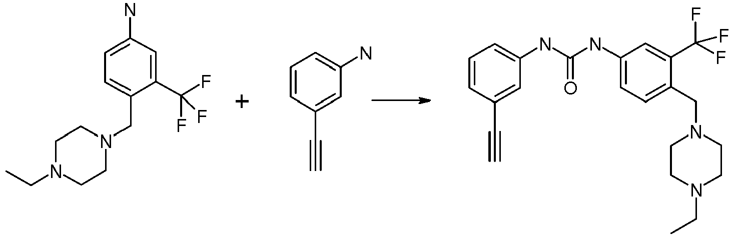

To a suspension of triphosgene (0.21 g, 0.71 mmol) and Na₂CO₃ (0.45 g, 4.27 mmol) in DCM (18 mL), kept at 0 °C under argon, 4-(4-ethyl-piperazin-1-ylmethyl)-3-trifluoromethyl-phenylamine (0.61 g, 2.13 mmol) was added. The reaction was monitored by HPLC (following the formation of 4-ethyl-piperazine-1-carboxylic acid [4-(4-ethyl-piperazin-1-ylmethyl)-3-trifluoromethyl-phenyl]-amide by treating a sample of the reaction mixture with N-ethylpiperazine). After 1 h, 3-ethynyl-phenylamine (0.26 g, 2.18 mmol) was added and the reaction was let under stirring overnight at r.t.. The mixture was diluted with DCM, washed with water (3 x 10 mL), dried over anhydrous Na₂SO₄ and concentrated under vacuum. Purification by flash column chromatography (DCM/MeOH 95/5) afforded the product as yellow solid (0.64 g, 70%).
¹H NMR (600 MHz, DMSO-*d*₆) δ ppm 0.95 - 1.00 (m, 3 H) 2.22 - 2.47 (m, 10 H) 3.52 (s, 2 H) 4.14 (s, 1 H) 7.09 (d, *J*=7.69 Hz, 1 H) 7.30 (t, *J*=7.88 Hz, 1 H) 7.41 (dd, *J*=8.24, 1.28 Hz, 1 H) 7.56 (dd, *J*=8.61, 1.83 Hz, 1 H) 7.61 - 7.64 (m, 1 H) 7.66 (t, *J*=1.65 Hz, 1 H) 7.95 (d, *J*=2.01 Hz, 1 H) 8.85 (s, 1 H) 9.02 (s, 1 H).

According to this same methodology, but employing suitable substituted derivatives, the following intermediates were prepared:

### 1-[3-(4-Ethyl-piperazin-1-ylmethyl)-5-trifluoromethyl-phenyl]-3-(3-ethynyl-phenyl)-urea

¹H NMR (401 MHz, DMSO-*d*₆) δ ppm 0.98 (t, *J*=7.14 Hz, 3 H) 3.51 (s, 2 H) 4.15 (s, 1 H) 7.10 (d, *J*=7.57 Hz, 1 H) 7.22 (s, 1 H) 7.30 (t, *J*=7.81 Hz, 1 H) 7.41 (d, *J*=8.06 Hz, 1 H) 7.53 (s, 1 H) 7.67 (s, 1 H) 7.88 (s, 1 H) 8.84 (s, 1 H) 9.11 (s, 1 H).

### 1-[4-(4-Ethyl-piperazin-1-ylmethyl)-3-trifluoromethyl-phenyl]-3-(4-ethynyl-phenyl)-urea

(ESI) *m*/*z* 431 [(M + H)⁺]. HRMS (ESI) calculated for C₂₃H₂₆F₃N₄O [(M + H)⁺] 431.2053; found 431.2071.

### Preparation 33

**2-[2-Amino-5-(3-amino-phenylethynyl)-pyrimidin-4-yl]-1-methyl-4-oxo-1,4,6,7-tetrahydro-pyrrolo[3,2-c]pyridine-5-carboxylic acid tert-butyl ester**

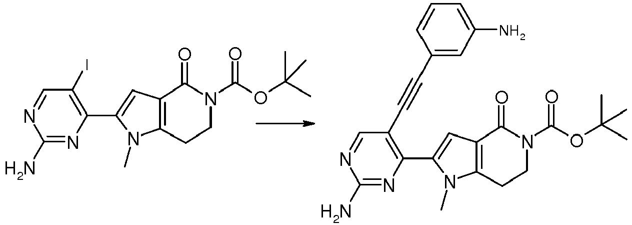

The chemical procedure for the synthesis of this intermediate is the same already described in patent WO2010/145998.
¹H NMR (401 MHz, DMSO-*d₆*) δ ppm 1.48 (s, 9 H) 3.00 (t, J=6.28 Hz, 2 H) 3.85 (s, 3 H) 4.01 (t, J=6.28 Hz, 2 H) 5.17 (s, 2 H) 6.58 (ddd, J=8.11, 2.26, 0.98 Hz, 1 H) 6.62 (dt, J=7.53, 1.17 Hz, 1 H) 6.69 (t, J=1.83 Hz, 1 H) 7.03 (t, J=7.74 Hz, 1 H) 7.08 (s, 2 H) 7.53 (s, 1 H) 8.43 (s, 1 H).

According to this same methodology, but employing suitable substituted derivatives, the following intermediates were prepared:

### 2-[2-Amino-5-(3-amino-phenylethynyl)-pyrimidin-4-yl]-1-(2-tert-butoxycarbonylamino-ethyl)-4-oxo-1,4,6,7-tetrahydro-pyrrolo[3,2-c]pyridine-5-carboxylic acid tert-butyl ester

(ESI) *m*/*z* 588 [(M + H)⁺]. HRMS (ESI) calculated for C₃₁H₃₈N₇O₅ [(M + H)⁺] 588.2929; found 588.2943.

### 2-[2-Amino-5-(4-amino-phenylethynyl)-pyrimidin-4-yl]-1-methyl-4-oxo-1,4,6,7-tetrahydro-pyrrolo[3,2-c] pyridine-5-carboxylic acid tert-butyl ester

(ESI) *m*/*z* 459 [(M + H)⁺]. HRMS (ESI) calculated for C₂₅H₂₇N₆O₃ [(M + H)⁺] 459.2139; found 459.2153.

### 2-[2-Amino-5-(3-amino-4-fluoro-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 477 [(M + H)⁺]. HRMS (ESI) calculated for C₂₅H₂₆N₆O₃ [(M + H)⁺] 477.2045; found 477.2026.

### Preparation 34

### 2-{2-Amino-5-[3-(3-phenyl-ureido)-phenylethynyl]-pyrimidin-4-yl}-1-methyl-4-oxo-1,4,6,7-tetrahydro-pyrrolo [3,2-c]pyridine-5-carboxylic acid tert-butyl ester

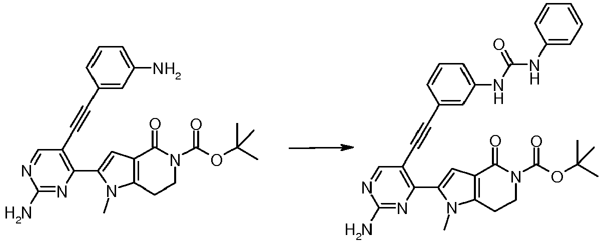

2-[2-Amino-5-(3-amino-phenylethynyl)-pyrimidin-4-yl]-1-methyl-4-oxo-1,4,6,7-tetrahydro-pyrrolo[3,2-c]pyridine-5-carboxylic acid tert-butyl ester (0.10 g, 0.22 mmol) was suspended in DCM (2 mL), dioxane (2 mL) and DMA (0.5 mL) and treated with phenyl-isocyanate (0.026 mL, 0.24 mmol). The mixture was let under stirring overnight, then the solvent was removed under vacuum. The crude was treated with Et₂O affording the title product as white solid (94.2 mg, 75%).
¹H NMR (401 MHz, DMSO-*d*₆) δ ppm 1.46 (s, 9 H) 3.00 (t, J=6.35 Hz, 2 H) 3.84 (s, 3 H) 4.01 (t, J=6.35 Hz, 2 H) 6.95 - 7.03 (m, 1 H) 7.07 (dt, J=7.63, 1.31 Hz, 1 H) 7.13 (s, 2 H) 7.24 - 7.34 (m, 3 H) 7.35 - 7.41 (m, 1 H) 7.42 - 7.47 (m, 2 H) 7.64 (t, J=1.77 Hz, 1 H) 8.49 (s, 1 H) 8.68 (s, 1 H) 8.75 (s, 1 H).

According to this same methodology, but employing suitable substituted derivatives, the following intermediates were prepared:

### 2-{2-Amino-5-[3-(3-p-tolyl-ureido)-phenylethynyl]-pyrimidin-4-yl}-1-methyl-4-oxo-1,4,6,7-tetrahydro-pyrrolo [3,2-c]pyridine-5-carboxylic acid tert-butyl ester

¹H NMR (401 MHz, DMSO-*d*₆) δ ppm 1.38 - 1.50 (m, 9 H) 2.24 (s, 3 H) 3.00 (t, J=6.29 Hz, 2 H) 3.84 (s, 3 H) 4.01 (t, J=6.35 Hz, 2 H) 7.02 - 7.11 (m, 3 H) 7.11 - 7.16 (m, 2 H) 7.25 - 7.36 (m, 3 H) 7.36 - 7.41 (m, 1 H) 7.49 - 7.50 (m, 1 H) 7.63 (t, J=1.71 Hz, 1 H) 8.49 (s, 1 H) 8.57 (s, 1 H) 8.70 (s, 1 H).

### 2-(2-Amino-5-{3-[3-(4-dimethylamino-phenyl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (401 MHz, DMSO-*d*₆) δ ppm 1.46 (s, 9 H) 2.84 (s, 6 H) 3.00 (t, J=6.29 Hz, 2 H) 3.84 (s, 3 H) 3.98 - 4.03 (m, 2 H) 6.70 (d, J=8.67 Hz, 2 H) 7.04 (dt, J=7.63, 1.25 Hz, 1 H) 7.12 (s, 2 H) 7.22 - 7.31 (m, 3 H) 7.35 - 7.40 (m, 1 H) 7.49 (s, 1 H) 7.62 (t, J=1.71 Hz, 1 H) 8.31 (s, 1 H) 8.48 (s, 1 H) 8.61 (s, 1 H).

### 2-{2-Amino-5-[3-(3-pyridin-3-yl-ureido)-phenylethynyl]-pyrimidin-4-yl}-1-methyl-4-oxo-1,4,6,7-tetrahydro-pyrrolo[3,2-c]pyridine-5-carboxylic acid tert-butyl ester

¹H NMR (401 MHz, DMSO-*d*₆) δ ppm 1.46 (s, 9 H) 3.00 (t, J=6.23 Hz, 2 H) 3.85 (s, 3 H) 4.01 (t, J=6.23 Hz, 2 H) 7.04 - 7.17 (m, 3 H) 7.27 - 7.38 (m, 2 H) 7.39 - 7.44 (m, 1 H) 7.50 (s, 1 H) 7.64 (s, 1 H) 7.95 (d, 1 H) 8.21 (br. s., 1 H) 8.49 (s, 1 H) 8.62 (br. s., 1 H) 8.90 (d, J=6.47 Hz, 2 H).

### 2-(2-Amino-5-{3-[3-(2,6-dichloro-pyridin-4-yl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (401 MHz, DMSO-*d*₆) δ ppm 1.44 (s, 9 H) 3.00 (t, J=6.35 Hz, 2 H) 3.85 (s, 3 H) 4.01 (t, J=6.23 Hz, 2 H) 7.10 - 7.18 (m, 3 H) 7.31 - 7.38 (m, 1 H) 7.38 - 7.42 (m, 1 H) 7.49 (s, 1 H) 7.55 - 7.57 (m, 2 H) 7.65 (t, J=1.65 Hz, 1 H) 8.49 (s, 1 H) 9.23 (s, 1 H) 9.59 (s, 1 H).

### 2-(2-Amino-5-{3-[3-(3-trifluoromethyl-phenyl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (401 MHz, DMSO-*d*₆) δ ppm 1.45 (s, 9 H) 3.00 (t, J=6.29 Hz, 2 H) 3.85 (s, 3 H) 4.00 (t, J=6.29 Hz, 2 H) 7.10 (dt, J=7.66, 1.24 Hz, 2 H) 7.13 (s, 2 H) 7.26 - 7.37 (m, 2 H) 7.36 - 7.44 (m, 1 H) 7.47 - 7.55 (m, 2 H) 7.57 (s, 1 H) 7.66 (t, J=1.71 Hz, 1 H) 8.00 (s, 1 H) 8.49 (s, 1 H) 8.88 (s, 1 H) 9.06 (s, 1 H).

### 2-(2-Amino-5-{3-[3-(4-chloro-3-trifluoromethyl-phenyl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (401 MHz, DMSO-*d*₆) δ ppm 1.44 (s, 9 H) 3.00 (t, J=6.23 Hz, 2 H) 3.84 (s, 3 H) 4.00 (t, J=6.35 Hz, 2 H) 7.10 (dt, J=7.63, 1.31 Hz, 1 H) 7.13 (s, 2 H) 7.27 - 7.37 (m, 1 H) 7.37 - 7.42 (m, 1 H) 7.48 - 7.51 (m, 1 H) 7.57 - 7.68 (m, 2 H) 8.10 (d, J=2.32 Hz, 1 H) 8.49 (s, 1 H) 8.97 (s, 1 H) 9.23 (s, 1 H).

### 2-(2-Amino-5-{3-[3-(4-fluoro-phenyl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (401 MHz, DMSO-*d*₆) δ ppm 1.42 (s, 9 H) 2.97 (t, J=6.35 Hz, 2 H) 3.81 (s, 3 H) 3.98 (t, J=6.35 Hz, 2 H) 6.96 - 7.13 (m, 5 H) 7.22 - 7.30 (m, 1 H) 7.32 - 7.38 (m, 1 H) 7.41 - 7.45 (m, 2 H) 7.46 (s, 1 H) 7.56 - 7.65 (m, 1 H) 8.46 (s, 1 H) 8.69 (s, 1 H) 8.72 (s, 1 H).

### 2-(2-Amino-5-{3-[3-(3-fluoro-phenyl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (401 MHz, DMSO-*d*₆) δ ppm 1.45 (s, 9 H) 3.00 (t, J=6.16 Hz, 2 H) 3.85 (s, 3 H) 4.01 (t, J=6.10 Hz, 2 H) 6.79 (td, J=8.51, 2.50 Hz, 1 H) 7.02 - 7.16 (m, 4 H) 7.23 - 7.34 (m, 1 H) 7.36 - 7.42 (m, 1 H) 7.43 - 7.54 (m, 2 H) 7.64 (s, 1 H) 8.49 (s, 1 H) 8.82 (s, 1 H) 8.92 (s, 1 H).

### 2-(2-Amino-5-{3-[3-(4-trifluoromethyl-phenyl)-thioureido]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (401 MHz, DMSO-*d*₆) δ ppm 1.46 (s, 9 H) 2.99 (t, *J*=6.29 Hz, 2 H) 3.84 (s, 3 H) 3.97 - 4.02 (m, 2 H) 7.14 (s, 2 H) 7.25 (dt, *J*=7.72, 1.27 Hz, 1 H) 7.38 (t, *J*=7.87 Hz, 1 H) 7.46 - 7.50 (m, 1 H) 7.58 (t, *J*=1.83 Hz, 1 H) 7.65 - 7.77 (m, 4 H) 8.48 (s, 1 H) 10.09 (s, 1 H) 10.18 (s, 1 H).

### 2-(2-Amino-5-{3-[3-(4-trifluoromethyl-phenyl)-ureido]-phenylethynyl}-pyrimidin-4-yl)-1-(2-tert-butoxycarbonylamino-ethyl)-4-oxo-1,4,6,7-tetrahydro-pyrrolo[3,2-c]pyridine-5-carboxylic acid tert-butyl ester

(ESI) *m*/*z* 755 [(M + H)⁺]. HRMS (ESI) calculated for C₃₉H₄₂F₃N₈O₆ [(M + H)⁺] 775.3174; found 775.3189.

### 2-(2-Amino-5-{4-[3-(4-trifluoromethyl-phenyl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester.

(ESI) *m*/*z* 646 [(M + H)⁺]. HRMS (ESI) calculated for C₃₃H₃₁F₃N₇O₄ [(M + H)⁺] 646.2384; found 646.2369.

### Preparation 35

### 2-(2-Amino-5-{3-[3-(2-diethylamino-ethyl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester

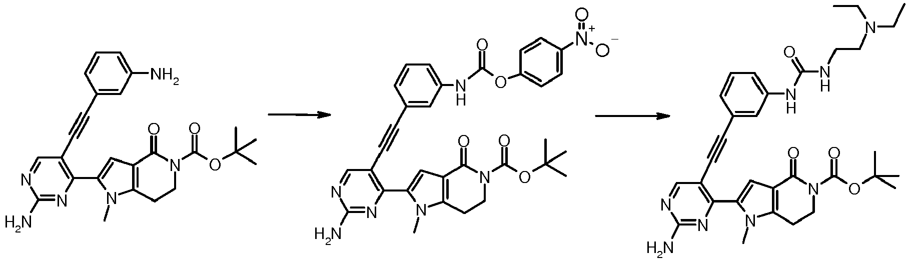

### Step 1. 2-{2-Amino-5-[3-(4-nitro-phenoxycarbonylamino)-phenylethynyl]-pyrimidin-4-yl}-1-methyl-4-oxo-1,4,6,7-tetrahydro-pyrrolo[3,2-c]pyridine-5-carboxylic acid tert-butyl ester

2-[2-Amino-5-(3-amino-phenylethynyl)-pyrimidin-4-yl]-1-methyl-4-oxo-1,4,6,7-tetrahydro-pyrrolo[3,2-c]pyridine-5-carboxylic acid tert-butyl ester (0.52 g, 1.13 mmol) was suspended in DCM (10 mL) then pyridine (0.91 mL, 11.34 mmol) and 4-nitrophenyl chloroformate (0.46 g, 2.27 mmol) were added and let under stirring. After 2.5 h the mixture was diluted with DCM, washed with water and brine, dried over anhydrous Na₂SO₄ and evaporated under vacuum. The crude was treated with a reduced volume of DCM and Hex and the resulting solid was filtered (0.61 g) and used in the next step without further purification.

### Step 2. 2-(2-Amino-5-{3-[3-(2-diethylamino-ethyl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester

A suspension of 2-{2-amino-5-[3-(4-nitro-phenoxycarbonylamino)-phenylethynyl]-pyrimidin-4-yl}-1-methyl-4-oxo-1,4,6,7-tetrahydro-pyrrolo[3,2-c]pyridine-5-carboxylic acid tert-butyl ester (0.11 g, 0.18 mmol) and *N,N-*diethylethylenediamine (0.04 mL, 0.27 mmol) in MeCN (5 mL) and DMSO (0.5 mL) was stirred at r.t. for 2 h following the reaction by HPLC-MS and TLC (DCM/MeOH 95/5). The mixture was poured in water and extracted with DCM (3 x 10 mL). The organic layer was dried over anhydrous Na₂SO₄ and taken to dryness under vacuo. After purification by flash column chromatography (DCM/MeOH 90/10 to 80/20), the product was isolated as white solid (21.7 mg, 23 %).
¹H NMR (401 MHz, DMSO-*d*₆) δ ppm 0.97 (t, J=7.08 Hz, 6 H) 1.45 (s, 9 H) 2.42 - 2.49 (m, 6 H) 2.99 (t, J=6.35 Hz, 2 H) 3.13 (q, J=6.23 Hz, 2 H) 3.84 (s, 3 H) 4.00 (t, J=6.16 Hz, 2 H) 6.09 (t, J=5.49 Hz, 1 H) 6.98 (d, J=7.57 Hz, 1 H) 7.11 (s, 2 H) 7.20 - 7.27 (m, 1 H) 7.29 - 7.34 (m, 1 H) 7.46 - 7.50 (m, 1 H) 7.58 (s, 1 H) 8.47 (s, 1 H) 8.77 (s, 1 H).

According to this same methodology, but employing suitable substituted derivatives, the following intermediates were prepared:

### 2-[2-Amino-5-(3-{3-[3-(4-ethyl-piperazin-1-ylmethyl)-4-trifluoromethyl-phenyl]-ureido}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (401 MHz, DMSO-*d*₆) δ ppm 0.99 (t, J=7.14 Hz, 3 H) 1.44 (s, 9 H) 2.27 - 2.47 (m, 10 H) 3.00 (t, J=6.23 Hz, 2 H) 3.56 (s, 2 H) 3.84 (s, 3 H) 4.01 (t, J=6.23 Hz, 2 H) 7.08 - 7.11 (m, 1 H) 7.13 (s, 2 H) 7.30 - 7.36 (m, 1 H) 7.37 - 7.42 (m, 1 H) 7.48 - 7.51 (m, 2 H) 7.57 - 7.61 (m, 1 H) 7.63 (t, J=1.71 Hz, 1 H) 7.64 - 7.72 (m, 3 H) 8.49 - 8.50 (m, 1 H) 8.79 - 8.85 (m, 1 H) 9.16 (s, 1 H).

### 2-(2-Amino-5-{3-[3-(4-tert-butoxycarbonylamino-cyclohexyl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (401 MHz, DMSO-*d*₆) δ ppm 1.38 (s, 9 H) 1.48 (br. s., 9 H) 1.72 - 1.90 (m, 8 H) 2.99 (t, J=6.35 Hz, 2 H) 3.84 (s, 3 H) 4.01 (t, J=6.29 Hz, 2 H) 6.06 (d, J=7.69 Hz, 1 H) 6.69 (d, J=7.69 Hz, 1 H) 6.98 (dt, J=7.63, 1.19 Hz, 1 H) 7.11 (s, 2 H) 7.23 (t, J=7.87 Hz, 1 H) 7.29 - 7.34 (m, 1 H) 7.48 (s, 1 H) 7.54 (t, J=1.83 Hz, 1 H) 8.37 (s, 1 H) 8.46 (s, 1 H).

### 2-[2-Amino-5-(3-{3-[4-(4-methyl-piperazin-1-ylmethyl)-phenyl]-ureido}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 690 [(M + H)⁺]. HRMS (ESI) calculated for C₃₈H₄₄N₉O₄ [(M + H)⁺] 690.3511; found 690.3532.

### 2-(2-Amino-5-{3-[3-(4-morpholin-4-ylmethyl-phenyl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester

(ESI) *mlz* 677 [(M + H)⁺]. HRMS (ESI) calculated for C₃₇H₄₁N₈O₅ [(M + H)⁺] 677.3194; found 677.3198.

### 2-[2-Amino-5-(3-{3-[3-(4-methyl-piperazin-1-ylmethyl)-phenyl]-ureido}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 690 [(M + H)⁺]. HRMS (ESI) calculated for C₃₈H₄₄N₉O₄ [(M + H)⁺] 690.3511; found 690.3502.

### 2-(2-Amino-5-{3-[3-(3-morpholin-4-ylmethyl-phenyl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester

(ESI) *m*/*z* 677 [(M + H)⁺]. HRMS (ESI) calculated for C₃₇H₄₁N₈O₅ [(M + H)⁺] 677.3194; found 677.3189.

### 2-(2-Amino-5-{3-[3-(3-trifluoromethyl-benzyl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester

(ESI) *m*/*z* 660 [(M + H)⁺]. HRMS (ESI) calculated for C₃₄H₃₃F₃N₇O₄ [(M + H)⁺] 660.2541; found 660.2535.

### 2-(2-Amino-5-{3-[3-(5-trifluoromethyl-[1,3,4]thiadiazol-2-yl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester

(ESI) *mlz* 654 [(M + H)⁺]. HRMS (ESI) calculated for C₂₉H₂₇F₃N₉O₄S [(M + H)⁺] 654.1853; found 654.1860.

### 2-(2-Amino-5-{3-[3-(2,4-dimethoxy-benzyl)-3-(4-trifluoromethyl-cyclohexyl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester (cis isomer)

(ESI) *m*/*z* 802 [(M + H)⁺]. HRMS (ESI) calculated for C₄₂H₄₇F₃N₇O₆ [(M + H)⁺] 802.3534; found 802.3526.

### 2-(2-Amino-5-{3-[3-(2,4-dimethoxy-benzyl)-3-(4-trifluoromethyl-cyclohexyl)-ureido]-phenylethynyl)-pyrimidin-4-yl)-1-methyl-4-oxo-1,4,6,7-tetrahydro-pyrrolo[3,2-c]pyridine-5-carboxylic acid tert-butyl ester (trans isomer)

(ESI) *m*/*z* 802 [(M + H)⁺]. HRMS (ESI) calculated for C₄₂H₄₇F₃N₇O₆ [(M + H)⁺] 802.3534; found 802.3540.

### 2-[2-Amino-5-(3-{3-[4-(4-ethyl-piperazin-1-ylmethyl)-3-trifluoromethyl-phenyl]-ureido}-phenylethynyl)-pyrimidin-4-yl]-1-(2-tert-butoxycarbonylamino-ethyl)-4-oxo-1,4,6,7-tetrahydro-pyrrolo[3,2-c]pyridine-5-carboxylic acid tert-butyl ester

(ESI) *m*/*z* 901 [(M + H)⁺]. HRMS (ESI) calculated for C₄₆H₅₆F₃N₁₀O₆ [(M + H)⁺] 901.4331; found 901.4312.

### Preparation 36

### 2-[2-Amino-5-(3-{3-[4-(4-ethyl-piperazin-1-ylmethyl)-3-trifluoromethyl-phenyl]-ureido}-phenylethynyl)-pyrimidin-4-yl]-1-methyl-4-oxo-1,4,6,7-tetrahydro-pyrrolo[3,2-c]pyridine-5-carboxylic acid tert-butyl ester

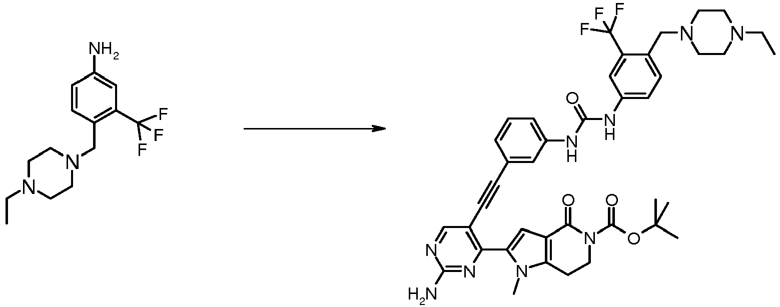

To a suspension of triphosgene (17.2 mg, 0.058 mmol) and Na₂CO₃ (37 mg, 0.348 mmol) in DCM (2 mL), kept at 0°C under argon, 4-(4-ethyl-piperazin-1-ylmethyl)-3-trifluoromethyl-phenylamine (50 mg, 0.174 mmol) was added. The reaction was monitored by HPLC (following the formation of 4-ethyl-piperazine-1-carboxylic acid [4-(4-ethylpiperazin-1-ylmethyl)-3-trifluoromethyl-phenyl]-amide by treating a sample of the reaction mixture with *N-*ethylpiperazine). After 1 h a solution of 2-[2-amino-5-(3-amino-phenylethynyl)-pyrimidin-4-yl]-1-methyl-1,5,6,7-tetrahydro-pyrrolo[3,2-c]pyridin-4-one (104 mg, 0.226 mmol) in DMA (1.5 mL) was added and the reaction was let under stirring overnight. The mixture was diluted with DCM, washed with water (3 x 10 mL), dried over anhydrous Na₂SO₄ and taken to dryness under vacuum. Purification by flash column chromatography (DCM/MeOH 95/5) afforded the product as yellow solid (70 mg, 52%).
¹H NMR (401 MHz, DMSO-*d*₆) δ ppm 0.97 (t, J=7.20 Hz, 3 H) 1.45 (s, 9 H) 2.23 - 2.45 (m, 10 H) 3.00 (t, J=6.3 Hz, 2 H) 3.51 (s, 2 H) 3.84 (s, 3 H) 4.00 (t, J=6.16 Hz, 2 H) 7.06 (d, J=7.69 Hz, 1 H) 7.11 (s, 2 H) 7.25 - 7.31 (m, 1 H) 7.43 (d, J=8.06 Hz, 1 H) 7.49 (s, 1 H) 7.56 - 7.65 (m, 2 H) 7.72 (t, J=1.71 Hz, 1 H) 8.01 (d, J=1.71 Hz, 1 H) 8.50 (s, 1 H), 10.00 (bs, 1 H), 10.19 (bs, 1 H).

According to this same methodology, but employing suitable substituted derivatives, the following intermediates were prepared:

### 2-[2-Amino-5-(3-{3-[4-(4-dimethylamino-piperidin-1-ylmethyl)-3-trifluoromethyl-phenyl]-ureido}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 786 [(M + H)⁺]. HRMS (ESI) calculated for C₄₁H₄₇F₃N₉O₄ [(M + H)⁺] 786.3698; found 786.3711.

### 2-[2-Amino-5-(3-{3-[4-(4-methyl-piperazin-1-ylmethyl)-3-trifluoromethyl-phenyl]-ureido}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 758 [(M + H)⁺]. HRMS (ESI) calculated for C₃₉H₄₃F₃N₉O₄ [(M + H)⁺] 758.3385; found 758.3398.

### 2-[2-Amino-5-(3-{3-[4-(4-methyl-piperazin-1-yl)-phenyl]-ureido}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (401 MHz, DMSO-*d*₆) δ ppm 1.46 (s, 9 H) 2.21 (s, 3 H) 2.41 - 2.47 (m, 4 H) 3.00 (t, J=6.23 Hz, 2 H) 3.02 - 3.09 (m, 4 H) 3.84 (s, 3 H) 4.01 (t, J=6.29 Hz, 2 H) 6.82 - 6.92 (m, 2 H) 7.05 (d, J=7.57 Hz, 1 H) 7.12 (s, 2 H) 7.24 - 7.32 (m, 2 H) 7.34 - 7.42 (m, 1 H) 7.49 (s, 1 H) 7.62 (t, J=1.65 Hz, 1 H) 8.41 (s, 1 H) 8.48 (s, 1 H) 8.64 (s, 1 H).

### 2-[2-Amino-5-(3-{3-[3-(4-methyl-piperazin-1-yl)-phenyl]-ureido}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 676 [(M + H)⁺]. HRMS (ESI) calculated for C₃₇H₄₂N₉O₄ [(M + H)⁺] 676.3354; found 676.3341.

### 2-[2-Amino-5-(3-{3-[4-(4-methyl-piperazin-1-yl)-3-trifluoromethyl-phenyl]-ureido}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (401 MHz, DMSO-*d*₆) δ ppm 1.44 (s, 9 H) 2.22 (s, 3 H) 2.33 - 2.47 (m, 4 H) 2.77 - 2.85 (m, 4 H) 3.00 (t, J=6.23 Hz, 2 H) 3.84 (s, 3 H) 4.00 (t, J=6.35 Hz, 2 H) 7.09 (d, J=7.57 Hz, 1 H) 7.12 (s, 2 H) 7.31 (t, J=7.81 Hz, 1 H) 7.36 - 7.41 (m, 1 H) 7.44 - 7.53 (m, 3 H) 7.58 (dd, J=8.61, 2.50 Hz, 1 H) 7.65 (s, 1 H) 7.89 (d, J=2.44 Hz, 1 H) 8.49 (s, 1 H) 8.82 (s, 1 H) 8.95 (s, 1 H).

### 2-[2-Amino-5-(3-{3-[3-(4-ethyl-piperazine-1-carbonyl)-5-trifluoromethyl-phenyl]-ureido}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 786 [(M + H)⁺]. HRMS (ESI) calculated for C₄₀H₄₃F₃N₉O₅ [(M + H)⁺] 786.3334; found 786.3356.

### Preparation 37

### 2-[2-Amino-5-(3-{3-[3-(4-ethyl-piperazin-1-ylmethyl)-5-trifluoromethyl-phenyl]-ureido}-phenylethynyl)-pyrimidin-4-yl]-1-methyl-4-oxo-1,4,6,7-tetrahydro-pyrrolo[3,2-c]pyridine-5-carboxylic acid tert-butyl ester

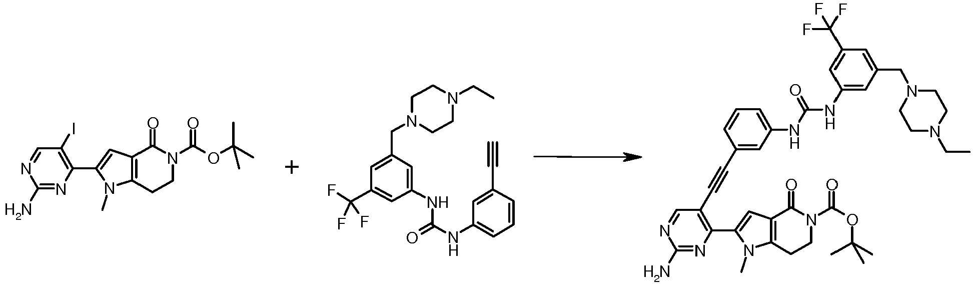

To a solution of 2-(2-amino-5-iodo-pyrimidin-4-yl)-1-methyl-1,5,6,7-tetrahydro-pyrrolo[3,2-c]pyridin-4-one (25 mg, 0.054 mmol), 1-[3-(4-ethyl-piperazin-1-ylmethyl)-5-trifluoromethyl-phenyl]-3-(3-ethynyl-phenyl)-urea (30 mg, 0.070 mmol) and CuI (0.5 mg, 0.003 mmol) in degassed MeCN (2.5 mL), TEA (0.075 mL, 0.54 mmol) and PdCl₂(PPh₃)₂ (2 mg, 0.003 mmol) were added. The resulting mixture was degassed three times back filling with argon each time and then heated at 80 °C for 4.5 h. The mixture was diluted with DCM and washed with NH₃, water and brine. The organic layer was dried over anhydrous Na₂SO₄, filtered and concentrated under reduced pressure. The residue was purified by flash column chromatography (DCM/MeOH 95/5) affording the title product (15 mg, 36%).
¹H NMR (401 MHz, DMSO-*d*₆) δ ppm 0.98 (t, J=7.20 Hz, 3 H) 1.44 (s, 9 H) 2.28 - 2.34 (m, 2 H) 2.39 (br. s., 8 H) 3.00 (t, J=6.29 Hz, 2 H) 3.51 (s, 2 H) 3.85 (s, 3 H) 4.00 (t, J=6.35 Hz, 2 H) 7.10 (dt, J=7.60, 1.27 Hz, 1 H) 7.13 (s, 2 H) 7.22 (s, 1 H) 7.32 (t, J=7.87 Hz, 1 H) 7.38 - 7.43 (m, 1 H) 7.50 (s, 1 H) 7.53 (s, 1 H) 7.65 (t, J=1.71 Hz, 1 H) 7.89 (s, 1 H) 8.49 (s, 1 H) 8.83 (s, 1 H) 9.09 (s, 1 H).

According to this same methodology, but employing suitable substituted derivatives, the following intermediates were prepared:

### 2-[2-Amino-5-(4-{3-[4-(4-ethyl-piperazin-1-ylmethyl)-3-trifluoromethyl-phenyl]-ureido}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 772 [(M + H)⁺]. HRMS (ESI) calculated for C₄₀H₄₅F₃N₉O₄ [(M + H)⁺] 772.3541; found 772.3523.

### 2-(2-Amino-5-{3-[3-(4-trifluoromethyl-phenyl)-ureido]-phenylethynyl}-pyrimidin-4-yl)-4-oxo-1,4,6,7-tetrahydro-pyrrolo[3,2-c]pyridine-5-carboxylic acid tert-butyl ester

(ESI) *m*/*z* 632 [(M + H)⁺]. HRMS (ESI) calculated for C₃₂H₂₉F₃N₇O₄ [(M + H)⁺] 632.2228; found 632.2236.

### 1-[4-(4-Ethyl-piperazin-1-ylmethyl)-3-trifluoromethyl-phenyl]-3-{3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-urea (cmpd 41)

¹H NMR (600 MHz, DMSO-*d*₆) δ ppm 0.98 (t, J=7.23 Hz, 3 H) 2.31 (q, J=7.02 Hz, 3 H) 2.33 - 2.46 (m, 6 H) 2.88 (d, J=4.76 Hz, 3 H) 2.89 - 2.96 (m, 2 H) 3.42 - 3.47 (m, 3 H) 3.52 (s, 2 H) 3.80 - 3.93 (m, 3 H) 7.11 (d, J=7.14 Hz, 2 H) 7.31 (t, J=7.88 Hz, 1 H) 7.41 (d, J=8.06 Hz, 1 H) 7.48 - 7.55 (m, 2 H) 7.56 (dd, J=8.52, 1.74 Hz, 1 H) 7.60 - 7.64 (m, 2 H) 7.96 (d, J=2.01 Hz, 1 H) 8.46 (br. S, 1 H), 8.85 (s, 1 H) 9.04 (s, 1 H).
(ESI) *m*/*z* 686 [(M + H)⁺]. HRMS (ESI) calculated for C₃₆H₃₉F₃N₉O₂ [(M + H)⁺] 686.3174; found 686.3187.

### 1-{3-[2-Methylamino-4-(1-methyl-4-oxo-4,5,6,7-tetrahydro-1H-pyrrolo[3,2-c]pyridin-2-yl)-pyrimidin-5-ylethynyl]-phenyl}-3-[4-(4-methyl-piperazin-1-ylmethyl)-3-trifluoromethyl-phenyl]-urea (cmpd 42)

¹H NMR (600 MHz, DMSO-*d*₆) δ ppm 2.74 (br. s., 3 H) 2.83 - 2.97 (m, 13 H) 3.42 - 3.48 (m, 2 H) 3.62 (s, 2 H) 3.90 (br. s., 3 H) 7.08 - 7.14 (m, 2 H) 7.32 (t, J=7.97 Hz, 1 H) 7.41 (d, J=8.24 Hz, 1 H) 7.45 - 7.57 (m, 2 H) 7.58 - 7.67 (m, 3 H) 7.95 (s, 1 H) 8.50 (br. s, 1 H) 8.87 (s, 1 H) 9.07 (s, 1 H).
(ESI) *m*/*z* 672 [(M + H)⁺]. HRMS (ESI) calculated for C₃₅H₃₇F₃N₉O₂ [(M + H)⁺] 6723017; found 672.3009.

### 1-[4-(4-Ethyl-piperazin-1-ylmethyl)-3-trifluoromethyl-phenyl]-3-{3-[2-(2-hydroxy-ethylamino)-4-(1-methyl-4-oxo-4,5,6,7-tetrahydro-1H-pyrrolo[3,2-c]pyridin-2-yl)-pyrimidin-5-ylethynyl]-phenyl}-urea (cmpd 43)

¹H NMR (600 MHz, DMSO-*d*₆) δ ppm 0.98 (t, J=7.14 Hz, 3 H) 2.26 - 2.34 (m, 2 H) 2.34 - 2.46 (m, 4 H) 2.90 (t, J=6.87 Hz, 2 H) 3.39 - 3.47 (m, 4 H) 3.50 - 3.57 (m, 4 H) 3.86 (s, 3 H) 4.72 (t, J=5.31 Hz, 1 H) 7.05 - 7.16 (m, 2 H) 7.31 (t, J=7.97 Hz, 1 H) 7.41 (d, J=8.42 Hz, 1 H) 7.44 - 7.54 (m, 2 H) 7.56 (dd, J=8.43, 1.83 Hz, 1 H) 7.60 - 7.67 (m, 2 H) 7.96 (d, J=2.01 Hz, 1 H) 8.47 (br. s., 1 H) 8.84 (s, 1 H) 9.03 (s, 1 H).
(ESI) *m*/*z* 716 [(M + H)⁺]. HRMS (ESI) calculated for C₃₅H₃₇F₃N₉O₂ [(M + H)⁺] 716.3279; found 716.3265.

### Preparation 38

### 1-Methyl-4-oxo-2-(2-[(pyridin-4-ylmethyl)-amino]-5-{3-[3-(4-trifluoromethyl-phenyl)-ureido]-phenylethynyl}-pyrimidin-4-yl)-1,4,6,7-tetrahydro-pyrrolo[3,2-c]pyridine-5-carboxylic acid tert-butyl ester

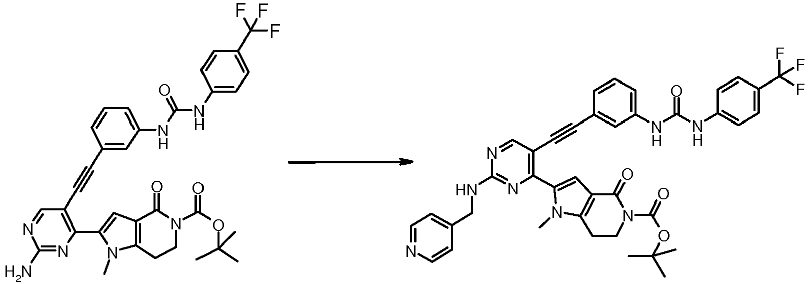

A suspension of 2-(2-amino-5-{3-[3-(4-trifluoromethyl-phenyl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester (80 mg, 0.124 mmol) in dry DMF (0.65 mL), pyridine-4-carbaldehyde (0.036 mL, 0.382 mmol) and TFA (0.062 mL, 0.805 mmol) was let under stirring at r.t. for 30 min. NaBH(OAc)₃ (94 mg, 0.443 mmol) was then added and the reaction let overnight. The mixture was diluted with EtOAc, poured into a solution of water/NaOH 1N (1:1) and extracted with EtOAc (2 x 10 mL). The organic layer was furthermore washed with water and brine, dried over anhydrous Na₂SO₄, filtered and concentrated under reduced pressure. The product was purified by flash column chromatography (EtOAc/MeOH 98/2) and isolated as white solid (12.5 mg, 14%).
¹H NMR (401 MHz, DMSO-*d*₆) δ ppm 1.44 (s, 9 H) 2.87 - 3.09 (m, 2 H) 3.46 (br. s., 3 H) 3.84 - 4.06 (m, 2 H) 4.60 (d, J=6.35 Hz, 2 H) 7.10 (d, J=7.57 Hz, 1 H) 7.28 - 7.34 (m, 3 H) 7.37 - 7.44 (m, 1 H) 7.54 (s, 1 H) 7.58 - 7.71 (m, 5 H) 8.31 (br. s., 1 H) 8.49 (d, J=5.86 Hz, 2 H) 8.58 (br. s., 1 H) 8.94 (s, 1 H) 9.17 (s, 1 H).

According to this same methodology, but employing suitable substituted derivatives, the following intermediates were prepared:

### 2-(2-(1-tert-Butoxycarbonyl-piperidin-4-ylamino)-5-{3-[3-(4-trifluoromethyl-phenyl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (401 MHz, DMSO-d6) δ ppm 1.41 (s, 9 H) 1.45 (s, 9 H) 1.35 - 1.48 (m, 2 H) 1.81 - 1.92 (m, 2 H) 2.87 (br. s., 2 H) 3.01 (t, J=6.35 Hz, 2 H) 3.85 (s, 3 H) 3.87 - 3.98 (m, 3 H) 4.02 (t, J=6.71 Hz, 2 H) 7.10 (d, J=7.45 Hz, 1 H) 7.33 (t, J=8.06 Hz, 1 H) 7.39 (d, J=8.30 Hz, 1 H) 7.58 - 7.74 (m, 7 H) 8.52 (br. s., 1 H) 8.91 (s, 1 H) 9.14 (s, 1 H).

### 2-(2-(1-tert-Butoxycarbonyl-piperidin-4-ylamino)-5-{3-[3-(4-chloro-3-trifluoromethyl-phenyl)-ureido]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (401 MHz, DMSO-*d*₆) δ ppm 1.41 (s, 9 H) 1.44 (s, 9 H) 1.36 - 1.47 (m, 2 H) 1.86 (d, J=11.47 Hz, 2 H) 2.87 (br. s., 2 H) 3.00 (t, J=6.29 Hz, 2 H) 3.85 (s, 3 H) 3.87 - 3.98 (m, 3 H) 4.01 (t, J=6.16 Hz, 2 H) 7.11 (d, J=7.45 Hz, 1 H) 7.33 (t, J=7.9 Hz, 1 H) 7.37 - 7.42 (m, 1 H) 7.50 - 7.76 (m, 5 H) 8.10 (d, J=2.08 Hz, 1 H) 8.52 (br. s., 1 H) 8.93 (s, 1 H) 9.18 (s, 1 H).

### Example 3

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-phenyl-urea hydrochloride (cmpd 1)

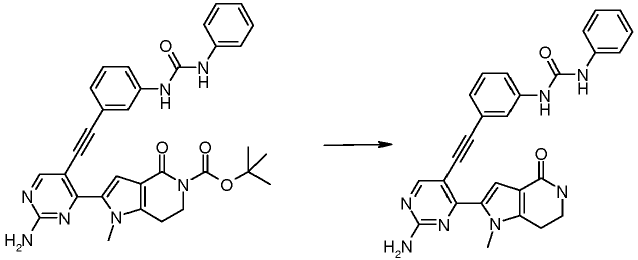

2-{2-Amino-5-[3-(3-phenyl-ureido)-phenylethynyl]-pyrimidin-4-yl}-1-methyl-4-oxo-1,4,6,7-tetrahydro-pyrrolo[3,2-c] pyridine-5-carboxylic acid tert-butyl ester (90 mg, 0.156 mmol) was suspended in dioxane (3 mL) and treated with 4 M HCl in dioxane (3 mL). After 3 h the solvent was removed, the compound was triturated with Et₂O and isolated by filtration as yellow solid (78 mg, quantitative yield).
¹H NMR (401 MHz, DMSO-*d₆*) δ ppm 2.91 (t, J=6.77 Hz, 2 H) 3.52 (m, 2 H overlapped by water signal) 3.86 (s, 3 H) 6.94 - 7.00 (m, 1 H) 7.10 (dt, J=7.57, 1.28 Hz, 1 H) 7.12 (br. s., 1 H) 7.24 - 7.34 (m, 3 H) 7.39 - 7.43 (m, 1 H) 7.45 (dd, J=8.67, 1.10 Hz, 2 H) 7.57 (s, 1 H) 7.64 (t, J=1.71 Hz, 1 H) 8.51 (s, 1 H) 8.87 (s, 1 H) 8.96 (s, 1 H).
(ESI) *mlz* 478 [(M + H)⁺]. HRMS (ESI) calculated for C₂₇H₂₅ClN₇O₂ [(M + H)⁺] 478.1986; found 478.1988.

According to this same methodology, but employing suitable substituted derivatives, the following compounds were prepared:

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-p-tolyl-urea hydrochloride (cmpd 2)

¹H NMR (401 MHz, DMSO-*d₆*) δ ppm 2.24 (s, 3 H) 2.91 (t, J=6.84 Hz, 2 H) 3.42 - 3.47 (m, 2 H overlapped by water signal) 3.86 (s, 3 H) 7.05 - 7.12 (m, 3 H) 7.18 (br. s., 1 H) 7.28 - 7.31 (m, 1 H) 7.32 - 7.35 (m, 2 H) 7.40 (ddd, J=8.21, 2.17, 0.98 Hz, 1 H) 7.58 (s, 1 H) 7.64 (t, J=1.71 Hz, 1 H) 8.51 (s, 1 H) 8.77 (s, 1 H) 8.93 (s, 1 H).
(ESI) *m*/*z* 492 [(M + H)⁺]. HRMS (ESI) calculated for C₂₈H₂₇ClN₇O₂ [(M + H)⁺] 492.2143; found 492.2143.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(4-dimethylamino-phenyl)-urea dihydrochloride (cmpd 3)

¹H NMR (401 MHz, DMSO-*d₆*) δ ppm 2.92 (t, J=6.60 Hz, 2 H) 3.11 (s, 6 H) 3.39 (m, 2 H overlapped by water signal) 3.86 (s, 3 H) 7.11 (d, J=7.57 Hz, 1 H) 7.16 (br. s., 1 H) 7.32 (t, J=7.87 Hz, 1 H) 7.40 - 7.45 (m, 1 H) 7.55 (s, 1 H) 7.58 (br. s., 4 H) 7.64 (s, 1 H) 8.50 (s, 1 H) 9.21 (s, 1 H) 9.32 (br. s., 1 H).
(ESI) *m*/*z* 521 [(M + H)⁺]. HRMS (ESI) calculated for C₂₉H₃₁Cl₂N₈O₂ [(M + H)⁺] 521.2408; found 521.2405.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-pyridin-3-yl-urea (cmpd 4)

¹H NMR (401 MHz, DMSO-*d₆*) δ ppm 2.89 (t, J=6.90 Hz, 2 H) 3.45 (td, J=6.90, 2.56 Hz, 2 H) 3.85 (s, 3 H) 7.07 (s, 2 H) 7.08 - 7.13 (m, 2 H) 7.26 - 7.36 (m, 2 H) 7.41 (dd, J=2.20, 0.98 Hz, 1 H) 7.44 (s, 1 H) 7.61 (t, J=1.71 Hz, 1 H) 7.90 - 7.97 (m, 1 H) 8.20 (dd, J=4.70, 1.40 Hz, 1 H) 8.46 (s, 1 H) 8.61 (d, J=2.44 Hz, 1 H) 8.86 - 8.94 (m, 2 H).
(ESI) *m*/*z* 479 [(M + H)⁺]. HRMS (ESI) calculated for C₂₆H₂₃N₃O₂ [(M + H)⁺] 479.1939; found 479.1931.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(2,6-dichloro-pyridin-4-yl)-urea (cmpd 5)

¹H NMR (401 MHz, DMSO-*d₆*) δ ppm 2.90 (t, J=6.84 Hz, 2 H) 3.46 (td, J=6.90, 2.44 Hz, 2 H) 3.86 (s, 3 H) 7.08 (s, 2 H) 7.11 (br. s., 1 H) 7.16 (dt, J=7.60, 1.27 Hz, 1 H) 7.35 (t, J=7.93 Hz, 1 H) 7.41 - 7.45 (m, 2 H) 7.56 (s, 2 H) 7.63 (t, J=1.71 Hz, 1 H) 8.47 (s, 1 H) 9.40 (s, 1 H) 9.88 (s, 1 H).
(ESI) *m*/*z* 547 [(M + H)⁺]. HRMS (ESI) calculated for C₂₆H₂₁Cl₂N₈O₂ [(M + H)⁺] 547.1159; found 547.1159.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(3-trifluoromethyl-phenyl)-urea (cmpd 6)

¹H NMR (401 MHz, DMSO-*d₆*) δ ppm 2.89 (t, J=6.90 Hz, 2 H) 3.45 (td, J=6.84, 2.56 Hz, 2 H) 3.85 (s, 3 H) 7.06 (s, 2 H) 7.08 - 7.14 (m, 2 H) 7.27 - 7.35 (m, 2 H) 7.38 - 7.44 (m, 1 H) 7.44 (s, 1 H) 7.53 (d, J=7.81 Hz, 1 H) 7.55 - 7.61 (m, 1 H) 7.63 (t, J=1.71 Hz, 1 H) 8.01 (s, 1 H) 8.46 (s, 1 H) 8.89 (s, 1 H) 9.10 (s, 1 H).
(ESI) *m*/*z* 546 [(M + H)⁺]. HRMS (ESI) calculated for C₂₈H₂₃F₃N₇O₂ [(M + H)⁺] 546.1860; found 546.1856.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[3-(4-methyl-piperazin-1-ylmethyl)-phenyl]-urea (cmpd 7)

¹H NMR (401 MHz, DMSO-*d₆*) δ ppm 2.22 (br. s., 3 H) 2.32 - 2.48 (m, 8 H) 2.89 (t, J=6.8 Hz, 2 H) 3.37 - 3.48 (m, 4 H) 3.85 (s, 3 H) 6.90 (d, J=7.6 Hz, 1 H) 7.02 - 7.13 (m, 4 H) 7.22 (t, J=7.8 Hz, 1 H) 7.30 (t, J=7.9 Hz, 2 H) 7.36 - 7.42 (m, 1 H) 7.41 - 7.43 (m, 1 H) 7.44 (s, 1 H) 7.61 (t, J=1.8 Hz, 1 H) 8.46 (s, 1 H) 8.69 - 8.76 (m, 2 H).
(ESI) *m*/*z* 590 [(M + H)⁺]. HRMS (ESI) calculated for C₃₃H₃₆N₉O₂ [(M + H)⁺] 590.2986; found 590.2986.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[4-(4-ethyl-piperazin-1-ylmethyl)-3-trifluoromethyl-phenyl]-urea (cmpd 8)

¹H NMR (600 MHz, DMSO-*d₆*) δ ppm 0.98 (t, J=7.14 Hz, 3 H) 2.30 (q, J=7.27 Hz, 2 H) 2.34 - 2.44 (m, 8 H) 2.89 (t, J=6.78 Hz, 2 H) 3.44 (td, J=6.82, 2.47 Hz, 2 H) 3.53 (s, 2 H) 3.85 (s, 3 H) 7.07 (s, 2 H) 7.09 - 7.13 (m, 2 H) 7.31 (t, J=7.88 Hz, 1 H) 7.41 (dd, J=8.33, 1.19 Hz, 1 H) 7.44 (s, 1 H) 7.56 (dd, J=8.43, 1.83 Hz, 1 H) 7.60 - 7.66 (m, 2 H) 7.96 (d, J=2.01 Hz, 1 H) 8.46 (s, 1 H) 8.84 (s, 1 H) 9.03 (s, 1 H).
(ESI) *m*/*z* 672 [(M + H)⁺]. HRMS (ESI) calculated for C₃₅H₃₇F₃N₉O₂ [(M + H)⁺] 672.3017; found 672.3018.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(4-chloro-3-trifluoromethyl-phenyl)-urea (cmpd 9)

¹H NMR (401 MHz, DMSO-*d₆*) δ ppm 2.89 (t, J=6.84 Hz, 2 H) 3.44 (td, J=6.77, 2.56 Hz, 2 H) 3.85 (s, 3 H) 7.07 (br. s., 2 H) 7.09 - 7.15 (m, 2 H) 7.32 (t, J=7.93 Hz, 1 H) 7.41 (m, 1 H) 7.43 (s, 1 H) 7.56 - 7.72 (m, 3 H) 8.10 (d, J=2.32 Hz, 1 H) 8.46 (s, 1 H) 8.93 (s, 1 H) 9.20 (s, 1 H).
(ESI) *m*/*z* 580 [(M + H)⁺]. HRMS (ESI) calculated for C₂₈H₂₂ClF₃N₇O₂ [(M + H)⁺] 580.1470; found 580.1448.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[4-(4-methyl-piperazin-1-ylmethyl)-phenyl]-urea (cmpd 10)

¹H NMR (401 MHz, DMSO-*d₆*) δ ppm 2.18 (s, 3 H) 2.26 - 2.45 (m, 8 H) 2.89 (t, J=6.8 Hz, 2 H) 3.39 (br. s., 2 H) 3.45 (td, J=6.9, 2.4 Hz, 2 H) 3.85 (s, 3 H) 7.04 - 7.13 (m, 4 H) 7.19 (d, J=8.4 Hz, 2 H) 7.30 (t, J=7.9 Hz, 1 H) 7.35 - 7.42 (m, 3 H) 7.44 (s, 1 H) 7.61 (t, J=1.7 Hz, 1 H) 8.45 (s, 1 H) 8.68 (s, 1 H) 8.74 (s, 1 H).
(ESI) *m*/*z* 590 [(M + H)⁺]. HRMS (ESI) calculated for C₃₃H₃₆N₉O₂ [(M + H)⁺] 590.2987; found 590.2985.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(4-morpholin-4-ylmethyl-phenyl)-urea (cmpd 11)

¹H NMR (401 MHz, DMSO-*d₆*) δ ppm 2.33 (m, 4 H) 2.89 (t, J=6.9 Hz, 2 H) 3.39 (br. s., 2 H) 3.45 (td, J=6.8, 2.4 Hz, 2 H) 3.52 - 3.59 (m, 4 H) 3.85 (s, 3 H) 7.03 - 7.12 (m, 4 H) 7.20 (d, J=8.5 Hz, 2 H) 7.30 (t, J=7.9 Hz, 1 H) 7.40 (d, J=8.5 Hz, 3 H) 7.44 (s, 1 H) 7.60 (t, J=1.8 Hz, 1 H) 8.45 (s, 1 H) 8.67 (s, 1 H) 8.73 (s, 1 H).
(ESI) *m*/*z* 577 [(M + H)⁺]. HRMS (ESI) calculated for C₃₂H₃₃N₈O₃ [(M + H)⁺] 577.2670; found 577.2661.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(3-morpholin-4-ylmethyl-phenyl)-urea (cmpd 12)

¹H NMR (401 MHz, DMSO-*d₆*) δ ppm 2.29 - 2.45 (m, 4 H) 2.89 (t, J=6.8 Hz, 2 H) 3.31 (s, 2 H overlapped by water signal) 3.45 (td, J=6.8, 2.6 Hz, 2 H) 3.59 (br. s., 4 H) 3.85 (s, 3 H) 6.93 (br. s., 1 H) 7.01 - 7.14 (m, 4 H) 7.24 (br. s., 1 H) 7.27 - 7.36 (m, 2 H) 7.37 - 7.42 (m, 1 H) 7.44 (s, 2 H) 7.62 (s, 1 H) 8.46 (s, 1 H) 8.74 (br. s., 2 H).
(ESI) *m*/*z* 577 [(M + H)⁺]. HRMS (ESI) calculated for C₃₂H₃₃N₈O₃ [(M + H)⁺] 577.2670; found 577.2670.

### 1-(4-Chloro-3-trifluoromethyl-phenyl)-3-{3-[4-(1-methyl-4-oxo-4,5,6,7-tetrahydro-1H-pyrrolo[3,2-c]pyridin-2-yl)-2-(piperidin-4-ylamino)-pyrimidin-5-ylethynyl]-phenyl}-urea (cmpd 13)

¹H NMR (401 MHz, DMSO-*d₆*) δ ppm 1.43 - 1.60 (m, 2 H) 1.84 - 1.98 (m, 2 H) 2.67 - 2.75 (m, 2 H) 2.90 (t, J=6.84 Hz, 2 H) 3.06 - 3.13 (m, 2 H) 3.45 (td, J=6.77, 2.32 Hz, 2 H) 3.86 (s, 3 H) 3.89 - 4.02 (m, 1 H) 7.12 (d, J=7.32 Hz, 2 H) 7.32 (t, J=7.87 Hz, 1 H) 7.41 (d, J=8.30 Hz, 1 H) 8.11 (d, J=2.20 Hz, 1 H) 8.50 (s, 1 H) 9.09 (s, 1 H) 9.37 (s, 1 H).
(ESI) *m*/*z* 663 [(M + H)⁺]. HRMS (ESI) calculated for C₃₃H₃₁ClF₃N₈O₂ [(M + H)⁺] 663.2205; found 663.2217.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(2-diethylamino-ethyl)-urea (cmpd 14)

¹H NMR (401 MHz, DMSO-*d₆*) δ ppm 0.97 (t, J=7.14 Hz, 6 H) 2.41 - 2.49 (m, 6 H) 2.62 - 2.71 (m, 1 H) 2.89 (t, J=6.84 Hz, 2 H) 3.14 (q, J=6.27 Hz, 2 H) 3.44 (td, J=6.84, 2.44 Hz, 2 H) 3.84 (s, 3 H) 6.07 (t, J=5.43 Hz, 1 H) 7.00 (dt, J=7.63, 1.25 Hz, 1 H) 7.04 (s, 2 H) 7.10 (t, J=1.95 Hz, 1 H) 7.23 (t, J=7.93 Hz, 1 H) 7.29 - 7.36 (m, 1 H) 7.42 (s, 1 H) 7.56 (t, J=1.77 Hz, 1 H) 8.44 (s, 1 H) 8.77 (s, 1 H).
(ESI) *m*/*z* 501 [(M + H)⁺]. HRMS (ESI) calculated for C₂₇H₃₃N₈O₂ [(M + H)⁺] 501.2721; found 501.2727.

### 1-(4-Amino-cyclohexyl)-3-{3-[2-amino-4-(1-methyl-4-oxo-4,5,6,7-tetrahydro-1H-pyrrolo[3,2-c]pyridin-2-yl)-pyrimidin-5-ylethynyl]-phenyl}-urea (trans isomer) (cmpd 15)

¹H NMR (401 MHz, DMSO-*d₆*) δ ppm 1.16 - 1.48 (m, 4 H) 1.94 (br. s., 4 H) 2.89 (t, J=6.84 Hz, 2 H) 2.98 (br. s., 1 H) 3.44 (td, J=6.77, 2.44 Hz, 2 H) 3.85 (s, 3 H) 6.22 (d, J=7.57 Hz, 1 H) 6.96 - 7.08 (m, 1 H) 7.10 (br. s., 1 H) 7.23 (t, J=7.87 Hz, 1 H) 7.27 (s, 2 H) 7.33 (dd, J=7.75, 1.65 Hz, 1 H) 7.42 (s, 1 H) 7.52 (t, J=1.77 Hz, 1 H) 7.75 - 7.92 (m, 2 H) 8.43 (s, 1 H) 8.57 (s, 1 H).
(ESI) *m*/*z* 499 [(M + H)⁺]. HRMS (ESI) calculated for C₂₇H₃₁N₈O₂ [(M + H)⁺] 499.2565; found 499.2542.

### 1-{3-[2-Amino-4-(4-oxo-4,5,6,7-tetrahydro-1H-pyrrolo[3,2-c]pyridin-2-yl)-pyrimidin-5-ylethynyl]-phenyl}-3-(4-trifluoromethyl-phenyl)-urea (cmpd 16)

¹H NMR (401 MHz, DMSO-*d₆*) δ ppm 2.87 (t, J=6.9 Hz, 2 H) 3.37 - 3.43 (m, 2 H) 6.78 (br. s., 2 H) 7.10 (s, 1 H) 7.18 (d, J=7.8 Hz, 1 H) 7.37 (t, J=7.9 Hz, 1 H) 7.47 - 7.52 (m, 1 H) 7.59 - 7.66 (m, 3 H) 7.66 - 7.72 (m, 3 H) 8.43 (s, 1 H) 9.30 (br. s., 1 H) 9.51 (br. s., 1 H) 11.73 (br. s., 1 H).
(ESI) *m*/*z* 532 [(M + H)⁺]. HRMS (ESI) calculated for C₂₇H₂₁F₃N₇O₂ [(M + H)⁺] 532.1704; found 532.1686.

### 1-(3-{4-(1-Methyl-4-oxo-4,5,6,7-tetrahydro-1H-pyrrolo[3,2-c]pyridin-2-yl)-2-[(pyridin-4-ylmethyl)-amino]-pyrimidin-5-ylethynyl}-phenyl)-3-(4-trifluoromethyl-phenyl)-urea (cmpd 17)

¹H NMR (401 MHz, DMSO-*d₆*) δ ppm 2.77 - 2.93 (m, 2 H) 3.38 - 3.53 (m, 2 H) 3.89 (br. s., 3 H) 4.61 (d, J=6.23 Hz, 2 H) 7.12 (d, J=7.81 Hz, 2 H) 7.28 - 7.36 (m, 3 H) 7.39 - 7.45 (m, 1 H) 7.48 (s, 1 H) 7.59 - 7.69 (m, 6 H) 8.24 (br. s., 1 H) 8.49 (d, J=5.86 Hz, 2 H) 8.54 (br. s., 1 H) 8.96 (s, 1 H) 9.21 (s, 1 H).
(ESI) *m*/*z* 637 [(M + H)⁺]. HRMS (ESI) calculated for C₃₄H₂₈F₃N₈O₂ [(M + H)⁺] 637.2282; found 637.2276.

### 1-{3-[4-(1-Methyl-4-oxo-4,5,6,7-tetrahydro-1H-pyrrolo[3,2-c]pyridin-2-yl)-2-(piperidin-4-ylamino)-pyrimidin-5-ylethynyl]-phenyl}-3-(4-trifluoromethyl-phenyl)-urea hydrochloride (cmpd 18)

¹H NMR (401 MHz, DMSO-*d₆*) δ ppm 1.61 - 1.75 (m, 2 H) 1.96 - 2.09 (m, 2 H) 2.87 - 2.92 (m, 2 H) 2.93 - 3.01 (m, 2 H) 3.22 - 3.36 (m, 2 H overlapped by water signal) 3.46 (td, J=6.84, 2.44 Hz, 2 H) 3.86 (s, 3 H) 3.98 - 4.07 (m, 1 H) 7.08 - 7.17 (m, 2 H) 7.32 (t, J=7.87 Hz, 1 H) 7.40 (d, J=8.67 Hz, 1 H) 7.48 (s, 1 H) 7.59 - 7.71 (m, 5 H) 7.82 (br. s., 2 H) 8.52 (s, 1 H) 9.09 (s, 1 H) 9.33 (s, 1 H).
(ESI) *m*/*z* 629 [(M + H)⁺]. HRMS (ESI) calculated for C₃₃H₃₃ClF₃N₈O₂ [(M + H)⁺] 629.2595; found 629.2582.

### 1-(3-{2-Amino-4-[1-(2-amino-ethyl)-4-oxo-4,5,6,7-tetrahydro-1H-pyrrolo[3,2-c]pyridin-2-yl]-pyrimidin-5-ylethynyl}-phenyl)-3-(4-trifluoromethyl-phenyl)-urea hydrochloride (cmpd 19)

¹H NMR (401 MHz, DMSO-*d₆*) δ ppm 2.95 (t, J=6.8 Hz, 2 H) 3.21 - 3.25 (m, 2 H) 3.48 (t, J=6.96 Hz, 2 H) 4.57 - 4.68 (m, 2 H) 7.13 - 7.17 (m, 1 H) 7.21 - 7.25 (m, 2 H) 7.33 (t, J=7.9 Hz, 1 H) 7.40 - 7.46 (m, 1 H) 7.49 (m, 3 H) 7.56 - 7.71 (m, 4 H) 7.77 (s, 1 H) 7.97 - 8.19 (m, 2 H) 8.49 (s, 1 H) 9.26 (s, 1 H) 9.50 (s, 1 H).
(ESI) *m*/*z* 575 [(M + H)⁺]. HRMS (ESI) calculated for C₂₉H₂₇ClF₃N₈O₂ [(M + H)⁺] 575.2126; found 575.2125.

### 1-(3-{2-Amino-4-[1-(2-amino-ethyl)-4-oxo-4,5,6,7-tetrahydro-1H-pyrrolo[3,2-c]pyridin-2-yl]-pyrimidin-5-ylethynyl}-phenyl)-3-[4-(4-ethyl-piperazin-1-ylmethyl)-3-trifluoromethyl-phenyl]-urea (cmpd 20)

¹H NMR (401 MHz, DMSO-*d₆*) δ ppm 0.98 (t, J=7.7 Hz, 3 H) 2.30 (q, J=7.4 Hz, 2 H) 2.35 - 2.44 (m, 8 H) 2.93 (t, J=6.8 Hz, 2 H) 3.43 - 3.48 (m, 2 H) 3.52 (s, 2 H) 4.42 - 4.47 (m, 2 H) 7.09 (s, 2 H) 7.11 - 7.14 (m, 1 H) 7.15 (br. s., 1 H) 7.31 (t, J=7.9 Hz, 1 H) 7.40 - 7.44 (m, 1 H) 7.55 - 7.59 (m, 1 H) 7.60 - 7.64 (m, 3 H) 7.97 (d, J=2.1 Hz, 1 H) 8.47 (s, 1 H) 9.09 (br. s., 1 H) 9.27 (br. s., 1 H).
(ESI) *m*/*z* 701 [(M + H)⁺]. HRMS (ESI) calculated for C₃₆H₄₀F₃N₁₀O₂ [(M + H)⁺] 701.3283; found 701.3276.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(3-trifluoromethyl-benzyl)-urea (cmpd 21)

¹H NMR (401 MHz, DMSO-*d₆*) δ ppm 2.88 (t, J=6.9 Hz, 2 H) 3.44 (td, J=6.8, 2.4 Hz, 2 H) 3.84 (s, 3 H) 4.39 (d, J=6.0 Hz, 2 H) 6.81 (t, J=6.1 Hz, 1 H) 6.99 - 7.06 (m, 3 H) 7.10 (m, 1 H) 7.25 (t, J=7.9 Hz, 1 H) 7.37 (ddd, J=8.2, 2.1, 1.0 Hz, 1 H) 7.42 (s, 1 H) 7.53 - 7.68 (m, 5 H) 8.43 (s, 1 H) 8.76 (s, 1 H).
(ESI) *m*/*z* 560 [(M + H)⁺]. HRMS (ESI) calculated for C₂₉H₂₅F₃N₇O₂ [(M + H)⁺] 560.2017; found 560.2015.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[3-(4-ethyl-piperazin-1-ylmethyl)-4-trifluoromethyl-phenyl]-urea (cmpd 22)

¹H NMR (401 MHz, DMSO-*d₆*) δ ppm 0.98 (t, J=7.14 Hz, 3 H) 2.27 - 2.36 (q, J=7.6, 2 H) 2.36 - 2.46 (m, 8 H) 2.89 (t, J=6.90 Hz, 2 H) 3.45 (td, J=6.90, 2.56 Hz, 2 H) 3.56 (s, 2 H) 3.85 (s, 3 H) 7.07 (s, 2 H) 7.09 - 7.13 (m, 2 H) 7.32 (t, J=7.93 Hz, 1 H) 7.41 - 7.45 (m, 1 H) 7.44 (s, 1 H) 7.57 - 7.60 (m, 1 H) 7.61 (t, J=1.83 Hz, 1 H) 7.63 - 7.69 (m, 1 H) 7.71 - 7.73 (m, 1 H) 8.46 (s, 1 H) 8.89 (s, 1 H) 9.25 (s, 1 H).
(ESI) *m*/*z* 672 [(M + H)⁺]. HRMS (ESI) calculated for C₃₅H₃₇F₃N₉O₂ [(M + H)⁺] 672.3017; found 672.3007.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(5-trifluoromethyl-[1,3,4]thiadiazol-2-yl)-urea trifluoroacetate (cmpd 23)

(ESI) *m*/*z* 554 [(M + H)⁺]. HRMS (ESI) calculated for C₂₄H₁₈F₃N₉O₂S [(M + H)⁺] 554.1329; found 554.1345.

### 1-(3-{2-Amino-4-[1-(2-amino-ethyl)-4-oxo-4,5,6,7-tetrahydro-1H-pyrrolo[3,2-c]pyridin-2-yl]-pyrimidin-5-ylethynyl}-phenyl)-3-[4-(4-ethyl-piperazin-1-ylmethyl)-3-trifluoromethyl-phenyl]-urea tri-hydrochloride (cmpd 24)

¹H NMR (401 MHz, DMSO-*d₆*) δ ppm 0.98 (t, J=7.45 Hz, 3 H) 2.27 - 2.34 (q, J=7.45 Hz, 2 H) 2.35 - 2.44 (m, 8 H) 2.93 (t, J=6.84 Hz, 3 H) 3.43 - 3.48 (m, 2 H) 3.52 (s, 2 H) 4.42 - 4.47 (m, 2 H) 7.09 (s, 2 H) 7.12 (dt, J=7.66, 1.24 Hz, 1 H) 7.15 (br. s., 1 H) 7.31 (t, J=7.87 Hz, 1 H) 7.40 - 7.44 (m, 1 H) 7.55 - 7.59 (m, 1 H) 7.61 (br. s., 1 H) 7.63 (s, 1 H) 7.65 (t, J=1.71 Hz, 1 H) 7.97 (d, J=2.07 Hz, 1 H) 8.47 (s, 1 H) 9.09 (s, 1 H) 9.27 (s, 1 H).

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(4-trifluoromethyl-phenyl)-thiourea (cmpd 25)

¹H NMR (401 MHz, DMSO-*d₆*) δ ppm 2.88 (t, J=6.77 Hz, 2 H) 3.41 - 3.49 (m, 2 H) 3.85 (s, 3 H) 7.08 (s, 2 H) 7.11 (br. s., 1 H) 7.26 (d, J=7.69 Hz, 1 H) 7.38 (t, J=7.87 Hz, 1 H) 7.44 (s, 1 H) 7.47 - 7.51 (m, 1 H) 7.56 (s, 1 H) 7.66 - 7.76 (m, 4 H) 8.45 (s, 1 H) 10.09 (s, 1 H) 10.18 (s, 1 H).
(ESI) *m*/*z* 562 [(M + H)⁺]. HRMS (ESI) calculated for C₂₈H₂₃F₃N₇OS [(M + H)⁺] 562.1631; found 562.1636.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(4-trifluoromethyl-cyclohexyl)-urea (cis isomer) (cmpd 26)

¹H NMR (401 MHz, DMSO-*d₆*) δ ppm 1.40 - 1.52 (m, 2 H) 1.53 - 1.63 (m, 2 H) 1.66 - 1.79 (m, 4 H) 2.26 - 2.34 (m, 1 H) 2.89 (t, J=6.8 Hz, 2 H) 3.44 (td, J=6.8, 2.6 Hz, 2 H) 3.85 (s, 3 H) 3.85 - 3.90 (m, 1 H) 6.47 (d, J=7.4 Hz, 1 H) 7.01 (dt, J=7.5, 1.3 Hz, 1 H) 7.05 (s, 2 H) 7.10 (s, 1 H) 7.24 (t, J=7.7 Hz, 1 H) 7.29 - 7.33 (m, 1 H) 7.42 (s, 1 H) 7.55 (t, J=1.8 Hz, 1 H) 8.40 (s, 1 H) 8.44 (s, 1 H).
(ESI) *m*/*z* 552 [(M + H)⁺]. HRMS (ESI) calculated for C₂₈H₂₉F₃N₇O₂ [(M + H)⁺] 552.2330; found 552.2330.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(4-trifluoromethyl-cyclohexyl)-urea (trans isomer) (cmpd 27)

¹H NMR (401 MHz, DMSO-*d₆*) δ ppm 1.15 - 1.28 (m, 2 H) 1.28 - 1.41 (m, 2 H) 1.83 - 2.02 (m, 4 H) 2.17 - 2.29 (m, 1 H) 2.88 (t, J=6.8 Hz, 2 H) 3.38 - 3.42 (m, 1 H) 3.42 - 3.48 (m, 2 H) 3.84 (s, 3 H) 6.13 (d, J=7.7 Hz, 1 H) 6.97 - 7.02 (m, 1 H) 7.04 (s, 2 H) 7.09 (br. s., 1 H) 7.23 (t, J=7.9 Hz, 1 H) 7.29 - 7.34 (m, 1 H) 7.41 (s, 1 H) 7.52 (t, J=1.8 Hz, 1 H) 8.39 (s, 1 H) 8.43 (s, 1 H).
(ESI) *m*/*z* 552 [(M + H)⁺]. HRMS (ESI) calculated for C₂₈H₂₉F₃N₇O₂ [(M + H)⁺] 552.2330; found 552.2317.

### 1-{4-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(4-trifluoromethyl-phenyl)-urea (cmpd 28)

¹H NMR (401 MHz, DMSO-*d₆*) δ ppm 2.89 (t, J=6.84 Hz, 2 H) 3.45 (td, J=6.77, 2.32 Hz, 2 H) 3.85 (s, 3 H) 7.01 (s, 2 H) 7.11 (s, 1 H) 7.41 (d, J=8.67 Hz, 2 H) 7.48 - 7.53 (m, 3 H) 7.60 - 7.70 (m, 4 H) 8.41 (s, 1 H) 9.00 (s, 1 H) 9.16 (s, 1 H).
(ESI) *m*/*z* 546 [(M + H)⁺]. HRMS (ESI) calculated for C₂₈H₂₃F₃N₇O₂ [(M + H)⁺] 546.1860; found 546.1839.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[3-(4-ethyl-piperazin-1-ylmethyl)-5-trifluoromethyl-phenyl]-urea (cmpd 29)

¹H NMR (401 MHz, DMSO-*d₆*) δ ppm 0.98 (t, J=7.08 Hz, 3 H) 2.24 - 2.47 (m, 10 H) 2.90 (t, J=6.71 Hz, 2 H) 3.41 - 3.48 (m, 2 H) 3.59 (s, 2 H) 3.85 (s, 3 H) 7.06 (s, 2 H) 7.10 - 7.15 (m, 1 H) 7.22 (s, 1 H) 7.32 (t, J=7.69 Hz, 1 H) 7.38 - 7.43 (m, 1 H) 7.44 (s, 1 H) 7.52 - 7.55 (m, 1 H) 7.62 - 7.65 (m, 2 H) 7.87 - 7.92 (m, 1 H) 8.47 (s, 1 H) 8.84 (s, 1 H) 9.12 (s, 1 H).
(ESI) *m*/*z* 672 [(M + H)⁺]. HRMS (ESI) calculated for C₃₅H₃₇F₃N₉O₂ [(M + H)⁺] 672.3017; found 672.2999.

### 1-{4-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[4-(4-ethyl-piperazin-1-ylmethyl)-3-trifluoromethyl-phenyl]-urea (cmpd 30)

¹H NMR (401 MHz, DMSO-*d₆*) δ ppm 1.02 (br. s., 3 H) 2.32 - 2.67 (m,10 H) 2.89 (t, J=6.8 Hz, 2 H) 3.45 (td, J=6.8, 2.4 Hz, 2 H) 3.55 (s, 2 H) 3.85 (s, 3 H) 7.01 (s, 2 H) 7.10 (s, 1 H) 7.40 (d, J=8.7 Hz, 2 H) 7.48 - 7.53 (m, 3 H) 7.56 - 7.66 (m, 2 H) 7.95 (d, J=1.8 Hz, 1 H) 8.41 (s, 1 H) 8.99 (s, 1 H) 9.09 (s, 1 H).
(ESI) *m*/*z* 672 [(M + H)⁺]. HRMS (ESI) calculated for C₃₅H₃₇F₃N₉O₂ [(M + H)⁺] 672.3017; found 672.2985.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[3-(4-ethyl-piperazine-1-carbonyl)-5-trifluoromethyl-phenyl]-urea (cmpd 31)

¹H NMR (401 MHz, DMSO-*d₆*) δ ppm 1.00 (t, J=7.14 Hz, 3 H) 2.25 - 2.47 (m, 6 H) 2.89 (t, J=6.90 Hz, 2 H) 3.30 (m, 2 H overlapped by water signal) 3.44 (td, J=6.80, 2.14 Hz, 2 H) 3.63 (br. s., 2 H) 3.85 (s, 3 H) 7.00 - 7.16 (m, 4 H) 7.28 (s, 1 H) 7.32 (t, J=7.87 Hz, 1 H) 7.42 (s, 1 H) 7.44 (s, 1 H) 7.63 - 7.68 (m, 2 H) 8.00 (s, 1 H) 8.46 (s, 1 H) 9.09 (br. s., 1 H) 9.35 (br. s., 1 H).
(ESI) *m*/*z* 686 [(M + H)⁺]. HRMS (ESI) calculated for C₃₅H₃₅F₃N₉O₃ [(M + H)⁺] 686.2809; found 686.2796.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[4-(4-methyl-piperazin-1-yl)-phenyl]-urea (cmpd 32)

¹H NMR (401 MHz, DMSO-*d₆*) δ ppm 2.21 (s, 3 H) 2.39 - 2.47 (m, 4 H) 2.89 (t, J=6.90 Hz, 2 H) 3.00 - 3.09 (m, 4 H) 3.45 (td, J=6.84, 2.44 Hz, 2 H) 3.85 (s, 3 H) 6.81 - 6.92 (m, 2 H) 7.04 - 7.13 (m, 4 H) 7.22 - 7.33 (m, 3 H) 7.37 - 7.41 (m, 1 H) 7.43 (s, 1 H) 7.60 (s, 1 H) 8.26 (s, 1 H) 8.45 - 8.47 (m, 1 H) 8.68 (s, 1 H).
(ESI) *m*/*z* 576 [(M + H)⁺]. HRMS (ESI) calculated for C₃₂H₃₄N₉O₂ [(M + H)⁺] 576.2830; found 576.2827.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[3-(4-methyl-piperazin-1-yl)-phenyl]-urea (cmpd 33)

¹H NMR (401 MHz, DMSO-*d₆*) δ ppm 2.22 (s, 3 H) 2.41 - 2.48 (m, 4 H) 2.89 (t, J=6.77 Hz, 2 H) 3.06 - 3.14 (m, 4 H) 3.44 (td, J=6.77, 2.44 Hz, 2 H) 3.85 (s, 3 H) 6.57 (dd, J=8.24, 1.77 Hz, 1 H) 6.77 (d, J=7.69 Hz, 1 H) 7.02 - 7.13 (m, 5 H) 7.17 (s, 1 H) 7.29 (t, J=7.93 Hz, 1 H) 7.35 - 7.40 (m, 1 H) 7.44 (s, 1 H) 7.62 (s, 1 H) 8.46 (s, 1 H) 8.60 (s, 1 H) 8.75 (s, 1 H).
(ESI) *m*/*z* 576 [(M + H)⁺]. HRMS (ESI) calculated for C₃₂H₃₄N₉O₂ [(M + H)⁺] 576.2830; found 576.2831.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[4-(4-methyl-piperazin-1-yl)-3-trifluoromethyl-phenyl]-urea (cmpd 34)

¹H NMR (401 MHz, DMSO-*d₆*) δ ppm 2.22 (s, 3 H) 2.43 (br. s., 4 H) 2.78 - 2.84 (m, 4 H) 2.89 (t, J=6.84 Hz, 2 H) 3.44 (td, J=6.77, 2.20 Hz, 2 H) 3.85 (s, 3 H) 7.00 - 7.14 (m, 4 H) 7.31 (t, J=7.93 Hz, 1 H) 7.38 - 7.43 (m, 1 H) 7.44 (s, 1 H) 7.48 - 7.52 (m, 1 H) 7.56 - 7.60 (m, 1 H) 7.62 (s, 1 H) 7.90 (d, J=2.44 Hz, 1 H) 8.46 (s, 1 H) 8.83 (s, 1 H) 8.97 (s, 1 H).
(ESI) *m*/*z* 644 [(M + H)⁺]. HRMS (ESI) calculated for C₃₅H₃₅F₃N₉O₃ [(M + H)⁺] 644.2704; found 644.2726.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(4-fluoro-phenyl)-urea (cmpd 35)

¹H NMR (401 MHz, DMSO-*d₆*) d ppm 2.89 (t, J=6.90 Hz, 2 H) 3.45 (td, J=6.77, 2.69 Hz, 2 H) 3.85 (s, 3 H) 7.02 - 7.15 (m, 6 H) 7.30 (t, J=7.93 Hz, 1 H) 7.38 - 7.42 (m, 1 H) 7.43 - 7.50 (m, 3 H) 7.60 (t, J=1.65 Hz, 1 H) 8.45 (s, 1 H) 8.73 (s, 1 H).
(ESI) *m*/*z* 496 [(M + H)⁺]. HRMS (ESI) calculated for C₂₇H₂₃FN₇O₂ [(M + H)⁺] 496.1892; found 496.1907.

### 1-{5-[2-Amino-4-(1-methyl-4-oxo-4,5,6,7-tetrahydro-1H-pyrrolo[3,2-c]pyridin-2-yl)-pyrimidin-5-ylethynyl]-2-fluoro-phenyl}-3-[4-(4-ethyl-piperazin-1-ylmethyl)-3-trifluoromethyl-phenyl]-urea (cmpd 36)

¹H NMR (401 MHz, DMSO-*d₆*) δ ppm 0.99 (t, J=7.1 Hz, 3 H) 2.18 - 2.45 (m, 10 H) 2.89 (t, J=6.8 Hz, 2 H) 3.44 (td, J=6.9, 2.4 Hz, 2 H) 3.54 (s, 2 H) 3.85 (s, 3 H) 6.99 - 7.10 (m, 3 H) 7.13 (ddd, J=8.5, 4.8, 2.1 Hz, 1 H) 7.30 (dd, J=11.1, 8.5 Hz, 1 H) 7.40 (s, 1 H) 7.52 (m, J=6.7 Hz, 1 H) 7.65 (d, J=8.5 Hz, 1 H) 7.98 (d, J=2.2 Hz, 1 H) 8.23 (dd, J=7.8, 2.0 Hz, 1 H) 8.47 (s, 1 H) 8.70 (d, J=2.1 Hz, 1 H) 9.39 (s, 1 H).
(ESI) *m*/*z* 690 [(M + H)⁺]. HRMS (ESI) calculated for C₃₅H₃₆F₄N₉O₂ [(M + H)⁺] 690.2923; found 690.2919.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(3-fluoro-phenyl)-urea (cmpd 37)

¹H NMR (401 MHz, DMSO-*d₆*) δ ppm 2.89 (t, J=6.96 Hz, 2 H) 3.45 (td, J=6.84, 2.32 Hz, 2 H) 3.85 (s, 3 H) 6.79 (td, J=8.45, 2.50 Hz, 1 H) 7.06 (s, 2 H) 7.25 - 7.35 (m, 2 H) 7.39 - 7.42 (m, 1 H) 7.44 (s, 1 H) 7.46 - 7.53 (m, 1 H) 7.59 - 7.62 (m, 1 H) 8.45 (s, 1 H) 8.82 (s, 1 H) 8.94 (s, 1 H).
(ESI) *m*/*z* 496 [(M + H)⁺]. HRMS (ESI) calculated for C₂₇H₂₃FN₇O₂ [(M + H)⁺] 496.1892; found 496.1889.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[4-(4-dimethylamino-piperidin-1-ylmethyl)-3-trifluoromethyl-phenyl]-urea (cmpd 39)

¹H NMR (600 MHz, DMSO-*d₆*) δ ppm 1.68 - 1.92 (m, 2 H) 2.10 - 2.21 (m, 2 H) 2.77 (br. s., 6 H) 2.89 (t, J=6.87 Hz, 2 H) 3.44 (m, 2 H overlapped by water signal) 3.85 (s, 3 H) 7.05 - 7.19 (m, 4 H) 7.33 (t, J=7.97 Hz, 1 H) 7.40 - 7.47 (m, 2 H) 7.65 (s, 1 H) 7.67 - 7.81 (m, 2 H) 8.05 (br. s., 1 H) 8.46 (s, 1 H) 9.12 (br. s., 1 H) 9.42 (br. s., 1 H).
(ESI) *m*/*z* 686 [(M + H)⁺]. HRMS (ESI) calculated for C₃₆H₃₇F₃N₉O₂ [(M + H)⁺] 686.3174; found 686.3199.

### 1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[4-(4-methyl-piperazin-1-ylmethyl)-3-trifluoromethyl-phenyl]-urea (cmpd 40)

¹H NMR (600 MHz, DMSO-*d₆*) δ ppm 2.18 (s, 3 H) 2.25 - 2.47 (m, 8 H) 2.89 (t, J=6.78 Hz, 2 H) 3.44 (td, J=6.87, 2.56 Hz, 2 H) 3.53 (s, 2 H) 3.85 (s, 3 H) 7.06 (s, 2 H) 7.09 - 7.13 (m, 2 H) 7.31 (t, J=7.88 Hz, 1 H) 7.41 (dd, J=8.24, 1.28 Hz, 1 H) 7.44 (s, 1 H) 7.57 (dd, J=8.33, 1.92 Hz, 1 H) 7.60 - 7.64 (m, 2 H) 7.96 (d, J=2.01 Hz, 1 H) 8.46 (s, 1 H) 8.85 (s, 1 H) 9.04 (s, 1 H).
(ESI) *m*/*z* 658 [(M + H)⁺]. HRMS (ESI) calculated for C₃₄H₃₅F₃N₉O₂ [(M + H)⁺] 658.2861; found 658.2868.

### 6-[2-Amino-4-(1-methyl-4-oxo-4,5,6,7-tetrahydro-1H-pyrrolo[3,2-c]pyridin-2-yl)-pyrimidin-5-ylethynyl]-2,3-dihydro-indole-1-carboxylic acid [4-(4-ethyl-piperazin-1-ylmethyl)-3-trifluoromethyl-phenyl]-amide (cmpd 44)

¹H NMR (600 MHz, DMSO-*d₆*) δ ppm 1.07 (br. s., 3 H) 2.34 - 2.67 (m, 10 H) 2.88 (t, J=6.87 Hz, 2 H) 3.21 (t, J=8.52 Hz, 2 H) 3.44 (td, J=6.87, 2.38 Hz, 2 H) 3.58 (br. s., 2 H) 3.84 (s, 3 H) 4.16 (t, J=8.61 Hz, 2 H) 7.03 (s, 2 H) 7.05 - 7.07 (m, 1 H) 7.08 (br. s., 1 H) 7.23 (d, J=7.69 Hz, 1 H) 7.41 (s, 1 H) 7.63 (d, J=8.79 Hz, 1 H) 7.85 (d, J=8.06 Hz, 1 H) 7.94 (s, 1 H) 8.00 (d, J=1.83 Hz, 1 H) 8.45 (s, 1 H) 8.86 (s, 1 H).
(ESI) *m*/*z* 698 [(M + H)⁺]. HRMS (ESI) calculated for C₃₇H₃₉F₃N₉O₂ [(M + H)⁺] 698.3174; found 698.3170.

### 5-[2-Amino-4-(1-methyl-4-oxo-4,5,6,7-tetrahydro-1H-pyrrolo[3,2-c]pyridin-2-yl)-pyrimidin-5-ylethynyl]-2,3-dihydro-indole-1-carboxylic acid [4-(4-ethyl-piperazin-1-ylmethyl)-3-trifluoromethyl-phenyl]-amide (cmpd 45)

¹H NMR (600 MHz, DMSO-*d₆*) δ ppm 0.98 (t, J=7.23 Hz, 3 H) 2.31 (q, J=7.14 Hz, 2 H) 2.38 (br. s., 8 H) 2.89 (t, J=6.87 Hz, 2 H) 3.19 (t, J=8.52 Hz, 2 H) 3.45 (td, J=6.82, 2.47 Hz, 2 H) 3.54 (s, 2 H) 3.85 (s, 3 H) 4.17 (t, J=8.70 Hz, 2 H) 7.00 (s, 2 H) 7.12 (br. s., 1 H) 7.27 (d, J=8.43 Hz, 1 H) 7.33 (s, 1 H) 7.50 (s, 1 H) 7.64 (d, J=8.43 Hz, 1 H) 7.83 - 7.85 (m, 1 H) 7.87 (d, J=8.43 Hz, 1 H) 7.98 (d, J=2.01 Hz, 1 H) 8.40 (s, 1 H) 8.86 (s, 1 H).
(ESI) *m*/*z* 698 [(M + H)⁺]. HRMS (ESI) calculated for C₃₇H₃₉F₃N₉O₂ [(M + H)⁺] 698.3174; found 698.3185.

### Example 4

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-phenyl-acetamide (cmpd 79)

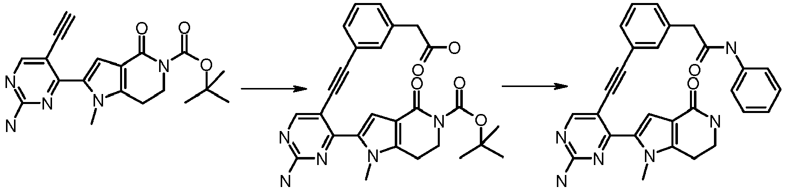

### Step 1. 2-[2-Amino-5-(3-carboxymethyl-phenylethynyl)-pyrimidin-4-yl]-1-methyl-4-oxo-1,4,6,7-tetrahydro-pyrrolo[3,2-c]pyridine-5-carboxylic acid tert-butyl ester

To a suspension of 2-(2-Amino-5-ethynyl-pyrimidin-4-yl)-1-methyl-4-oxo-1,4,6,7-tetrahydro-pyrrolo[3,2-c]pyridine-5-carboxylic acid tert-butyl ester (1.56 g, 4.25 mmol) in degassed MeCN (25 mL), CuI (10% mol, 81 mg, 0.425 mmol), 3-iodophenylacetic acid (1.17 g, 4.25 mmol), TEA (5.92 mL, 42.48 mmol) and PdCl₂(PPh₃)₂ (10% mol, 0.30 g, 0.425 mmol) were added. The mixture was degassed for three times back filling with argon each time and let under stirring at r.t. for 1 h. The reaction was quenched with 2 N HCl (21.2 mL) and the solvent was removed under reduced pressure. The residue was suspended in water and the resulting solid was filtered and rinsed with water affording the title product and brownish solid (1.62 g, 76%).
(ESI) *m*/*z* 502 [(M + H)⁺]. HRMS (ESI) calculated for C₂₇H₂₈N₅O₅ [(M + H)⁺] 502.2085; found 502.2074.

### Step 2. 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-phenyl-acetamide (cmpd 79)

A mixture of 2-[2-amino-5-(3-carboxymethyl-phenylethynyl)-pyrimidin-4-yl]-1-methyl-4-oxo-1,4,6,7-tetrahydropyrrolo[3,2-c]pyridine-5-carboxylic acid tert-butyl ester (70 mg, 0.14 mmol), TBTU (47 mg, 0.15 mmol), aniline (0.015 mL, 0.17 mmol) and DIPEA (0.03 mL, 0.18 mmol) in DMA (4 mL) was let under stirring at r.t. overnight. The reaction was diluted with EtOAc and washed with saturated solution of NaHCO₃, water and brine. The organic layer was dried over anhydrous Na₂SO₄, filtered, concentrated and finally treated with 4 M HCl in dioxane for 2 h. After removal of the solvent, the compound was purified by preparative HPLC using a XTerra C18 column (19x100 mm; 5 µm). Mobile phase A was 0.05% NH₃/CH₃CN 95/5, and mobile layer B was MeCN. The gradient was from 10 to 90% B in 8 min then hold for 2 min before re-equilibration.
¹H NMR (401 MHz, DMSO- *d₆*) δ ppm 2.90 (t, J=6.90 Hz, 2 H) 3.46 (td, J=6.87, 2.50 Hz, 2 H) 3.65 (s, 2 H) 3.85 (s, 3 H) 7.01 - 7.05 (m, 1 H) 7.07 (s, 2 H) 7.21 (s, 1 H) 7.26 - 7.32 (m, 2 H) 7.32 - 7.39 (m, 3 H) 7.51 - 7.54 (m, 2 H) 7.56 - 7.61 (m, 2 H) 8.44 (s, 1 H) 10.17 (s, 1 H).
(ESI) *m*/*z* 477 [(M + H)⁺]. HRMS (ESI) calculated for C₂₈H₂₅N₆O₂ [(M + H)⁺] 477.2034; found 477.2031.

According to this same methodology, but employing suitable substituted derivatives, the following compounds were prepared:

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-benzyl-acetamide (cmpd 80)

¹H NMR (401 MHz, DMSO-*d₆*) δ ppm 2.87 (t, J=6.84 Hz, 2 H) 3.44 (td, J=6.80, 2.26 Hz, 2 H) 3.49 (s, 2 H) 3.85 (s, 3 H) 4.27 (d, J=5.86 Hz, 2 H) 7.06 (s, 2 H) 7.16 (br. s., 1 H) 7.18 - 7.24 (m, 4 H) 7.24 - 7.35 (m, 6 H) 7.47 (s, 1 H) 7.51 (s, 1 H) 8.43 (s, 1 H) 8.55 (s, 1 H).
(ESI) *m*/*z* 491 [(M + H)⁺]. HRMS (ESI) calculated for C₂₈H₂₅N₆O₂ [(M + H)⁺] 491.2190; found 491.2193.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-trifluoromethyl-phenyl)-acetamide (cmpd 81)

¹H NMR (401 MHz, DMSO-*d₆*) δ ppm 2.90 (t, J=6.90 Hz, 2 H) 3.46 (td, J=6.90, 2.44 Hz, 2 H) 3.70 (s, 2 H) 3.85 (s, 3 H) 7.07 (s, 2 H) 7.21 (s, 1 H) 7.30 - 7.40 (m, 3 H) 7.50 - 7.54 (m, 2 H) 7.66 (d, J=8.79 Hz, 2 H) 7.81 (d, J=8.67 Hz, 2 H) 8.43 (s, 1 H) 10.54 (s, 1 H).
(ESI) *m*/*z* 545 [(M + H)⁺]. HRMS (ESI) calculated for C₂₉H₂₄F₃N₆O₂ [(M + H)⁺] 545.1908; found 545.1906.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[3-(4-ethyl-piperazin-1-ylmethyl)-4-trifluoromethyl-phenyl]-acetamide (cmpd 82)

¹H NMR (600 MHz, DMSO-*d₆*) δ ppm 0.96 (t, J=7.23 Hz, 3 H) 2.28 (q, J=7.20 Hz, 2 H) 2.32 - 2.47 (m, 8 H) 2.90 (t, J=6.87 Hz, 2 H) 3.46 (td, J=6.78, 2.56 Hz, 2 H) 3.54 (s, 2 H) 3.69 (s, 2 H) 3.85 (s, 3 H) 7.07 (s, 2 H) 7.19 (br. s., 1 H) 7.31 - 7.41 (m, 3 H) 7.51 - 7.55 (m, 2 H) 7.60 (d, J=8.79 Hz, 1 H) 7.79 (d, J=8.42 Hz, 1 H) 7.89 (s, 1 H) 8.43 (s, 1 H) 10.52 (s, 1 H).
(ESI) *m*/*z* 671 [(M + H)⁺]. HRMS (ESI) calculated for C₃₆H₃₈F₃N₈O₂ [(M + H)⁺] 671.3065; found 671.3067.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(1-methyl-piperidin-4-yloxy)-phenyl]-acetamide (cmpd 83)

¹H NMR (401 MHz, DMSO-*d₆*) δ ppm 1.49 - 1.64 (m, 2 H) 1.83 - 1.92 (m, 2 H) 2.08 - 2.14 (m, 2 H) 2.15 (s, 3 H) 2.54 - 2.63 (m, 2 H) 2.90 (t, J=6.90 Hz, 2 H) 3.46 (td, J=6.80, 2.38 Hz, 2 H) 3.60 (s, 2 H) 3.85 (s, 3 H) 4.21 - 4.30 (m, 1 H) 6.83 - 6.90 (m, 2 H) 7.06 (s, 2 H) 7.21 (s, 1 H) 7.29 - 7.38 (m, 3 H) 7.43 - 7.49 (m, 2 H) 7.51 (s, 1 H) 7.53 (s, 1 H) 8.43 (s, 1 H) 10.02 (s, 1 H).
(ESI) *m*/*z* 590 [(M + H)⁺]. HRMS (ESI) calculated for C₃₄H₃₆N₇O₃ [(M + H)⁺] 590.2874; found 590.2886.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-fluoro-benzyl)-acetamide (cmpd 84)

¹H NMR (401 MHz, DMSO-*d₆*) δ ppm 2.84 (t, J=6.90 Hz, 2 H) 3.81 (s, 3 H) 4.21 (d, J=5.74 Hz, 2 H) 6.98 - 7.17 (m, 4 H) 7.17 - 7.27 (m, 3 H) 7.29 (d, J=5.13 Hz, 2 H) 7.41 (s, 1 H) 8.40 (s, 1 H) 8.51 (t, J=5.80 Hz, 1 H).
(ESI) *m*/*z* 509 [(M + H)⁺]. HRMS (ESI) calculated for C₂₉H₂₆FN₆O₂ [(M + H)⁺] 509.2096; found 509.2091.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-isoquinolin-5-yl-acetamide (cmpd 85)

¹H NMR (401 MHz, DMSO-*d₆*) δ ppm 2.88 (t, J=6.84 Hz, 3 H) 3.44 (td, J=6.87, 2.26 Hz, 3 H) 3.84 - 3.88 (m, 5 H) 7.07 (s, 2 H) 7.15 - 7.19 (m, 1 H) 7.30 - 7.45 (m, 4 H) 7.55 (s, 1 H) 7.61 (s, 1 H) 7.66 (t, J=7.93 Hz, 1 H) 7.86 - 7.97 (m, 2 H) 8.00 (d, J=7.45 Hz, 1 H) 8.43 - 8.45 (m, 1 H) 8.50 (d, J=5.98 Hz, 1 H) 9.31 (s, 1 H) 10.27 (s, 1 H).
(ESI) *m*/*z* 528 [(M + H)⁺]. HRMS (ESI) calculated for C₃₁H₂₆N₇O₂ [(M + H)⁺] 528.2143; found 528.2153.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3-oxazol-5-yl-phenyl)-acetamide (cmpd 86)

¹H NMR (401 MHz, DMSO-*d₆*) δ ppm 2.90 (t, J=6.90 Hz, 2 H) 3.43 - 3.49 (m, 2 H) 3.68 (s, 2 H) 3.84 - 3.87 (m, 3 H) 7.07 (s, 2 H) 7.22 (s, 1 H) 7.34 - 7.38 (m, 3 H) 7.40 - 7.43 (m, 2 H) 7.52 - 7.58 (m, 3 H) 7.61 (s, 1 H) 8.03 (s, 1 H) 8.43 (d, J=2.93 Hz, 2 H) 10.32 - 10.36 (m, 1 H) 10.34 (s, 1 H).
(ESI) *m*/*z* 544 [(M + H)⁺]. HRMS (ESI) calculated for C₃₁H₂₆N₇O₃ [(M + H)⁺] 544.2091; found 544.2101.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(1H-indazol-5-yl)-acetamide (cmpd 87)

¹H NMR (401 MHz, DMSO-*d₆*) δ ppm 2.89 (d, J=7.08 Hz, 2 H) 3.46 (d, J=6.47 Hz, 2 H) 3.66 (s, 2 H) 3.85 (s, 3 H) 7.06 (br. s, 2 H) 7.22 (br. s, 1 H) 7.32 - 7.39 (m, 3 H) 7.40 - 7.51 (m, 2 H) 7.51 - 7.56 (m, 2 H) 7.99 (s, 1 H) 8.10 (s, 1 H) 8.44 (s, 1 H) 10.17 (s, 1 H) 12.94 (br. s, 1 H).
(ESI) *m*/*z* 517 [(M + H)⁺]. HRMS (ESI) calculated for C₂₉H₂₅N₈O₂ [(M + H)⁺] 517.2095; found 517.2096.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3-pyridin-4-yl-phenyl)-acetamide (cmpd 88)

¹H NMR (401 MHz, DMSO-*d₆*) δ ppm 2.89 (t, J=6.90 Hz, 2 H) 3.46 (td, J=6.96, 2.32 Hz, 2 H) 3.69 (s, 2 H) 3.86 (s, 3 H) 7.07 (s, 2 H) 7.25 (s, 1 H) 7.36 (s, 3 H) 8.03 (s, 1 H) 8.43 (s, 1 H) 8.59 - 8.66 (m, 2 H) 10.36 (s, 1 H).
(ESI) *m*/*z* 554 [(M + H)⁺]. HRMS (ESI) calculated for C₃₃H₂₈N₇O₂ [(M + H)⁺] 554.2299; found 554.2310.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-pyrazol-1-yl-phenyl)-acetamide (cmpd 89)

¹H NMR (401 MHz, DMSO-*d₆*) δ ppm 2.90 (t, J=6.90 Hz, 2 H) 3.46 (td, J=6.90, 2.32 Hz, 2 H) 3.67 (s, 2 H) 3.86 (s, 3 H) 6.49 - 6.52 (m, 1 H) 7.07 (br. s., 2 H) 7.22 (br. s., 1 H) 7.34 - 7.39 (m, 3 H) 7.51 - 7.66 (m, 2 H) 7.69 (m, 1 H) 7.70 - 7.78 (m, 4 H) 8.40 (d, J=2.44 Hz, 1 H) 8.44 (s, 1 H) 10.31 (s, 1 H).
(ESI) *m*/*z* 543 [(M + H)⁺]. HRMS (ESI) calculated for C₃₁H₂₇N₈O₂ [(M + H)⁺] 543.2252; found 543.2254.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3-pyrazol-1-ylmethyl-phenyl)-acetamide (cmpd 90)

¹H NMR (401 MHz, DMSO-*d₆*) δ ppm 2.86 (t, J=6.90 Hz, 2 H) 3.42 (td, J=6.84, 2.69 Hz, 2 H) 3.59 (s, 2 H) 3.82 (s, 3 H) 5.24 (s, 2 H) 6.21 (t, J=2.08 Hz, 1 H) 6.84 (d, J=7.57 Hz, 1 H) 7.03 (s, 2 H) 7.16 (s, 1 H) 7.19 - 7.24 (m, 1 H) 7.26 - 7.34 (m, 3 H) 7.73 (d, J=2.20 Hz, 1 H) 8.40 (s, 1 H) 10.14 (s, 1 H).
(ESI) *m*/*z* 557 [(M + H)⁺]. HRMS (ESI) calculated for C₃₂H₂₉N₈O₂ [(M + H)⁺] 557.2408; found 557.2410.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3-piperidin-1-yl-phenyl)-acetamide (cmpd 91)

¹H NMR (401 MHz, DMSO-*d₆*) δ ppm 1.45 - 1.67 (m, 6 H) 2.90 (t, J=6.77 Hz, 2 H) 3.03 - 3.12 (m, 4 H) 3.46 (td, J=6.80, 2.38 Hz, 2 H) 3.62 (s, 1 H) 3.86 (s, 3 H) 6.60 (dd, J=8.30, 2.07 Hz, 1 H) 6.98 (d, J=8.79 Hz, 1 H) 7.05 - 7.13 (m, 3 H) 7.20 - 7.25 (m, 2 H) 7.30 - 7.39 (m, 4 H) 7.54 (s, 1 H) 7.55 (s, 1 H) 8.43 (s, 1 H) 10.00 (s, 1 H).
(ESI) *m*/*z* 560 [(M + H)⁺]. HRMS (ESI) calculated for C₃₃H₃₄N₇O₂ [(M + H)⁺] 560.2769; found 560.2764.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3-trifluoromethyl-phenyl)-acetamide (cmpd 92)

¹H NMR (401 MHz, DMSO-*d₆*) δ ppm 2.90 (t, J=6.84 Hz, 2 H) 3.46 (td, J=6.93, 2.26 Hz, 2 H) 3.69 (s, 2 H) 3.85 (s, 3 H) 7.07 (s, 2 H) 7.21 (s, 1 H) 7.32 - 7.42 (m, 4 H) 7.52 - 7.58 (m, 3 H) 7.78 (d, J=8.18 Hz, 1 H) 8.09 (s, 1 H) 8.44 (s, 1 H) 10.52 (s, 1 H).
(ESI) *m*/*z* 545 [(M + H)⁺]. HRMS (ESI) calculated for C₂₉H₂₄F₃N₆O₂ [(M + H)⁺] 545.1908; found 545.1911.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(5,6,7,8-tetrahydro-naphthalen-1-yl)-acetamide (cmpd 93)

¹H NMR (401 MHz, DMSO-*d₆*) δ ppm 1.66 (t, J=3.23 Hz, 4 H) 2.54 (br. s., 2 H) 2.70 (br. s., 2 H) 2.89 (t, J=6.84 Hz, 2 H) 3.45 (td, J=6.87, 2.50 Hz, 2 H) 3.67 (s, 2 H) 3.85 (s, 3 H) 6.89 (d, J=7.08 Hz, 1 H) 7.03 (t, J=7.75 Hz, 1 H) 7.07 (s, 1 H) 7.14 - 7.19 (m, 2 H) 7.36 (s, 3 H) 7.54 (s, 1 H) 7.56 (s, 1 H) 8.44 (s, 1 H) 9.35 (s, 1 H).
(ESI) *m*/*z* 531 [(M + H)⁺]. HRMS (ESI) calculated for C₃₂H₃₁N₆O₂ [(M + H)⁺] 531.2503; found 531.2510.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(2-trifluoromethyl-phenyl)-acetamide (cmpd 94)

(ESI) *m*/*z* 545 [(M + H)⁺]. HRMS (ESI) calculated for C₂₉H₂₄F₃N₆O₂ [(M + H)⁺] 545.1908; found 545.192.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-naphthalen-1-yl-acetamide (cmpd 95)

¹H NMR (401 MHz, DMSO-*d₆*) δ ppm 2.87 (t, J=6.90 Hz, 2 H) 3.44 (td, J=6.74, 2.50 Hz, 2 H) 3.83 (s, 2 H) 3.86 (s, 3 H) 7.07 (s, 1 H) 7.18 (t, J=2.62 Hz, 1 H) 7.31 - 7.49 (m, 1 H) 7.67 (d, J=7.20 Hz, 1 H) 7.75 (d, J=8.18 Hz, 1 H) 7.88 - 7.97 (m, 1 H) 8.01 - 8.09 (m, 1 H) 8.44 (s, 1 H) 10.16 (s, 1 H).
(ESI) *m*/*z* 527 [(M + H)⁺]. HRMS (ESI) calculated for C₃₂H₂₇N₆O₂ [(M + H)⁺] 527.2190; found 527.2190.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-benzyloxy-phenyl)-acetamide (cmpd 96)

¹H NMR (401 MHz, DMSO-*d₆*) δ ppm 2.90 (t, J=6.84 Hz, 2 H) 3.46 (td, J=6.84, 2.44 Hz, 2 H) 3.61 (s, 2 H) 3.85 (s, 3 H) 5.05 (s, 2 H) 6.89 - 6.98 (m, 1 H) 7.06 (s, 1 H) 7.21 (t, J=2.32 Hz, 1 H) 7.26 - 7.46 (m, 1 H) 7.46 - 7.54 (m, 1 H) 8.43 (s, 1 H) 10.04 (s, 1 H).
(ESI) *m*/*z* 583 [(M + H)⁺]. HRMS (ESI) calculated for C₃₅H₃₁N₆O₃ [(M + H)⁺] 583.2452; found 583.2467.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3-methyl-cinnolin-5-yl)-acetamide (cmpd 97)

(ESI) *m*/*z* 543 [(M + H)⁺]. HRMS (ESI) calculated for C₃₁H₂₇N₈O₂ [(M + H)⁺] 543.2252; found 543.2253.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3-benzyloxy-phenyl)-acetamide (cmpd 98)

¹H NMR (401 MHz, DMSO-*d₆*) δ ppm 2.89 (t, J=6.84 Hz, 2 H) 3.46 (td, J=6.80, 2.38 Hz, 2 H) 3.64 (s, 2 H) 3.85 (s, 3 H) 5.05 (s, 2 H) 6.69 (dt, J=9.28, 1.10 Hz, 1 H) 7.07 (s, 2 H) 7.10 - 7.15 (m, 1 H) 7.16 - 7.21 (m, 1 H) 7.22 (t, J=2.14 Hz, 1 H) 7.28 - 7.46 (m, 9 H) 7.52 (s, 1 H) 7.54 (s, 1 H) 8.44 (s, 1 H) 10.16 (s, 1 H).
(ESI) *m*/*z* 583 [(M + H)⁺]. HRMS (ESI) calculated for C₃₅H₃₁N₆O₃ [(M + H)⁺] 583.2452; found 583.2452.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-benzo[1,3]dioxol-5-yl-acetamide (cmpd 99)

¹H NMR (401 MHz, DMSO-*d₆*) δ ppm 2.90 (t, J=6.96 Hz, 2 H) 3.46 (td, J=6.90, 2.44 Hz, 2 H) 3.60 (s, 2 H) 3.85 (s, 3 H) 5.96 (s, 2 H) 6.83 (d, J=8.42 Hz, 1 H) 6.96 (dd, J=8.42, 2.08 Hz, 1 H) 7.07 (s, 2 H) 7.21 (s, 1 H) 7.29 (d, J=2.07 Hz, 1 H) 7.30 - 7.39 (m, 3 H) 7.50 (s, 1 H) 7.53 (s, 1 H) 8.44 (s, 1 H) 10.08 (s, 1 H).
(ESI) *m*/*z* 521 [(M + H)⁺]. HRMS (ESI) calculated for C₂₉H₂₅N₆O₄ [(M + H)⁺] 521.1932; found 521.1923.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-indan-5-yl-acetamide (cmpd 100)

¹H NMR (401 MHz, DMSO-*d₆*) δ ppm 1.93 - 2.03 (m, 2 H) 2.74 - 2.84 (m, 4 H) 2.90 (t, J=6.84 Hz, 2 H) 3.46 (td, J=6.87, 2.50 Hz, 2 H) 3.62 (s, 2 H) 3.85 (s, 3 H) 7.06 (s, 2 H) 7.11 (d, J=8.06 Hz, 1 H) 7.21 (s, 1 H) 7.28 (dd, J=8.12, 1.77 Hz, 1 H) 7.31 - 7.39 (m, 3 H) 7.48 - 7.54 (m, 3 H) 8.43 (s, 1 H) 10.03 (s, 1 H).
(ESI) *m*/*z* 517 [(M + H)⁺]. HRMS (ESI) calculated for C₃₁H₂₉N₆O₂ [(M + H)⁺] 517.2347; found 517.2346.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-phenoxy-phenyl)-acetamide (cmpd 101)

¹H NMR (401 MHz, DMSO-*d₆*) δ ppm 2.90 (t, J=6.90 Hz, 2 H) 3.46 (d, J=2.44 Hz, 2 H) 3.64 (s, 2 H) 3.85 (s, 3 H) 6.91 - 7.00 (m, 4 H) 7.07 (s, 2 H) 7.08 - 7.12 (m, 1 H) 7.21 (s, 1 H) 7.30 - 7.40 (m, 5 H) 7.52 (br. s., 1 H) 7.53 (s, 1 H) 7.58 - 7.64 (m, 2 H) 8.44 (s, 1 H) 10.20 (s, 1 H).
(ESI) *m*/*z* 569 [(M + H)⁺]. HRMS (ESI) calculated for C₃₄H₂₉N₆O₃ [(M + H)⁺] 569.2296; found 569.2306.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-benzothiazol-6-yl-acetamide (cmpd 102)

(ESI) *m*/*z* 534 [(M + H)⁺]. HRMS (ESI) calculated for C₂₉H₂₄N₇O₂S [(M + H)⁺] 534.1707; found 534.1707.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(2,3-dihydro-benzo[1,4]dioxin-6-yl)-acetamide (cmpd 103)

¹H NMR (401 MHz, DMSO-*d₆*) δ ppm 2.90 (t, J=6.84 Hz, 2 H) 3.46 (td, J=6.84, 2.44 Hz, 2 H) 3.59 (s, 2 H) 3.85 (s, 3 H) 4.15 - 4.22 (m, 4 H) 6.76 (d, J=8.67 Hz, 1 H) 6.97 (dd, J=8.79, 2.44 Hz, 1 H) 7.06 (s, 2 H) 7.21 (d, J=2.44 Hz, 1 H) 7.27 - 7.38 (m, 3 H) 7.49 (d, J=1.46 Hz, 1 H) 7.52 - 7.54 (m, 1 H) 8.43 (s, 1 H) 10.00 (s, 1 H).
(ESI) *m*/*z* 535 [(M + H)⁺]. HRMS (ESI) calculated for C₃₀H₂₇N₆O₄ [(M + H)⁺] 535.2089; found 535.2090.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(5-tert-butyl-isoxazol-3-yl)-acetamide (cmpd 104)

(ESI) *m*/*z* 524 [(M + H)⁺]. HRMS (ESI) calculated for C₂₉H₃₀N₇O₃ [(M + H)⁺] 524.2405; found 524.2411.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(5-tert-butyl-2-methyl-2H-pyrazol-3-yl)-acetamide (cmpd 105)

¹H NMR (600 MHz, DMSO-*d₆*) δ ppm 1.18 (s, 10 H) 2.54 (s, 8 H) 2.89 (t, J=6.87 Hz, 2 H) 3.45 (td, J=6.87, 2.38 Hz, 2 H) 3.56 (s, 3 H) 3.68 (s, 2 H) 3.85 (s, 3 H) 6.05 (s, 1 H) 7.07 (s, 2 H) 7.18 (br. s., 1 H) 7.30 - 7.39 (m, 3 H) 7.50 - 7.54 (m, 2 H) 8.44 (s, 1 H) 10.07 (s, 1 H).
(ESI) *m*/*z* 537 [(M + H)⁺]. HRMS (ESI) calculated for C₃₀H₃₃N₈O₂ [(M + H)⁺] 537.2721; found 537.2733.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-quinolin-3-yl-acetamide (cmpd 106)

¹H NMR (600 MHz, DMSO-*d₆*) δ ppm 2.90 (t, J=6.87 Hz, 2 H) 3.46 (td, J=6.87, 2.38 Hz, 2 H) 3.77 (s, 2 H) 3.86 (s, 3 H) 7.07 (s, 2 H) 7.22 (br. s., 1 H) 7.34 - 7.41 (m, 3 H) 7.90 (d, J=7.69 Hz, 1 H) 7.94 (d, J=8.43 Hz, 1 H) 8.44 (s, 1 H) 8.70 (d, J=2.38 Hz, 1 H) 8.93 (d, J=2.56 Hz, 1 H) 10.67 (s, 1 H).
(ESI) *m*/*z* 528 [(M + H)⁺]. HRMS (ESI) calculated for C₃₁H₂₆N₇O₂ [(M + H)⁺] 528.2143; found 528.2151.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(2,5-dimethyl-2H-pyrazol-3-yl)-acetamide (cmpd 107)

(ESI) *m*/*z* 495 [(M + H)⁺]. HRMS (ESI) calculated for C₂₇H₂₇N₈O₂ [(M + H)⁺] 495.2252; found 495.2255.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3-methyl-isothiazol-5-yl)-acetamide (cmpd 108)

¹H NMR (600 MHz, DMSO-*d₆*) δ ppm 2.30 (s, 3 H) 2.90 (t, J=6.87 Hz, 2 H) 3.46 (td, J=6.73, 2.29 Hz, 2 H) 3.79 (s, 2 H) 3.85 (s, 3 H) 6.73 (s, 1 H) 7.08 (s, 2 H) 7.20 (br. s., 1 H) 7.31 (m, J=4.40 Hz, 1 H) 7.34 - 7.41 (m, 2 H) 7.49 (s, 1 H) 7.51 - 7.53 (m, 1 H) 8.44 (s, 1 H) 12.02 (s, 1 H).
(ESI) *m*/*z* 498 [(M + H)⁺]. HRMS (ESI) calculated for C₂₆H₂₄N₇O₂S [(M + H)⁺] 498.1707; found 498.1705.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(R)-1,2,3,4-tetrahydro-naphthalen-1-yl-acetamide (cmpd 109)

¹H NMR (600 MHz, DMSO-*d₆*) δ ppm 1.60 - 1.79 (m, 2 H) 1.80 - 1.90 (m, 2 H) 2.65 - 2.79 (m, 2 H) 2.87 (td, J=6.87, 3.48 Hz, 2 H) 3.40 - 3.52 (m, 4 H) 3.85 (s, 3 H) 4.89 - 4.97 (m, 1 H) 7.02 - 7.14 (m, 8 H) 7.26 - 7.37 (m, 3 H) 7.48 (s, 1 H) 7.49 (s, 1 H) 8.43 (s, 1 H) 8.45 (d, J=8.42 Hz, 1 H).
(ESI) *m*/*z* 531 [(M + H)⁺]. HRMS (ESI) calculated for C₃₂H₃₁N₆O₂ [(M + H)⁺] 531.2503; found 531.2511.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(S)-1,2,3,4-tetrahydro-naphthalen-1-yl-acetamide (cmpd 110)

¹H NMR (600 MHz, DMSO-*d₆*) δ ppm 1.62 - 1.94 (m, 5 H) 2.64 - 2.80 (m, 2 H) 2.87 (td, J=6.87, 3.48 Hz, 2 H) 3.40 - 3.45 (m, 2 H) 3.45 - 3.51 (m, 2 H) 3.85 (s, 3 H) 4.88 - 4.96 (m, 1 H) 7.02 - 7.16 (m, 8 H) 7.27 - 7.37 (m, 3 H) 7.48 (s, 1 H) 7.49 (s, 1 H) 8.43 (s, 1 H) 8.45 (d, J=8.61 Hz, 1 H).
(ESI) *m*/*z* 531 [(M + H)⁺]. HRMS (ESI) calculated for C₃₂H₃₁N₆O₂ [(M + H)⁺] 531.2503; found 531.2515.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3-trifluoromethyl-benzyl)-acetamide (cmpd 111)

¹H NMR (600 MHz, DMSO-*d₆*) δ ppm 2.87 (t, J=6.78 Hz, 2 H) 3.44 (td, J=6.87, 2.38 Hz, 2 H) 3.51 (s, 2 H) 3.85 (s, 3 H) 4.36 (d, J=5.86 Hz, 2 H) 7.07 (s, 2 H) 7.17 (br. s., 1 H) 7.26 - 7.35 (m, 3 H) 7.47 (s, 1 H) 8.42 (s, 1 H) 8.67 (t, J=6.04 Hz, 1 H).
(ESI) *m*/*z* 559 [(M + H)⁺]. HRMS (ESI) calculated for C₃₀H₂₆F₃N₆O₂ [(M + H)⁺] 559.2064; found 559.2065.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-trifluoromethyl-benzyl)-acetamide (cmpd 112)

¹H NMR (600 MHz, DMSO-*d₆*) δ ppm 2.87 (t, J=6.96 Hz, 2 H) 3.44 (td, J=6.87, 2.56 Hz, 2 H) 3.51 (s, 2 H) 3.85 (s, 3 H) 4.36 (d, J=5.86 Hz, 2 H) 7.07 (s, 2 H) 7.17 (br. s., 1 H) 7.26 - 7.35 (m, 3 H) 7.43 (d, J=7.88 Hz, 2 H) 7.47 (s, 1 H) 7.51 (s, 1 H) 8.43 (s, 1 H) 8.66 (t, J=5.95 Hz, 1 H).
(ESI) *m*/*z* 559 [(M + H)⁺]. HRMS (ESI) calculated for C₃₀H₂₆F₃N₆O₂ [(M + H)⁺] 559.2064; found 559.2064.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-((S)-1-phenyl-ethyl)-acetamide (cmpd 113)

¹H NMR (600 MHz, DMSO-*d₆*) δ ppm 1.35 (d, J=6.96 Hz, 3 H) 2.88 (td, J=6.82, 2.47 Hz, 2 H) 3.41 - 3.50 (m, 5 H) 3.85 (s, 3 H) 4.88 (m, 1 H) 7.07 (s, 2 H) 7.15 - 7.29 (m, 6 H) 7.31 (d, J=5.13 Hz, 2 H) 7.46 (s, 1 H) 7.53 (s, 1 H) 8.43 (s, 1 H) 8.53 (d, J=8.06 Hz, 1 H).
(ESI) *m*/*z* 505 [(M + H)⁺]. HRMS (ESI) calculated for C₃₀H₂₉N₆O₂ [(M + H)⁺] 505.2347; found 505.2355.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3-fluoro-benzyl)-acetamide (cmpd 114)

¹H NMR (600 MHz, DMSO-*d₆*) δ ppm 2.87 (t, J=6.87 Hz, 2 H) 3.44 (t, J=5.68 Hz, 2 H) 3.50 (s, 2 H) 3.85 (s, 3 H) 4.29 (d, J=5.86 Hz, 2 H) 6.95 - 7.09 (m, 5 H) 7.17 (br. s., 1 H) 7.25 - 7.38 (m, 4 H) 7.48 (s, 1 H) 7.51 (s, 1 H) 8.43 (s, 1 H) 8.60 (t, J=5.59 Hz, 1 H).
(ESI) *m*/*z* 509 [(M + H)⁺]. HRMS (ESI) calculated for C₂₉H₂₆FN₆O₂ [(M + H)⁺] 509.2096; found 509.2106.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(2-fluoro-benzyl)-acetamide (cmpd 115)

¹H NMR (600 MHz, DMSO-*d₆*) δ ppm 2.84 (t, J=6.87 Hz, 2 H) 3.41 (td, J=6.82, 2.47 Hz, 2 H) 3.47 (s, 2 H) 3.82 (s, 3 H) 4.28 (d, J=5.49 Hz, 2 H) 7.04 (s, 2 H) 7.13 (s, 1 H) 7.44 (s, 1 H) 7.48 (s, 1 H) 8.41 (s, 1 H) 8.53 (t, J=5.68 Hz, 1 H).
(ESI) *m*/*z* 509 [(M + H)⁺]. HRMS (ESI) calculated for C₂₉H₂₆FN₆O₂ [(M + H)⁺] 509.2096; found 509.2100.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-((R)-1-phenyl-ethyl)-acetamide (cmpd 116)

¹H NMR (600 MHz, DMSO-*d₆*) δ ppm 1.35 (d, J=6.96 Hz, 3 H) 2.88 (td, J=6.82, 2.66 Hz, 2 H) 3.42 - 3.49 (m, 4 H) 3.85 (s, 3 H) 4.84 - 4.92 (m, 1 H) 7.07 (s, 2 H) 7.16 - 7.33 (m, 8 H) 7.46 (s, 1 H) 7.53 (s, 1 H) 8.43 (s, 1 H) 8.53 (d, J=8.1 Hz, 1 H).
(ESI) *m*/*z* 505 [(M + H)⁺]. HRMS (ESI) calculated for C₃₀H₂₉N₆O₂ [(M + H)⁺] 505.2347; found 50.2350.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(2-trifluoromethyl-benzyl)-acetamide (cmpd 117)

¹H NMR (600 MHz, DMSO-*d₆*) δ ppm 2.84 (t, J=6.87 Hz, 2 H) 3.43 (td, J=6.82, 2.66 Hz, 2 H) 3.55 (s, 2 H) 3.85 (s, 3 H) 4.45 (br. s., 2 H) 7.07 (s, 2 H) 7.17 (s, 1 H) 7.27 - 7.37 (m, 3 H) 7.41 - 7.47 (m, 2 H) 7.51 (s, 1 H) 7.69 (d, J=7.69 Hz, 1 H) 8.43 (s, 1 H) 8.63 (t, J=5.86 Hz, 1 H).
(ESI) *m*/*z* 559 [(M + H)⁺]. HRMS (ESI) calculated for C₃₀H₂₆F₃N₆O₂ [(M + H)⁺] 559.2064; found 559.2068.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-((S)-1-phenyl-2-pyrrolidin-1-yl-ethyl)-acetamide (cmpd 118)

¹H NMR (600 MHz, DMSO-*d₆*) δ ppm 1.56 - 1.67 (m, 5 H) 2.33 - 2.45 (m, 5 H) 2.77 (dd, J=12.18, 9.80 Hz, 1 H) 2.89 (t, J=6.78 Hz, 2 H) 3.40 - 3.55 (m, 5 H) 3.85 (s, 3 H) 4.90 (td, J=8.75, 5.40 Hz, 1 H) 7.07 (s, 2 H) 7.16 - 7.24 (m, 2 H) 7.25 - 7.34 (m, 8 H) 7.49 (d, J=2.56 Hz, 1 H) 7.55 (s, 1 H) 8.41 (s, 1 H) 8.53 (d, J=8.24 Hz, 1 H).
(ESI) *m*/*z* 574 [(M + H)⁺]. HRMS (ESI) calculated for C₃₄H₃₆N₇O₂ [(M + H)⁺] 574.2925; found 574.2935.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-((S)-2-morpholin-4-yl-1-phenyl-ethyl)-acetamide (cmpd 119)

¹H NMR (600 MHz, DMSO-*d₆*) δ ppm 2.22 - 2.44 (m, 5 H) 2.58 - 2.64 (m, 1 H) 2.89 (t, J=6.87 Hz, 2 H) 3.36 - 3.52 (m, 8 H) 3.55 (d, J=13.55 Hz, 1 H) 3.86 (s, 3 H) 4.99 (d, J=3.66 Hz, 1 H) 7.07 (s, 2 H) 7.18 - 7.24 (m, 2 H) 7.27 - 7.34 (m, 7 H) 7.54 (s, 1 H) 7.56 (s, 1 H) 8.42 (s, 1 H) 8.51 (d, J=8.24 Hz, 1 H).
(ESI) *m*/*z* 590 [(M + H)⁺]. HRMS (ESI) calculated for C₃₄H₃₆N₇O₃ [(M + H)⁺] 590.2874; found 590.2881.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3-chloro-4-fluoro-phenyl)-acetamide (cmpd 130)

¹H NMR (600 MHz, DMSO-*d₆*) δ ppm 2.90 (t, J=6.87 Hz, 2 H) 3.46 (td, J=6.87, 2.56 Hz, 2 H) 3.65 (s, 2 H) 3.86 (s, 3 H) 7.07 (s, 2 H) 7.22 (br. s., 1 H) 7.30 - 7.38 (m, 5 H) 7.46 - 7.50 (m, 1 H) 7.51 (s, 1 H) 7.52 (s, 1 H) 7.91 (dd, J=6.78, 2.56 Hz, 1 H) 8.44 (s, 1 H) 10.40 (s, 1 H).
(ESI) *m*/*z* 529 [(M + H)⁺]. HRMS (ESI) calculated for C₂₈H₂₃ClFN₆O₂ [(M + H)⁺] 529.1550; found 529.1541.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-methyl-3-trifluoromethyl-phenyl)-acetamide (cmpd 131)

¹H NMR (600 MHz, DMSO-*d₆*) δ ppm 2.35 - 2.38 (m, 3 H) 2.90 (t, J=6.87 Hz, 2 H) 3.46 (td, J=6.91, 2.47 Hz, 2 H) 3.65 - 3.70 (m, 2 H) 3.86 (s, 3 H) 7.07 (s, 2 H) 7.21 (br. s., 1 H) 7.30 - 7.40 (m, 4 H) 7.51 (s, 1 H) 7.53 (s, 1 H) 7.70 (dd, J=8.24, 1.83 Hz, 1 H) 8.01 (d, J=2.01 Hz, 1 H) 8.43 (s, 1 H) 10.41 (s, 1 H).
(ESI) *m*/*z* 559 [(M + H)⁺]. HRMS (ESI) calculated for C₃₀H₂₆F₃N₆O₂ [(M + H)⁺] 559.2064; found 559.2076.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-chloro-phenyl)-acetamide (cmpd 132)

(ESI) *m*/*z* 511 [(M + H)⁺]. HRMS (ESI) calculated for C₂₈H₂₄ClN₆O₂ [(M + H)⁺] 511.1644; found 511.1644.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-isopropyl-phenyl)-acetamide (cmpd 133)

¹H NMR (600 MHz, DMSO-*d₆*) δ ppm 1.16 (d, J=6.78 Hz, 6 H) 2.82 (dt, J=13.74, 6.87 Hz, 1 H) 2.90 (t, J=6.87 Hz, 2 H) 3.46 (td, J=6.91, 2.47 Hz, 2 H) 3.59 - 3.64 (m, 2 H) 3.85 (s, 3 H) 7.07 (s, 2 H) 7.11 - 7.17 (m, 2 H) 7.21 (br. s., 1 H) 7.30 - 7.37 (m, 3 H) 7.46 - 7.50 (m, 2 H) 7.51 (s, 1 H) 7.53 (s, 1 H) 8.43 (s, 1 H) 10.09 (s, 1 H).
(ESI) *m*/*z* 519 [(M + H)⁺]. HRMS (ESI) calculated for C₃₁H₃₁N₆O₂ [(M + H)⁺] 519.2503; found 519.2513.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-chloro-3-methyl-phenyl)-acetamide (cmpd 134)

¹H NMR (600 MHz, DMSO-*d₆*) δ ppm 2.28 (s, 4 H) 2.90 (t, J=6.78 Hz, 2 H) 3.46 (td, J=6.87, 2.56 Hz, 2 H) 3.64 (s, 2 H) 3.86 (s, 3 H) 7.07 (s, 2 H) 7.22 (br. s., 1 H) 7.28 - 7.38 (m, 5 H) 7.44 (dd, J=8.61, 2.38 Hz, 1 H) 7.51 (s, 1 H) 7.53 (s, 1 H) 7.58 (d, J=2.20 Hz, 1 H) 8.43 (s, 1 H) 10.23 (s, 1 H).
(ESI) *m*/*z* 525 [(M + H)⁺]. HRMS (ESI) calculated for C₂₉H₂₆ClN₆O₂ [(M + H)⁺] 525.1801; found 525.1809.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-tert-butyl-phenyl)-acetamide (cmpd 135)

¹H NMR (600 MHz, DMSO-*d₆*) δ ppm 1.24 (s, 9 H) 2.90 (t, J=6.78 Hz, 2 H) 3.46 (td, J=6.87, 2.38 Hz, 2 H) 3.63 (s, 2 H) 3.86 (s, 3 H) 7.07 (s, 2 H) 7.21 (br. s., 1 H) 7.30 (d, J=8.79 Hz, 3 H) 7.31 - 7.37 (m, 4 H) 7.47 - 7.52 (m, 4 H) 7.53 (s, 1 H) 8.43 (s, 1 H) 10.10 (s, 1 H).
(ESI) *m*/*z* 533 [(M + H)⁺]. HRMS (ESI) calculated for C₃₂H₃₂N₆O₂ [(M + H)⁺] 533.2660; found 533.2659.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-phenyl-thiazol-2-yl)-acetamide (cmpd 136)

¹H NMR (600 MHz, DMSO-*d₆*) δ ppm 2.86 (t, J=6.87 Hz, 2 H) 3.43 (td, J=6.82, 2.29 Hz, 2 H) 3.79 (s, 2 H) 3.83 (s, 3 H) 7.04 (s, 2 H) 7.12 (br. s., 1 H) 7.24 - 7.36 (m, 5 H) 7.40 (t, J=7.69 Hz, 2 H) 7.45 - 7.51 (m, 3 H) 7.58 (s, 1 H) 7.87 (d, J=7.33 Hz, 2 H) 8.42 (s, 1 H) 12.46 (br. s., 1 H).
(ESI) *m*/*z* 560 [(M + H)⁺]. HRMS (ESI) calculated for C₃₁H₂₆N₇O₂S [(M + H)⁺] 560.1863; found 560.1868.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3-chloro-4-methyl-phenyl)-acetamide (cmpd 137)

¹H NMR (600 MHz, DMSO-*d₆*) δ ppm 2.26 (s, 3 H) 2.90 (t, J=6.87 Hz, 2 H) 3.46 (td, J=6.82, 2.47 Hz, 2 H) 3.64 (s, 2 H) 3.86 (s, 3 H) 7.07 (s, 2 H) 7.22 (br. s., 1 H) 7.26 (d, J=8.61 Hz, 1 H) 7.31 - 7.39 (m, 5 H) 7.50 - 7.54 (m, 2 H) 7.78 (d, J=2.01 Hz, 1 H) 8.44 (s, 1 H) 10.26 (s, 1 H).
(ESI) *m*/*z* 525 [(M + H)⁺]. HRMS (ESI) calculated for C₂₉H₂₆ClN₆O₂ [(M + H)⁺] 525.1801; found 525.1796.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-chloro-3-(4-ethyl-piperazin-1-ylmethyl)-phenyl]-acetamide (cmpd 138)

¹H NMR (600 MHz, DMSO-*d₆*) δ ppm 0.96 (t, J=7.14 Hz, 3 H) 2.28 (q, J=7.14 Hz, 2 H) 2.32 - 2.47 (m, 6 H) 2.46 (br. s., 0 H) 2.90 (t, J=6.87 Hz, 2 H) 3.46 (td, J=6.87, 2.38 Hz, 2 H) 3.48 (s, 2 H) 3.65 (s, 2 H) 3.86 (s, 3 H) 7.07 (s, 2 H) 7.21 (s, 1 H) 7.30 - 7.40 (m, 4 H) 7.51 (s, 1 H) 7.53 (s, 1 H) 7.60 (dd, J=8.61, 2.56 Hz, 1 H) 7.68 (d, J=2.56 Hz, 1 H) 8.43 (s, 1 H) 10.30 (s, 1 H).
(ESI) *m*/*z* 637 [(M + H)⁺]. HRMS (ESI) calculated for C₃₅H₃₈ClN₈O₂ [(M + H)⁺] 637.2801; found 637.2800.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(6-chloro-pyridin-3-yl)-acetamide (cmpd 139)

¹H NMR (600 MHz, DMSO-*d₆*) δ ppm 2.87 (t, J=6.87 Hz, 2 H) 3.42 - 3.46 (m, 2 H) 3.67 (s, 2 H) 3.83 (s, 3 H) 7.05 (s, 2 H) 7.18 (br. s., 1 H) 7.26 - 7.37 (m, 3 H) 7.43 (d, J=8.61 Hz, 1 H) 7.48 (s, 1 H) 7.50 (s, 1 H) 8.06 (dd, J=8.79, 2.75 Hz, 1 H) 8.41 (s, 1 H) 8.58 (d, J=2.38 Hz, 1 H) 10.52 (br. s., 1 H).
(ESI) *m*/*z* 512 [(M + H)⁺]. HRMS (ESI) calculated for C₂₇H₂₃ClN₇O₂ [(M + H)⁺] 512.1597; found 512.1594.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-pyridin-3-yl-acetamide (cmpd 140)

¹H NMR (600 MHz, DMSO-*d₆*) δ ppm 2.90 (t, J=6.87 Hz, 2 H) 3.46 (td, J=6.87, 2.56 Hz, 2 H) 3.69 (s, 2 H) 3.86 (s, 3 H) 7.07 (s, 2 H) 7.22 (br. s., 1 H) 7.29 - 7.41 (m, 4 H) 7.51 - 7.54 (m, 2 H) 8.02 - 8.06 (m, 1 H) 8.25 (dd, J=4.67, 1.37 Hz, 1 H) 8.44 (s, 1 H) 8.74 (d, J=2.20 Hz, 1 H) 10.40 (s, 1 H).
(ESI) *m*/*z* 478 [(M + H)⁺]. HRMS (ESI) calculated for C₂₇H₂₄N₇O₂ [(M + H)⁺] 478.1986; found 478.1985.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-chloro-3-trifluoromethyl-phenyl)-acetamide (cmpd 141)

¹H NMR (600 MHz, DMSO-*d₆*) δ ppm 2.90 (t, J=6.87 Hz, 2 H) 3.46 (td, J=6.82, 2.47 Hz, 2 H) 3.69 (s, 2 H) 3.86 (s, 3 H) 7.07 (s, 2 H) 7.21 (br. s., 1 H) 7.29 - 7.40 (m, 4 H) 7.50 - 7.53 (m, 2 H) 7.65 (d, J=8.79 Hz, 1 H) 7.84 (dd, J=8.61, 2.38 Hz, 1 H) 8.19 (d, J=2.56 Hz, 1 H) 8.43 (s, 1 H) 10.64 (br. s., 1 H).
(ESI) *m*/*z* 579 [(M + H)⁺]. HRMS (ESI) calculated for C₂₉H₂₃ClF₃N₇O₂ [(M + H)⁺] 579.1518; found 579.1526.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3,4-dimethoxy-phenyl)-acetamide (cmpd 142)

¹H NMR (600 MHz, DMSO-*d₆*) δ ppm 2.90 (t, J=6.78 Hz, 2 H) 3.46 (td, J=6.91, 2.47 Hz, 2 H) 3.61 (s, 2 H) 3.70 (d, 6 H) 3.86 (s, 3 H) 6.87 (d, J=8.79 Hz, 1 H) 7.07 (s, 2 H) 7.09 (dd, J=8.61, 2.38 Hz, 1 H) 7.23 (br. s., 1 H) 7.30 (d, J=2.38 Hz, 1 H) 7.32 - 7.39 (m, 3 H) 7.52 (br. s., 1 H) 7.54 (s, 1 H) 8.44 (s, 1 H) 10.04 (s, 1 H).
(ESI) *m*/*z* 537 [(M + H)⁺]. HRMS (ESI) calculated for C₃₀H₂₉N₆O₄ [(M + H)⁺] 537.2245; found 537.2258.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3-hydroxy-4-methoxy-phenyl)-acetamide (cmpd 143)

(ESI) *m*/*z* 523 [(M + H)⁺]. HRMS (ESI) calculated for C₂₉H₂₇N₆O₄ [(M + H)⁺] 523.2089; found 523.2084.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3,4-dichloro-phenyl)-acetamide (cmpd 144)

(ESI) *m*/*z* 545 [(M + H)⁺]. HRMS (ESI) calculated for C₂₈H₂₃Cl₂N₆O₂ [(M + H)⁺] 545.1254; found 545.1262.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3-chloro-4-hydroxy-phenyl)-acetamide (cmpd 145)

(ESI) *m*/*z* 527 [(M + H)⁺]. HRMS (ESI) calculated for C₂₈H₂₄ClN₆O₃ [(M + H)⁺] 527.1593; found 527.1595.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(6-methoxy-pyridin-3-yl)-acetamide (cmpd 146)

(ESI) *m*/*z* 508 [(M + H)⁺]. HRMS (ESI) calculated for C₂₈H₂₆N₇O₃ [(M + H)⁺] 508.2092; found 508.2095.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(5-cyclopropyl-[1,3,4]thiadiazol-2-yl)-acetamide (cmpd 147)

¹H NMR (600 MHz, DMSO-*d₆*) δ ppm 0.82 - 0.94 (m, 2 H) 1.01 - 1.08 (m, 2 H) 2.22 - 2.32 (m, 1 H) 2.89 (t, J=6.87 Hz, 2 H) 3.45 (td, J=6.91, 2.47 Hz, 2 H) 3.68 (s, 2 H) 3.85 (s, 3 H) 7.06 (s, 2 H) 7.12 (br. s., 1 H) 7.26 - 7.35 (m, 3 H) 7.44 (s, 1 H) 7.49 (s, 1 H) 8.44 (s, 1 H).
(ESI) *m*/*z* 525 [(M + H)⁺]. HRMS (ESI) calculated for C₂₇H₂₅N₈O₂S [(M + H)⁺] 525.1816; found 525.1831.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-pyridin-4-yl-acetamide (cmpd 151)

(ESI) *m*/*z* 478 [(M + H)⁺]. HRMS (ESI) calculated for C₂₇H₂₄N₇O₂ [(M + H)⁺] 478.1986; found 478.1987.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(3-chloro-phenyl)-acetamide (cmpd 152)

¹H NMR (600 MHz, DMSO-*d₆*) δ ppm 2.90 (t, J=6.87 Hz, 2 H) 3.46 (td, J=6.87, 2.56 Hz, 2 H) 3.66 (s, 2 H) 3.86 (s, 3 H) 7.07 (s, 2 H) 7.09 - 7.11 (m, 1 H) 7.22 (br. s., 1 H) 7.28 - 7.39 (m, 4 H) 7.44 - 7.47 (m, 1 H) 7.51 (s, 1 H) 7.53 (s, 1 H) 7.80 (t, J=1.92 Hz, 1 H) 8.44 (s, 1 H) 10.37 (s, 1 H).
(ESI) *m*/*z* 511 [(M + H)⁺]. HRMS (ESI) calculated for C₂₈H₂₄ClN₆O₂ [(M + H)⁺] 511.1644; found 511.1642.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[3-chloro-4-(4-ethyl-piperazin-1-ylmethyl)-phenyl]-acetamide (cmpd 169)

¹H NMR (600 MHz, DMSO-*d₆*) δ ppm 0.96 (t, J=7.23 Hz, 3 H) 2.23 - 2.46 (m, 10 H) 2.90 (t, J=6.78 Hz, 2 H) 3.44 - 3.50 (m, 4 H) 3.65 (s, 2 H) 3.86 (s, 3 H) 7.07 (s, 2 H) 7.22 (br. s., 1 H) 7.30 - 7.38 (m, 4 H) 7.43 (dd, J=8.52, 1.92 Hz, 1 H) 7.51 (s, 1 H) 7.53 (s, 1 H) 7.79 (d, J=2.01 Hz, 1 H) 8.44 (s, 1 H) 10.32 (s, 1 H).
(ESI) *m*/*z* 637 [(M + H)⁺]. HRMS (ESI) calculated for C₃₅H₃₈ClN₈O₂ [(M + H)⁺] 637.2801; found 637.2799.

### Preparation 39

### 2-[2-Amino-5-(3-{[4-(4-propyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

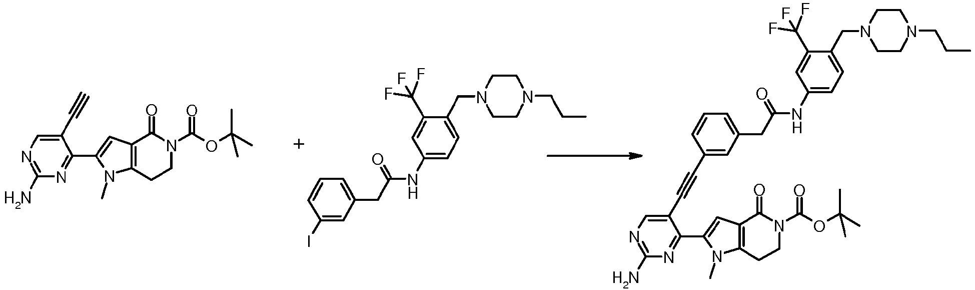

A solution of 2-(2-amino-5-ethynyl-pyrimidin-4-yl)-1-methyl-4-oxo-1,4,6,7-tetrahydro-pyrrolo[3,2-c]pyridine-5-carboxylic acid tert-butyl ester (0.10 g, 0.27 mmol), 2-(3-iodo-phenyl)-N-[4-(4-propyl-piperazin-1-ylmethyl)-3-trifluoromethyl-phenyl]-acetamide (0.14 g, 0.26 mmol), CuI (10% mol, 5 mg, 0.026 mmol), PdCl₂(PPh₃)₂ (10% mol, 18 mg, 0.026 mmol) and TEA (0.35 mL, 2.60 mmol) in DMF (3 mL) was degassed with argon and stirred at r.t. for 4 h. The reaction was poured in water (15 mL) and extracted with EtOAc (2 x 15 mL). The organic phase was washed with brine, dried with anhydrous Na₂SO₄ and evaporated under vacuum to give a crude that was purified by flash column chromatography (DCM/MeOH/NH₃ 90/10/0.4). The title product (0.10 g, 49%) was obtained as yellowish solid.
¹H NMR (600 MHz, DMSO-*d*₆) δ ppm 0.83 (t, *J*=7.33 Hz, 3 H) 1.37 - 1.43 (m, 2 H) 1.45 (s, 9 H) 2.20 (t, *J*=7.05 Hz, 2 H) 2.27 - 2.42 (m, 8 H) 3.00 (t, *J*=6.32 Hz, 2 H) 3.52 (s, 2 H) 3.68 (s, 2 H) 3.85 (s, 3 H) 4.01 (t, *J*=6.32 Hz, 2 H) 7.13 (s, 2 H) 7.24 - 7.40 (m, 3 H) 7.47 (s, 1 H) 7.57 (s, 1 H) 7.64 (d, *J*=8.61 Hz, 1 H) 7.74 - 7.80 (m, 1 H) 8.03 (d, *J*=1.83 Hz, 1 H) 8.47 (s, 1 H) 10.42 (s, 1 H).

According to this same methodology, but employing suitable substituted derivatives, the following intermediates were prepared:

### 2-[2-Amino-5-(3-{[4-(4-ethyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.97 (t, *J*=7.14 Hz, 4 H) 1.44 (s, 11 H) 3.00 (t, *J*=6.32 Hz, 2 H) 3.52 (s, 2 H) 3.68 (s, 2 H) 3.85 (s, 3 H) 4.01 (t, *J*=6.23 Hz, 2 H) 7.13 (s, 2 H) 7.31 - 7.40 (m, 4 H) 7.47 (s, 1 H) 7.57 (s, 1 H) 7.64 (d, *J*=8.61 Hz, 1 H) 7.77 (d, *J*=8.06 Hz, 1 H) 8.03 (d, *J*=1.65 Hz, 1 H) 8.47 (s, 1 H) 10.42 (s, 1 H).

### 2-[2-(Acetyl-methyl-amino)-5-(3-{[4-(4-ethyl-piperazin-1-ylmethyl)-3-trifluoromethyl-phenylcarbamoyl]-methyl)-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.97 (t, *J*=7.14 Hz, 3 H) 1.45 (s, 9 H) 2.29 (q, *J*=7.27 Hz, 3 H) 2.31 - 2.39 (m, 5 H) 2.42 (s, 3 H) 3.03 (t, *J*=6.23 Hz, 2 H) 3.39 - 3.43 (m, 3 H) 3.52 (s, 2 H) 3.71 (s, 2 H) 3.86 (s, 3 H) 3.99 - 4.06 (m, 2 H) 7.34 - 7.48 (m, 3 H) 7.56 (s, 1 H) 7.65 (d, *J*=8.43 Hz, 1 H) 7.75 (s, 1 H) 7.77 (d, *J*=6.78 Hz, 1 H) 8.04 (d, *J*=1.65 Hz, 1 H) 8.92 (s, 1 H) 10.44 (s, 1 H).

### 2-[2-Amino-5-(3-{[4-(4-ethyl-piperazin-1-ylmethyl)-3-fluoro-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.95 (t, *J*=7.14 Hz, 3 H) 1.45 (s, 9 H) 2.19 - 2.43 (m, 11 H) 3.00 (t, *J*=6.32 Hz, 2 H) 3.66 (s, 2 H) 3.84 (s, 3 H) 4.01 (t, *J*=6.23 Hz, 2 H) 7.13 (s, 1 H) 7.23 - 7.30 (m, 2 H) 7.31 - 7.39 (m, 3 H) 7.46 (s, 1 H) 7.54 (dd, *J*=12.36, 1.56 Hz, 1 H) 7.55 - 7.57 (m, 1 H) 7.70 - 7.77 (m, 2 H) 8.47 (s, 1 H) 10.32 (s, 1 H).

### 2-[2-Amino-5-(3-{[4-(2-dimethylamino-ethylcarbamoyl)-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.45 (s, 9 H) 2.19 (br. s., 6 H) 2.41 (br. s., 2 H) 3.00 (t, *J*=6.32 Hz, 2 H) 3.69 (s, 2 H) 3.85 (s, 3 H) 4.01 (t, *J*=6.32 Hz, 2 H) 7.13 (s, 2 H) 7.30 - 7.41 (m, 3 H) 7.47 (s, 1 H) 7.57 (s, 1 H) 7.65 (d, *J*=8.79 Hz, 2 H) 7.78 (d, *J*=8.61 Hz, 2 H) 8.24 (t, *J*=5.40 Hz, 1 H) 8.47 (s, 1 H) 10.35 (s, 1 H).

### 2-[2-Amino-5-(4-{[4-(4-ethyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.97 (t, *J*=7.23 Hz, 3 H) 1.45 (s, 10 H) 2.18 - 2.47 (m, 10 H) 2.98 (t, *J*=6.23 Hz, 2 H) 3.52 (s, 2 H) 3.69 (s, 2 H) 3.84 (s, 3 H) 3.99 (t, *J*=6.23 Hz, 2 H) 7.11 (s, 2 H) 7.36 (d, *J*=8.24 Hz, 2 H) 7.41 - 7.45 (m, 2 H) 7.55 (s, 1 H) 7.65 (d, *J*=8.61 Hz, 1 H) 7.77 (d, *J*=8.61 Hz, 1 H) 8.04 (d, *J*=1.83 Hz, 1 H) 8.46 (s, 1 H) 10.47 (s, 1 H).

### 2-[2-Amino-5-(3-{[2-(4-ethyl-piperazin-1-ylmethyl)-5-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 771 [(M + H)⁺]. HRMS (ESI) calculated for C₄₁H₄₆F₃N₈O₄⁺ [(M + H)⁺] 771.8424, found 771.8422.

### 2-[2-Amino-5-(3-{[4-chloro-3-(4-ethyl-piperazine-1-carbonyl)-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 752 [(M + H)⁺]. HRMS (ESI) calculated for C₄₀H₄₄ClN₈O₅⁺ [(M + H)⁺] 752.2730, found 752.2731.

### 2-[2-Amino-5-(3-{[3-(4-ethyl-piperazine-1-carbonyl)-4-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 785 [(M + H)⁺]. HRMS (ESI) calculated for C₄₁H₄₄F₃N₈O₅⁺ [(M + H)⁺] 785.8259, found 785.8261.

### 2-[2-Amino-5-(3-{[4-(4-methyl-[1,4]diazepan-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.45 (s, 9 H) 1.71 (quin, *J*=5.82 Hz, 2 H) 2.27 (br. s., 3 H) 2.53 - 2.67 (m, 8 H) 3.00 (t, *J*=6.32 Hz, 2 H) 3.67 (d, *J*=7.88 Hz, 4 H) 3.85 (s, 3 H) 4.01 (t, *J*=6.32 Hz, 2 H) 7.13 (s, 2 H) 7.26 - 7.41 (m, 3 H) 7.47 (s, 1 H) 7.57 (s, 1 H) 7.70 (d, *J*=8.61 Hz, 1 H) 7.77 (dd, *J*=8.61, 1.65 Hz, 1 H) 8.02 (d, *J*=1.83 Hz, 1 H) 8.47 (s, 1 H) 10.41 (s, 1 H).

### 2-[2-Amino-5-(3-{[4-((S)-3-dimethylamino-pyrrolidin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.43 - 1.46 (m, 9 H) 1.61 (dd, *J*=12.64, 8.24 Hz, 1 H) 1.84 (dd, *J*=13.28, 5.04 Hz, 1 H) 2.05 - 2.11 (m, 6 H) 2.29 - 2.34 (m, 1 H) 2.43 - 2.48 (m, 1 H) 2.53 - 2.58 (m, 1 H) 2.59 - 2.64 (m, 1 H) 2.73 (d, *J*=0.55 Hz, 1 H) 3.00 (t, *J*=6.32 Hz, 2 H) 3.54 - 3.70 (m, 4 H) 3.85 (s, 3 H) 4.01 (t, *J*=6.32 Hz, 2 H) 7.13 (s, 2 H) 7.31 - 7.40 (m, 3 H) 7.47 (s, 1 H) 7.57 (s, 1 H) 7.63 (d, *J*=8.61 Hz, 1 H) 7.77 (dd, *J*=8.52, 1.74 Hz, 1 H) 8.02 (d, *J*=2.01 Hz, 1 H) 8.47 (s, 1 H) 10.41 (s, 1 H).

### 2-[2-Amino-5-(3-{[4-(4-ethyl-piperazine-1-carbonyl)-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.99 (t, *J*=7.14 Hz, 3 H) 1.46 (s, 9 H) 2.25 - 2.44 (m, 6 H) 3.00 (t, *J*=6.32 Hz, 2 H) 3.33 - 3.64 (m, 4 H) 3.68 (s, 2 H) 3.85 (s, 3 H) 4.01 (t, *J*=6.32 Hz, 2 H) 7.13 (s, 2 H) 7.31 - 7.34 (m, 2 H) 7.35 - 7.40 (m, 2 H) 7.47 (s, 1 H) 7.56 (s, 1 H) 7.64 (d, *J*=8.61 Hz, 2 H) 8.47 (s, 1 H) 10.31 (s, 1 H).

### 2-[2-Amino-5-(3-{[3-cyclopropyl-4-(4-methyl-piperazin-1-ylmethyl)-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.46 - 0.58 (m, 2 H) 0.84 - 0.94 (m, 2 H) 1.45 (s, 9 H) 2.10 - 2.18 (m, 4 H) 2.21 - 2.48 (m, 7 H) 3.00 (t, *J*=6.32 Hz, 2 H) 3.51 (s, 2 H) 3.62 (s, 2 H) 3.85 (s, 2 H) 4.01 (t, *J*=6.32 Hz, 2 H) 7.08 - 7.18 (m, 4 H) 7.29 - 7.39 (m, 4 H) 7.46 (s, 1 H) 7.57 (s, 1 H) 8.47 (s, 1 H) 10.01 (s, 1 H).

### 2-[2-Amino-5-(3-{[4-((R)-3-dimethylamino-pyrrolidin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.43 - 1.47 (m, 10 H) 1.56 - 1.68 (m, 1 H) 1.77 - 1.88 (m, 1 H) 2.07 (s, 6 H) 2.32 (d, *J*=7.51 Hz, 1 H) 2.46 (d, *J*=5.68 Hz, 1 H) 2.52 - 2.59 (m, 1 H) 2.60 - 2.64 (m, 1 H) 2.73 (s, 1 H) 3.00 (t, *J*=6.23 Hz, 2 H) 3.56 - 3.67 (m, 2 H) 3.68 (s, 2 H) 3.85 (s, 3 H) 4.01 (t, *J*=6.32 Hz, 2 H) 7.13 (s, 2 H) 7.32 - 7.41 (m, 3 H) 7.47 (s, 1 H) 7.57 (s, 1 H) 7.63 (d, *J*=8.43 Hz, 1 H) 7.77 (dd, *J*=8.52, 1.56 Hz, 1 H) 8.02 (d, *J*=2.01 Hz, 1 H) 8.47 (s, 1 H) 10.41 (s, 1 H).

### 2-[2-Amino-5-(3-{[4-(4-isopropyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.95 (d, *J*=5.49 Hz, 6 H) 2.27 - 2.47 (m, 8 H) 2.56 - 2.64 (m, 1 H) 3.00 (t, *J*=6.32 Hz, 2 H) 3.51 (br. s., 2 H) 3.68 (s, 2 H) 3.85 (s, 2 H) 4.01 (t, *J*=6.32 Hz, 2 H) 7.11 - 7.15 (m, 2 H) 7.32 - 7.40 (m, 3 H) 7.47 (s, 1 H) 7.57 (s, 1 H) 7.61 - 7.69 (m, 1 H) 7.77 (d, *J*=8.24 Hz, 1 H) 8.03 (d, *J*=1.83 Hz, 1 H) 8.47 (s, 1 H) 10.42 (s, 1 H).

### 2-(2-Amino-5-{3-[(4-piperidin-1-ylmethyl-3-trifluoromethyl-phenylcarbamoyl)-methyl]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.44 (s, 10 H) 1.46 - 1.54 (m, 4 H) 2.31 (br. s., 4 H) 3.00 (t, *J*=6.41 Hz, 2 H) 3.48 (s, 2 H) 3.68 (s, 2 H) 3.85 (s, 3 H) 4.01 (t, *J*=6.32 Hz, 2 H) 7.13 (s, 2 H) 7.29 - 7.39 (m, 3 H) 7.47 (s, 1 H) 7.57 (s, 1 H) 7.66 (d, *J*=8.61 Hz, 1 H) 7.76 (dd, *J*=8.70, 1.56 Hz, 1 H) 8.03 (d, *J*=1.83 Hz, 1 H) 8.47 (s, 1 H) 10.41 (s, 1 H).

### 2-(2-Amino-5-{3-[(4-morpholin-4-ylmethyl-3-trifluoromethyl-phenylcarbamoyl)-methyl]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.45 (s, 10 H) 2.35 (br. s., 4 H) 3.00 (t, J=6.32 Hz, 2 H) 3.53 (s, 2 H) 3.57 (t, *J*=4.40 Hz, 4 H) 3.68 (s, 2 H) 3.85 (s, 3 H) 4.01 (t, *J*=6.32 Hz, 2 H) 7.13 (s, 2 H) 7.32 - 7.39 (m, 3 H) 7.47 (s, 1 H) 7.57 (s, 1 H) 7.67 (d, *J*=8.43 Hz, 1 H) 7.78 (dd, *J*=8.61, 1.83 Hz, 1 H) 8.04 (d, *J*=2.02 Hz, 1 H) 8.47 (s, 1 H) 10.43 (s, 1 H).

### 2-[2-Amino-5-(3-{[4-(4-methyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 757 [(M + H)⁺]. HRMS (ESI) calculated for C₄₀H₄₄F₃N₈O₄⁺ [(M + H)⁺] 757.8158, found 757.8154.

### 2-[2-Amino-5-(3-{[4-(3-pyrrolidin-1-yl-azetidin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.45 (s, 9 H) 1.60 - 1.71 (m, 4 H) 2.34 (br. s., 4 H) 2.91 (t, *J*=6.59 Hz, 2 H) 3.00 (t, *J*=6.32 Hz, 2 H) 3.03 (m, *J*=6.23 Hz, 1 H) 3.33 - 3.37 (m, 2 H) 3.65 (s, 2 H) 3.68 (s, 2 H) 3.85 (s, 3 H) 4.01 (t, *J*=6.32 Hz, 2 H) 7.13 (s, 2 H) 7.30 - 7.40 (m, 3 H) 7.47 (s, 1 H) 7.54 - 7.62 (m, 2 H) 7.76 (dd, *J*=8.70, 1.56 Hz, 1 H) 8.01 (d, *J*=2.01 Hz, 1 H) 8.47 (s, 1 H) 10.40 (s, 1 H).

### 2-[2-Amino-5-(3-{[4-(4-ethyl-piperazine-1-carbonyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.98 (t, *J*=7.14 Hz, 3 H) 1.45 (s, 10 H) 2.13 - 2.45 (m, 2 H) 2.32 (q, *J*=7.20 Hz, 3 H) 3.00 (t, *J*=6.41 Hz, 2 H) 3.02 - 3.13 (m, 2 H) 3.52 - 3.65 (m, 2 H) 3.71 (s, 2 H) 3.85 (s, 3 H) 4.01 (t, *J*=6.32 Hz, 2 H) 7.13 (s, 2 H) 7.26 (br. s., 1 H) 7.32 - 7.41 (m, 5 H) 7.48 (s, 1 H) 7.57 (s, 1 H) 7.84 (dd, *J*=8.33, 1.56 Hz, 1 H) 8.11 (d, *J*=1.83 Hz, 1 H) 8.47 (s, 1 H) 10.58 (s, 1 H).

### 2-(2-Amino-5-{3-[(4-{[methyl-(1-methyl-piperidin-4-yl)-amino]-methyl}-3-trifluoromethyl-phenylcarbamoyl)-methyl]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.41 - 1.45 (m, 8 H) 1.51 (d, *J*=9.34 Hz, 2 H) 1.67 (d, *J*=11.54 Hz, 2 H) 1.75 - 1.96 (m, 2 H) 2.09 (s, 3 H) 2.15 (br. s., 3 H) 2.34 (br. s., 1 H) 2.81 (br. s., 2 H) 3.00 (t, *J*=6.32 Hz, 2 H) 3.62 (s, 2 H) 3.68 (s, 2 H) 3.85 (s, 3 H) 4.01 (t, *J*=6.41 Hz, 2 H) 7.13 (s, 2 H) 7.31 - 7.39 (m, 3 H) 7.47 (s, 1 H) 7.57 (s, 1 H) 7.68 (d, *J*=8.42 Hz, 1 H) 7.76 (dd, *J*=8.43, 1.65 Hz, 1 H) 8.02 (d, *J*=2.01 Hz, 1 H) 8.47 (s, 1 H) 10.41 (s, 1 H).

### 2-[2-Amino-5-(3-{[4-(4-dimethylamino-piperidin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.38 (d, *J*=8.97 Hz, 2 H) 1.44 (s, 9 H) 1.70 (d, *J*=11.72 Hz, 2 H) 1.95 (t, *J*=10.81 Hz, 2 H) 1.99 - 2.24 (m, 7 H) 2.78 (d, *J*=11.17 Hz, 2 H) 3.00 (t, *J*=6.23 Hz, 2 H) 3.50 (s, 2 H) 3.68 (s, 2 H) 3.85 (s, 3 H) 4.01 (t, *J*=6.32 Hz, 2 H) 7.13 (s, 2 H) 7.30 - 7.40 (m, 3 H) 7.47 (s, 1 H) 7.57 (s, 1 H) 7.66 (d, *J*=8.61 Hz, 1 H) 7.75 - 7.79 (m, 1 H) 8.03 (d, *J*=2.01 Hz, 1 H) 8.47 (s, 1 H) 10.42 (s, 1 H).

### 2-[2-Amino-5-(3-{[3-bromo-4-(4-ethyl-piperazin-1-ylmethyl)-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 782 [(M + H)⁺]. HRMS (ESI) calculated for C₄₀H₄₆BrN₈O₄⁺ [(M + H)⁺] 782.7405, found 782.7408.

### 2-{5-[3-({4-[4-(2-Acetoxy-ethyl)-piperazin-1-ylmethyl]-3-trifluoromethyl-phenylcarbamoyl}-methyl)-phenylethynyl]-2-amino-pyrimidin-4-yl}-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.44 (s, 9 H) 1.99 (s, 3 H) 2.28 - 2.48 (m, 8 H) 3.00 (t, *J*=6.23 Hz, 2 H) 3.52 (s, 2 H) 3.68 (s, 2 H) 3.85 (s, 3 H) 4.01 (t, *J*=6.32 Hz, 2 H) 4.08 (t, *J*=5.86 Hz, 2 H) 7.13 (s, 2 H) 7.29 - 7.40 (m, 3 H) 7.47 (s, 1 H) 7.57 (s, 1 H) 7.64 (d, *J*=8.61 Hz, 1 H) 7.77 (dd, *J*=8.70, 1.74 Hz, 1 H) 7.95 (s, 1 H) 8.03 (d, *J*=1.83 Hz, 1 H) 8.47 (s, 1 H) 10.42 (s, 1 H).

### 2-[2-Amino-5-(3-{[4-(4-cyclopropylmethyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.05 (br. s., 2 H) 0.44 (d, J=4.21 Hz, 2 H) 0.80 (br. s., 1 H) 1.44 (s, 9 H) 2.16 (br. s., 2 H) 2.19 - 2.47 (m, 9 H) 3.00 (t, *J*=6.23 Hz, 2 H) 3.53 (br. s., 2 H) 3.68 (s, 2 H) 3.85 (s, 3 H) 3.98 - 4.03 (m, 2 H) 7.12 - 7.14 (m, 2 H) 7.30 - 7.39 (m, 4 H) 7.47 (s, 1 H) 7.57 (s, 1 H) 7.62 - 7.67 (m, 1 H) 7.77 (d, *J*=7.88 Hz, 1 H) 8.03 (d, *J*=1.47 Hz, 1 H) 8.47 (s, 1 H) 10.42 (s, 1 H).

### 2-[2-Amino-5-(3-{[4-(1-methyl-piperidin-4-yloxy)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.42 (s, 9 H) 1.63 (d, *J*=8.24 Hz, 2 H) 1.80 - 1.91 (m, 2 H) 2.14 (br. s., 3 H) 2.20 (br. s., 2 H) 2.97 (t, *J*=6.23 Hz, 2 H) 3.61 (s, 2 H) 3.82 (s, 3 H) 3.98 (t, *J*=6.32 Hz, 2 H) 4.50 (br. s., 1 H) 7.10 (s, 2 H) 7.22 (d, *J*=9.16 Hz, 1 H) 7.28 - 7.38 (m, 3 H) 7.44 (s, 1 H) 7.54 (s, 1 H) 7.69 (dd, *J*=8.97, 2.38 Hz, 1 H) 7.89 (d, *J*=2.56 Hz, 1 H) 8.44 (s, 1 H) 10.21 (s, 1 H).

### 2-[2-Amino-5-(3-{[4-(4-ethyl-piperazin-1-yl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.01 (t, *J*=7.23 Hz, 3 H) 1.45 (s, 9 H) 2.26 - 2.49 (m, 9 H) 2.81 (t, *J*=4.40 Hz, 4 H) 3.00 (t, *J*=6.23 Hz, 2 H) 3.67 (s, 2 H) 3.85 (s, 3 H) 4.01 (t, *J*=6.32 Hz, 2 H) 7.13 (s, 2 H) 7.31 - 7.39 (m, 3 H) 7.46 (s, 1 H) 7.50 - 7.53 (m, 1 H) 7.57 (s, 1 H) 7.77 (dd, *J*=8.70, 2.11 Hz, 1 H) 7.98 (d, *J*=2.38 Hz, 1 H) 8.47 (s, 1 H) 10.34 -10.38 (m, 1 H).

### 2-[2-(Acetyl-methyl-amino)-5-(3-{[4-(4-methyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 813 [(M + H)⁺]. HRMS (ESI) calculated for C₄₃H₄₈F₃N₈O₅⁺ [(M + H)⁺] 813.8791, found 813.8788.

### 2-[2-(Acetyl-methyl-amino)-5-(3-{[4-(4-cyclopropyl-piperazin-1-ylmethyl)-3-trifluoromethyl-phenylcarbamoyl]-methyl)-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 839 [(M + H)⁺]. HRMS (ESI) calculated for C₄₅H₅₀F₃N₈O₅⁺ [(M + H)⁺] 839.9164, found 839.9167.

### 2-[2-(Acetyl-methyl-amino)-5-(3-{[4-(4-isopropyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 841 [(M + H)⁺]. HRMS (ESI) calculated for C₄₅H₅₂F₃N₈O₅⁺ [(M + H)⁺] 841.9322, found 841.9319.

### 2-[2-Amino-5-(3-{[4-(1-methyl-piperidin-4-ylamino)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.45 (s, 9 H) 1.47 - 1.52 (m, 2 H) 1.86 (d, *J*=11.54 Hz, 3 H) 2.06 (d, *J*=10.26 Hz, 3 H) 2.16 (s, 4 H) 2.65 (br. s., 2 H) 3.00 (t, *J*=6.41 Hz, 2 H) 3.60 (s, 2 H) 3.85 (s, 3 H) 4.01 (t, *J*=6.32 Hz, 2 H) 4.36 (d, *J*=7.69 Hz, 1 H) 7.25 - 7.39 (m, 4 H) 7.46 (s, 1 H) 7.51 - 7.58 (m, 2 H) 7.78 (d, *J*=2.01 Hz, 1 H) 8.47 (s, 1 H) 10.03 (s, 1 H).

### 2-{2-Amino-5-[3-({4-[methyl-(1-methyl-piperidin-4-yl)-amino]-3-trifluoromethyl-phenylcarbamoyl}-methyl)-phenylethynyl]-pyrimidin-4-yl}-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.29 - 1.39 (m, 2 H) 1.45 (s, 9 H) 1.62 (d, *J*=12.45 Hz, 2 H) 1.78 (br. s., 2 H) 2.10 (s, 3 H) 2.62 - 2.79 (m, 3 H) 3.00 (t, *J*=6.23 Hz, 2 H) 3.67 (s, 2 H) 3.85 (s, 3 H) 4.01 (t, *J*=6.32 Hz, 2 H) 7.13 (s, 2 H) 7.31 - 7.40 (m, 3 H) 7.47 (s, 1 H) 7.49 - 7.52 (m, 1 H) 7.57 (s, 1 H) 7.78 (dd, *J*=8.88, 2.11 Hz, 1 H) 7.96 (d, *J*=2.38 Hz, 1 H) 8.47 (s, 1 H) 10.36 (s, 1 H).

### 2-[2-(Acetyl-methyl-amino)-5-(3-{[4-(4-propyl-piperazin-1-ylmethyl)-3-trifluoromethyl-phenylcarbamoyl]-methyl}-phenylethynyl)-pyrimidin-4-yl]-1-methyl--oxo-1,4,6,7-tetrahydro-pyrrolo[3,2-c]pyridine-5-carboxylic acid tert-butyl ester

(ESI) *m*/*z* 841 [(M + H)⁺]. HRMS (ESI) calculated for C₄₅H₅₂F₃N₈O₅⁺ [(M + H)⁺] 841.9322, found 841.9315.

### 2-[2-Amino-5-(3-{[3-trifluoromethyl-4-((2S,5R)-2,4,5-trimethyl-piperazin-1-ylmethyl)-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.83 (d, *J*=6.23 Hz, 3 H) 0.99 (d, *J*=6.04 Hz, 3 H) 1.43 (br. s., 9 H) 1.75 (t, *J*=10.71 Hz, 1 H) 1.89 (d, *J*=10.44 Hz, 3 H) 2.11 (br. s., 4 H) 2.31 - 2.44 (m, 3 H) 2.66 (d, *J*=10.44 Hz, 1 H) 3.00 (t, *J*=6.32 Hz, 2 H) 3.09 (d, *J*=14.65 Hz, 1 H) 3.68 (s, 2 H) 3.85 (s, 3 H) 3.99 - 4.02 (m, 2 H) 4.05 (s, 1 H) 7.13 (s, 2 H) 7.31 - 7.40 (m, 4 H) 7.47 (s, 1 H) 7.57 (s, 1 H) 7.69 - 7.72 (m, 1 H) 7.75 - 7.77 (m, 1 H) 8.03 (d, *J*=1.83 Hz, 1 H) 8.47 (s, 1 H) 10.41 (s, 1 H).

### 2-[2-Amino-5-(3-{[3-trifluoromethyl-4-(3,4,5-trimethyl-piperazin-1-ylmethyl)-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 785 [(M + H)⁺]. HRMS (ESI) calculated for C₄₂H₄₈F₃N₈O₄⁺ [(M + H)⁺] 785.8690, found 785.8688.

### 2-[2-Amino-5-(3-{[4-(4-tert-butoxycarbonyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.38 (s, 9 H) 1.43 - 1.46 (m, 9 H) 2.31 (t, *J*=4.85 Hz, 5 H) 3.00 (t, *J*=6.23 Hz, 2 H) 3.55 (s, 2 H) 3.68 (s, 2 H) 3.85 (s, 3 H) 4.01 (t, *J*=6.23 Hz, 2 H) 7.13 (s, 2 H) 7.32 - 7.40 (m, 4 H) 7.47 (s, 1 H) 7.53 - 7.55 (m, 1 H) 7.57 (s, 1 H) 7.60 - 7.65 (m, 2 H) 7.66 (d, *J*=8.43 Hz, 1 H) 7.78 (dd, *J*=8.42, 1.83 Hz, 1 H) 8.04 (d, *J*=2.01 Hz, 1 H) 8.47 (s, 1 H) 10.43 (s, 1 H).

### 2-[2-Amino-5-(3-{[4-(3-oxo-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.44 (s, 9 H) 2.51 - 2.55 (m, 3 H) 2.93 (s, 2 H) 3.00 (t, *J*=6.41 Hz, 2 H) 3.13 (br. s., 2 H) 3.61 (s, 2 H) 3.68 (s, 2 H) 3.85 (s, 3 H) 4.01 (t, *J*=6.23 Hz, 2 H) 7.13 (s, 2 H) 7.31 - 7.39 (m, 3 H) 7.47 (s, 1 H) 7.57 (s, 1 H) 7.66 (d, *J*=8.61 Hz, 1 H) 7.74 (s, 1 H) 7.79 (dd, *J*=8.43, 1.83 Hz, 1 H) 8.05 (d, *J*=2.01 Hz, 1 H) 8.47 (s, 1 H) 10.45 (s, 1 H).

### 2-{2-Amino-5-[3-({4-[3-(tert-butyl-dimethyl-silanyloxy)-piperidin-1-ylmethyl]-3-trifluoromethyl-phenylcarbamoyl}-methyl)-phenylethynyl]-pyrimidin-4-yl}-1-methyl-4-oxo-1,4,6,7-tetrahydro-pyrrolo[3,2-c]pyridine-5-carboxylic acid tert-butyl ester

(ESI) *m*/*z* 873 [(M + H)⁺]. HRMS (ESI) calculated for C₄₆H₅₇F₃N₇O₅Si⁺ [(M + H)⁺] 873.0614, found 873.0611.

### 2-[2-Amino-5-(3-cyclopropylcarbamoylmethyl-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.36 - 0.41 (m, 2 H) 0.57 - 0.61 (m, 2 H) 1.47 (s, 9 H) 2.60 - 2.63 (m, 1 H) 3.00 (t, *J*=6.32 Hz, 2 H) 3.34 - 3.36 (m, 2 H) 3.85 (s, 3 H) 4.01 (t, *J*=6.32 Hz, 2 H) 7.13 (s, 2 H) 7.24 (d, *J*=6.96 Hz, 1 H) 7.29 - 7.35 (m, 2 H) 7.37 (s, 1 H) 7.55 (s, 1 H) 8.09 (d, *J*=3.85 Hz, 1 H) 8.47 (s, 1 H).

### 2-[2-Amino-5-(3-{[4-(4-methyl-2-oxo-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.44 (s, 10 H) 2.23 (s, 4 H) 2.55 - 2.64 (m, 3 H) 3.00 (t, *J*=6.32 Hz, 2 H) 3.06 (s, 2 H) 3.18 (t, *J*=5.31 Hz, 2 H) 3.69 (s, 2 H) 3.85 (s, 3 H) 4.01 (t, *J*=6.32 Hz, 2 H) 4.62 (s, 2 H) 7.13 (s, 2 H) 7.24 (d, *J*=8.79 Hz, 1 H) 7.31 - 7.40 (m, 4 H) 7.47 (s, 1 H) 7.57 (s, 1 H) 7.75 - 7.79 (m, 1 H) 7.95 (s, 1 H) 8.11 (d, *J*=1.83 Hz, 1 H) 8.47 (s, 1 H) 10.48 (s, 1 H).

### 2-{2-Amino-5-[3-({4-[4-(tert-butyl-dimethyl-silanyloxy)-piperidin-1-ylmethyl]-3-trifluoromethyl-phenylcarbamoyl}-methyl)-phenylethynyl]-pyrimidin-4-yl}-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.03 (s, 6 H) 1.39 - 1.48 (m, 11 H) 1.68 (br. s., 2 H) 2.14 (t, *J*=8.70 Hz, 2 H) 3.68 (s, 2 H) 3.72 (br. s., 1 H) 3.85 (s, 3 H) 4.01 (t, *J*=6.23 Hz, 2 H) 7.13 (s, 2 H) 7.31 - 7.39 (m, 3 H) 7.47 (s, 1 H) 7.52 - 7.58 (m, 2 H) 7.66 (d, *J*=8.61 Hz, 1 H) 7.76 (d, *J*=8.97 Hz, 1 H) 8.02 (s, 1 H) 8.47 (s, 1 H) 10.41 (s, 1 H).

### tert-Butyl 2-(2-amino-5-{[3-(2-{[4-{[(2-{[tert-butyl(dimethyl)silyl]oxy}ethyl)(methyl)amino]methyl}-3-(trifluoro methyl)phenyl]amino}-2-oxoethyl)phenyl]ethynyl}pyrimidin-4-yl)-1-methyl-4-oxo-1,4,6,7-tetrahydro-5H-pyrrolo[3,2-c]pyridine-5-carboxylate

¹H NMR (600 MHz, DMSO-*d*₆) δ ppm -0.01 - 0.01 (m, 7 H) 0.83 (s, 10 H) 1.44 (s, 9 H) 2.19 (s, 4 H) 3.00 (t, *J*=6.32 Hz, 2 H) 3.61 (s, 2 H) 3.65 - 3.72 (m, 5 H) 3.85 (s, 3 H) 4.01 (t, *J*=6.32 Hz, 2 H) 7.11 - 7.14 (m, 2 H) 7.27 - 7.40 (m, 4 H) 7.47 (s, 1 H) 7.57 (s, 2 H) 7.59 - 7.64 (m, 1 H) 7.69 - 7.74 (m, 1 H) 7.74 - 7.78 (m, 1 H) 8.02 (d, *J*=2.01 Hz, 1 H) 8.47 (s, 1 H) 10.40 (s, 1 H).

### 2-[2-(Acetyl-methyl-amino)-5-(2-fluoro-5-{[4-(4-methyl-piperazin-1-ylmethyl)-3-trifluoromethyl-phenyl carbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 831 [(M + H)⁺]. HRMS (ESI) calculated for C₄₃H₄₇F₄N₈O₅⁺ [(M + H)⁺] 831.8696, found 831.8690.

### 2-[2-Amino-5-(3-{[4-(4-ethyl-piperazin-1-ylmethyl)-3-trifluoromethyl-phenylcarbamoyl]-methyl}-phenylethynyl)-pyrimidin-4-yl]-4-oxo-1-[2-(tetrahydro-pyran-2-yloxy)-ethyl]-1,4,6,7-tetrahydro-pyrrolo[3,2-c]pyridine-5-carboxylic acid tert-butyl ester

(ESI) *m*/*z* 885 [(M + H)⁺]. HRMS (ESI) calculated for C₄₇H₅₆F₃N₈O₆⁺ [(M + H)⁺] 885.9848, found 885.9845.

### 2-(2-Amino-5-{3-[(4-{[(2,2-dimethyl-[1,3]dioxolan-4-ylmethyl)-methyl-amino]-methyl}-3-trifluoromethyl-phenylcarbamoyl)-methyl]-phenylethynyl}-pyrimidin-4-yl)-1-methyl-4-oxo-1,4,6,7-tetrahydro-pyrrolo[3,2-c]pyridine-5-carboxylic acid tert-butyl ester

(ESI) *m*/*z* 802 [(M + H)⁺]. HRMS (ESI) calculated for C₄₂H₄₇F₃N₇O₆⁺ [(M + H)⁺] 802.8531, found 802.8532.

### 2-[2-(Acetyl-ethyl-amino)-5-(3-{[4-(4-methyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 827 [(M + H)⁺]. HRMS (ESI) calculated for C₄₄H₅₀F₃N₈O₅⁺ [(M + H)⁺] 827.9057, found 827.9060.

### 2-[2-(Acetyl-methyl-amino)-5-(3-{[4-(4-cyclopropylmethyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.06 (br. s., 2 H) 0.44 (d, *J*=6.59 Hz, 2 H) 0.80 (br. s., 1 H) 2.06 - 2.24 (m, 2 H) 2.26 - 2.48 (m, 9 H) 2.93 (t, *J*=6.87 Hz, 2 H) 3.41 (s, 3 H) 3.48 (td, *J*=6.73, 2.29 Hz, 2 H) 3.53 (s, 2 H) 3.71 (s, 2 H) 3.87 (s, 3 H) 7.29 (br. s., 1 H) 7.40 - 7.48 (m, 3 H) 7.60 (s, 1 H) 7.65 (d, *J*=8.42 Hz, 1 H) 7.72 (s, 1 H) 7.78 (d, *J*=8.06 Hz, 1 H) 8.05 (d, *J*=1.65 Hz, 1 H) 8.87 (s, 1 H) 10.48 (s, 1 H).

### 2-[2-(Acetyl-methyl-amino)-5-(3-{[4-((S)-3-dimethylamino-pyrrolidin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 827 [(M + H)⁺]. HRMS (ESI) calculated for C₄₄H₅₀F₃N₈O₅⁺ [(M + H)⁺] 827.9057, found 827.9054.

### 2-(2-Amino-5-{5-[4-(4-ethyl-piperazin-1-ylmethyl)-3-trifluoromethyl-phenylcarbamoyl]-2-methyl-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.98 (t, *J*=7.14 Hz, 3 H) 1.41 (s, 9 H) 2.30 (q, *J*=7.14 Hz, 2 H) 2.48 (br. s., 3 H) 2.99 (t, *J*=6.31 Hz, 2 H) 3.56 (s, 3 H) 3.84 (s, 3 H) 3.96 - 4.00 (m, 2 H) 7.23 (s, 2 H) 7.48 (d, *J*=8.24 Hz, 1 H) 7.55 (s, 1 H) 7.70 (d, *J*=8.51 Hz, 1 H) 7.86 (dd, *J*=7.96, 1.65 Hz, 1 H) 8.00 - 8.06 (m, 2 H) 8.21 (d, *J*=1.92 Hz, 1 H) 8.50 - 8.61 (m, 2 H) 10.52 (s, 1 H).

### 2-[2-Acetylamino-5-(3-{[4-(4-methyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.45 (s, 9 H) 2.19 (s, 5 H) 2.22 - 2.47 (m, 7 H) 3.03 (t, *J*=6.32 Hz, 2 H) 3.53 (s, 2 H) 3.71 (s, 2 H) 3.97 (s, 3 H) 4.02 (t, *J*=6.32 Hz, 2 H) 7.37 - 7.46 (m, 3 H) 7.55 (s, 1 H) 7.64 (d, *J*=8.61 Hz, 1 H) 7.74 - 7.79 (m, 2 H) 8.03 (d, *J*=1.83 Hz, 1 H) 8.82 (s, 1 H) 10.44 (s, 1 H) 10.80 (s, 1 H).

### 2-[2-Amino-5-(3-{[4-chloro-3-(4-ethyl-piperazin-1-ylmethyl)-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 738 [(M + H)⁺]. HRMS (ESI) calculated for C₄₀H₄₆ClN₈O₄⁺ [(M + H)⁺] 738.2895, found 738.2898.

### 2-(2-Amino-5-{3-[(5-bromo-pyridin-3-ylcarbamoyl)-methyl]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.45 (s, 9 H) 3.00 (t, *J*=6.32 Hz, 2 H) 3.72 (s, 2 H) 3.85 (s, 3 H) 4.02 (quin, *J*=6.87 Hz, 4 H) 7.13 (s, 2 H) 7.32 - 7.40 (m, 3 H) 7.47 (s, 1 H) 7.56 (s, 1 H) 8.36 - 8.39 (m, 2 H) 8.47 (s, 1 H) 8.66 (d, *J*=2.01 Hz, 1 H) 10.56 (s, 1 H)

### 2-(2-Amino-5-{1-[4-(4-ethyl-piperazin-1-ylmethyl)-3-trifluoromethyl-phenylcarbamoyl]-2,3-dihydro-1H-indol-6-ylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.98 (t, J=7.14 Hz, 3 H) 1.45 (s, 10 H) 2.29 - 2.32 (m, 2 H) 2.33 - 2.48 (m, 7 H) 2.99 (t, J=6.32 Hz, 2 H) 3.21 (t, J=8.61 Hz, 1 H) 3.54 (s, 2 H) 3.84 (s, 2 H) 3.97 - 4.01 (m, 2 H) 4.17 (t, J=8.79 Hz, 2 H) 7.04 (dd, J=7.60, 1.37 Hz, 1 H) 7.09 (s, 1 H) 7.24 (d, J=7.69 Hz, 1 H) 7.47 (s, 1 H) 7.64 (d, J=8.61 Hz, 1 H) 7.83 (dd, J=8.42, 1.83 Hz, 1 H) 7.94 (d, J=0.92 Hz, 1 H) 7.98 (d, J=2.02 Hz, 1 H) 8.47 - 8.51 (m, 1 H) 8.84 (s, 1 H).

### 2-(2-Amino-5-{1-[4-(4-ethyl-piperazin-1-ylmethyl)-3-trifluoromethyl-phenylcarbamoyl]-2,3-dihydro-1H-indol-5-ylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (500 MHz, DMSO-*d*₆) δ ppm 1.02 - 1.17 (m, 3 H) 1.44 - 1.50 (m, 10 H) 2.99 (t, J=6.33 Hz, 2 H) 3.20 (t, J=8.39 Hz, 2 H) 3.58 (br. s., 2 H) 3.84 (s, 3 H) 4.00 (t, J=6.33 Hz, 2 H) 4.17 (t, J=8.77 Hz, 2 H) 7.09 (s, 2 H) 7.23 - 7.29 (m, 1 H) 7.32 (s, 1 H) 7.56 (s, 1 H) 7.64 (d, J=8.69 Hz, 1 H) 7.83 - 7.90 (m, 2 H) 7.99 (d, J=2.14 Hz, 1 H) 8.43 (s, 1 H) 8.92 (s, 1 H).

### 2-(2-(Acetyl-methyl-amino)-5-{1-[4-(4-ethyl-piperazin-1-ylmethyl)-3-trifluoromethyl-phenylcarbamoyl]-2,3-dihydro-1H-indol-6-ylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (600 MHz, DMSO-*d₆*) δ ppm 0.98 (t, J=7.14 Hz, 3 H) 1.45 (s, 9 H) 2.31 (q, J=7.14 Hz, 2 H) 2.38 (br. s., 8 H) 2.41 (s, 3 H) 3.03 (t, J=6.23 Hz, 2 H) 3.24 (t, J=8.61 Hz, 2 H) 3.40 (s, 3 H) 3.54 (s, 2 H) 3.85 (s, 3 H) 4.02 (t, J=6.32 Hz, 2 H) 4.18 (t, J=8.70 Hz, 2 H) 7.11 - 7.14 (m, 1 H) 7.29 (d, J=7.69 Hz, 1 H) 7.64 (d, J=8.61 Hz, 1 H) 7.66 (s, 1 H) 7.81 - 7.88 (m, 1 H) 7.98 (d, J=2.01 Hz, 1 H) 8.03 (s, 1 H) 8.85 (s, 1 H) 8.93 (s, 1 H).

### 2-(2-(Acetyl-methyl-amino)-5-{1-[4-(4-ethyl-piperazin-1-ylmethyl)-3-trifluoromethyl-phenylcarbamoyl]-2,3-dihydro-1H-indol-5-ylethynyl}-pyrimidin-4-yl)-1-methyl-4-oxo-1,4,6,7-tetrahydro-pyrrolo[3,2-c]pyridine-5-carboxylic acid tert-butyl ester

1H NMR (600 MHz, DMSO-*d₆*) δ ppm 0.98 (t, J=7 Hz, 3 H) 1.48 (s, 9 H) 2.31 (q, J=7.02 Hz, 2 H) 2.38 (br. s., 8 H) 2.41 (s, 4 H) 3.03 (t, J=6.23 Hz, 2 H) 3.22 (t, J=8.52 Hz, 2 H) 3.40 (s, 3 H) 3.54 (s, 2 H) 3.86 (s, 3 H) 4.03 (t, J=6.41 Hz, 2 H) 4.19 (t, J=8.61 Hz, 2 H) 7.36 (d, J=8.06 Hz, 1 H) 7.41 (s, 1 H) 7.64 (d, J=8.79 Hz, 1 H) 7.76 (s, 1 H) 7.85 (d, J=8.42 Hz, 1 H) 7.93 (d, J=8.42 Hz, 1 H) 7.99 (d, J=1.83 Hz, 1 H) 8.87 (s, 1 H) 8.90 (s, 1 H).

### 2-[2-Acetylamino-5-(3-{[4-(4-methyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester.

¹H NMR (600 MHz, DMSO-*d*₆) δ ppm 1.45 (s, 9 H) 2.19 (s, 5 H) 2.22 - 2.47 (m, 7 H) 3.03 (t, *J*=6.32 Hz, 2 H) 3.53 (s, 2 H) 3.71 (s, 2 H) 3.97 (s, 3 H) 4.02 (t, *J*=6.32 Hz, 2 H) 7.37 - 7.46 (m, 3 H) 7.55 (s, 1 H) 7.64 (d, *J*=8.61 Hz, 1 H) 7.74 - 7.79 (m, 2 H) 8.03 (d, *J*=1.83 Hz, 1 H) 8.82 (s, 1 H) 10.44 (s, 1 H) 10.80 (s, 1 H).

### 2-[2-(Acetyl-isopropyl-amino)-5-(3-{[4-(4-methyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester.

¹H NMR (600 MHz, DMSO-*d*₆) δ ppm 1.25 (d, *J*=6.96 Hz, 6 H) 1.45 (s, 9 H) 1.99 (s, 3 H) 2.14 (s, 3 H) 2.16 - 2.46 (m, 8 H) 3.03 (t, *J*=6.23 Hz, 2 H) 3.52 (s, 2 H) 3.72 (s, 2 H) 3.84 (s, 3 H) 4.03 (t, *J*=6.32 Hz, 2 H) 4.73 - 4.80 (m, 1 H) 7.40 - 7.51 (m, 3 H) 7.58 (s, 1 H) 7.64 (d, *J*=8.43 Hz, 1 H) 7.75 - 7.79 (m, 2 H) 8.04 (d, *J*=1.83 Hz, 1 H) 9.01 (s, 1 H) 10.45 (s, 1 H).

### 2-[2-(Acetyl-methyl-amino)-5-(5-{[4-(4-ethyl-piperazin-1-ylmethyl)-3-trifluoromethyl-phenylcarbamoyl]-methyl}-2-fluoro-phenylethynyl)-pyrimidin-4-yl]-1-methyl-4-oxo-1,4,6,7-tetrahydro-pyrrolo[3,2-c]pyridine-5-carboxylic acid tert-butyl ester.

¹H NMR (600 MHz, DMSO-*d*₆) δ ppm 0.99 (br. s., 3 H) 1.44 (s, 10 H) 2.26 - 2.47 (m, 3 H) 2.42 (s, 4 H) 3.03 (t, *J*=6.32 Hz, 2 H) 3.53 (br. s., 2 H) 3.71 (s, 2 H) 3.85 (s, 3 H) 4.01 (t, *J*=6.23 Hz, 2 H) 7.33 (t, *J*=8.88 Hz, 1 H) 7.43 - 7.48 (m, 1 H) 7.60 (dd, *J*=6.69, 1.92 Hz, 1 H) 7.65 (d, *J*=8.42 Hz, 1 H) 7.71 (s, 1 H) 7.77 (d, *J*=8.79 Hz, 1 H) 8.03 (d, *J*=1.65 Hz, 1 H) 8.92 (s, 1 H) 10.45 (s, 1 H).

### 2-[2-(Acetyl-ethyl-amino)-5-(2-fluoro-5-{[4-(4-methyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester.

¹H NMR (600 MHz, DMSO-*d*₆) δ ppm 1.16 (t, *J*=7.05 Hz, 3 H) 1.44 (s, 9 H) 2.14 (s, 3 H) 2.36 - 2.38 (m, 4 H) 3.03 (t, *J*=6.32 Hz, 2 H) 3.52 (s, 2 H) 3.71 (s, 2 H) 3.84 (s, 3 H) 3.98 - 4.07 (m, 4 H) 7.33 (t, *J*=8.97 Hz, 1 H) 7.43 - 7.48 (m, 1 H) 7.59 (dd, *J*=6.78, 2.20 Hz, 1 H) 7.64 (d, *J*=8.61 Hz, 1 H) 7.70 (s, 1 H) 7.76 (dd, *J*=8.52, 1.92 Hz, 1 H) 8.03 (d, *J*=1.83 Hz, 1 H) 8.92 (s, 1 H) 10.43 (s, 1 H).

### 2-(2-(Acetyl-methyl-amino)-5-{2-methyl-5-[4-(4-methyl-piperazin-1-ylmethyl)-3-trifluoromethyl-phenylcarbamoyl]-phenylethynyl}-pyrimidin-4-yl)-1-methyl-4-oxo-1,4,6,7-tetrahydro-pyrrolo[3,2-c]pyridine-5-carboxylic acid tert-butyl ester.

¹H NMR (600 MHz, DMSO-*d*₆) δ ppm 1.40 - 1.44 (m, 9 H) 2.16 (s, 3 H) 2.26 - 2.42 (m, 7 H) 2.43 (s, 3 H) 2.54 (s, 3 H) 3.03 (t, *J*=6.29 Hz, 2 H) 3.42 (s, 3 H) 3.56 (s, 2 H) 3.86 (s, 3 H) 4.01 (t, *J*=6.29 Hz, 2 H) 7.53 (d, *J*=8.18 Hz, 1 H) 7.70 (d, *J*=8.67 Hz, 1 H) 7.75 (s, 1 H) 7.94 (dd, *J*=7.99, 1.89 Hz, 1 H) 8.04 (dd, *J*=8.42, 2.08 Hz, 1 H) 8.12 (d, *J*=1.95 Hz, 1 H) 8.20 (d, *J*=2.20 Hz, 1 H) 8.98 (s, 1 H) 10.52 (s, 1 H).

### Preparation 40

### 2-[2-Amino-5-(5-{[4-(4-ethyl-piperazin-1-ylmethyl)-3-trifluoromethyl-phenylcarbamoyl]-methyl}-pyridin-3-ylethynyl)-pyrimidin-4-yl]-1-methyl-4-oxo-1,4,6,7-tetrahydro-pyrrolo[3,2-c]pyridine-5-carboxylic acid tert-butyl ester

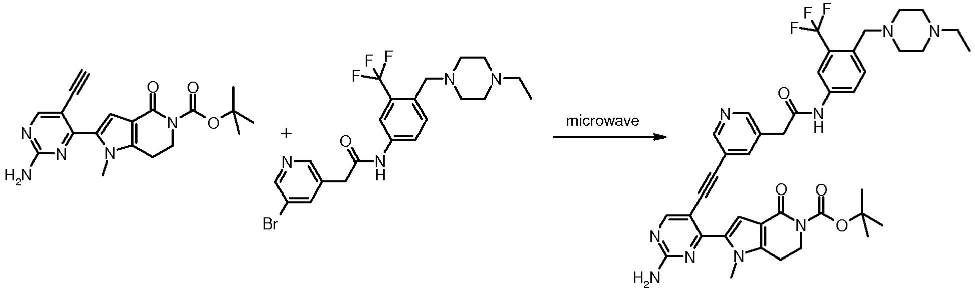

A solution of 2-(2-amino-5-ethynyl-pyrimidin-4-yl)-1-methyl-4-oxo-1,4,6,7-tetrahydro-pyrrolo[3,2-c]pyridine-5-carboxylic acid tert-butyl ester (0.05 g, 0.14 mmol), 2-(5-bromo-pyridin-3-yl)-N-[4-(4-ethyl-piperazin-1-ylmethyl)-3-trifluoromethyl-phenyl]-acetamide (0.05 g, 0.10 mmol), Cul (10% mol, 3 mg, 0.014 mmol), PdCl₂(PPh₃)₂ (10% mol, 10 mg, 0.014 mmol) and TEA (0.19 mL, 1.40 mmol) in DMF (2 mL) was degassed with argon and heated at 60 °C, in microwave apparatus, for 1 h. The reaction was cooled at r.t., poured in water (15 mL) and extracted with EtOAc (2 x 15 mL). The organic phase was washed with brine, dried with anhydrous Na₂SO₄ and evaporated under vacuum to give a crude that was purified by flash column chromatography (DCM/MeOH/NH₃ 90/10/0.5). The title product (0.02 g, 26%) was obtained as yellowish solid.
¹H NMR (600 MHz, DMSO-*d*₆) δ ppm 0.97 (t, *J*=7.23 Hz, 3 H) 1.41 - 1.47 (m, 9 H) 2.11 - 2.46 (m, 10 H) 2.99 (t, *J*=6.32 Hz, 2 H) 3.52 (s, 2 H) 3.76 (s, 2 H) 3.84 (s, 3 H) 4.00 (t, *J*=6.32 Hz, 2 H) 7.20 (s, 2 H) 7.54 (s, 1 H) 7.65 (d, *J*=8.61 Hz, 1 H) 7.76 (dd, *J*=8.52, 1.74 Hz, 1 H) 7.83 (t, *J*=2.01 Hz, 1 H) 8.03 (d, *J*=1.83 Hz, 1 H) 8.49 (d, *J*=2.01 Hz, 1 H) 8.50 (s, 1 H) 8.54 (d, *J*=1.83 Hz, 1 H) 10.50 (s, 1 H).

According to this same methodology, but employing suitable substituted derivatives, the following intermediates were prepared:

### 2-[2-Amino-5-(5-{[4-(4-ethyl-piperazin-1-ylmethyl)-3-trifluoromethyl-phenylcarbamoyl]-methyl}-thiophen-3-ylethynyl)-pyrimidin-4-yl]-1-methyl-4-oxo-1,4,6,7-tetrahydro-pyrrolo[3,2-c]pyridine-5-carboxylic acid tert-butyl ester

ESI) *m*/*z* 777 [(M + H)⁺]. HRMS (ESI) calculated for C₃₉H₄₄F₃N₈O₄S⁺ [(M + H)⁺] 777.8701, found 777.8705.

### 2-[2-Amino-5-(4-{[4-(4-ethyl-piperazin-1-ylmethyl)-3-trifluoromethyl-phenylcarbamoyl]-methyl}-pyridin-2-ylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 772 [(M + H)⁺]. HRMS (ESI) calculated for C₄₀H₄₅F₃N₉O₄⁺ [(M + H)⁺] 772.8305, found 772.8302.

### 2-[2-Amino-5-(5-{[4-(4-ethyl-piperazin-1-ylmethyl)-3-trifluoromethyl-phenylcarbamoyl]-methyl}-thiophen-2-ylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 777 [(M + H)⁺]. HRMS (ESI) calculated for C₃₉H₄₄F₃N₈O₄S⁺ [(M + H)⁺] 777.8701, found 777.8698.

### 2-[2-Amino-5-(5-{[4-(4-ethyl-piperazin-1-ylmethyl)-3-trifluoromethyl-phenylcarbamoyl]-methyl}-2-fluoro-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.97 (t, *J*=7.14 Hz, 3 H) 1.44 (s, 9 H) 2.20 - 2.47 (m, 9 H) 2.99 (t, *J*=6.32 Hz, 2 H) 3.52 (s, 2 H) 3.67 (s, 2 H) 3.84 (s, 3 H) 4.00 (t, *J*=6.32 Hz, 2 H) 7.19 (s, 2 H) 7.27 (t, *J*=8.97 Hz, 1 H) 7.35 - 7.39 (m, 1 H) 7.51 (dd, *J*=6.78, 2.01 Hz, 1 H) 7.54 (s, 1 H) 7.65 (d, *J*=8.43 Hz, 1 H) 7.76 (dd, *J*=8.70, 1.74 Hz, 1 H) 8.03 (d, *J*=2.01 Hz, 1 H) 8.48 (s, 1 H) 10.41 (s, 1 H).

### 2-[2-Amino-5-(3-{[4-(4-ethyl-piperazin-1-ylmethyl)-3-trifluoromethyl-phenylcarbamoyl]-methyl}-2-fluoro-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.98 (t, *J*=7.14 Hz, 3 H) 1.45 (s, 9 H) 2.20 - 2.47 (m, 9 H) 2.98 (t, *J*=6.32 Hz, 2 H) 3.52 (s, 2 H) 3.67 (s, 2 H) 3.84 (s, 3 H) 4.00 (t, *J*=6.32 Hz, 2 H) 7.19 (s, 2 H) 7.30 (t, *J*=8.97 Hz, 1 H) 7.35 - 7.39 (m, 1 H) 7.48 (dd, *J*=6.78, 2.01 Hz, 1 H) 7.50 (s, 1 H) 7.65 (d, *J*=8.43 Hz, 1 H) 7.76 (dd, *J*=8.70, 1.74 Hz, 1 H) 8.04 (d, *J*=2.01 Hz, 1 H) 8.47 (s, 1 H) 10.52 (s, 1 H).

### 2-[2-(Acetyl-methyl-amino)-5-(2-fluoro-5-{[4-(4-isopropyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 859 [(M + H)⁺]. HRMS (ESI) calculated for C₄₅H₅₁F₄N₈O₅⁺ [(M + H)⁺] 859.9227, found 859.9228.

### 2-[2-(Acetyl-methyl-amino)-5-(5-{[4-(4-ethyl-piperazin-1-ylmethyl)-3-trifluoromethyl-phenylcarbamoyl]-methyl}-thiophen-2-ylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 833 [(M + H)⁺]. HRMS (ESI) calculated for C₄₂H₄₈F₃N₈O₅S⁺ [(M + H)⁺] 833.9334, found 833.9330.

### 2-[2-(Acetyl-methyl-amino)-5-(2-fluoro-5-{[4-(4-propyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 859 [(M + H)⁺]. HRMS (ESI) calculated for C₄₅H₅₁F₄N₈O₅⁺ [(M + H)⁺] 859.9227, found 859.9233.

### 2-(2-Amino-5-{5-[4-(4-ethyl-piperazin-1-ylmethyl)-3-trifluoromethyl-phenylcarbamoyl]-thiophen-2-ylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.99 (t, *J*=7.14 Hz, 3 H) 1.47 (s, 9 H) 2.22 - 2.48 (m, 9 H) 2.99 (t, *J*=6.32 Hz, 2 H) 3.56 (s, 2 H) 3.84 (s, 3 H) 4.00 (t, *J*=6.32 Hz, 2 H) 7.25 (s, 2 H) 7.37 (d, *J*=3.85 Hz, 1 H) 7.43 (s, 1 H) 7.71 (d, *J*=8.61 Hz, 1 H) 7.96 - 7.98 (m, 1 H) 7.99 (d, *J*=4.03 Hz, 1 H) 8.12 (d, *J*=2.20 Hz, 1 H) 8.50 (s, 1 H) 10.57 (s, 1 H).

### 2-(2-Amino-5-{5-[4-(4-ethyl-piperazin-1-ylmethyl)-3-trifluoromethyl-phenylcarbamoyl]-thiazol-2-ylethynyl}-pyrimidin-4-yl)-1-methyl-4-oxo-1,4,6,7-tetrahydro-pyrrolo[3,2-c]pyridine-5-carboxylic acid tert-butyl ester

(ESI) *m*/*z* 764 [(M + H)⁺]. HRMS (ESI) calculated for C₃₇H₄₁F₃N₉O₄S⁺ [(M + H)⁺] 764.8316, found 764.8318.

### 2-(2-Amino-5-{5-[4-(4-ethyl-piperazin-1-ylmethyl)-3-trifluoromethyl-phenylcarbamoyl]-pyridin-3-ylethynyl}-pyrimidin-4-yl)-1-methyl-4-oxo-1,4,6,7-tetrahydro-pyrrolo[3,2-c]pyridine-5-carboxylic acid tert-butyl ester

(ESI) *m*/*z* 758 [(M + H)⁺]. HRMS (ESI) calculated for C₃₉H₄₃F₃N₉O₄⁺ [(M + H)⁺] 758.8039, found 758.8045.

### 2-(2-Amino-5-{3-[4-(4-ethyl-piperazin-1-ylmethyl)-3-trifluoromethyl-phenylcarbamoyl]-5-fluoro-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.98 (t, *J*=7.14 Hz, 3 H) 2.31 (q, *J*=7.08 Hz, 3 H) 2.33 - 2.47 (m, 5 H) 3.00 (t, *J*=6.32 Hz, 2 H) 3.57 (s, 2 H) 3.85 (s, 3 H) 4.00 (t, *J*=6.32 Hz, 2 H) 7.24 (s, 2 H) 7.51 - 7.53 (m, 1 H) 7.54 (s, 1 H) 7.72 (d, *J*=8.42 Hz, 1 H) 7.78 (dd, *J*=8.97, 1.83 Hz, 1 H) 7.89 (s, 1 H) 8.02 (dd, *J*=8.61, 1.83 Hz, 1 H) 8.19 (d, *J*=2.01 Hz, 1 H) 8.52 (s, 1 H) 10.61 (s, 1 H).

### 2-(2-Amino-5-{3-[4-(4-ethyl-piperazin-1-ylmethyl)-3-trifluoromethyl-phenylcarbamoyl]-2-fluoro-phenylethynyl}-pyrimidin-4-yl)-1-methyl-4-oxo-1,4,6,7-tetrahydro-pyrrolo[3,2-c]pyridine-5-carboxylic acid tert-butyl ester

(ESI) *m*/*z* 775 [(M + H)⁺]. HRMS (ESI) calculated for C₄₀H₄₃F₄N₈O₄⁺ [(M + H)⁺] 775.8063, found 775.8064.

### 2-(2-Amino-5-{3-[4-(4-ethyl-piperazin-1-ylmethyl)-3-trifluoromethyl-phenylsulfamoyl]-phenylethynyl}-pyrimidin-4-yl)-1-methyl-4-oxo-1,4,6,7-tetrahydro-pyrrolo[3,2-c]pyridine-5-carboxylic acid tert-butyl ester

(ESI) *m*/*z* 793 [(M + H)⁺]. HRMS (ESI) calculated for C₃₉H₄₄F₃N₈O₅S⁺ [(M + H)⁺] 793.8695, found 793.8698.

### 2-[2-Amino-5-(3-cyclopropylsulfamoyl-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.34 - 0.41 (m, 2 H) 0.47 - 0.53 (m, 2 H) 1.47 (s, 9 H) 2.15 (td, *J*=6.69, 3.30 Hz, 1 H) 3.00 (t, *J*=6.32 Hz, 2 H) 3.85 (s, 3 H) 4.00 (t, *J*=6.32 Hz, 2 H) 7.21 (s, 2 H) 7.51 (s, 1 H) 7.64 - 7.68 (m, 1 H) 7.69 - 7.72 (m, 1 H) 7.79 (dt, *J*=7.69, 1.47 Hz, 1 H) 7.82 - 7.84 (m, 1 H) 7.90 (d, *J*=2.93 Hz, 1 H) 8.53 (s, 1 H).

### Preparation 41

### 2-(2-Amino-5-{3-[(4-chloro-3-trifluoromethyl-phenylcarbamoyl)-methyl]-phenylethynyl}-pyrimidin-4-yl)-4-oxo-1-[2-(tetrahydro-pyran-2-yloxy)-ethyl]-1,4,6,7-tetrahydro-pyrrolo[3,2-c]pyridine-5-carboxylic acid tert-butyl ester

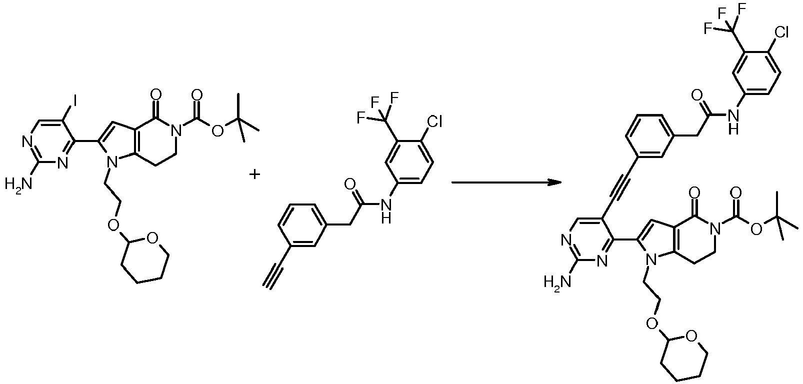

A solution of 2-(2-amino-5-iodo-pyrimidin-4-yl)-4-oxo-1-[2-(tetrahydro-pyran-2-yloxy)-ethyl]-1,4,6,7-tetrahydro-pyrrolo[3,2-c]pyridine-5-carboxylic acid tert-butyl ester (0.22 g, 0.38 mmol), N-(4-chloro-3-trifluoromethyl-phenyl)-2-(3-ethynyl-phenyl)-acetamide (0.15 g, 0.46 mmol), Cul (10% mol, 7 mg, 0.038 mmol), PdCl₂(PPh₃)₂ (10% mol, 27 mg, 0.038 mmol) and TEA (0.53 mL, 3.80 mmol) in MeCN (7 mL) was degassed with argon and stirred at r.t. for 2 h. The reaction was poured in water (15 mL) and extracted with EtOAc (2 x 15 mL). The organic phase was washed with brine, dried with anhydrous Na₂SO₄ and evaporated under vacuum to give a crude that was purified by flash column chromatography (DCM/MeOH 98/2) to afford the title product (0.15 g, 50% yield) was obtained as white solid.
(ESI) *m*/*z* 794 [(M + H)⁺]. HRMS (ESI) calculated for C₄₀H₄₁ClF₃N₆O₆⁺ [(M + H)⁺] 794.2304, found 794.2301. According to this same methodology, but employing suitable substituted derivatives, the following compounds or intermediates were prepared:

### 2-(3-{2-Amino-4-[1-(1-methyl-piperidin-4-yl)-4-oxo-4,5,6,7-tetrahydro-1H-pyrrolo[3,2-c]pyridin-2-yl]-pyrimidin-5-ylethynyl}-phenyl)-N-(4-chloro-3-trifluoromethyl-phenyl)-acetamide (cmpd 195)

¹H NMR (600 MHz, DMSO-*d*₆) δ ppm 1.94 (d, *J*=9.71 Hz, 4 H) 2.08 - 2.16 (m, 2 H) 2.18 (s, 3 H) 2.84 (d, *J*=10.07 Hz, 2 H) 3.04 (t, *J*=6.69 Hz, 2 H) 3.41 - 3.44 (m, 2 H) 3.68 (s, 2 H) 4.64 (t, *J*=12.27 Hz, 1 H) 7.03 (br. s., 2 H) 7.25 (s, 1 H) 7.27 (br. s., 1 H) 7.30 - 7.38 (m, 4 H) 7.48 (s, 1 H) 7.65 (d, *J*=8.79 Hz, 1 H) 7.84 (dd, *J*=8.79, 2.20 Hz, 1 H) 8.19 (d, *J*=2.20 Hz, 1 H) 8.46 (s, 1 H) 10.62 (s, 1 H).

### 2-(2-Amino-5-{3-[4-(4-ethyl-piperazin-1-ylmethyl)-3-trifluoromethyl-phenylcarbamoyl]-phenylethynyl}-pyrimidin-4-yl)-1-methyl-4-oxo-1,4,6,7-tetrahydro-pyrrolo[3,2-c]pyridine-5-carboxylic acid tert-butyl ester

(ESI) *m*/*z* 757 [(M + H)⁺]. HRMS (ESI) calculated for C₄₀H₄₄F₃N₈O₄⁺ [(M + H)⁺] 757.8158, found 757.8160.

### N-[4-(4-Ethyl-piperazin-1-ylmethyl)-3-trifluoromethyl-phenyl]-3-[2-methylamino-4-(1-methyl-4-oxo-4,5,6,7-tetrahydro-1H-pyrrolo[3,2-c]pyridin-2-yl)-pyrimidin-5-ylethynyl]-benzamide (cmpd 49)

¹H NMR (600 MHz, DMSO-*d*₆) δ ppm 0.98 (t, *J*=7.14 Hz, 3 H) 2.31 (q, *J*=7.20 Hz, 2 H) 2.33 - 2.49 (m, 7 H) 2.89 (d, *J*=4.76 Hz, 3 H) 2.90 - 2.92 (m, 1 H) 3.43 - 3.48 (m, 2 H) 3.57 (s, 2 H) 3.81 - 3.95 (m, 3 H) 7.14 (br. s., 1 H) 7.51 - 7.62 (m, 3 H) 7.70 (dd, *J*=15.57, 8.06 Hz, 2 H) 7.95 (d, *J*=7.88 Hz, 1 H) 8.05 (d, *J*=8.43 Hz, 1 H) 8.06 (s, 1 H) 8.22 (d, *J*=2.01 Hz, 1 H) 8.46 - 8.57 (m, 1 H) 10.56 (br. s., 1 H).

(ESI) *m*/*z* 671 [(M + H)⁺]. HRMS (ESI) calculated for C₃₆H₃₈F₃N₈O₂⁺ [(M + H)⁺] 671.3065, found 671.3063.

### 2-[2-Amino-5-(3-cyclopropylcarbamoyl-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 0.56 - 0.61 (m, 2 H) 0.66 - 0.71 (m, 2 H) 1.47 (s, 9 H) 2.82 - 2.88 (m, 1 H) 2.99 (t, *J*=6.32 Hz, 2 H) 3.84 (s, 3 H) 4.00 (t, *J*=6.32 Hz, 2 H) 7.16 (s, 2 H) 7.45 - 7.50 (m, 1 H) 7.52 (s, 1 H) 7.58 (dt, *J*=7.74, 1.26 Hz, 1 H) 7.80 (dt, *J*=7.83, 1.40 Hz, 1 H) 7.86 (s, 1 H) 8.46 - 8.51 (m, 2 H).

### 2-[2-Amino-5-(3-phenylcarbamoylmethoxy-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.44 (s, 9 H) 2.99 (t, *J*=6.16 Hz, 2 H) 3.85 (s, 3 H) 4.00 (t, *J*=6.23 Hz, 2 H) 4.74 (s, 2 H) 7.00 - 7.12 (m, 4 H) 7.13 (s, 2 H) 7.28 - 7.38 (m, 3 H) 7.57 (s, 1 H) 7.63 (d, *J*=8.18 Hz, 2 H) 8.46 (s, 1 H) 10.01 (s, 1 H).

### 2-(2-Amino-5-{3-[(1-phenylcarbamoyl-cyclopropanecarbonyl)-amino]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.35 - 1.58 (m, 13 H) 2.99 (t, *J*=6.35 Hz, 2 H) 3.84 (s, 3 H) 3.96-4.04 (m, 2 H) 7.03 - 7.09 (m, 1 H) 7.12 (s, 2 H) 7.17 (d, *J*=7.57 Hz, 1 H) 7.31 (dt, *J*=15.72, 7.83 Hz, 4 H) 7.48 (s, 1 H) 7.56 (d, *J*=8.91 Hz, 1 H) 7.61 (d, *J*=8.18 Hz, 2 H) 7.80 (s, 1 H) 7.95 (s, 1 H) 8.47 (s, 1 H) 10.00 (s, 1 H) 10.09 (s, 1 H).

### 2-[2-Amino-5-(3-{[1-(4-trifluoromethyl-phenylcarbamoyl)-cyclopropanecarbonyl]-amino}-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 714 [(M + H)⁺]. HRMS (ESI) calculated for C₃₇H₃₅F₃N₇O₅⁺ [(M + H)⁺] 714.7050, found 714.7055.

### 2-[2-Amino-5-(3-phenethyloxy-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.46 (s, 9 H) 3.01 (dt, *J*=16.02, 6.45 Hz, 4 H) 3.85 (s, 3 H) 4.00 (t, *J*=5.98 Hz, 2 H) 4.23 (t, *J*=6.71 Hz, 2 H) 6.95 (d, *J*=8.30 Hz, 1 H) 6.98 - 7.04 (m, 2 H) 7.12 (s, 2 H) 7.18 - 7.24 (m, 1 H) 7.26 - 7.36 (m, 5 H) 7.57 (s, 1 H) 8.46 (s, 1 H).

### 2-{2-Amino-5-[3-(3-phenyl-propionylamino)-phenylethynyl]-pyrimidin-4-yl}-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.46 (s, 8 H) 2.60 - 2.66 (m, 2 H) 2.88 - 2.93 (m, 2 H) 2.99 (t, *J*=6.29 Hz, 2 H) 3.84 (s, 3 H) 4.01 (t, *J*=6.35 Hz, 2 H) 7.11 - 7.15 (m, 3 H) 7.16 - 7.21 (m, 1 H) 7.22 - 7.37 (m, 5 H) 7.47 (s, 1 H) 7.49 - 7.52 (m, 1 H) 7.76 (s, 1 H) 8.48 (s, 1 H) 9.98 (s, 1 H).

### 2-(2-Amino-5-{3-[((1S,2S)-2-phenyl-cyclopropanecarbonyl)-amino]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.31 - 1.56 (m, 12 H) 2.02 - 2.11 (m, 1 H) 2.35 - 2.40 (m, 1 H) 2.99 (t, *J*=6.23 Hz, 2 H) 3.84 (s, 3 H) 4.00 (t, *J*=6.23 Hz, 2 H) 7.09 - 7.16 (m, 3 H) 7.16 - 7.23 (m, 3 H) 7.26 - 7.36 (m, 3 H) 7.47 (s, 1 H) 7.51 - 7.55 (m, 1 H) 7.77 (s, 1 H) 10.32 (s, 1 H).

### 2-{2-Amino-5-[3-(3-phenyl-propoxy)-phenylethynyl]-pyrimidin-4-yl}-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.45 (s, 9 H) 1.99 - 2.05 (m, 2 H) 2.72 - 2.76 (m, 2 H) 2.99 (t, *J*=6.23 Hz, 2 H) 3.85 (s, 3 H) 3.97 - 4.03 (m, 4 H) 6.96 (dd, *J*=8.33, 2.47 Hz, 1 H) 6.99 (s, 1 H) 7.03 (d, *J*=7.69 Hz, 1 H) 7.13 (s, 2 H) 7.16 - 7.19 (m, 1 H) 7.21 - 7.24 (m, 2 H) 7.25 - 7.32 (m, 3 H) 7.59 (s, 1 H) 8.46 (s, 1 H).

### 2-{2-Amino-5-[3-(benzoylamino-methyl)-phenylethynyl]-pyrimidin-4-yl}-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.44 - 1.49 (m, 9 H) 2.99 (t, *J*=6.32 Hz, 2 H) 3.84 (s, 3 H) 4.00 (t, *J*=6.32 Hz, 2 H) 7.12 (s, 2 H) 7.30 - 7.39 (m, 3 H) 7.44 - 7.50 (m, 3 H) 7.51 - 7.55 (m, 1 H) 7.56 (s, 1 H) 7.87 - 7.91 (m, 2 H) 8.46 (s, 1 H) 9.03 (t, *J*=5.95 Hz, 1 H).

### 2-{2-Amino-5-[3-(2-phenylcarbamoyl-ethyl)-phenylethynyl]-pyrimidin-4-yl}-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.44 (s, 9 H) 2.65 (t, *J*=7.69 Hz, 2 H) 2.92 (t, *J*=7.60 Hz, 2 H) 3.00 (t, *J*=6.32 Hz, 2 H) 3.85 (s, 3 H) 4.01 (t, *J*=6.32 Hz, 2 H) 6.98 - 7.03 (m, 1 H) 7.12 (s, 2 H) 7.23 - 7.30 (m, 4 H) 7.31 - 7.34 (m, 1 H) 7.40 (s, 1 H) 7.55 (d, *J*=7.69 Hz, 2 H) 7.60 (s, 1 H) 8.45 (s, 1 H) 9.85 (s, 1 H).

### 2-[2-Amino-5-(3-benzyloxymethyl-phenylethynyl)-pyrimidin-4-yl]-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.46 (s, 9 H) 2.99 (t, *J*=6.32 Hz, 2 H) 3.85 (s, 3 H) 4.00 (t, *J*=6.32 Hz, 1 H) 4.54 (s, 2 H) 4.55 (s, 2 H) 7.13 (s, 2 H) 7.29 (m, *J*=4.03 Hz, 1 H) 7.36 (d, *J*=4.40 Hz, 3 H) 7.38 - 7.42 (m, 2 H) 7.47 (s, 1 H) 7.57 (s, 1 H) 8.47 (s, 1 H).

### 2-(2-Amino-5-{3-[(benzyl-tert-butoxycarbonyl-amino)-methyl]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.36 (br. s., 9 H) 1.43 (s, 9 H) 2.99 (t, *J*=6.32 Hz, 2 H) 3.85 (s, 3 H) 4.00 (t, *J*=6.32 Hz, 2 H) 4.18 - 4.51 (m, 4 H) 7.13 (s, 2 H) 7.19 - 7.28 (m, 4 H) 7.29 - 7.41 (m, 5 H) 7.56 (s, 1 H) 8.46 (s, 1 H).

### 2-(2-Amino-5-{3-[2-(3-trifluoromethyl-phenyl)-acetylamino]-phenylethynyl}-pyrimidin-4-yl)-1-methyl-4-oxo-1,4,6,7-tetrahydro-pyrrolo[3,2-c]pyridine-5-carboxylic acid tert-butyl ester

¹H NMR (600 MHz, DMSO-*d*₆) δ ppm 1.43 - 1.47 (m, 9 H) 2.99 (t, *J*=6.41 Hz, 2 H) 3.79 (s, 2 H) 3.83 (s, 3 H) 3.99 (t, *J*=6.32 Hz, 2 H) 7.13 (s, 2 H) 7.15 (d, *J*=7.88 Hz, 1 H) 7.34 (t, *J*=7.97 Hz, 1 H) 7.47 (s, 1 H) 7.52 - 7.65 (m, 5 H) 7.69 (s, 1 H) 7.76 (s, 1 H) 8.47 (s, 1 H) 10.31 (s, 1 H).

### 2-(2-Amino-5-{3-[3-(4-trifluoromethyl-phenyl)-propionylamino]-phenylethynyl}-pyrimidin-4-yl)-1-methyl-4-oxo-1,4,6,7-tetrahydro-pyrrolo[3,2-c]pyridine-5-carboxylic acid tert-butyl ester

(ESI) *m*/*z* 659 [(M + H)⁺]. HRMS (ESI) calculated for C₃₅H₃₄F₃N₆O₄⁺ [(M + H)⁺] 659.6695, found 659.6697.

### 2-(2-Amino-5-{3-[3-(3-trifluoromethyl-phenyl)-propionylamino]-phenylethynyl}-pyrimidin-4-yl)-1-methyl-4-oxo-1,4,6,7-tetrahydro-pyrrolo[3,2-c]pyridine-5-carboxylic acid tert-butyl ester

(ESI) *m*/*z* 659 [(M + H)⁺]. HRMS (ESI) calculated for C₃₅H₃₄F₃N₆O₄⁺ [(M + H)⁺] 659.6695, found 659.6690.

### 2-{2-Amino-5-[3-(3-trifluoromethyl-benzylcarbamoyl)-phenylethynyl]-pyrimidin-4-yl}-1-methyl-4-oxo-1,4,6,7-tetrahydro-pyrrolo[3,2-c]pyridine-5-carboxylic acid tert-butyl ester

(ESI) *m*/*z* 645 [(M + H)⁺]. HRMS (ESI) calculated for C₃₄H₃₂F₃N₆O₄⁺ [(M + H)⁺] 645.6430, found 645.6432.

### Preparation 42

### 2-{2-Amino-5-[3-(4-trifluoromethyl-benzoylamino)-phenylethynyl]-pyrimidin-4-yl}-1-methyl-4-oxo-1,4,6,7-tetrahydro-pyrrolo[3,2-c]pyridine-5-carboxylic acid tert-butyl ester

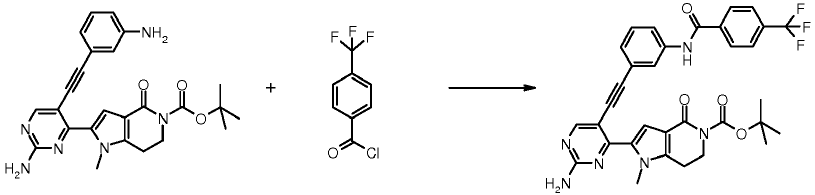

To a solution of 2-[2-amino-5-(3-amino-phenylethynyl)-pyrimidin-4-yl]-1-methyl-4-oxo-1,4,6,7-tetrahydro-pyrrolo[3,2-c]pyridine-5-carboxylic acid tert-butyl ester (0.12 g, 0.26 mmol) and pyridine (2 mL) in anhydrous THF (2 mL), cooled at 0-5 °C, 4-trifluoromethyl-benzoyl chloride (0.05 mL, 0.31 mmol) dissolved in anhydrous THF (1 mL) was added drop wise. The reaction was stirred at r.t. for 2 h, then poured in water (20 mL) and extracted with EtOAc (2 x 15 mL). The organic phase was washed with brine, dried with anhydrous Na₂SO₄ and evaporated under vacuum to obtain a crude that was grounded with isopropyl ether. The title product (0.12 g, 78%) was obtained as grey solid.
¹H NMR (600 MHz, DMSO-*d*₆) δ ppm 1.35 - 1.43 (m, 9 H) 2.97 (t, *J*=6.23 Hz, 2 H) 3.82 (s, 3 H) 3.93 - 4.01 (m, 2 H) 7.11 (s, 2 H) 7.21 (dt, *J*=7.78, 1.11 Hz, 1 H) 7.38 (t, *J*=7.99 Hz, 1 H) 7.66 - 7.77 (m, 1 H) 7.87 - 7.95 (m, 3 H) 8.13 (d, *J*=8.18 Hz, 1 H) 8.47 (s, 1 H) 10.49 (s, 1 H).

According to this same methodology, but employing suitable substituted derivatives, the following intermediate was prepared:

### 2-[2-Amino-5-(3-phenylacetylamino-phenylethynyl)-pyrimidin-4-yl]-1-methyl-4-oxo-1,4,6,7-tetrahydro-pyrrolo[3,2-c]pyridine-5-carboxylic acid tert-butyl ester

(ESI) *m*/*z* 577 [(M + H)⁺]. HRMS (ESI) calculated for C₃₃H₃₃N₆O₄⁺ [(M + H)⁺] 577.6450, found 577.6449.

### Preparation 43

### 2-(2-Amino-5-{3-[2-(4-trifluoromethyl-phenyl)-acetylamino]-phenylethynyl}-pyrimidin-4-yl)-1-methyl-4-oxo-1,4,6,7-tetrahydro-pyrrolo[3,2-c]pyridine-5-carboxylic acid tert-butyl ester

A mixture of (4-trifluoromethyl-phenyl)-acetic acid (0.06 g, 0.29 mmol), 2-[2-amino-5-(3-amino-phenylethynyl)-pyrimidin-4-yl]-1-methyl-4-oxo-1,4,6,7-tetrahydro-pyrrolo[3,2-c]pyridine-5-carboxylic acid tert-butyl ester (0.09 g, 0.19 mmol), TBTU (0.10 g, 0.30 mmol) and DIPEA (0.10 mL, 0.57 mmol) in anhydrous DMF (3 mL) was stirred at r.t. for 2 h. The reaction was poured in a satured solution of NaHCO₃ and extracted with EtOAc (2 x 15 mL), the organic phase was washed with brine, dried with anhydrous Na₂SO₄ and evaporated under vacuum. The crude was purified by flash column chromatography (EtOAc-Hex 65:35) to obtain the title compound (0.06 g, 49%) as white solid.
¹H NMR (600 MHz, DMSO-*d*₆) δ ppm 1.46 (s, 9 H) 2.99 (t, *J*=6.35 Hz, 2 H) 3.78 (s, 2 H) 3.83 (s, 3 H) 4.00 (t, *J*=6.23 Hz, 2 H) 7.13 (s, 2 H) 7.15 (d, *J*=7.81 Hz, 1 H) 7.34 (t, *J*=7.99 Hz, 1 H) 7.47 (s, 1 H) 7.50 - 7.58 (m, 3 H) 7.69 (d, *J*=8.30 Hz, 2 H) 7.76 (s, 1 H) 8.47 (s, 1 H) 10.31 (s, 1 H).

### Example 6

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-propyl-piperazin-1-ylmethyl)-3-trifluoromethyl-phenyl]-acetamide (cmpd 164)

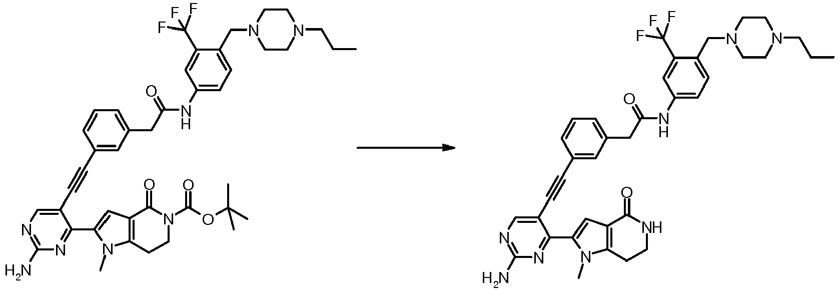

To a solution of 2-[2-amino-5-(3-{[4-(4-propyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester (0.09 g, 0.12 mmol) in DCM (5 mL), 4 M HCl in dioxane (0.15 mL, 0.60 mmol) was added. The reaction was stirred at r.t. for 1 h, then evaporated under vacuum to obtain a crude that was purified by flash column chromatography (DCM-MeOH-NH₃ 90:10:0.8). The title compound (0.06 g, 74%) was obtained as white solid.
¹H NMR (600 MHz, DMSO-*d*₆) δ ppm 0.83 (t, *J*=7.33 Hz, 3 H) 1.36 - 1.45 (m, 2 H) 2.20 (br. s., 2 H) 2.26 - 2.47 (m, 7 H) 2.90 (t, *J*=6.87 Hz, 2 H) 3.46 (td, *J*=6.87, 2.56 Hz, 2 H) 3.52 (s, 2 H) 3.67 (s, 2 H) 3.86 (s, 4 H) 7.07 (s, 2 H) 7.21 (br. s., 1 H) 7.31 - 7.38 (m, 4 H) 7.52 (s, 1 H) 7.53 (s, 1 H) 7.64 (d, *J*=8.42 Hz, 1 H) 7.77 (dd, *J*=8.33, 1.74 Hz, 1 H) 8.04 (d, *J*=2.01 Hz, 1 H) 8.43 (s, 1 H) 10.46 (s, 1 H). (ESI) *m*/*z* 685 [(M + H)⁺].
HRMS (ESI) calculated for C₃₇H₄₀F₃N₈O₂⁺ [(M + H)⁺] 685.3221, found 685.3226.

According to this same methodology, but employing suitable substituted derivatives, the following compounds were prepared:

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-ethyl-piperazin-1-ylmethyl)-3-trifluoromethyl-phenyl]-acetamide (cmpd 77)

¹H NMR (600 MHz, DMSO-*d*₆) ppm 0.97 (t, *J*=7.14 Hz, 3 H) 2.29 (q, *J*=7.14 Hz, 3 H) 2.33 - 2.43 (m, 6 H) 2.90 (t, *J*=6.87 Hz, 2 H) 3.46 (td, *J*=6.82, 2.47 Hz, 2 H) 3.52 (s, 2 H) 3.67 (s, 2 H) 3.85 (s, 3 H) 7.07 (s, 2 H) 7.21 (br. s., 1 H) 7.29 - 7.39 (m, 3 H) 7.50 - 7.56 (m, 2 H) 7.65 (d, *J*=8.61 Hz, 1 H) 7.75 - 7.79 (m, 1 H) 8.04 (d, *J*=2.01 Hz, 1 H) 8.43 (s, 1 H) 10.48 (s, 1 H).
(ESI) *m*/*z* 671 [(M + H)⁺]. HRMS (ESI) calculated for C₃₆H₃₈F₃N₈O₂⁺ [(M + H)⁺] 671.3064, found 671.3064.

### 2-{5-[2-Amino-4-(1-methyl-4-oxo-4,5,6,7-tetrahydro-1H-pyrrolo[3,2-c]pyridin-2-yl)-pyrimidin-5-ylethynyl]-pyridin-3-yl}-N-[4-(4-ethyl-piperazin-1-ylmethyl)-3-trifluoromethyl-phenyl]-acetamide (cmpd 122)

¹H NMR (600 MHz, DMSO-*d*₆) δ ppm 0.92 - 1.07 (m, 3 H) 2.86 - 2.93 (m, 2 H) 3.42 - 3.47 (m, 2 H) 3.52 - 3.56 (m, 2 H) 3.73 - 3.78 (m, 2 H) 3.83 - 3.88 (m, 3 H) 7.12 - 7.15 (m, 2 H) 7.16 - 7.19 (m, 1 H) 7.47 - 7.49 (m, 1 H) 7.64 - 7.67 (m, 1 H) 7.75 - 7.79 (m, 1 H) 7.86 - 7.87 (m, 1 H) 8.02 - 8.04 (m, 1 H) 8.46 - 8.47 (m, 1 H) 8.48 - 8.49 (m, 1 H) 8.53 - 8.54 (m, 1 H) 10.49 - 10.55 (m, 1 H).
(ESI) *m*/*z* 672 [(M + H)⁺]. HRMS (ESI) calculated for C₃₅H₃₇F₃N₉O₂⁺ [(M + H)⁺] 672.3017, found 672.3015.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-ethyl-piperazin-1-ylmethyl)-3-fluoro-phenyl]-acetamide (cmpd 120)

¹H NMR (600 MHz, DMSO-*d*₆) δ ppm 0.95 (t, *J*=7.23 Hz, 3 H) 2.27 (q, *J*=7.20 Hz, 2 H) 2.30 - 2.41 (m, 6 H) 2.90 (t, *J*=6.87 Hz, 2 H) 3.42 (s, 2 H) 3.46 (td, *J*=6.82, 2.47 Hz, 2 H) 3.65 (s, 2 H) 3.86 (s, 3 H) 7.07 (s, 1 H) 7.22 (br. s., 1 H) 7.24 - 7.30 (m, 1 H) 7.31 - 7.39 (m, 1 H) 7.51 (s, 1 H) 7.53 (s, 1 H) 7.53 - 7.56 (m, 1 H) 8.43 (s, 1 H) 10.36 (s, 1 H).
(ESI) *m*/*z* 621 [(M + H)⁺]. HRMS (ESI) calculated for C₃₅H₃₈FN₈O₂⁺ [(M + H)⁺] 621.3097, found 621.3105.

### 4-(2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-acetylamino)-N-(2-dimethylamino-ethyl)-benzamide dihydrochloride (cmpd 121)

¹H NMR (600 MHz, DMSO-*d*₆) δ ppm 2.82 (d, *J*=4.95 Hz, 6 H) 2.91 (t, *J*=6.87 Hz, 2 H) 3.24 (q, *J*=5.98 Hz, 2 H) 3.45 - 3.48 (m, 4 H) 3.59 (q, *J*=5.74 Hz, 2 H) 3.70 (s, 2 H) 3.86 (s, 3 H) 7.15 - 7.34 (m, 2 H) 7.37 (s, 3 H) 7.53 (s, 1 H) 7.59 (s, 1 H) 7.70 (d, *J*=8.97 Hz, 2 H) 7.85 (d, *J*=8.61 Hz, 2 H) 8.46 (s, 1 H) 8.65 (t, *J*=5.40 Hz, 1 H) 9.75 (br. s., 1 H) 10.50 (s, 1 H).
(ESI) *m*/*z* 591 [(M + H)⁺]. HRMS (ESI) calculated for C₃₃H₃₇Cl₂N₈O₃⁺ [(M + H)⁺] 591.2827, found 591.2826.

### 2-{4-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-ethyl-piperazin-1-ylmethyl)-3-trifluoromethyl-phenyl]-acetamide (cmpd 123)

¹H NMR (600 MHz, DMSO-*d*₆) δ ppm 0.97 (t, *J*=7.23 Hz, 3 H) 2.27 - 2.31 (m, 3 H) 2.32 - 2.46 (m, 6 H) 2.88 (t, *J*=6.87 Hz, 2 H) 3.44 (td, *J*=6.87, 2.56 Hz, 2 H) 3.52 (s, 2 H) 3.69 (s, 2 H) 3.85 (s, 3 H) 7.05 (s, 2 H) 7.09 (br. s., 1 H) 7.35 (d, *J*=8.43 Hz, 2 H) 7.43 (d, *J*=8.24 Hz, 2 H) 7.48 - 7.50 (m, 1 H) 7.65 (d, *J*=8.61 Hz, 1 H) 7.77 (dd, *J*=8.42, 1.83 Hz, 1 H) 8.03 (d, *J*=2.01 Hz, 1 H) 8.42 (s, 1 H) 10.46 (s, 1 H).
(ESI) *m*/*z* 671 [(M + H)⁺]. HRMS (ESI) calculated for C₃₆H₃₈F₃N₈O₂⁺ [(M + H)⁺] 671.3065, found 671.3074.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[2-(4-ethyl-piperazin-1-ylmethyl)-5-trifluoromethyl-phenyl]-acetamide (cmpd 124)

¹H NMR (600 MHz, DMSO-*d*₆) δ ppm 0.85 (t, *J*=7.14 Hz, 3 H) 2.18 - 2.22 (m, 3 H) 2.23 - 2.36 (m, 4 H) 2.86 (t, *J*=6.78 Hz, 2 H) 3.39 - 3.44 (m, 2 H) 3.55 (s, 2 H) 3.74 (s, 2 H) 3.82 (s, 3 H) 7.05 (s, 2 H) 7.10 (br. s., 1 H) 7.27 - 7.39 (m, 4 H) 7.40 - 7.42 (m, 1 H) 7.52 (s, 2 H) 8.36 - 8.40 (m, 2 H) 10.76 (s, 1 H).
(ESI) *m*/*z* 671 [(M + H)⁺]. HRMS (ESI) calculated for C₃₆H₃₈F₃N₈O₂⁺ [(M + H)⁺] 671.3065, found 671.3079.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-chloro-3-(4-ethyl-piperazine-1-carbonyl)-phenyl]-acetamide dihydrochloride (cmpd 125)

¹H NMR (600 MHz, DMSO-*d*₆) δ ppm 1.22 (br. s., 3 H) 2.91 (t, *J*=6.78 Hz, 2 H) 3.45 - 3.48 (m, 4 H) 3.68 (br. s., 2 H) 3.86 (s, 3 H) 4.51 - 4.65 (m, 1 H) 7.24 (br. s., 1 H) 7.33 - 7.39 (m, 3 H) 7.43 - 7.53 (m, 3 H) 7.59 (br. s., 1 H) 8.45 (s, 1 H).
(ESI) *m*/*z* 651 [(M + H)⁺] HRMS (ESI) calculated for C₃₅H₃₈Cl₃N₈O₃⁺ [(M + H)⁺] 651.2594, found 651.2591.

### 2-{4-[2-Amino-4-(1-methyl-4-oxo-4,5,6,7-tetrahydro-1H-pyrrolo[3,2-c]pyridin-2-yl)-pyrimidin-5-ylethynyl]-thiophen-2-yl}-N-[4-(4-ethyl-piperazin-1-ylmethyl)-3-trifluoromethyl-phenyl]-acetamide (cmpd 126)

¹H NMR (600 MHz, DMSO-*d*₆) δ ppm 0.98 (t, *J*=6.87 Hz, 3 H) 2.00 - 2.48 (m, 10 H) 2.88 (t, *J*=6.87 Hz, 2 H) 3.44 (td, *J*=6.87, 2.38 Hz, 2 H) 3.54 (s, 2 H) 3.84 (s, 3 H) 3.91 (s, 2 H) 7.03 (s, 2 H) 7.08 (s, 1 H) 7.11 (br. s., 1 H) 7.45 (s, 1 H) 7.61 (d, *J*=1.28 Hz, 1 H) 7.67 (d, *J*=8.61 Hz, 1 H) 7.78 (d, *J*=8.24 Hz, 1 H) 8.03 (d, *J*=1.83 Hz, 1 H) 8.39 (s, 1 H) 10.52 (s, 1 H).
(ESI) *m*/*z* 677 [(M + H)⁺]. HRMS (ESI) calculated for C₃₄H₃₆F₃N₈O₂S⁺ [(M + H)⁺] 677.2629, found 677.2647.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[3-(4-ethyl-piperazine-1-carbonyl)-4-trifluoromethyl-phenyl]-acetamide (cmpd 127)

¹H NMR (600 MHz, DMSO-*d*₆) δ ppm 0.84 - 1.07 (m, 3 H) 2.15 - 2.44 (m, 6 H) 2.90 (t, *J*=6.87 Hz, 2 H) 2.99 - 3.17 (m, 2 H) 3.46 (td, *J*=6.87, 2.38 Hz, 2 H) 3.50 - 3.67 (m, 2 H) 3.69 - 3.74 (m, 2 H) 3.86 (s, 3 H) 7.07 (s, 2 H) 7.22 (br. s., 1 H) 7.28 - 7.41 (m, 3 H) 7.51 - 7.57 (m, 2 H) 7.67 (br. s., 1 H) 7.73 - 7.80 (m, 2 H) 8.43 (s, 1 H) 10.65 (s, 1 H).
(ESI) *m*/*z* 685 [(M + H)⁺]. HRMS (ESI) calculated for C₃₆H₃₆F₃N₈O₃⁺ [(M + H)⁺] 685.2857, found 685.2858.

### 2-{2-[2-Amino-4-(1-methyl-4-oxo-4,5,6,7-tetrahydro-1H-pyrrolo[3,2-c]pyridin-2-yl)-pyrimidin-5-ylethynyl]-pyridin-4-yl}-N-[4-(4-ethyl-piperazin-1-ylmethyl)-3-trifluoromethyl-phenyl]-acetamide (cmpd 128)

¹H NMR (600 MHz, DMSO-*d*₆) δ ppm 0.97 (t, *J*=7.20 Hz, 3 H) 2.24 - 2.33 (m, 2 H) 2.36 (br. s., 8 H) 2.90 (t, *J*=6.90 Hz, 2 H) 3.46 (td, *J*=6.87, 2.50 Hz, 2 H) 3.52 (s, 2 H) 3.73 (s, 2 H) 3.86 (s, 3 H) 7.18 (s, 2 H) 7.23 (t, *J*=2.08 Hz, 1 H) 7.32 (dd, *J*=5.07, 1.53 Hz, 1 H) 7.47 (s, 1 H) 7.59 (s, 1 H) 7.66 (d, *J*=8.54 Hz, 1 H) 7.77 (dd, *J*=8.54, 1.83 Hz, 1 H) 8.03 (d, *J*=1.95 Hz, 1 H) 8.49 (s, 1 H) 8.50 (d, *J*=5.13 Hz, 1 H) 10.53 (s, 1 H).
(ESI) *m*/*z* 672 [(M + H)⁺]. HRMS (ESI) calculated for C₃₅H₃₇F₃N₉O₂⁺ [(M + H)⁺] 672.3017, found 672.3025.

### 2-{5-[2-Amino-4-(1-methyl-4-oxo-4,5,6,7-tetrahydro-1H-pyrrolo[3,2-c]pyridin-2-yl)-pyrimidin-5-ylethynyl]-thiophen-2-yl}-N-[4-(4-ethyl-piperazin-1-ylmethyl)-3-trifluoromethyl-phenyl]-acetamide (cmpd 129)

¹H NMR (600 MHz, DMSO-*d*₆) δ ppm 0.98 (t, *J*=6.87 Hz, 3 H) 2.08 - 2.48 (m, 7 H) 2.88 (t, *J*=6.87 Hz, 1 H) 3.43 (td, *J*=6.82, 2.47 Hz, 1 H) 3.54 (s, 1 H) 3.84 (s, 2 H) 3.92 (s, 1 H) 6.97 (d, *J*=3.48 Hz, 1 H) 7.08 (s, 2 H) 7.17 (d, *J*=3.66 Hz, 1 H) 7.35 (s, 1 H) 7.67 (d, *J*=8.61 Hz, 1 H) 7.77 (d, *J*=8.24 Hz, 1 H) 8.02 (d, *J*=2.01 Hz, 1 H) 8.41 (s, 1 H) 10.52 (s, 1 H).
(ESI) *m*/*z* 677 [(M + H)⁺]. HRMS (ESI) calculated for C₃₄H₃₆F₃N₈O₂S⁺ [(M + H)⁺] 677.2629, found 677.2631.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-bromo-3-(4-ethyl-piperazine-1-carbonyl)-phenyl]-acetamide dihydrochloride (cmpd 148)

¹H NMR (600 MHz, DMSO-*d*₆) δ ppm 1.21 (br. s., 3 H) 2.92 (t, *J*=6.78 Hz, 2 H) 3.45 - 3.48 (m, 4 H) 3.69 (br. s., 2 H) 3.87 (s, 3 H) 4.50 - 4.64 (m, 1 H) 7.25 (br. s., 1 H) 7.34 - 7.40 (m, 3 H) 7.45 - 7.54 (m, 3 H) 7.60 (br. s., 1 H) 8.47 (s, 1 H) 10.59 (br. s, 1 H).
(ESI) *m*/*z* 769 [(M + H)⁺]. HRMS (ESI) calculated for C₃₅H₃₈BrCl₂N₈O₃⁺ [(M + H)⁺] 769.5301, found 769.5304.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-[1,4]diazepan-1-ylmethyl)-3-trifluoromethyl-phenyl]-acetamide (cmpd 149)

¹H NMR (600 MHz, DMSO-*d*₆) δ ppm 1.71 (t, *J*=5.68 Hz, 2 H) 2.27 (br. s., 3 H) 2.54 - 2.66 (m, 7 H) 2.90 (t, *J*=6.87 Hz, 2 H) 3.46 (td, *J*=6.87, 2.38 Hz, 2 H) 3.67 (s, 4 H) 3.86 (s, 3 H) 7.07 (s, 2 H) 7.22 (br. s., 1 H) 7.29 - 7.40 (m, 3 H) 7.50 - 7.56 (m, 2 H) 7.70 (d, *J*=8.43 Hz, 1 H) 7.78 (dd, *J*=8.52, 1.56 Hz, 1 H) 8.03 (d, *J*=2.02 Hz, 1 H) 8.43 (s, 1 H) 10.46 (s, 1 H).
(ESI) *m*/*z* 671 [(M + H)⁺]. HRMS (ESI) calculated for C₃₆H₃₈F₃N₈O₂⁺ [(M + H)⁺] 671.3065, found 671.3074.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-((S)-3-dimethylamino-pyrrolidin-1-ylmethyl)-3-trifluoromethyl-phenyl]-acetamide (cmpd 150)

¹H NMR (600 MHz, DMSO-*d*₆) δ ppm 1.63 (dd, *J*=12.27, 6.96 Hz, 1 H) 1.86 (dd, *J*=13.28, 5.22 Hz, 1 H) 2.05 - 2.17 (m, 5 H) 2.34 (br. s., 1 H) 2.44 - 2.48 (m, 1 H) 2.53 - 2.57 (m, 1 H) 2.60 - 2.64 (m, 1 H) 2.77 (br. s., 1 H) 2.90 (t, *J*=6.87 Hz, 2 H) 3.46 (td, *J*=6.87, 2.38 Hz, 2 H) 3.57 - 3.66 (m, 2 H) 3.67 (s, 2 H) 3.86 (s, 3 H) 7.07 (s, 2 H) 7.21 (br. s., 1 H) 7.31 - 7.39 (m, 3 H) 7.52 (s, 1 H) 7.53 (s, 1 H) 7.63 (d, *J*=8.61 Hz, 1 H) 7.78 (dd, *J*=8.43, 1.83 Hz, 1 H) 8.03 (d, *J*=2.01 Hz, 1 H) 8.43 (s, 1 H) 10.46 (s, 1 H).
(ESI) *m*/*z* 671 [(M + H)⁺]. HRMS (ESI) calculated for C₃₆H₃₈F₃N₈O₂⁺ [(M + H)⁺] 671.3065, found 671.3063.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-ethyl-piperazine-1-carbonyl)-phenyl]-acetamide dihydrochloride (cmpd 153)

¹H NMR (600 MHz, DMSO-*d*₆) δ ppm 1.24 (t, *J*=7.33 Hz, 3 H) 2.92 (t, *J*=6.78 Hz, 2 H) 2.97 - 3.05 (m, 2 H) 3.08 - 3.15 (m, 2 H) 3.87 (s, 3 H) 7.27 (br. s., 1 H) 7.35 - 7.39 (m, 3 H) 7.42 (d, *J*=8.61 Hz, 2 H) 7.54 (s, 1 H) 7.64 (s, 1 H) 7.70 (d, *J*=8.61 Hz, 2 H) 8.48 (s, 1 H) 10.50 (s, 1 H) 10.68 (br. s., 1 H).
(ESI) *m*/*z* 617 [(M + H)⁺]. HRMS (ESI) calculated for C₃₅H₃₉Cl₂N₈O₃⁺ [(M + H)⁺] 617.2983, found 769.2990.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[3-cyclopropyl-4-(4-methyl-piperazin-1-ylmethyl)-phenyl]-acetamide (cmpd 154)

¹H NMR (600 MHz, DMSO-*d*₆) δ ppm 0.49 - 0.55 (m, 2 H) 0.84 - 0.95 (m, 2 H) 2.06 - 2.24 (m, 5 H) 2.23 - 2.48 (m, 7 H) 2.90 (t, *J*=6.87 Hz, 2 H) 3.47 (td, *J*=6.82, 2.47 Hz, 2 H) 3.51 (s, 2 H) 3.61 (s, 2 H) 3.86 (s, 3 H) 7.07 (s, 2 H) 7.12 (d, *J*=8.24 Hz, 1 H) 7.15 (d, *J*=2.01 Hz, 1 H) 7.24 (br. s., 1 H) 7.25 - 7.36 (m, 3 H) 7.38 (dd, *J*=8.24, 1.83 Hz, 1 H) 7.53 (s, 1 H) 7.55 (s, 1 H) 8.43 (s, 1 H) 10.06 (s, 1 H).
(ESI) *m*/*z* 629 [(M + H)⁺]. HRMS (ESI) calculated for C₃₇H₄₁N₈O₂⁺ [(M + H)⁺] 629.3347, found 629.3356.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-4-fluoro-phenyl}-N-[4-(4-ethyl-piperazin-1-ylmethyl)-3-trifluoromethyl-phenyl]-acetamide (cmpd 155)

¹H NMR (600 MHz, DMSO-*d*₆) δ ppm 0.99 (br. s., 3 H) 2.12 - 2.49 (m, 11 H) 2.89 (t, *J*=6.87 Hz, 2 H) 3.45 (td, *J*=6.87, 2.56 Hz, 2 H) 3.53 (br. s., 2 H) 3.67 (s, 2 H) 3.85 (s, 3 H) 7.13 (s, 2 H) 7.19 (br. s., 1 H) 7.26 (t, *J*=8.97 Hz, 1 H) 7.35 - 7.39 (m, 1 H) 7.48 (s, 1 H) 7.56 (dd, *J*=6.78, 2.01 Hz, 1 H) 7.65 (d, *J*=8.61 Hz, 1 H) 7.77 (d, *J*=8.24 Hz, 1 H) 8.03 (d, *J*=1.83 Hz, 1 H) 8.44 (s, 1 H).
(ESI) *m*/*z* 689 [(M + H)⁺]. HRMS (ESI) calculated for C₃₆H₃₇F₄N₈O₂⁺ [(M + H)⁺] 689.2970, found 689.2987.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-2-fluoro-phenyl}-N-[4-(4-ethyl-piperazin-1-ylmethyl)-3-trifluoromethyl-phenyl]-acetamide (cmpd 156)

¹H NMR (600 MHz, DMSO-*d*₆) δ ppm 0.99 (br. s., 3 H) 2.15 - 2.50 (m, 45 H) 2.88 (t, *J*=6.87 Hz, 2 H) 3.43 (td, *J*=6.82, 2.47 Hz, 2 H) 3.54 (br. s., 2 H) 3.79 (s, 2 H) 3.84 (s, 3 H) 7.08 (br. s., 1 H) 7.12 (s, 2 H) 7.20 (t, *J*=7.69 Hz, 1 H) 7.41 (t, *J*=7.42 Hz, 1 H) 7.43 (s, 1 H) 7.44 - 7.46 (m, 1 H) 7.66 (d, *J*=8.43 Hz, 1 H) 7.78 (d, *J*=8.24 Hz, 1 H) 8.05 (d, *J*=1.83 Hz, 1 H) 8.43 (s, 1 H) 10.53 (s, 1 H).
(ESI) *m*/*z* 689 [(M + H)⁺]. HRMS (ESI) calculated for C₃₆H₃₇F₄N₈O₂⁺ [(M + H)⁺] 689.2970, found 689.2986.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-((R)-3-dimethylamino-pyrrolidin-1-ylmethyl)-3-trifluoromethyl-phenyl]-acetamide (cmpd 157)

¹H NMR (600 MHz, DMSO-*d*₆) δ ppm 1.63 (dd, *J*=12.73, 6.69 Hz, 1 H) 1.80 - 1.91 (m, 1 H) 2.00 - 2.18 (m, 6 H) 2.33 (d, *J*=6.04 Hz, 1 H) 2.53 - 2.57 (m, 1 H) 2.59 - 2.64 (m, 1 H) 2.77 (br. s., 1 H) 2.90 (t, *J*=6.87 Hz, 2 H) 3.46 (td, *J*=6.87, 2.56 Hz, 2 H) 3.57 - 3.67 (m, 2 H) 3.67 (s, 2 H) 3.86 (s, 3 H) 7.07 (s, 2 H) 7.21 (s, 1 H) 7.31 - 7.39 (m, 3 H) 7.52 (s, 1 H) 7.53 (s, 1 H) 7.63 (d, *J*=8.61 Hz, 1 H) 7.78 (dd, *J*=8.52, 1.74 Hz, 1 H) 8.03 (d, *J*=2.01 Hz, 1 H) 8.43 (s, 1 H) 10.46 (s, 1 H).
(ESI) *m*/*z* 671 [(M + H)⁺]. HRMS (ESI) calculated for C₃₆H₃₈F₃N₈O₂⁺ [(M + H)⁺] 671.3065, found 671.3067.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-isopropyl-piperazin-1-ylmethyl)-3-trifluoromethyl-phenyl]-acetamide (cmpd 158)

¹H NMR (600 MHz, DMSO-*d*₆) δ ppm 0.95 (d, *J*=5.68 Hz, 6 H) 2.23 - 2.47 (m, 8 H) 2.61 (d, *J*=1.83 Hz, 1 H) 2.90 (t, *J*=6.87 Hz, 2 H) 3.46 (td, *J*=6.82, 2.47 Hz, 2 H) 3.51 (s, 2 H) 3.67 (s, 2 H) 3.86 (s, 3 H) 7.07 (s, 2 H) 7.21 (s, 1 H) 7.31 - 7.39 (m, 3 H) 7.52 (s, 1 H) 7.53 (s, 1 H) 7.65 (d, *J*=8.43 Hz, 1 H) 7.76 - 7.79 (m, 1 H) 8.04 (d, *J*=2.01 Hz, 1 H) 8.43 (s, 1 H) 10.47 (s, 1 H).
(ESI) *m*/*z* 685 [(M + H)⁺]. HRMS (ESI) calculated for C₃₇H₄₀F₃N₈O₂⁺ [(M + H)⁺] 685.3221, found 685.3224.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-piperidin-1-ylmethyl-3-trifluoromethyl-phenyl)-acetamide (cmpd 159)

¹H NMR (600 MHz, DMSO-*d*₆) δ ppm 1.36 (br. s., 2 H) 1.43 - 1.51 (m, 4 H) 2.28 (br. s., 4 H) 2.82 - 2.91 (m, 2 H) 3.40 - 3.49 (m, 4 H) 3.65 (s, 2 H) 3.83 (s, 3 H) 7.05 (s, 2 H) 7.19 (br. s., 1 H) 7.30 - 7.36 (m, 3 H) 7.49 (s, 1 H) 7.51 (s, 1 H) 7.64 (d, *J*=8.61 Hz, 1 H) 7.74 (d, *J*=8.79 Hz, 1 H) 8.01 (s, 1 H) 8.41 (s, 1 H) 10.43 (s, 1 H).
(ESI) *m*/*z* 642 [(M + H)⁺]. HRMS (ESI) calculated for C₃₅H₃₅F₃N₇O₂⁺ [(M + H)⁺] 642.2799, found 642.2798.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-morpholin-4-ylmethyl-3-trifluoromethyl-phenyl)-acetamide (cmpd 160)

¹H NMR (600 MHz, DMSO-*d*₆) δ ppm 2.33 - 2.38 (m, 4 H) 2.90 (t, *J*=6.87 Hz, 2 H) 3.46 (td, *J*=6.82, 2.47 Hz, 2 H) 3.53 (s, 2 H) 3.57 (t, *J*=4.40 Hz, 4 H) 3.67 (s, 2 H) 3.86 (s, 3 H) 7.07 (s, 1 H) 7.21 (br. s., 1 H) 7.31 - 7.39 (m, 3 H) 7.52 (s, 1 H) 7.53 (s, 1 H) 7.67 (d, *J*=8.61 Hz, 1 H) 7.78 (dd, *J*=8.52, 1.56 Hz, 1 H) 8.05 (d, *J*=2.01 Hz, 1 H) 8.43 (s, 1 H) 10.47 (s, 1 H).
(ESI) *m*/*z* 644 [(M + H)⁺]. HRMS (ESI) calculated for C₃₄H₃₃F₃N₇O₃⁺ [(M + H)⁺] 644.2592, found 644.2605.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide (cmpd 161)

¹H NMR (600 MHz, DMSO-*d*₆) δ ppm 2.12 (s, 2 H) 2.15 - 2.44 (m, 8 H) 2.87 (t, *J*=6.87 Hz, 2 H) 3.43 (td, *J*=6.87, 2.56 Hz, 2 H) 3.49 (s, 2 H) 3.64 (s, 2 H) 3.83 (s, 3 H) 7.05 (s, 2 H) 7.19 (br. s., 1 H) 7.28 - 7.37 (m, 3 H) 7.49 (s, 1 H) 7.50 (s, 1 H) 7.61 (d, *J*=8.61 Hz, 1 H) 7.75 (dd, *J*=8.52, 1.92 Hz, 1 H) 8.01 (d, *J*=2.01 Hz, 1 H) 8.41 (s, 1 H) 10.44 (s, 1 H).
(ESI) *m*/*z* 657 [(M + H)⁺]. HRMS (ESI) calculated for C₃₅H₃₆F₃N₈O₂⁺ [(M + H)⁺] 657.2908, found 657.2905.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(5-bromo-pyridin-3-yl)-acetamide (cmpd 162)

¹H NMR (600 MHz, DMSO-*d*₆) δ ppm 2.90 (t, *J*=6.87 Hz, 2 H) 3.46 (td, *J*=6.87, 2.38 Hz, 2 H) 3.71 (s, 2 H) 3.86 (s, 3 H) 7.07 (s, 2 H) 7.22 (br. s., 1 H) 7.30 - 7.38 (m, 3 H) 7.52 (s, 1 H) 7.53 (s, 1 H) 8.34 - 8.40 (m, 2 H) 8.44 (s, 1 H) 8.67 (d, *J*=1.83 Hz, 1 H) 10.60 (s, 1 H)
(ESI) *m*/*z* 556 [(M + H)⁺]. HRMS (ESI) calculated for C₂₇H₂₃BrN₇O₂⁺ [(M + H)⁺] 556.1091, found 556.1105.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(3-pyrrolidin-1-yl-azetidin-1-ylmethyl)-3-trifluoromethyl-phenyl]-acetamide (cmpd 163)

¹H NMR (600 MHz, DMSO-*d*₆) δ ppm 1.67 (br. s., 4 H) 2.21 - 2.43 (m, 4 H) 2.90 (t, *J*=6.87 Hz, 2 H) 2.93 (br. s., 1 H) 3.02 - 3.11 (m, 1 H) 3.36 (t, *J*=6.32 Hz, 2 H) 3.46 (td, *J*=6.87, 2.56 Hz, 2 H) 3.65 (br. s., 1 H) 3.67 (s, 2 H) 3.86 (s, 3 H) 7.07 (s, 1 H) 7.21 (br. s., 1 H) 7.29 - 7.39 (m, 1 H) 7.52 (s, 1 H) 7.53 (s, 1 H) 7.58 (d, *J*=8.42 Hz, 1 H) 7.77 (d, *J*=8.06 Hz, 1 H) 8.02 (d, *J*=1.83 Hz, 1 H) 8.43 (s, 1 H) 10.45 (s, 1 H).
(ESI) *m*/*z* 683 [(M + H)⁺]. HRMS (ESI) calculated for C₃₇H₃₈F₃N₈O₂⁺ [(M + H)⁺] 683.3065, found 683.3077.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-ethyl-piperazine-1-carbonyl)-3-trifluoromethyl-phenyl]-acetamide (cmpd 165)

¹H NMR (600 MHz, DMSO-*d*₆) δ ppm 0.98 (t, *J*=7.14 Hz, 3 H) 2.13 - 2.46 (m, 2 H) 2.32 (q, *J*=7.08 Hz, 3 H) 2.90 (t, *J*=6.87 Hz, 2 H) 2.98 - 3.16 (m, 2 H) 3.46 (td, *J*=6.82, 2.47 Hz, 2 H) 3.60 (d, *J*=15.57 Hz, 2 H) 3.70 (s, 2 H) 3.86 (s, 3 H) 7.07 (s, 2 H) 7.22 (s, 1 H) 7.32 - 7.40 (m, 4 H) 7.51 - 7.54 (m, 2 H) 7.85 (dd, *J*=8.43, 1.65 Hz, 1 H) 8.12 (d, *J*=1.83 Hz, 1 H) 8.44 (s, 1 H) 10.62 (s, 1 H).
(ESI) *m*/*z* 685 [(M + H)⁺]. HRMS (ESI) calculated for C₃₆H₃₆F₃N₈O₂⁺ [(M + H)⁺] 685.2857, found 685.2866.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-{[methyl-(1-methyl-piperidin-4-yl)-amino]-methyl}-3-trifluoromethyl-phenyl)-acetamide (cmpd 166)

(ESI) *m*/*z* 685 [(M + H)⁺]. HRMS (ESI) calculated for C₃₇H₄₀F₃N₈O₂⁺ [(M + H)⁺] 685.3221, found 685.3221.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-dimethylamino-piperidin-1-ylmethyl)-3-trifluoromethyl-phenyl]-acetamide (cmpd 167)

¹H NMR (600 MHz, DMSO-*d*₆) δ ppm 1.31 - 1.44 (m, 2 H) 1.70 (d, *J*=11.54 Hz, 2 H) 1.95 (t, *J*=10.90 Hz, 2 H) 2.05 - 2.14 (m, 1 H) 2.18 (br. s., 5 H) 2.78 (d, *J*=11.72 Hz, 2 H) 2.86 - 2.92 (m, 2 H) 3.46 (td, *J*=6.91, 2.47 Hz, 2 H) 3.50 (s, 2 H) 3.67 (s, 2 H) 3.86 (s, 3 H) 7.07 (s, 2 H) 7.21 (s, 1 H) 7.31 - 7.41 (m, 3 H) 7.52 (s, 1 H) 7.53 (s, 1 H) 7.66 (d, *J*=8.61 Hz, 1 H) 7.77 (dd, *J*=8.52, 1.74 Hz, 1 H) 8.04 (d, *J*=2.01 Hz, 1 H) 8.43 (s, 1 H) 10.47 (s, 1 H).
(ESI) *m*/*z* 685 [(M + H)⁺]. HRMS (ESI) calculated for C₃₇H₄₀F₃N₈O₂⁺ [(M + H)⁺] 685.3221, found 685.3224.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[3-bromo-4-(4-ethyl-piperazin-1-ylmethyl)-phenyl]-acetamide (cmpd 168)

¹H NMR (600 MHz, DMSO-*d*₆) δ ppm 0.97 (t, *J*=7.14 Hz, 3 H) 2.20 - 2.48 (m, 9 H) 2.90 (t, *J*=6.87 Hz, 2 H) 3.43 - 3.49 (m, 5 H) 3.65 (s, 2 H) 3.86 (s, 3 H) 7.07 (s, 2 H) 7.22 (br. s., 1 H) 7.37 (br. s., 5 H) 7.47 - 7.50 (m, 1 H) 7.51 (s, 1 H) 7.53 (s, 1 H) 7.96 (d, *J*=2.01 Hz, 1 H) 8.43 (s, 1 H) 10.31 (s, 1 H).
(ESI) *m*/*z* 681 [(M + H)⁺]. HRMS (ESI) calculated for C₃₅H₃₈BrN₈O₂⁺ [(M + H)⁺] 681.2296, found 681.2308.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-{4-[4-(2-hydroxy-ethyl)-piperazin-1-ylmethyl]-3-trifluoromethyl-phenyl}-acetamide dihydrochloride (cmpd 170)

¹H NMR (600 MHz, DMSO-*d*₆) δ ppm 2.89 - 2.94 (m, 2 H) 3.71 (s, 2 H) 3.75 (t, *J*=4.67 Hz, 3 H) 3.87 (s, 3 H) 7.21 - 7.30 (m, 1 H) 7.32 - 7.41 (m, 3 H) 7.54 (s, 1 H) 7.64 (d, *J*=3.85 Hz, 1 H) 7.81 (br. s., 1 H) 7.88 (d, *J*=6.96 Hz, 1 H) 8.11 (br. s., 1 H) 8.46 - 8.49 (m, 1 H) 10.20 (br. s., 1 H) 10.71 (br. s., 1 H).
(ESI) *m*/*z* 687 [(M + H)⁺]. HRMS (ESI) calculated for C₃₆H₄₀Cl₂F₃N₈O₃⁺ [(M + H)⁺] 687.3014, found 687.3027.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-cyclopropylmethyl-piperazin-1-ylmethyl)-3-trifluoromethyl-phenyl]-acetamide (cmpd 171)

¹H NMR (600 MHz, DMSO-*d*₆) δ ppm 0.07 (br. s., 2 H) 0.45 (br. s., 2 H) 0.81 (br. s., 1 H) 1.98 - 2.49 (m, 9 H) 2.90 (t, *J*=6.87 Hz, 2 H) 3.43 - 3.48 (m, 2 H) 3.53 (br. s., 2 H) 3.67 (s, 2 H) 3.86 (s, 3 H) 7.07 (s, 2 H) 7.21 (s, 1 H) 7.32 - 7.39 (m, 3 H) 7.52 (s, 1 H) 7.53 (s, 1 H) 7.65 (d, *J*=8.42 Hz, 1 H) 7.78 (d, *J*=8.24 Hz, 1 H) 8.04 (d, *J*=2.01 Hz, 1 H) 8.43 (s, 1 H) 10.47 (s, 1 H).
(ESI) *m*/*z* 697 [(M + H)⁺]. HRMS (ESI) calculated for C₃₈H₄₀F₃N₈O₂⁺ [(M + H)⁺] 697.3221, found 697.3225.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(1-methyl-piperidin-4-yloxy)-3-trifluoromethyl-phenyl]-acetamide (cmpd 172)

¹H NMR (600 MHz, DMSO-*d*₆) δ ppm 1.66 (d, *J*=7.88 Hz, 2 H) 1.84 - 1.94 (m, 2 H) 2.17 (br. s., 3 H) 2.23 (br. s., 2 H) 2.90 (t, *J*=6.87 Hz, 2 H) 3.46 (td, *J*=6.82, 2.47 Hz, 2 H) 3.63 (s, 2 H) 3.86 (s, 3 H) 4.53 (br. s., 1 H) 7.07 (s, 2 H) 7.22 (br. s., 1 H) 7.25 (d, *J*=8.97 Hz, 1 H) 7.31 - 7.39 (m, 3 H) 7.51 (s, 1 H) 7.53 (s, 1 H) 7.73 (dd, *J*=8.97, 2.38 Hz, 1 H) 7.92 (d, *J*=2.56 Hz, 1 H) 8.43 (s, 1 H) 10.28 (s, 1 H).
(ESI) *m*/*z* 658 [(M + H)⁺]. HRMS (ESI) calculated for C₃₅H₃₅F₃N₇O₃⁺ [(M + H)⁺] 658.2748, found 658.2747.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-ethyl-piperazin-1-yl)-3-trifluoromethyl-phenyl]-acetamide (cmpd 173)

¹H NMR (600 MHz, DMSO-*d*₆) δ ppm 0.97 - 1.06 (m, 3 H) 2.30 - 2.48 (m, 5 H) 2.81 (br. s., 4 H) 2.90 (t, *J*=6.87 Hz, 2 H) 3.46 (td, *J*=6.78, 2.38 Hz, 2 H) 3.66 (s, 2 H) 3.86 (s, 3 H) 7.07 (s, 2 H) 7.21 (br. s., 1 H) 7.31 - 7.39 (m, 3 H) 7.50 - 7.55 (m, 3 H) 7.78 (d, *J*=8.24 Hz, 1 H) 7.99 (d, *J*=2.20 Hz, 1 H) 8.43 (s, 1 H) 10.41 (s, 1 H).
(ESI) *m*/*z* 657 [(M + H)⁺]. HRMS (ESI) calculated for C₃₅H₃₆F₃N₈O₂⁺ [(M + H)⁺] 657.2908, found 657.2908.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(1-methyl-piperidin-4-ylamino)-3-trifluoromethyl-phenyl]-acetamide (cmpd 177)

¹H NMR (600 MHz, DMSO-*d*₆) δ ppm 1.48 (q, *J*=9.83 Hz, 2 H) 1.86 (d, *J*=10.99 Hz, 2 H) 2.06 (d, *J*=7.51 Hz, 2 H) 2.16 (s, 3 H) 2.66 (br. s., 2 H) 2.90 (t, *J*=6.87 Hz, 2 H) 3.46 (td, *J*=6.69, 2.20 Hz, 2 H) 3.60 (s, 2 H) 3.85 (s, 3 H) 4.36 (d, *J*=7.69 Hz, 1 H) 6.88 (d, *J*=9.16 Hz, 1 H) 7.07 (s, 2 H) 7.21 (br. s., 1 H) 7.29 - 7.39 (m, 3 H) 7.50 (s, 1 H) 7.53 (s, 1 H) 7.55 (d, *J*=8.97 Hz, 1 H) 7.79 (d, *J*=2.20 Hz, 1 H) 8.43 (s, 1 H) 10.08 (s, 1 H).
(ESI) *m*/*z* 657 [(M + H)⁺]. HRMS (ESI) calculated for C₃₅H₃₆F₃N₈O₂⁺ [(M + H)⁺] 657.2908, found 657.2915.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-{4-[methyl-(1-methyl-piperidin-4-yl)-amino]-3-trifluoromethyl-phenyl}-acetamide (cmpd 178)

¹H NMR (600 MHz, DMSO-*d*₆) δ ppm 1.30 - 1.42 (m, 2 H) 1.63 (d, *J*=11.17 Hz, 2 H) 1.85 (br. s., 2 H) 2.14 (br. s., 3 H) 2.62 - 2.79 (m, 3 H) 2.90 (t, *J*=6.87 Hz, 2 H) 3.44 - 3.49 (m, 2 H) 3.66 (s, 2 H) 3.86 (s, 3 H) 7.07 (s, 2 H) 7.22 (br. s., 1 H) 7.30 - 7.38 (m, 3 H) 7.50 (d, *J*=6.04 Hz, 3 H) 7.77 - 7.81 (m, 1 H) 7.98 (d, *J*=2.01 Hz, 1 H) 8.43 (s, 1 H) 10.42 (s, 1 H).
(ESI) *m*/*z* 671 [(M + H)⁺]. HRMS (ESI) calculated for C₃₆H₃₈F₃N₈O₂⁺ [(M + H)⁺] 671.3065, found 671.3066.

### 2-(3-{2-Amino-4-[1-(2-hydroxy-ethyl)-4-oxo-4,5,6,7-tetrahydro-1H-pyrrolo[3,2-c]pyridin-2-yl]-pyrimidin-5-ylethynyl}-phenyl)-N-(4-chloro-3-trifluoromethyl-phenyl)-acetamide (cmpd 180)

¹H NMR (600 MHz, DMSO-*d*₆) δ ppm 2.95 (t, *J*=6.87 Hz, 2 H) 3.44 (td, *J*=6.82, 2.47 Hz, 3 H) 3.64 (q, *J*=5.68 Hz, 2 H) 3.70 (s, 2 H) 4.50 (t, *J*=5.59 Hz, 2 H) 4.83 (t, *J*=5.49 Hz, 1 H) 7.02 (br. s., 2 H) 7.23 (s, 1 H) 7.30 - 7.41 (m, 4 H) 7.55 (s, 1 H) 7.65 (d, *J*=8.79 Hz, 1 H) 7.67 (s, 1 H) 7.84 (dd, *J*=8.79, 2.56 Hz, 1 H) 8.18 (d, *J*=2.38 Hz, 1 H) 8.43 (s, 1 H) 10.63 (s, 1 H).
(ESI) *m*/*z* 609 [(M + H)⁺]. HRMS (ESI) calculated for C₃₀H₂₅ClF₃N₆O₃⁺ [(M + H)⁺] 609.1624, found 609.1634.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[3-trifluoromethyl-4-((2S,5R)-2,4,5-trimethyl-piperazin-1-ylmethyl)-phenyl]-acetamide (cmpd 181)

¹H NMR (600 MHz, DMSO-*d*₆) δ ppm 0.83 (d, *J*=6.23 Hz, 3 H) 0.99 (d, *J*=6.04 Hz, 3 H) 1.76 (t, *J*=10.71 Hz, 1 H) 1.90 (d, *J*=11.36 Hz, 2 H) 2.11 (br. s., 3 H) 2.34 - 2.43 (m, 2 H) 2.66 (d, *J*=10.62 Hz, 1 H) 2.90 (t, *J*=6.87 Hz, 2 H) 3.09 (d, *J*=15.20 Hz, 1 H) 3.46 (td, *J*=6.82, 2.47 Hz, 2 H) 3.67 (s, 2 H) 3.86 (s, 3 H) 4.04 (d, *J*=14.84 Hz, 1 H) 7.07 (s, 2 H) 7.21 (br. s., 1 H) 7.30 - 7.40 (m, 3 H) 7.52 (s, 1 H) 7.53 (s, 1 H) 7.69 - 7.72 (m, 1 H) 7.75 - 7.78 (m, 1 H) 8.04 (d, *J*=2.01 Hz, 1 H) 8.43 (s, 1 H) 10.46 (s, 1 H).
(ESI) *m*/*z* 685 [(M + H)⁺]. HRMS (ESI) calculated for C₃₇H₄₀F₃N₈O₂⁺ [(M + H)⁺] 685.3221, found 685.3231.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[3-trifluoromethyl-4-(3,4,5-trimethyl-piperazin-1-ylmethyl)-phenyl]-acetamide (cmpd 182)

¹H NMR (600 MHz, DMSO-*d*₆) δ ppm 0.95 (br. s., 5 H) 1.81 (br. s., 2 H) 2.16 (d, *J*=15.02 Hz, 4 H) 2.55 - 2.70 (m, 2 H) 2.90 (t, *J*=6.87 Hz, 2 H) 3.42 - 3.50 (m, 4 H) 3.67 (s, 2 H) 3.86 (s, 3 H) 7.07 (s, 2 H) 7.22 (s, 1 H) 7.30 - 7.39 (m, 3 H) 7.52 (s, 1 H) 7.53 (s, 1 H) 7.64 (d, *J*=8.43 Hz, 1 H) 7.78 (d, *J*=8.24 Hz, 1 H) 8.03 (d, *J*=2.01 Hz, 1 H) 8.43 (s, 1 H) 10.48 (s, 1 H).
(ESI) *m*/*z* 685 [(M + H)⁺]. HRMS (ESI) calculated for C₃₇H₄₀F₃N₈O₂⁺ [(M + H)⁺] 685.3221, found 685.3224.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-piperazin-1-ylmethyl-3-trifluoromethyl-phenyl)-acetamide (cmpd 183)

¹H NMR (600 MHz, DMSO-*d*₆) δ ppm 2.28 (br. s., 4 H) 2.69 (br. s., 4 H) 2.90 (t, *J*=6.87 Hz, 2 H) 3.46 (td, *J*=6.91, 2.47 Hz, 2 H) 3.49 (s, 2 H) 3.67 (s, 2 H) 3.85 (s, 3 H) 7.07 (s, 2 H) 7.21 (br. s., 1 H) 7.30 - 7.39 (m, 3 H) 7.52 (s, 1 H) 7.53 (s, 1 H) 7.66 (d, *J*=8.61 Hz, 1 H) 7.77 (dd, *J*=8.61, 1.65 Hz, 1 H) 8.04 (d, *J*=1.83 Hz, 1 H) 8.43 (s, 1 H) 10.46 (s, 1 H).
(ESI) *m*/*z* 643 [(M + H)⁺]. HRMS (ESI) calculated for C₃₄H₃₄F₃N₈O₂⁺ [(M + H)⁺] 643.2752, found 643.2770.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(3-oxo-piperazin-1-ylmethyl)-3-trifluoromethyl-phenyl]-acetamide (cmpd 184)

¹H NMR (600 MHz, DMSO-*d*₆) δ ppm 2.52 - 2.54 (m, 2 H) 2.90 (t, *J*=6.87 Hz, 2 H) 2.94 (s, 2 H) 3.10 - 3.15 (m, 2 H) 3.46 (td, *J*=6.82, 2.47 Hz, 2 H) 3.61 (s, 2 H) 3.68 (s, 2 H) 3.85 (s, 3 H) 7.07 (s, 2 H) 7.21 (s, 1 H) 7.26 - 7.40 (m, 3 H) 7.52 (s, 1 H) 7.53 (s, 1 H) 7.66 (d, *J*=8.61 Hz, 1 H) 7.74 (s, 1 H) 7.80 (dd, *J*=8.52, 1.74 Hz, 1 H) 8.06 (d, *J*=2.01 Hz, 1 H) 8.43 (s, 1 H) 10.49 (s, 1 H).
(ESI) *m*/*z* 657 [(M + H)⁺]. HRMS (ESI) calculated for C₃₄H₃₂F₃N₈O₃⁺ [(M + H)⁺] 657.2544, found 657.2536.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(3-hydroxy-piperidin-1-ylmethyl)-3-trifluoromethyl-phenyl]-acetamide (cmpd 185)

¹H NMR (600 MHz, DMSO-*d*₆) δ ppm 1.02 - 1.12 (m, 1 H) 1.41 (d, *J*=12.45 Hz, 1 H) 1.56 - 1.64 (m, 1 H) 1.73 (t, *J*=9.71 Hz, 1 H) 1.79 (d, *J*=8.79 Hz, 1 H) 1.89 (t, *J*=10.26 Hz, 1 H) 2.55 - 2.62 (m, 1 H) 2.72 (d, *J*=8.97 Hz, 1 H) 2.88 - 2.91 (m, 2 H) 3.40 - 3.49 (m, 4 H) 3.53 - 3.57 (m, 1 H) 3.67 (s, 2 H) 3.85 (s, 3 H) 4.55 (d, *J*=4.95 Hz, 1 H) 7.07 (s, 2 H) 7.21 (s, 1 H) 7.32 - 7.37 (m, 3 H) 7.52 (s, 1 H) 7.53 (s, 1 H) 7.66 (d, *J*=8.43 Hz, 1 H) 7.77 (d, *J*=8.61 Hz, 1 H) 8.03 (d, *J*=1.83 Hz, 1 H) 8.43 (s, 1 H) 10.46 (s, 1 H).
(ESI) *m*/*z* 658 [(M + H)⁺]. HRMS (ESI) calculated for C₃₅H₃₅F₃N₇O₃⁺ [(M + H)⁺] 658.2748, found 658.2747.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-cyclopropyl-acetamide (cmpd 186)

¹H NMR (600 MHz, DMSO-*d*₆) δ ppm 0.36 - 0.42 (m, 2 H) 0.56 - 0.61 (m, 2 H) 2.59 - 2.64 (m, 1 H) 2.89 (t, *J*=6.87 Hz, 2 H) 3.35 (s, 2 H) 3.46 (td, *J*=6.87, 2.38 Hz, 2 H) 3.85 (s, 3 H) 7.07 (s, 2 H) 7.18 (br. s., 1 H) 7.22 - 7.26 (m, 1 H) 7.29 - 7.34 (m, 2 H) 7.41 (s, 1 H) 7.51 (s, 1 H) 8.13 (d, *J*=4.21 Hz, 1 H) 8.43 (s, 1 H).
(ESI) *m*/*z* 441 [(M + H)⁺]. HRMS (ESI) calculated for C₂₅H₂₅N₆O₂⁺ [(M + H)⁺] 441.2034, found 441.2025.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-2-oxo-piperazin-1-ylmethyl)-3-trifluoromethyl-phenyl]-acetamide (cmpd 187)

¹H NMR (600 MHz, DMSO-*d*₆) δ ppm 2.23 (s, 3 H) 2.57 - 2.63 (m, 2 H) 2.90 (t, *J*=6.87 Hz, 2 H) 3.06 (s, 2 H) 3.18 (t, *J*=5.40 Hz, 2 H) 3.46 (td, *J*=6.87, 2.56 Hz, 2 H) 3.68 (s, 2 H) 3.85 (s, 3 H) 4.62 (s, 2 H) 7.07 (s, 2 H) 7.21 (s, 1 H) 7.24 (d, *J*=8.61 Hz, 1 H) 7.32 - 7.39 (m, 3 H) 7.51 (s, 1 H) 7.53 (s, 1 H) 7.75 - 7.80 (m, 1 H) 8.11 (d, *J*=1.83 Hz, 1 H) 8.43 (s, 1 H) 10.52 (s, 1 H).
(ESI) *m*/*z* 671 [(M + H)⁺]. HRMS (ESI) calculated for C₃₅H₃₄F₃N₈O₃⁺ [(M + H)⁺] 671.2701, found 671.2691.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-hydroxy-piperidin-1-ylmethyl)-3-trifluoromethyl-phenyl]-acetamide (cmpd 188)

¹H NMR (600 MHz, DMSO-*d*₆) δ ppm 1.33 - 1.42 (m, 2 H) 1.69 (d, *J*=9.52 Hz, 2 H) 2.00 - 2.08 (m, 2 H) 2.60 - 2.66 (m, 2 H) 2.90 (t, *J*=6.87 Hz, 2 H) 3.46 (td, *J*=6.78, 2.56 Hz, 3 H) 3.49 (s, 2 H) 3.67 (s, 2 H) 3.86 (s, 3 H) 4.53 (d, *J*=4.21 Hz, 1 H) 7.07 (s, 2 H) 7.21 (br. s., 1 H) 7.30 - 7.39 (m, 3 H) 7.52 (s, 1 H) 7.53 (s, 1 H) 7.65 (d, *J*=8.43 Hz, 1 H) 7.77 (d, *J*=8.43 Hz, 1 H) 8.03 (d, *J*=2.01 Hz, 1 H) 8.42 - 8.45 (m, 1 H) 10.46 (s, 1 H).
(ESI) *m*/*z* 658 [(M + H)⁺]. HRMS (ESI) calculated for C₃₅H₃₅F₃N₇O₃⁺ [(M + H)⁺] 658.2748, found 658.2752.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-{[(2-hydroxy-ethyl)-methyl-amino]-methyl}-3-trifluoromethyl-phenyl)-acetamide (cmpd 189)

¹H NMR (600 MHz, DMSO-*d*₆) δ ppm 2.15 (s, 3 H) 2.44 (t, *J*=6.23 Hz, 2 H) 2.90 (t, *J*=6.87 Hz, 2 H) 3.46 (td, *J*=6.82, 2.29 Hz, 2 H) 3.50 (q, *J*=5.98 Hz, 2 H) 3.57 (s, 2 H) 3.67 (s, 2 H) 3.86 (s, 3 H) 4.37 (t, *J*=5.40 Hz, 1 H) 7.07 (s, 2 H) 7.21 (br. s., 1 H) 7.32 - 7.39 (m, 4 H) 7.52 (s, 1 H) 7.53 (s, 1 H) 7.70 - 7.75 (m, 1 H) 7.76 - 7.79 (m, 1 H) 8.03 (s, 1 H) 8.42 - 8.45 (m, 1 H) 10.46 (s, 1 H).
(ESI) *m*/*z* 632 [(M + H)⁺]. HRMS (ESI) calculated for C₃₃H₃₃F₃N₇O₃⁺ [(M + H)⁺] 632.2592, found 632.2602.

### 2-(3-{2-Amino-4-[1-(2-hydroxy-ethyl)-4-oxo-4,5,6,7-tetrahydro-1H-pyrrolo[3,2-c]pyridin-2-yl]-pyrimidin-5-ylethynyl}-phenyl)-N-[4-(4-ethyl-piperazin-1-ylmethyl)-3-trifluoromethyl-phenyl]-acetamide (cmpd 191)

¹H NMR (600 MHz, DMSO-*d*₆) δ ppm 0.99 (br. s., 3 H) 2.38 (br. s., 10 H) 2.95 (t, *J*=6.87 Hz, 2 H) 3.44 (td, *J*=6.78, 2.38 Hz, 2 H) 3.53 (s, 2 H) 3.64 (q, *J*=5.56 Hz, 2 H) 3.68 (s, 2 H) 4.50 (t, *J*=5.59 Hz, 2 H) 4.83 (t, *J*=5.49 Hz, 1 H) 7.02 (br. s., 2 H) 7.23 (br. s., 1 H) 7.29 - 7.42 (m, 4 H) 7.55 (s, 1 H) 7.65 (d, *J*=8.61 Hz, 1 H) 7.68 (s, 1 H) 7.78 (d, *J*=8.61 Hz, 1 H) 8.04 (d, *J*=1.83 Hz, 1 H) 8.43 (s, 1 H) 10.48 (s, 1 H).
(ESI) *m*/*z* 701 [(M + H)⁺]. HRMS (ESI) calculated for C₃₇H₄₀F₃N₈O₃⁺ [(M + H)⁺] 701.3170, found 701.3159.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-{[(2,3-dihydroxy-propyl)-methyl-amino]-methyl}-3-trifluoromethyl-phenyl)-acetamide (cmpd 192)

¹H NMR (600 MHz, DMSO-*d*₆) δ ppm 2.14 (s, 3 H) 2.30 (dd, *J*=12.55, 6.87 Hz, 1 H) 2.44 (dd, *J*=12.45, 5.13 Hz, 1 H) 2.90 (t, *J*=6.78 Hz, 2 H) 3.23 - 3.29 (m, 1 H) 3.33 - 3.37 (m, 2 H) 3.46 (td, *J*=6.73, 2.29 Hz, 2 H) 3.53 - 3.64 (m, 3 H) 3.67 (s, 2 H) 3.86 (s, 3 H) 4.38 (d, *J*=4.58 Hz, 1 H) 4.42 (t, *J*=5.40 Hz, 1 H) 7.07 (s, 2 H) 7.21 (br. s., 1 H) 7.31 - 7.40 (m, 3 H) 7.52 (s, 1 H) 7.53 (s, 1 H) 7.71 - 7.75 (m, 1 H) 7.76 - 7.80 (m, 1 H) 8.03 (s, 1 H) 8.44 (s, 1 H) 10.46 (s, 1 H).
(ESI) *m*/*z* 662 [(M + H)⁺]. HRMS (ESI) calculated for C₃₄H₃₅F₃N₇0₄⁺ [(M + H)⁺] 662.2697, found 662.2684.

### 2-{3-[2-Acetylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide (cmpd 200)

¹H NMR (600 MHz, DMSO-*d*₆) δ ppm 2.15 - 2.20 (m, 6 H) 2.23 - 2.48 (m, 8 H) 2.92 (t, *J*=6.78 Hz, 2 H) 3.47 (td, *J*=6.87, 2.38 Hz, 2 H) 3.52 (s, 2 H) 3.70 (s, 2 H) 3.98 (s, 3 H) 7.27 (br. s., 1 H) 7.37 - 7.46 (m, 3 H) 7.60 (s, 1 H) 7.64 (d, *J*=8.61 Hz, 1 H) 7.73 (s, 1 H) 7.78 (d, *J*=8.43 Hz, 1 H) 8.04 (d, *J*=1.65 Hz, 1 H) 8.78 (s, 1 H) 10.48 (s, 1 H) 10.75 (s, 1 H).
(ESI) *m*/*z* 699 [(M + H)⁺]. HRMS (ESI) calculated for C₃₇H₃₈F₃N₈O₃⁺[(M + H)⁺] 699.3014, found 699.3002.

### 3-[2-Amino-4-(1-methyl-4-oxo-4,5,6,7-tetrahydro-1H-pyrrolo[3,2-c]pyridin-2-yl)-pyrimidin-5-ylethynyl]-N-[4-(4-ethyl-piperazin-1-ylmethyl)-3-trifluoromethyl-phenyl]-benzenesulfonamide(cmpd75)

¹H NMR (600 MHz, DMSO-*d₆*) δ ppm 0.96 (t, *J*=7.05 Hz, 3 H) 2.12 - 2.48 (m, 10 H) 2.91 (t, *J*=6.87 Hz, 2 H) 3.44 - 3.51 (m, 5 H) 3.85 (s, 3 H) 7.17 (s, 2 H) 7.34 (s, 1 H) 7.36 - 7.41 (m, 2 H) 7.45 (s, 1 H) 7.54 - 7.62 (m, 2 H) 7.65 (d, *J*=7.88 Hz, 1 H) 7.69 - 7.72 (m, 1 H) 7.82 (s, 1 H) 8.46 (s, 1 H).
(ESI) *m*/*z* 693 [(M + H)⁺]. HRMS (ESI) calculated for C₃₄H₃₆F₃N₈O₃S⁺ [(M + H)⁺] 693.2578, found 693.2571.

### 3-[2-Amino-4-(1-methyl-4-oxo-4,5,6,7-tetrahydro-1H-pyrrolo[3,2-c]pyridin-2-yl)-pyrimidin-5-ylethynyl]-N-cyclopropyl-benzenesulfonamide hydrochloride (cmpd 76)

¹H NMR (600 MHz, DMSO-*d₆*) δ ppm 0.31 - 0.42 (m, 2 H) 0.47 - 0.53 (m, 2 H) 2.14 (td, *J*=6.59, 3.11 Hz, 1 H) 2.91 (t, *J*=6.78 Hz, 2 H) 3.86 (s, 3 H) 7.09 - 7.46 (m, 2 H) 7.53 (s, 1 H) 7.63 - 7.68 (m, 1 H) 7.72 (d, *J*=7.69 Hz, 1 H) 7.79 (d, *J*=7.69 Hz, 1 H) 7.86 (s, 1 H) 7.96 (d, *J*=2.56 Hz, 1 H) 8.52 (s, 1 H).
(ESI) *mlz* 463 [(M + H)⁺]. HRMS (ESI) calculated for C₂₃H₂₄CIN₆O₃S⁺ [(M + H)⁺] 463.1547, found 463.1538.

### N-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-4-trifluoromethyl-benzamide (cmpd 57)

¹H NMR (600 MHz, DMSO-*d₆*) δ ppm 2.90 (t, *J*=6.84 Hz, 2 H) 3.46 (td, *J*=6.87, 2.50 Hz, 2 H) 3.86 (s, 3 H) 7.08 (s, 2 H) 7.14 (s, 1 H) 7.24 (dt, *J*=7.81, 1.22 Hz, 1 H) 7.41 (t, *J*=7.93 Hz, 1 H) 7.47 (s, 1 H) 7.83 (ddd, *J*=8.27, 2.11, 0.98 Hz, 1 H) 7.91 - 7.96 (m, 3 H) 8.19 (d, *J*=8.18 Hz, 2 H) 8.46 (s, 1 H) 10.48 (s, 1 H). (ESI) *m*/*z* 531 [(M + H)⁺]. HRMS (ESI) calculated for C₂₈H₂₂F₃N₆O₂+ [(M + H)⁺] 531.1751, found 531.1742.

### N-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-2-(4-trifluoromethyl-phenyl)-acetamide (cmpd 58)

¹H NMR (600 MHz, DMSO-*d₆*) δ ppm 2.89 (t, *J*=6.84 Hz, 2 H) 3.44 (td, *J*=6.84, 2.32 Hz, 2 H) 3.79 (s, 2 H) 3.84 (s, 3 H) 7.07 (s, 2 H) 7.16 (d, *J*=7.81 Hz, 2 H) 7.33 (t, *J*=7.99 Hz, 1 H) 7.42 (s, 1 H) 7.54 - 7.59 (m, 3 H) 7.67 - 7.72 (m, 2 H) 7.74 (s, 1 H) 8.43 (s, 1 H) 10.31 (s, 1 H).
(ESI) *m*/*z* 545 [(M + H)⁺]. HRMS (ESI) calculated for C₂₉H₂₄F₃N₆O₂⁺ [(M + H)⁺] 545.1907, found 545.1932.

### 2-{3-[2-Amino-4-(1-methyl-4-oxo-4,5,6,7-tetrahydro-1H-pyrrolo[3,2-c]pyridin-2-yl)-pyrimidin-5-ylethynyl]-phenoxy}-N-phenyl-acetamide hydrochloride (cmpd 59)

¹H NMR (600 MHz, DMSO-*d₆*) δ ppm 2.91 (t, *J*=6.77 Hz, 2 H) 3.86 (s, 3 H) 4.75 - 4.78 (m, 2 H) 7.01 - 7.14 (m, 5 H) 7.29 - 7.38 (m, 4 H) 7.63 (t, *J*=4.09 Hz, 4 H) 8.47 (s, 1 H) 10.13 (s, 1 H).
(ESI) *mlz* 493 [(M + H)⁺]. HRMS (ESI) calculated for C₂₈H₂₆CIN₆O₃⁺ [(M + H)⁺] 493.1983, found 493.1985.

### Cyclopropane-1,1-dicarboxylic acid {3-[2-amino-4-(1-methyl-4-oxo-4,5,6,7-tetrahydro-1H-pyrrolo[3,2-c] pyridin-2-yl)-pyrimidin-5-ylethynyl]-phenyl}-amide phenylamide (cmpd 60)

¹H NMR (600 MHz, DMSO-*d₆*) δ ppm 1.47 (d, *J*=3.54 Hz, 4 H) 2.89 (t, *J*=6.96 Hz, 2 H) 3.44 (td, *J*=6.74, 2.01 Hz, 2 H) 3.85 (s, 3 H) 7.00 - 7.08 (m, 3 H) 7.10 (br. s., 1 H) 7.17 (d, *J*=7.81 Hz, 1 H) 7.26 - 7.35 (m, 3 H) 7.43 (s, 1 H) 7.57 (d, *J*=8.54 Hz, 1 H) 7.61 (d, *J*=8.18 Hz, 2 H) 7.80 (s, 1 H) 8.43 (s, 1 H) 10.02 (s, 1 H) 10.08 (s, 1 H).
(ESI) *m*/*z* 546 [(M + H)⁺]. HRMS (ESI) calculated for C₃₁H₂₈N₇O₃⁺ [(M + H)⁺] 546.2248, found 546.2250.

### 2-[2-Amino-5-(3-phenethyloxy-phenylethynyl)-pyrimidin-4-yl]-1-methyl-1,5,6,7 -tetrahydro-pyrrolo[3,2-c]pyridin-4-one (cmpd 61)

¹H NMR (600 MHz, DMSO-*d₆*) δ ppm 2.88 (t, *J*=6.96 Hz, 2 H) 3.03 (t, *J*=6.59 Hz, 2 H) 3.41 - 3.47 (m, 2 H) 3.85 (s, 3 H) 4.20 - 4.26 (m, 2 H) 6.93 (dd, *J*=8.36, 2.38 Hz, 1 H) 7.01 (d, *J*=5.98 Hz, 2 H) 7.06 (s, 2 H) 7.10 (br. s., 1 H) 7.18 - 7.24 (m, 1 H) 7.26 - 7.37 (m, 6 H) 8.43 (s, 1 H).
(ESI) *mlz* 464 [(M + H)⁺]. HRMS (ESI) calculated for C₂₈H₂₆N₅O₂⁺ [(M + H)⁺] 464.2081, found 464.2076.

### N-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-2-phenyl-acetamide (cmpd 62)

¹H NMR (600 MHz, DMSO-*d₆*) δ ppm 2.89 (t, *J*=6.90 Hz, 2 H) 3.42 - 3.47 (m, 3 H) 3.64 - 3.66 (m, 2 H) 3.83 - 3.86 (m, 3 H) 7.06 (s, 2 H) 7.11 - 7.17 (m, 2 H) 7.21 - 7.28 (m, 1 H) 7.29 - 7.37 (m, 5 H) 7.42 (s, 1 H) 7.58 (dd, *J*=8.30, 1.10 Hz, 1 H) 7.75 (t, *J*=1.65 Hz, 1 H) 8.43 (s, 1 H) 10.23 (s, 1 H).
(ESI) *mlz* 477 [(M + H)⁺]. HRMS (ESI) calculated for C₂₈H₂₅N₆O₂⁺ [(M + H)⁺] 477.2034, found 477.2035.

### N-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-phenyl-propionamide (cmpd 63)

¹H NMR (600 MHz, DMSO-*d₆*) δ ppm 2.64 (t, *J*=7.69 Hz, 2 H) 2.83 - 2.95 (m, 5 H) 3.44 (td, *J*=6.82, 2.47 Hz, 2 H) 3.84 (s, 3 H) 7.07 (s, 2 H) 7.10 - 7.15 (m, 2 H) 7.16 - 7.20 (m, 1 H) 7.22 - 7.33 (m, 5 H) 7.41 (s, 1 H) 7.54 (d, *J*=8.24 Hz, 1 H) 7.74 (s, 1 H) 8.45 (s, 1 H) 9.98 (s, 1 H).
(ESI) *m*/*z* 491 [(M + H)⁺]. HRMS (ESI) calculated for C₂₉H₂₇N₆O₂⁺ [(M + H)⁺] 491.2190, found 491.2190.

### (1R,2R)-2-Phenyl-cyclopropanecarboxylicacid{3-[2-amino-4-(1-methyl-4-oxo-4,5,6,7-tetrahydro-1H-pyrrolo[3,2-c]pyridin-2-yl)-pyrimidin-5-ylethynyl]-phenyl}-amide (cmpd 64)

¹H NMR (600 MHz, DMSO-*d₆*) δ ppm 1.35 - 1.53 (m, 2 H) 2.04 - 2.09 (m, 1 H) 2.36 - 2.42 (m, 1 H) 2.89 (t, *J*=6.96 Hz, 2 H) 3.44 (td, *J*=6.78, 2.56 Hz, 2 H) 3.84 (s, 3 H) 7.07 (s, 2 H) 7.11 (br. s., 1 H) 7.14 (d, *J*=7.88 Hz, 1 H) 7.17 - 7.22 (m, 3 H) 7.27 - 7.35 (m, 3 H) 7.41 (s, 1 H) 7.53 - 7.58 (m, 1 H) 7.75 (s, 1 H) 8.44 (s, 1 H) 10.32 (s, 1 H).
(ESI) *m*/*z* 503 [(M + H)⁺]. HRMS (ESI) calculated for C₃₀H₂₇N₆O₂⁺ [(M + H)⁺] 503.2190, found 503.2188.

### 2-{2-Amino-5-[3-(3-phenyl-propoxy)-phenylethynyl]-pyrimidin-4-yl}-1-methyl-1,5,6,7-tetrahydro-pyrrolo[3,2-c]pyridin-4-one (cmpd 65)

¹H NMR (600 MHz, DMSO-*d₆*) δ ppm 1.98 - 2.05 (m, 2 H) 2.72 - 2.76 (m, 2 H) 2.89 (t, *J*=6.87 Hz, 2 H) 3.44 (td, *J*=6.78, 2.38 Hz, 2 H) 3.85 (s, 3 H) 4.01 (t, *J*=6.41 Hz, 2 H) 6.94 (dd, *J*=8.24, 2.38 Hz, 1 H) 7.00 (s, 1 H) 7.03 (d, *J*=7.51 Hz, 1 H) 7.07 (s, 2 H) 7.12 (br. s., 1 H) 7.16 - 7.20 (m, 1 H) 7.23 - 7.25 (m, 2 H) 7.27 - 7.30 (m, 3 H) 7.51 (s, 1 H) 8.43 (s, 1 H).
(ESI) *mlz* 478 [(M + H)⁺]. HRMS (ESI) calculated for C₂₉H₂₈N₅O₂⁺ [(M + H)⁺] 478.2238, found 478.2243.

### N-{3-[2-Amino-4-(1-methyl-4-oxo-4,5,6,7-tetrahydro-1H-pyrrolo[3,2-c]pyridin-2-yl)-pyrimidin-5-ylethynyl]-benzyl}-benzamide hydrochloride (cmpd 66)

¹H NMR (600 MHz, DMSO-*d₆*) δ ppm 2.90 (t, *J*=6.69 Hz, 2 H) 3.86 (s, 3 H) 4.49 (d, *J*=5.86 Hz, 2 H) 7.25 (br. s., 1 H) 7.31 - 7.41 (m, 3 H) 7.43 - 7.56 (m, 5 H) 7.62 (s, 1 H) 7.90 (d, *J*=7.69 Hz, 2 H) 8.47 (d, *J*=1.65 Hz, 1 H) 9.07 (t, *J*=5.68 Hz, 1 H).
(ESI) *mlz* 477 [(M + H)⁺]. HRMS (ESI) calculated for C₂₈H₂₆CIN₆O₂⁺ [(M + H)⁺] 477.1983, found 477.1985.

### 3-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-phenyl-propionamide (cmpd 67)

¹H NMR (600 MHz, DMSO-*d₆*) δ ppm 2.67 (t, *J*=7.69 Hz, 2 H) 2.89 - 2.96 (m, 4 H) 3.46 (td, *J*=6.87, 2.56 Hz, 2 H) 3.84 - 3.88 (m, 3 H) 6.97 - 7.04 (m, 2 H) 7.06 (s, 2 H) 7.21 (br. s., 1 H) 7.24 - 7.34 (m, 5 H) 7.42 (s, 1 H) 7.54 (s, 1 H) 7.56 (d, *J*=7.69 Hz, 2 H) 8.42 (s, 1 H) 9.95 (s, 1 H).
(ESI) *m*/*z* 491 [(M + H)⁺]. HRMS (ESI) calculated for C₂₉H₂₇N₆O₂⁺ [(M + H)⁺] 491.2190, found 491.2193.

### Cyclopropane-1,1-dicarboxylic acid {3-[2-amino-4-(1-methyl-4-oxo-4,5,6,7-tetrahydro-1H-pyrrolo[3,2-c] pyridin-2-yl)-pyrimidin-5-ylethynyl]-phenyl}-amide (4-trifluoromethyl-phenyl)-amide hydrochloride (cmpd 68)

¹H NMR (600 MHz, DMSO-*d₆*) δ ppm 1.44 - 1.51 (m, 4 H) 2.91 (t, *J*=6.96 Hz, 2 H) 3.86 (s, 3 H) 7.19 (d, *J*=7.69 Hz, 2 H) 7.34 (t, *J*=7.97 Hz, 1 H) 7.54 (s, 1 H) 7.56 - 7.59 (m, 1 H) 7.66 (d, *J*=8.61 Hz, 2 H) 7.83 (s, 1 H) 7.86 (d, *J*=8.42 Hz, 2 H) 8.48 (s, 1 H) 10.01 (s, 1 H) 10.48 (s, 1 H).
(ESI) *m*/*z* 614 [(M + H)⁺]. HRMS (ESI) calculated for C₃₂H₂₈CIF₃N₇O₃⁺ [(M + H)⁺] 614.2122, found 614.2136.

### 2-[2-Amino-5-(3-benzyloxymethyl-phenylethynyl)-pyrimidin-4-yl]-1-methyl-1,5,6,7-tetrahydro-pyrrolo[3,2-c] pyridin-4-one (cmpd 69)

¹H NMR (600 MHz, DMSO-*d₆*) δ ppm 2.89 (t, *J*=6.87 Hz, 2 H) 3.44 (td, *J*=6.87, 2.38 Hz, 2 H) 3.85 (s, 3 H) 4.54 (s, 2 H) 4.55 (s, 2 H) 7.07 (s, 2 H) 7.13 (br. s., 1 H) 7.29 (td, *J*=5.40, 3.11 Hz, 1 H) 7.33 - 7.38 (m, 5 H) 7.38 - 7.41 (m, 2 H) 7.47 (s, 1 H) 7.50 (s, 1 H) 8.44 (s, 1 H).
(ESI) *mlz* 464 [(M + H)⁺]. HRMS (ESI) calculated for C₂₈H₂₆N₅O₂⁺ [(M + H)⁺] 464.2081, found 464.2076.

### 2-{2-Amino-5-[3-(benzylamino-methyl)-phenylethynyl]-pyrimidin-4-yl}-1-methyl-1,5,6,7-tetrahydro-pyrrolo[3,2-c]pyridin-4-one dihydrochloride (cmpd 70)

¹H NMR (600 MHz, DMSO-*d₆*) δ ppm 2.91 (t, *J*=6.87 Hz, 2 H) 3.86 (s, 3 H) 4.20 (q, *J*=5.98 Hz, 4 H) 7.25 (br. s., 1 H) 7.41 - 7.46 (m, 2 H) 7.49 (quin, *J*=7.92 Hz, 2 H) 7.53 - 7.56 (m, 2 H) 7.58 (s, 1 H) 7.73 (s, 1 H) 8.46 (s, 1 H) 9.46 (br. s., 1 H).
(ESI) *mlz* 463 [(M + H)⁺]. HRMS (ESI) calculated for C₂₈H₂₉Cl₂N₆O⁺ [(M + H)⁺] 463.2241, found 463.2241.

### N-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-2-(3-trifluoromethyl-phenyl)-acetamide hydrochloride (cmpd 71)

¹H NMR (600 MHz, DMSO-*d₆*) δ ppm 3.80 (s, 2 H) 3.85 (s, 3 H) 7.17 (d, *J*=7.69 Hz, 2 H) 7.34 (t, *J*=7.88 Hz, 1 H) 7.50 (s, 1 H) 7.52 - 7.66 (m, 7 H) 7.70 (s, 1 H) 7.77 (s, 1 H) 7.77 - 7.77 (m, 1 H) 8.46 (s, 1 H) 10.35 (s, 1 H).
(ESI) *m*/*z* 545 [(M + H)⁺]. HRMS (ESI) calculated for C₂₉H₂₅CIF₃N₆O₂⁺ [(M + H)⁺] 545.1908, found 545.1922.

### N-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(4-trifluoromethyl-phenyl)-propionamide hydrochloride (cmpd 72)

¹H NMR (600 MHz, DMSO-*d₆*) δ ppm 2.69 (t, *J*=7.60 Hz, 2 H) 2.90 (t, *J*=6.96 Hz, 2 H) 3.01 (t, *J*=7.42 Hz, 2 H) 3.85 (s, 3 H) 7.16 (d, *J*=7.69 Hz, 2 H) 7.32 (t, *J*=7.88 Hz, 1 H) 7.48 - 7.50 (m, 2 H) 7.51 - 7.55 (m, 2 H) 7.65 (d, *J*=8.24 Hz, 2 H) 7.77 (s, 1 H) 8.48 (s, 1 H) 10.06 (s, 1 H).
(ESI) *m*/*z* 559 [(M + H)⁺]. HRMS (ESI) calculated for C₃₀H₂₇CIF₃N₆O+ [(M + H)⁺] 559.2064, found 559.2080.

### N-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-(3-trifluoromethyl-phenyl)-propionamide hydrochloride (cmpd 73)

¹H NMR (600 MHz, DMSO-*d₆*) δ ppm 2.69 (t, *J*=7.60 Hz, 2 H) 2.90 (t, *J*=6.87 Hz, 2 H) 3.01 (t, *J*=7.69 Hz, 2 H) 3.85 (s, 3 H) 7.16 (d, *J*=7.88 Hz, 1 H) 7.18 (br. s., 1 H) 7.32 (t, *J*=7.97 Hz, 1 H) 7.50 - 7.59 (m, 5 H) 7.62 (s, 1 H) 7.75 (s, 1 H) 8.48 (s, 1 H) 10.04 (s, 1 H).
(ESI) *m*/*z* 559 [(M + H)⁺]. HRMS (ESI) calculated for C₃₀H₂₇CIF₃N₆O₂⁺ [(M + H)⁺] 559.2064, found 559.2079.

### 3-[2-Amino-4-(1-methyl-4-oxo-4,5,6,7-tetrahydro-1H-pyrrolo[3,2-c]pyridin-2-yl)-pyrimidin-5-ylethynyl]-N-(3-trifluoromethyl-benzyl)-benzamide hydrochloride (cmpd 74)

¹H NMR (600 MHz, DMSO-*d₆*) δ ppm 2.87 - 2.94 (m, 2 H) 3.86 (s, 3 H) 4.58 (d, *J*=5.86 Hz, 2 H) 7.27 (br. s., 2 H) 7.51 - 7.67 (m, 7 H) 7.69 (s, 1 H) 7.88 (d, *J*=7.88 Hz, 1 H) 7.97 (s, 1 H) 8.48 (s, 1 H) 9.22 (t, *J*=5.95 Hz, 1 H).
(ESI) *m*/*z* 545 [(M + H)⁺]. HRMS (ESI) calculated for C₂₉H₂₅CIF₃N₆O₂⁺ [(M + H)⁺] 545.1908, found 545.1925.

### 3-[2-Amino-4-(1-methyl-4-oxo-4,5,6,7-tetrahydro-1H-pyrrolo[3,2-c]pyridin-2-yl)-pyrimidin-5-ylethynyl]-N-[4-(4-ethyl-piperazin-1-ylmethyl)-3-trifluoromethyl-phenyl]-benzamide (cmpd 48)

¹H NMR (600 MHz, DMSO-*d₆*) δ ppm 1.00 (t, *J*=6.96 Hz, 3 H) 2.22 - 2.54 (m, 10 H) 2.89 (t, *J*=6.87 Hz, 2 H) 3.45 (td, *J*=6.82, 2.47 Hz, 2 H) 3.58 (s, 2 H) 3.86 (s, 3 H) 7.06 - 7.16 (m, 3 H) 7.51 (s, 1 H) 7.59 (t, *J*=7.78 Hz, 1 H) 7.69 (d, *J*=7.51 Hz, 1 H) 7.72 (d, *J*=8.42 Hz, 1 H) 7.95 (d, *J*=7.88 Hz, 1 H) 8.03 - 8.07 (m, 2 H) 8.22 (d, *J*=2.01 Hz, 1 H) 8.48 (s, 1 H) 10.55 (s, 1 H).
(ESI) *m*/*z* 657 [(M + H)⁺]. HRMS (ESI) calculated for C₃₅H₃₆F₃N₈O₂⁺ [(M + H)⁺] 657.2908, found 657.2914.

### 3-[2-Amino-4-(1-methyl-4-oxo-4,5,6,7-tetrahydro-1H-pyrrolo[3,2-c]pyridin-2-yl)-pyrimidin-5-ylethynyl]-N-[4-(4-ethyl-piperazin-1-ylmethyl)-3-trifluoromethyl-phenyl]-4-methyl-benzamide (cmpd 50)

¹H NMR (600 MHz, DMSO-*d₆*) δ ppm 0.99 (t, *J*=7.14 Hz, 3 H) 2.19 - 2.48 (m, 8 H) 2.89 (t, *J*=6.90 Hz, 2 H) 3.44 (td, *J*=6.87, 2.50 Hz, 2 H) 3.57 (s, 2 H) 3.86 (s, 3 H) 7.05 - 7.14 (m, 3 H) 7.47 (d, *J*=8.18 Hz, 1 H) 7.51 (s, 1 H) 7.71 (d, *J*=8.54 Hz, 1 H) 7.86 (dd, *J*=7.93, 1.95 Hz, 1 H) 8.01 - 8.07 (m, 2 H) 8.21 (d, *J*=2.20 Hz, 1 H) 8.50 (s, 1 H) 10.45 - 10.48 (m, 1 H).
(ESI) *m*/*z* 671 [(M + H)⁺]. HRMS (ESI) calculated for C₃₆H₃₈F₃N₈O₂⁺ [(M + H)⁺] 671.3065, found 671.3069.

### 5-[2-Amino-4-(1-methyl-4-oxo-4,5,6,7-tetrahydro-1H-pyrrolo[3,2-c]pyridin-2-yl)-pyrimidin-5-ylethynyl]-thiophene-2-carboxylic acid [4-(4-ethyl-piperazin-1-ylmethyl)-3-trifluoromethyl-phenyl]-amide (cmpd 51)

¹H NMR (600 MHz, DMSO-*d₆*) δ ppm 0.98 (t, *J*=7.23 Hz, 3 H) 2.31 (q, *J*=7.14 Hz, 2 H) 2.28 - 2.33 (m, 2 H) 2.39 (dd, *J*=3.66, 1.83 Hz, 6 H) 2.33 - 2.48 (m, 6 H) 2.89 (t, *J*=6.87 Hz, 2 H) 2.87 - 2.91 (m, 2 H) 3.45 (td, *J*=6.87, 2.56 Hz, 2 H) 3.56 (s, 2 H) 3.85 (s, 3 H) 7.11 - 7.14 (m, 1 H) 7.19 (s, 2 H) 7.36 (s, 1 H) 7.38 (d, *J*=4.03 Hz, 1 H) 7.71 (d, *J*=8.61 Hz, 1 H) 7.97 (dd, *J*=8.61, 2.01 Hz, 1 H) 7.99 (d, *J*=4.03 Hz, 1 H) 8.12 (d, *J*=2.01 Hz, 1 H) 8.47 (s, 1 H) 10.56 (s, 1 H).
(ESI) *m*/*z* 663 [(M + H)⁺]. HRMS (ESI) calculated for C₃₃H₃₄F₃N₈O₂S⁺ [(M + H)⁺] 663.2472, found 663.2473.

### 2-[2-Amino-4-(1-methyl-4-oxo-4,5,6,7-tetrahydro-1H-pyrrolo[3,2-c]pyridin-2-yl)-pyrimidin-5-ylethynyl]-thiazole-5-carboxylic acid [4-(4-ethyl-piperazin-1-ylmethyl)-3-trifluoromethyl-phenyl]-amide (cmpd 52)

¹H NMR (600 MHz, DMSO-*d₆*) δ ppm 1.00 (br. s., 3 H) 2.24 - 2.47 (m, 10 H) 2.90 (t, *J*=6.78 Hz, 2 H) 3.45 (td, *J*=6.87, 2.38 Hz, 2 H) 3.58 (br. s., 2 H) 3.85 (s, 3 H) 7.14 (br. s., 1 H) 7.29 (s, 1 H) 7.38 (s, 2 H) 7.74 (d, *J*=8.61 Hz, 1 H) 7.97 (dd, *J*=8.52, 1.01 Hz, 1 H) 8.11 (d, *J*=2.20 Hz, 1 H) 8.57 (s, 1 H) 8.66 (s, 1 H) 10.75 (s, 1 H).
(ESI) *m*/*z* 664 [(M + H)⁺]. HRMS (ESI) calculated for C₃₂H₃₃F₃N₉O₂S⁺ [(M + H)⁺] 664.2425, found 664.2425.

### 5-[2-Amino-4-(1-methyl-4-oxo-4,5,6,7-tetrahydro-1H-pyrrolo[3,2-c]pyridin-2-yl)-pyrimidin-5-ylethynyl]-N-[4-(4-ethyl-piperazin-1-ylmethyl)-3-trifluoromethyl-phenyl]-nicotinamide (cmpd 53)

¹H NMR (600 MHz, DMSO-*d₆*) δ ppm 1.00 (br. s., 3 H) 2.00 - 2.48 (m, 9 H) 2.90 (t, *J*=6.87 Hz, 2 H) 3.45 (td, *J*=6.87, 2.38 Hz, 2 H) 3.58 (br. s., 2 H) 3.86 (s, 3 H) 7.12 - 7.24 (m, 3 H) 7.49 - 7.52 (m, 1 H) 7.74 (d, *J*=8.61 Hz, 1 H) 8.03 (d, *J*=8.43 Hz, 1 H) 8.21 (d, *J*=2.01 Hz, 1 H) 8.37 (t, *J*=2.11 Hz, 1 H) 8.51 (s, 1 H) 8.82 (d, *J*=2.01 Hz, 1 H) 9.05 (d, *J*=2.20 Hz, 1 H) 10.71 (s, 1 H).
(ESI) *m*/*z* 658 [(M + H)⁺]. HRMS (ESI) calculated for C₃₄H₃₅F₃N₉O₂⁺ [(M + H)⁺] 658.2861, found 658.2861.

### 3-[2-Amino-4-(1-methyl-4-oxo-4,5,6,7-tetrahydro-1H-pyrrolo[3,2-c]pyridin-2-yl)-pyrimidin-5-ylethynyl]-N-[4-(4-ethyl-piperazin-1-ylmethyl)-3-trifluoromethyl-phenyl]-5-fluoro-benzamide(cmpd 54)

¹H NMR (600 MHz, DMSO-*d₆*) δ ppm 1.02 (br. s., 2 H) 2.21 - 2.48 (m, 7 H) 2.87 - 2.92 (m, 2 H) 3.43 - 3.47 (m, 3 H) 3.59 (br. s., 2 H) 3.86 (s, 3 H) 7.15 (br. s., 1 H) 7.18 (s, 2 H) 7.49 (s, 1 H) 7.53 (dd, *J*=8.97, 1.10 Hz, 1 H) 7.73 (d, *J*=8.43 Hz, 1 H) 7.76 - 7.79 (m, 1 H) 7.90 (s, 1 H) 8.05 (d, *J*=8.97 Hz, 1 H) 8.20 (d, *J*=2.01 Hz, 1 H) 8.49 (s, 1 H) 10.61 (s, 1 H).
(ESI) *m*/*z* 675 [(M + H)⁺]. HRMS (ESI) calculated for C₃₅H₃₅F₄N₈O₂⁺ [(M + H)⁺] 675.2814, found 675.2832.

### 3-[2-Amino-4-(1-methyl-4-oxo-4,5,6,7-tetrahydro-1H-pyrrolo[3,2-c]pyridin-2-yl)-pyrimidin-5-ylethynyl]-N-[4-(4-ethyl-piperazin-1-ylmethyl)-3-trifluoromethyl-phenyl]-2-fluoro-benzamide(cmpd 55)

¹H NMR (600 MHz, DMSO-*d₆*) δ ppm 0.98 (t, *J*=7.14 Hz, 3 H) 2.31 (q, *J*=7.20 Hz, 3 H) 2.33 - 2.48 (m, 5 H) 2.89 (t, *J*=6.96 Hz, 2 H) 3.44 (td, *J*=6.87, 2.38 Hz, 2 H) 3.57 (s, 2 H) 3.85 (s, 3 H) 7.11 (br. s., 1 H) 7.17 (s, 2 H) 7.37 (t, *J*=7.78 Hz, 1 H) 7.46 (s, 1 H) 7.63 - 7.76 (m, 3 H) 7.93 (d, *J*=8.42 Hz, 1 H) 8.17 (d, *J*=1.65 Hz, 1 H) 8.46 (s, 1 H) 10.75 (s, 1 H).
(ESI) *m*/*z* 675 [(M + H)⁺]. HRMS (ESI) calculated for C₃₅H₃₅F₄N₈O₂⁺ [(M + H)⁺] 675.2814, found 675.2804.

### 3-[2-Amino-4-(1-methyl-4-oxo-4,5,6,7-tetrahydro-1H-pyrrolo[3,2-c]pyridin-2-yl)-pyrimidin-5-ylethynyl]-N-cyclopropyl-benzamide (cmpd 56)

¹H NMR (600 MHz, DMSO-*d₆*) δ ppm 0.58 - 0.63 (m, 2 H) 0.67 - 0.72 (m, 2 H) 2.83 - 2.87 (m, 1 H) 2.89 (t, *J*=6.87 Hz, 2 H) 3.45 (td, *J*=6.87, 2.38 Hz, 2 H) 3.86 (s, 3 H) 7.08 - 7.12 (m, 2 H) 7.14 - 7.17 (m, 1 H) 7.47 - 7.50 (m, 1 H) 7.50 (s, 1 H) 7.59 (d, *J*=7.88 Hz, 1 H) 7.80 (d, *J*=7.88 Hz, 1 H) 7.87 (s, 1 H) 8.45 (s, 1 H) 8.46 (d, *J*=4.40 Hz, 1 H).
(ESI) *mlz* 427 [(M + H)⁺]. HRMS (ESI) calculated for C₂₄H₂₃N₆O₂⁺ [(M + H)⁺] 427.1877, found 427.1880.

### Example 7

### N-[4-(4-lsopropyl-piperazin-1-ylmethyl)-3-trifluoromethyl-phenyl]-2-{3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-acetamide (cmpd 176)

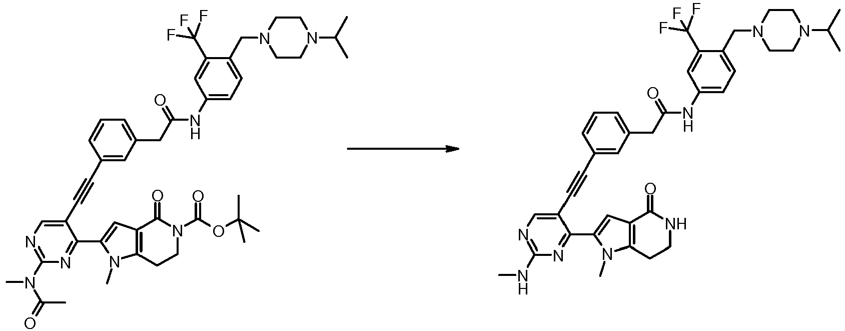

To a solution of 2-[2-(acetyl-methyl-amino)-5-(3-{[4-(4-isopropyl-piperazin-1-ylmethyl)-3-trifluoromethyl-phenylcarbamoyl]-methyl}-phenylethynyl)-pyrimidin-4-yl]-1-methyl-4-oxo-1,4,6,7-tetrahydro-pyrrolo[3,2-c]pyridine-5-carboxylic acid tert-butyl ester (0.49 g, 0.58 mmol) in DCM (15 mL), 4 M HCl in dioxane (0.73 mL, 2.90 mmol) was added. The resulting suspension was stirred at r.t. for 1 h, then the solvent was evaporated under vacuum. The solid was dissolved in MeOH (15 mL), K₂CO₃ (0.32 g, 2.32 mmol) was added and the mixture was stirred at reflux for 3 h. After removal of the solvent under vacuum the crude was purified by flash column chromatography (DCM/MeOH/NH₃ 90/10/0.5) to give the title compound (0.32 g, 78%) as white solid.
¹H NMR (600 MHz, DMSO-*d₆*) δ ppm 0.84 - 1.09 (m, 6 H) 2.24 - 2.48 (m, 6 H) 2.88 (d, *J*=4.76 Hz, 3 H) 2.90 (t, *J*=6.87 Hz, 2 H) 3.46 (td, *J*=6.73, 2.11 Hz, 2 H) 3.52 (br. s., 2 H) 3.67 (s, 2 H) 3.82 - 3.94 (m, 3 H) 7.24 (br. s., 1 H) 7.31 - 7.39 (m, 3 H) 7.50 - 7.62 (m, 3 H) 7.65 (d, *J*=8.43 Hz, 1 H) 7.78 (d, *J*=8.24 Hz, 1 H) 8.04 (d, *J*=2.01 Hz, 1 H) 8.39 - 8.55 (m, 1 H) 10.48 (s, 1 H).
(ESI) *m*/*z* 699 [(M + H)⁺]. HRMS (ESI) calculated for C₃₈H₄₂F₃N₈O₂⁺ [(M + H)⁺] 699.3378, found 699.3375.

According to this same methodology, but employing suitable substituted derivatives, the following compounds were prepared:

### 6-[2-Methylamino-4-(1-methyl-4-oxo-4,5,6,7-tetrahydro-1H-pyrrolo[3,2-c]pyridin-2-yl)-pyrimidin-5-ylethynyl]-2,3-dihydro-indole-1-carboxylic acid [4-(4-ethyl-piperazin-1-ylmethyl)-3-trifluoromethyl-phenyl]-amide (cmpd 46)

¹H NMR (600 MHz, DMSO-d₆) δ ppm 1.01 (t, J=6.69 Hz, 3 H) 2.23 - 2.46 (m, 10 H) 2.82 - 2.95 (m, 5 H) 3.18 - 3.25 (m, 2 H) 3.44 (td, J=6.78, 2.38 Hz, 2 H) 3.55 (s, 2 H) 3.89 (br. s., 3 H) 4.16 (t, J=8.70 Hz, 2 H) 7.06 (dd, J=7.60, 1.19 Hz, 1 H) 7.09 (br. s., 1 H) 7.23 (d, J=7.51 Hz, 1 H) 7.49 (s, 2 H) 7.59 - 7.68 (m, 1 H) 7.84 (dd, J=8.42, 1.83 Hz, 1 H) 7.95 (d, J=0.55 Hz, 1 H) 7.99 (d, J=2.01 Hz, 1 H) 8.36 - 8.58 (m, 1 H) 8.84 (s, 1 H).
(ESI) *m*/*z* 712 [(M + H)⁺]. HRMS (ESI) calculated for C₃₈H₄₁F₃N₉O₂+ [(M + H)⁺] 712.3330, found 712.3332.

### 5-[2-Methylamino-4-(1-methyl-4-oxo-4,5,6,7-tetrahydro-1H-pyrrolo[3,2-c]pyridin-2-yl)-pyrimidin-5-ylethynyl]-2,3-dihydro-indole-1-carboxylic acid [4-(4-ethyl-piperazin-1-ylmethyl)-3-trifluoromethyl-phenyl]-amide (cmpd 47)

¹H NMR (401 MHz, DMSO-*d₆*) δ ppm 0.98 (t, *J*=7.14 Hz, 3 H) 2.30 (q, *J*=7.12 Hz, 2 H) 2.38 (br. s., 8 H) 2.85 - 2.93 (m, 5 H) 3.14 - 3.24 (m, 2 H) 3.45 (td, *J*=6.71, 1.95 Hz, 2 H) 3.54 (s, 2 H) 3.88 (br. s., 3 H) 4.17 (t, *J*=8.61 Hz, 2 H) 7.12 (br. s., 1 H) 7.28 (d, *J*=8.91 Hz, 1 H) 7.33 (s, 1 H) 7.43 - 7.49 (m, 1 H) 7.56 (br. s., 1 H) 7.63 (d, *J*=8.79 Hz, 1 H) 7.81 - 7.90 (m, 2 H) 7.98 (d, *J*=1.95 Hz, 1 H) 8.42 (br. s., 1 H) 8.86 (s, 1 H).
(ESI) *m*/*z* 712 [(M + H)⁺]. HRMS (ESI) calculated for C₃₈H₄₁F₃N₉O₂+ [(M + H)⁺] 712.3330, found 712.3331.

### N-[4-(4-Ethyl-piperazin-1-ylmethyl)-3-trifluoromethyl-phenyl]-2-{3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-acetamide (cmpd 78)

¹H NMR (600 MHz, DMSO-*d₆*) δ ppm 0.97 (t, *J*=7.20 Hz, 3 H) 1.09 (t, *J*=7.02 Hz, 2 H) 2.29 (q, *J*=7.12 Hz, 4 H) 2.36 (br. s., 8 H) 2.81 - 2.96 (m, 8 H) 3.47 (td, *J*=6.84, 2.44 Hz, 3 H) 3.52 (s, 3 H) 3.67 (s, 3 H) 3.89 (d, *J*=6.35 Hz, 3 H) 7.22 (s, 1 H) 7.30 - 7.40 (m, 3 H) 7.53 (s, 1 H) 7.59 (br. s., 1 H) 7.65 (d, *J*=8.54 Hz, 1 H) 7.78 (dd, *J*=8.48, 2.01 Hz, 1 H) 8.04 (d, *J*=2.20 Hz, 1 H) 8.32 - 8.63 (m, 1 H) 10.46 (s, 1 H).
(ESI) *m*/*z* 685 [(M + H)⁺]. HRMS (ESI) calculated for C₃₇H₄₀F₃N₈O₂⁺ [(M + H)⁺] 685.3221, found 685.3223.

### 2-{3-[2-Methylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide (cmpd 174)

¹H NMR (600 MHz, DMSO-*d₆*) δ ppm 2.14 (s, 2 H) 2.22 - 2.43 (m, 8 H) 2.79 - 2.97 (m, 5 H) 3.43 - 3.49 (m, 2 H) 3.52 (s, 2 H) 3.67 (s, 2 H) 3.79 - 3.99 (m, 3 H) 7.22 (br. s., 1 H) 7.30 - 7.39 (m, 3 H) 7.53 (s, 2 H) 7.59 (br. s., 1 H) 7.64 (d, *J*=8.54 Hz, 1 H) 7.77 (dd, *J*=8.61, 1.77 Hz, 1 H) 8.04 (d, *J*=1.95 Hz, 1 H) 8.44 (br. s., 1 H) 10.47 (s, 1 H).
(ESI) *m*/*z* 671 [(M + H)⁺]. HRMS (ESI) calculated for C₃₆H₃₈F₃N₈O₂⁺ [(M + H)⁺] 671.3065, found 671.3066.

### N-[4-(4-Cyclopropyl-piperazin-1-ylmethyl)-3-trifluoromethyl-phenyl]-2-{3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-acetamide (cmpd 175)

¹H NMR (600 MHz, DMSO-*d₆*) δ ppm 0.26 (d, *J*=2.01 Hz, 2 H) 0.36 - 0.40 (m, 2 H) 1.55 - 1.61 (m, 1 H) 2.31 (br. s., 3 H) 2.88 (d, *J*=4.76 Hz, 3 H) 2.89 - 2.92 (m, 2 H) 3.47 (td, *J*=6.78, 2.38 Hz, 2 H) 3.50 (s, 2 H) 3.66 - 3.69 (m, 2 H) 3.81 - 3.94 (m, 3 H) 7.23 (br. s., 1 H) 7.31 - 7.39 (m, 3 H) 7.51 - 7.62 (m, 3 H) 7.65 (d, *J*=8.61 Hz, 1 H) 7.76 - 7.80 (m, 1 H) 8.04 (d, *J*=1.83 Hz, 1 H) 8.44 (br. s., 1 H) 10.47 (s, 1 H).
(ESI) *m*/*z* 697 [(M + H)⁺]. HRMS (ESI) calculated for C₃₈H₄₀F₃N₈O₂⁺ [(M + H)⁺] 697.3221, found 697.3244.

### 2-{3-[2-Methylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-propyl-piperazin-1-ylmethyl)-3-trifluoromethyl-phenyl]-acetamide (cmpd 179)

¹H NMR (600 MHz, DMSO-*d₆*) δ ppm 0.83 (t, *J*=7.33 Hz, 3 H) 1.42 (m, *J*=5.13 Hz, 2 H) 2.12 - 2.46 (m, 9 H) 2.83 - 2.93 (m, 5 H) 3.46 (td, *J*=6.55, 1.92 Hz, 1 H) 3.52 (br. s., 2 H) 3.67 (s, 2 H) 3.76 - 3.99 (m, 3 H) 7.22 (br. s., 1 H) 7.31 - 7.38 (m, 3 H) 7.50 - 7.61 (m, 3 H) 7.64 (d, *J*=8.61 Hz, 1 H) 7.78 (d, *J*=8.06 Hz, 1 H) 8.04 (s, 1 H) 8.36 - 8.61 (m, 1 H) 10.47 (s, 1 H).
(ESI) *m*/*z* 699 [(M + H)⁺]. HRMS (ESI) calculated for C₃₈H₄₂F₃N₈O₂⁺ [(M + H)⁺] 699.3378, found 699.3383.

### 2-{4-Fluoro-3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide (cmpd 190)

¹H NMR (600 MHz, DMSO-*d₆*) δ ppm 2.15 (s, 3 H) 2.21 - 2.46 (m, 8 H) 2.88 - 2.89 (m, 3 H) 2.90 - 2.92 (m, 2 H) 3.42 - 3.48 (m, 2 H) 3.52 (s, 2 H) 3.67 (s, 2 H) 3.79 - 3.93 (m, 3 H) 7.20 (br. s., 1 H) 7.26 (t, *J*=8.97 Hz, 1 H) 7.37 (ddd, *J*=8.15, 5.40, 2.20 Hz, 1 H) 7.53 - 7.62 (m, 3 H) 7.64 (d, *J*=8.43 Hz, 1 H) 7.75 - 7.78 (m, 1 H) 8.03 (d, *J*=1.83 Hz, 1 H) 8.40 - 8.55 (m, 1 H) 10.45 (s, 1 H).
(ESI) *m*/*z* 689 [(M + H)⁺]. HRMS (ESI) calculated for C₃₆H₃₇F₄N₈0₂⁺ [(M + H)⁺] 689.2970, found 689.2973.

### 2-{3-[2-Ethylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide (cmpd 193)

¹H NMR (600 MHz, DMSO-*d₆*) δ ppm 1.16 (t, *J*=7.14 Hz, 3 H) 2.15 (s, 3 H) 2.21 - 2.44 (m, 7 H) 2.90 (t, *J*=6.78 Hz, 2 H) 3.34 - 3.39 (m, 3 H) 3.46 (td, *J*=6.82, 2.29 Hz, 2 H) 3.52 (s, 2 H) 3.67 (s, 2 H) 3.87 (br. s., 3 H) 7.22 (br. s., 1 H) 7.28 - 7.40 (m, 3 H) 7.52 (s, 1 H) 7.59 (br. s., 2 H) 7.64 (d, *J*=8.43 Hz, 1 H) 7.77 (dd, *J*=8.61, 1.47 Hz, 1 H) 8.04 (d, *J*=1.83 Hz, 1 H) 8.44 (br. s., 1 H) 10.46 (s, 1 H).
(ESI) *m*/*z* 685 [(M + H)⁺]. HRMS (ESI) calculated for C₃₇H₄₀F₃N₈O₂⁺ [(M + H)⁺] 685.3221, found 685.3221.

### 2-{4-Fluoro-3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-isopropyl-piperazin-1-ylmethyl)-3-trifluoromethyl-phenyl]-acetamide (cmpd 194)

¹H NMR (600 MHz, DMSO-*d₆*) δ ppm 0.95 (d, *J*=5.86 Hz, 6 H) 2.20 - 2.48 (m, 7 H) 2.61 (br. s., 1 H) 2.83 - 2.94 (m, 5 H) 3.42 - 3.48 (m, 2 H) 3.51 (s, 2 H) 3.67 (s, 2 H) 3.77 - 3.94 (m, 3 H) 7.20 (br. s., 1 H) 7.26 (t, *J*=8.97 Hz, 1 H) 7.37 (td, *J*=5.49, 2.38 Hz, 1 H) 7.51 - 7.63 (m, 3 H) 7.65 (d, *J*=8.42 Hz, 1 H) 7.76 (d, J=8.61 Hz, 1 H) 8.03 (d, *J*=1.83 Hz, 1 H) 8.44 (br. s., 1 H) 10.45 (s, 1 H).
(ESI) *m*/*z* 717 [(M + H)⁺]. HRMS (ESI) calculated for C₃₈H₄₁F₄N₈O₂⁺ [(M + H)⁺] 717.3283, found 717.3288.

### N-[4-(4-Ethyl-piperazin-1-ylmethyl)-3-trifluoromethyl-phenyl]-2-{5-[2-methylamino-4-(1-methyl-4-oxo-4,5,6,7-tetrahydro-1H-pyrrolo[3,2-c]pyridin-2-yl)-pyrimidin-5-ylethynyl]-thiophen-2-yl}-acetamide (cmpd 196)

¹H NMR (600 MHz, DMSO-*d₆*) δ ppm 2.16 (s, 3 H) 2.38 (br. s., 7 H) 2.84 - 2.92 (m, 6 H) 3.41 - 3.47 (m, 2 H) 3.53 (s, 2 H) 3.81 - 3.90 (m, 3 H) 3.92 (s, 2 H) 6.97 (d, *J*=3.48 Hz, 1 H) 7.09 (br. s., 1 H) 7.17 (d, *J*=3.66 Hz, 1 H) 7.34 - 7.45 (m, 1 H) 7.55 (br. s., 1 H) 7.66 (d, *J*=8.42 Hz, 1 H) 7.77 (d, *J*=8.43 Hz, 1 H) 8.02 (d, *J*=1.83 Hz, 1 H) 8.41 (br. s., 1 H) 10.52 (s, 1 H).
(ESI) *m*/*z* 677 [(M + H)⁺]. HRMS (ESI) calculated for C₃₄H₃₅F₃N₈O₂S⁺ [(M + H)⁺] 677.2629, found 677.2623.

### N-[4-(4-Cyclopropylmethyl-piperazin-1-ylmethyl)-3-trifluoromethyl-phenyl]-2-{3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-acetamide (cmpd 197)

¹H NMR (600 MHz, DMSO-*d₆*) δ ppm 0.07 (br. s., 2 H) 0.45 (d, *J*=6.78 Hz, 2 H) 0.69 - 0.88 (m, 1 H) 2.82 - 2.94 (m, 5 H) 3.43 - 3.49 (m, 2 H) 3.53 (br. s., 2 H) 3.67 (s, 2 H) 3.90 (br. s., 3 H) 7.22 (br. s., 1 H) 7.29 - 7.39 (m, 3 H) 7.50 - 7.61 (m, 3 H) 7.65 (d, *J*=8.61 Hz, 1 H) 7.77 (d, *J*=7.69 Hz, 1 H) 8.04 (s, 1 H) 8.44 (br. s., 1 H) 10.47 (s, 1 H).
(ESI) *m*/*z* 711 [(M + H)⁺]. HRMS (ESI) calculated for C₃₉H₄₁F₃N₈O₂⁺ [(M + H)⁺] 711.3378, found 711.3376.

### N-[4-((S)-3-Dimethylamino-pyrrolidin-1-ylmethyl)-3-trifluoromethyl-phenyl]-2-{3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-acetamide (cmpd 198)

¹H NMR (600 MHz, DMSO-*d₆*) δ ppm 1.52 - 1.66 (m, 1 H) 1.84 (dd, *J*=13.28, 5.77 Hz, 1 H) 2.07 (s, 6 H) 2.27 - 2.35 (m, 1 H) 2.43 - 2.48 (m, 1 H) 2.52 - 2.57 (m, 2 H) 2.61 (t, *J*=8.06 Hz, 1 H) 2.73 (m, *J*=6.96 Hz, 1 H) 2.83 - 2.93 (m, 5 H) 3.46 (t, *J*=5.86 Hz, 3 H) 3.56 - 3.63 (m, 1 H) 3.65 (s, 1 H) 3.67 (s, 2 H) 3.77 - 3.98 (m, 3 H) 7.22 (br. s., 1 H) 7.28 - 7.41 (m, 3 H) 7.53 (s, 2 H) 7.59 (br. s., 1 H) 7.63 (d, *J*=8.24 Hz, 1 H) 7.77 (d, *J*=8.24 Hz, 1 H) 8.03 (s, 1 H) 8.44 (br. s., 1 H) 10.46 (s, 1 H).
(ESI) *m*/*z* 685 [(M + H)⁺]. HRMS (ESI) calculated for C₃₇H₄₀F₃N₈O₂⁺ [(M + H)⁺] 685.3221, found 685.3228.

### 2-{4-Fluoro-3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-propyl-piperazin-1-ylmethyl)-3-trifluoromethyl-phenyl]-acetamide (cmpd 199)

¹H NMR (600 MHz, DMSO-*d₆*) δ ppm 0.78 - 0.88 (m, 3 H) 1.40 (sxt, *J*=7.29 Hz, 2 H) 2.16 - 2.22 (m, 2 H) 2.26 - 2.46 (m, 7 H) 2.80 - 2.96 (m, 5 H) 3.45 (t, *J*=5.59 Hz, 2 H) 3.52 (s, 2 H) 3.67 (s, 2 H) 3.77 - 3.95 (m, 3 H) 7.20 (br. s., 1 H) 7.23 - 7.29 (m, 1 H) 7.36 (dt, *J*=5.54, 2.82 Hz, 1 H) 7.55 (br. s., 1 H) 7.57 (dd, *J*=6.78, 1.83 Hz, 1 H) 7.59 (br. s., 1 H) 7.64 (d, *J*=8.42 Hz, 1 H) 7.76 (dd, *J*=8.52, 1.19 Hz, 1 H) 8.03 (s, 1 H) 8.37 - 8.61 (m, 1 H) 10.46 (s, 1 H).
(ESI) *m*/*z* 717 [(M + H)⁺]. HRMS (ESI) calculated for C₃₈H₄₁F₄N₈O₂+ [(M + H)⁺] 717.3283, found 717.3284.

### 2-{3-[2-lsopropylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide (cmpd 202)

¹H NMR (600 MHz, DMSO-*d₆*) δ ppm 1.19 (d, *J*=6.41 Hz, 5 H) 2.15 (s, 3 H) 2.20 - 2.47 (m, 7 H) 2.90 (t, *J*=6.87 Hz, 2 H) 3.46 (td, *J*=6.87, 2.20 Hz, 2 H) 3.52 (s, 2 H) 3.67 (s, 2 H) 3.87 (s, 3 H) 4.11 (dd, *J*=14.29, 6.78 Hz, 1 H) 7.22 (br. s., 1 H) 7.31 - 7.40 (m, 3 H) 7.38 - 7.61 (m, 3 H) 7.64 (d, *J*=8.42 Hz, 1 H) 7.77 (dd, *J*=8.33, 1.56 Hz, 1 H) 8.04 (d, *J*=1.83 Hz, 1 H) 8.45 (br. s., 1 H) 10.47 (s, 1 H).
(ESI) *m*/*z* 699 [(M + H)⁺]. HRMS (ESI) calculated for C₃₈H₄₂F₃N₈O₂⁺ [(M + H)⁺] 699.3378, found 699.3372.

### N-[4-(4-Ethyl-piperazin-1-ylmethyl)-3-trifluoromethyl-phenyl]-2-{4-fluoro-3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-acetamide (cmpd 205)

¹H NMR (600 MHz, DMSO-*d₆*) δ ppm 1.01 (br. s., 3 H) 2.86 - 2.92 (m, 5 H) 3.43 - 3.48 (m, 2 H) 3.54 (br. s., 2 H) 3.67 (s, 2 H) 3.80 - 3.94 (m, 3 H) 7.20 (br. s., 1 H) 7.26 (t, *J*=8.97 Hz, 1 H) 7.33 - 7.39 (m, 1 H) 7.48 - 7.62 (m, 3 H) 7.65 (d, *J*=8.42 Hz, 1 H) 7.78 (d, *J*=7.51 Hz, 1 H) 8.03 (d, *J*=2.01 Hz, 1 H) 8.38 - 8.57 (m, 1 H) 10.47 (s, 1 H).
(ESI) *m*/*z* 703 [(M + H)⁺]. HRMS (ESI) calculated for C₃₇H₃₉F₄N₈O₂⁺ [(M + H)⁺] 703.3127, found 703.3138.

### 2-{3-[2-Ethylamino-4-(1-methyl-4-oxo-4,5,6,7-tetrahydro-1H-pyrrolo[3,2-c]pyridin-2-yl)-pyrimidin-5-ylethynyl]-4-fluoro-phenyl}-N-[4-(4-methyl-piperazin-1-ylmethyl)-3-trifluoromethyl-phenyl]-acetamide (cmpd 206)

¹H NMR (600 MHz, DMSO-*d₆*) δ ppm 1.16 (t, *J*=7.05 Hz, 3 H) 1.44 (s, 9 H) 2.14 (s, 3 H) 2.36 - 2.38 (m, 4 H) 3.03 (t, *J*=6.32 Hz, 2 H) 3.52 (s, 2 H) 3.71 (s, 2 H) 3.84 (s, 3 H) 3.98 - 4.07 (m, 4 H) 7.33 (t, *J*=8.97 Hz, 1 H) 7.43 - 7.48 (m, 1 H) 7.59 (dd, *J*=6.78, 2.20 Hz, 1 H) 7.64 (d, *J*=8.61 Hz, 1 H) 7.70 (s, 1 H) 7.76 (dd, *J*=8.52, 1.92 Hz, 1 H) 8.03 (d, *J*=1.83 Hz, 1 H) 8.92 (s, 1 H) 10.43 (s, 1 H).
(ESI) *m*/*z* 703 [(M + H)⁺]. HRMS (ESI) calculated for C₃₇H₃₉F₄N₈O₂⁺ [(M + H)⁺] 703.3127, found 703.3147.

### 4-Methyl-3-[2-methylamino-4-(1-methyl-4-oxo-4,5,6,7-tetrahydro-1H-pyrrolo[3,2-c]pyridin-2-yl)-pyrimidin-5-ylethynyl]-N-[4-(4-methyl-piperazin-1-ylmethyl)-3-trifluoromethyl-phenyl]-benzamide (cmpd 207)

¹H NMR (600 MHz, DMSO-*d₆*) δ ppm 1.40 - 1.44 (m, 9 H) 2.16 (s, 3 H) 2.26 - 2.42 (m, 7 H) 2.43 (s, 3 H) 2.54 (s, 3 H) 3.03 (t, *J*=6.29 Hz, 2 H) 3.42 (s, 3 H) 3.56 (s, 2 H) 3.86 (s, 3 H) 4.01 (t, *J*=6.29 Hz, 2 H) 7.53 (d, *J*=8.18 Hz, 1 H) 7.70 (d, *J*=8.67 Hz, 1 H) 7.75 (s, 1 H) 7.94 (dd, *J*=7.99, 1.89 Hz, 1 H) 8.04 (dd, *J*=8.42, 2.08 Hz, 1 H) 8.12 (d, *J*=1.95 Hz, 1 H) 8.20 (d, *J*=2.20 Hz, 1 H) 8.98 (s, 1 H) 10.52 (s, 1 H).
(ESI) *m*/*z* 671 [(M + H)⁺]. HRMS (ESI) calculated for C₃₆H₃₈F₃N₈O₂⁺ [(M + H)⁺] 671.3065, found 671.3061.

### Example 8

### N-[4-(4-Ethyl-piperazin-1-ylmethyl)-3-trifluoromethyl-phenyl]-2-{3-[6-methylamino-4-(1-methyl-4-oxo-4,5,6,7-tetrahydro-1H-pyrrolo[3,2-c]pyridin-2-yl)-pyridin-3-ylethynyl]-phenyl}-acetamide (cmpd 209)

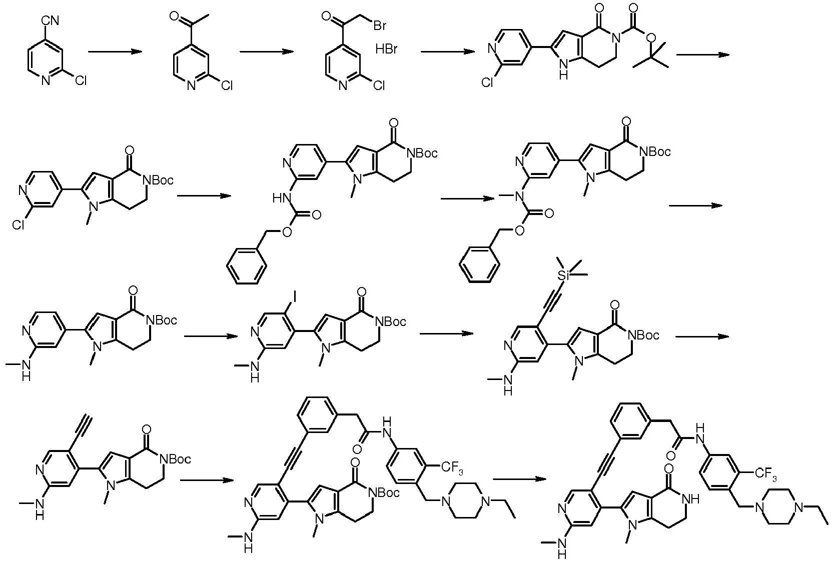

### Step 1. 1-(2-Chloro-pyridin-4-yl)-ethanone

To a solution of 2-chloro-isonicotinonitrile (5 g, 36.08 mmol) in dry Et₂O (50 mL), 1 M methylmagnesium bromide in BU₂O (72 mL, 72.16 mmol) was slowly added. The mixture was let under stirring overnight at room temperature, then the precipitate was filtered and washed con *i*-Pr₂O. The solid was suspended in a mixture of ice (50 g), water (37 mL) and HCl 6N (25 mL) and the product was extracted with EtOAc (4 x 12 mL). The organic layer was dried over Na₂SO₄ and evaporated to dryness. The crude yellow oil was treated with warm hexane (4 x 12 mL) and the extracts were let at 4 °C overnight. The product precipitated as white needles (2.66 g, 47%).
¹H NMR (600 MHz, DMSO-*d₆*) δ ppm 2.63 (s, 3 H) 7.81 (dd, J=5.04, 1.37 Hz, 1 H) 7.91 (dd, J=1.37, 0.64 Hz, 1 H) 8.63 (dd, J=5.04, 0.64 Hz, 1 H).

### Step 2. 2-Bromo-1-(2-chloro-pyridin-4-yl)-ethanone hydrobromide

To a solution of 1-(2-chloro-pyridin-4-yl)-ethanone (2.66 g, 17.09 mmol) and 48% HBr (2.9 mL) in AcOH (12 mL), a solution of Br₂ (0.875 mL, 17.09 mmol) in AcOH (2.5 mL) was added dropwise and the mixture was let under stirring overnight. The precipitate was filtered, washed with absolute ethanol and dried at 40 °C under vacuum affording the title compound as white solid (4.8 g, 53%).
¹H NMR (401 MHz, DMSO-*d*₆) δ ppm 4.81 (s, 2 H) 7.79 (dd, J=5.06, 1.40 Hz, 1 H) 7.89 (dd, J=1.34, 0.85 Hz, 1 H) 8.62 - 8.64 (m, 1 H).

### Step 3. 2-(2-Chloro-pyridin-4-yl)-4-oxo-1,4,6,7-tetrahydro-pyrrolo[3,2-c]pyridine-5-carboxylic acid tert-butyl ester

A mixture of 2-bromo-1-(2-chloro-pyridin-4-yl)-ethanone hydrobromide (1.528 g, 4.84 mmol), 2,4-dioxo-piperidine-1-carboxylic acid tert-butyl ester (2.58 g, 12.1 mmol) and ammonium acetate (2.49 g, 32.29 mmol) in absolute ethanol (42 mL) was let under stirring overnight. The product precipitated as a white solid that was filtered off and dried under vacuum (1.56, 93%).
¹H NMR (600 MHz, DMSO-d₆) δ ppm 1.46 (s, 9 H) 2.96 (t, J=6.32 Hz, 2 H) 3.98 (t, J=6.32 Hz, 2 H) 7.26 (s, 1 H) 7.67 (dd, J=5.31, 1.47 Hz, 1 H) 7.78 (d, J=0.92 Hz, 1 H) 8.33 (d, J=5.31 Hz, 1 H) 12.22 (br. s., 1 H).

### Step 4. 2-(2-Chloro-pyridin-4-yl)-1-methyl-4-oxo-1,4,6,7-tetrahydro-pyrrolo[3,2-c]pyridine-5-carboxylic acid tert-butyl ester

The mixture of 2-(2-chloro-pyridin-4-yl)-4-oxo-1,4,6,7-tetrahydro-pyrrolo[3,2-c]pyridine-5-carboxylic acid tert-butyl ester (1.56 g, 4.49 mmol), Cs₂CO₃ (2.19 g, 6.72 mmol) and Mel (0.363 mL, 5.83 mmol) in dry DMF (40 mL) was heated at 80 °C for 1 h. The suspension was then poured in ice and water and let under stirring for 30 min. The product was isolated by filtration as a white solid (1.46 g, 90%).
¹H NMR (500 MHz, DMSO-*d₆*) δ ppm 1.47 (s, 9 H) 2.97 (t, J=6.25 Hz, 2 H) 3.65 (s, 3 H) 3.98 (t, J=6.38 Hz, 2 H) 6.88 (s, 1 H) 7.55 (dd, J=5.22, 1.51 Hz, 1 H) 7.63 - 7.65 (m, 1 H) 8.42 (dd, J=5.22, 0.55 Hz, 1 H).

### Step 5. 2-(2-Benzyloxycarbonylamino-pyridin-4-yl)-1-methyl-4-oxo-1,4,6,7-tetrahydro-pyrrolo[3,2-c]pyridine-5-carboxylic acid tert-butyl ester

2-(2-Chloro-pyridin-4-yl)-1-methyl-4-oxo-1,4,6,7-tetrahydro-pyrrolo[3,2-c]pyridine-5-carboxylic acid tert-butyl ester (1.84 g, 5.07 mmol) and benzyl carbamate (3.83 g, 25.35 mmol) were charged in a Schlenk tube under argon and dissolved in dry dioxane (60 mL). Xantphos (440 mg, 0.76 mmol), Cs₂CO₃ (3.3 g, 10.14 mmol) and Pd(OAc)₂ (114 mg, 0.507 mmol) were added. The resulting mixture was degassed three times back filling with argon each time and then heated at 120 °C for 1.5 h. The solvent was removed under reduced pressure and the crude dissolved in DCM and washed with water and brine. The organic layer was dried over Na₂SO₄ and evaporated to dryness. The title compound was purified by column chromatography (Hexane/EtOAc 1/1) and isolated as white solid (1.41 g, 58%).
¹H NMR (401 MHz, DMSO-*d₆*) δ ppm 1.44 (s, 9 H) 2.96 (t, J=6.35 Hz, 2 H) 3.61 (s, 3 H) 3.98 (t, J=6.35 Hz, 2 H) 5.19 (s, 2 H) 6.68 (s, 1 H) 7.18 (dd, J=5.19, 1.65 Hz, 1 H) 7.26 - 7.48 (m, 8 H) 7.90 - 7.94 (m, 1 H) 8.29 (dd, J=5.31, 0.67 Hz, 1 H) 10.33 (s, 1 H).

### Step 6. 2-[2-(Benzyloxycarbonyl-methyl-amino)-pyridin-4-yl]-1-methyl-4-oxo-1,4,6,7-tetrahydro-pyrrolo[3,2-c]pyridine-5-carboxylic acid tert-butyl ester

2-(2-Benzyloxycarbonylamino-pyridin-4-yl)-1-methyl-4-oxo-1,4,6,7-tetrahydro-pyrrolo[3,2-c]pyridine-5-carboxylic acid tert-butyl ester (405 mg, 0.849 mmol) was dissolved in dry DMF, (17 mL) and treated with Cs₂CO₃ (684 mg, 2.1 mmol) and Mel (0.130 mL, 2.1 mmol). The mixture was let under stirring at room temperature for 2.5 h then it was diluted with EtOAc and washed with water and brine. The organic layer was dried over Na₂SO₄ and evaporated to dryness. The product was isolated as foaming solid (387 mg, 93%).
¹H NMR (401 MHz, DMSO-*d*₆) δ ppm 1.47 (s, 9 H) 2.95 (t, J=6.41 Hz, 2 H) 3.40 (s, 3 H) 3.54 (s, 3 H) 3.94 - 4.02 (m, 2 H) 5.21 (s, 2 H) 6.69 (s, 1 H) 7.29 (dd, J=5.19, 1.53 Hz, 1 H) 7.31 -7.44 (m, 6 H) 7.73 (d, J=0.73 Hz, 1 H) 8.42 (dd, J=5.25, 0.73 Hz, 1 H).

### Step 7. 1-Methyl-2-(2-methylamino-pyridin-4-yl)-4-oxo-1,4,6,7-tetrahydro-pyrrolo[3,2-c]pyridine-5-carboxylic acid tert-butyl ester

The suspension of 2-[2-(benzyloxycarbonyl-methyl-amino)-pyridin-4-yl]-1-methyl-4-oxo-1,4,6,7-tetrahydro-pyrrolo[3,2-c]pyridine-5-carboxylic acid tert-butyl ester (374 mg, 0.7632 mmol) with ammonium formate (333 mg, 4.892 mmol) and 5% Pd/C (50 mg) in ethanol was heated at reflux. The reaction was monitored by TLC (Hexane/EtOAc 1/1) and five subsequent additions of the reagents were required in order to reach total conversion of the starting material. The suspension was then filtered over a pad of celite that was repeatedly washed with ethanol. After solvent removal under reduced pressure, the crude was dissolved in DCM and washed with water and brine. The product was isolated as bright white solid (204 mg, 75%).
¹H NMR (401 MHz, DMSO-d₆) δ ppm 1.47 (s, 9 H) 2.80 (d, J=4.76 Hz, 3 H) 2.90 - 2.98 (m, 2 H) 3.57 (s, 3 H) 3.97 (t, J=6.35 Hz, 2 H) 6.49 (s, 1 H) 6.57 (s, 1 H) 6.59 (dd, J=5.31, 1.40 Hz, 1 H) 8.00 (d, J=5.25 Hz, 1 H).

According to this same methodology, but employing suitable substituted derivatives, the following intermediates were prepared:

### 2-(2-Amino-pyridin-4-yl)-1-methyl-4-oxo-1,4,6,7-tetrahydro-pyrrolo[3,2-c]pyridine-5-carboxylic acid tert-butyl ester

¹H NMR (401 MHz, DMSO-d₆) δ ppm 1.46 (s, 9 H) 2.94 (t, J=6.29 Hz, 2 H) 3.57 (s, 3 H) 3.92 - 4.01 (m, 2 H) 5.98 (s, 2 H) 6.51 (d, J=0.73 Hz, 1 H) 6.54 (s, 1 H) 6.58 (dd, J=5.37, 1.59 Hz, 1 H) 7.30 - 7.43 (m, 2 H) 7.93 (dd, J=5.25, 0.61 Hz, 1 H).

### Step 8. 2-(5-lodo-2-methylamino-pyridin-4-yl)-1-methyl-4-oxo-1,4,6,7-tetrahydro-pyrrolo[3,2-c]pyridine-5-carboxylic acid tert-butyl ester

To a solution of 1-methyl-2-(2-methylamino-pyridin-4-yl)-4-oxo-1,4,6,7-tetrahydro-pyrrolo[3,2-c]pyridine-5-carboxylic acid tert-butyl ester (156 mg, 0.437 mmol) in dry DMF (8 mL), silver acetate (84 mg, 0.505 mmol) and iodine (128 mg, 0.505 mmol) were added at 0 °C. The mixture was allowed to reach room temperature and let under stirring for 2 h. The suspension was filtered over a pad of celite that was exhaustively washed with EtOAC. The filtrate was concentrated under reduced pressure and the residue was treated with water. The yellow precipitate was filtered and dried under vacuum (130 mg, 61%).
¹H NMR (401 MHz, DMSO-d₆) δ ppm 1.47 (s, 9 H) 2.77 (d, J=3.42 Hz, 3 H) 2.93 (t, J=6.7 Hz, 2 H) 3.99 (t, J=6.29 Hz, 2 H) 6.35 (s, 1 H) 6.48 (s, 1 H) 6.87 (br. s., 1 H) 8.33 (d, J=0.61 Hz, 1 H).

According to this same methodology, but employing suitable substituted derivatives, the following intermediates were prepared:

### 2-(2-Amino-5-iodo-pyridin-4-yl)-1-methyl-4-oxo-1,4,6,7-tetrahydro-pyrrolo[3,2-c]pyridine-5-carboxylic acid tert-butyl ester

¹H NMR (401 MHz, DMSO-*d₆*) δ ppm 1.46 (s, 9 H) 2.93 (t, J=6.29 Hz, 2 H) 3.98 (t, J=6.35 Hz, 2 H) 6.37 (s, 1 H) 6.50 (s, 1 H) 8.26 (s, 1 H).

### Step 9. 1-Methyl-2-(2-methylamino-5-trimethylsilanylethynyl-pyridin-4-yl)-4-oxo-1,4,6,7-tetrahydro-pyrrolo[3,2-c]pyridine-5-carboxylic acid tert-butyl ester

As described for the synthesis of 2-(2-amino-5-trimethylsilanylethynyl-pyrimidin-4-yl)-1-methyl-4-oxo-1,4,6,7-tetrahydro-pyrrolo[3,2-c]pyridine-5-carboxylic acid tert-butyl ester, heating the reaction mixture at 50 °C for 1 h.
¹H NMR (401 MHz, DMSO-*d₆*) δ ppm 0.10 (s, 9 H) 1.46 (s, 9 H) 2.82 (d, J=4.76 Hz, 3 H) 2.92 (t, J=6.29 Hz, 2 H) 3.46 (s, 3 H) 3.97 (t, J=6.23 Hz, 2 H) 6.40 (s, 1 H) 6.54 (s, 1 H) 7.08 (d, J=4.88 Hz, 1 H) 8.17 (s, 1 H).

According to this same methodology, but employing suitable substituted derivatives, the following intermediates were prepared:

### 2-(2-Amino-5-trimethylsilanylethynyl-pyridin-4-yl)-1-methyl-4-oxo-1,4,6,7-tetrahydro-pyrrolo[3,2-c]pyridine-5-carboxylic acid tert-butyl ester

¹H NMR (401 MHz, DMSO-*d₆*) δ ppm 0.10 (s, 9 H) 1.46 (s, 9 H) 2.92 (t, J=6.41 Hz, 2 H) 3.46 (s, 3 H) 3.97 (t, J=6.2 Hz, 2 H) 6.40 (d, J=0.49 Hz, 1 H) 6.53 (br. s., 2 H) 6.55 (s, 1 H) 8.09 (s, 1 H).

### Step 10. 2-(5-Ethynyl-2-methylamino-pyridin-4-yl)-1-methyl-4-oxo-1,4,6,7-tetrahydro-pyrrolo[3,2-c]pyridine-5-carboxylic acid tert-butyl ester

As described for the sysnthesis of 2-(2-amino-5-ethynyl-pyrimidin-4-yl)-1-methyl-4-oxo-1,4,6,7-tetrahydro-pyrrolo[3,2-c]pyridine-5-carboxylic acid tert-butyl ester.
¹H NMR (401 MHz, DMSO-*d₆*) δ ppm 1.46 (s, 9 H) 2.81 (d, J=5 Hz, 3 H) 2.93 (t, J=6.35 Hz, 2 H) 3.45 (s, 3 H) 3.99 (t, J=6.22 Hz, 2 H) 3.98 (s, 1 H) 6.37 (s, 1 H) 6.53 (s, 1 H) 7.00 - 7.06 (m, 1 H) 8.21 (s, 1 H).

According to this same methodology, but employing suitable substituted derivatives, the following intermediates were prepared:

### 2-(2-Amino-5-ethynyl-pyridin-4-yl)-1-methyl-4-oxo-1,4,6,7-tetrahydro-pyrrolo[3,2-c]pyridine-5-carboxylic acid tert-butyl ester

(ESI) *m*/*z* 367 [(M + H)⁺]. HRMS (ESI) calculated for C₂₀H₂₃N₄O₃⁺ [(M + H)⁺] 367.1765, found 367.1783.

### Step 11. 2-[5-(3-{[4-(4-Ethyl-piperazin-1-ylmethyl)-3-trifluoromethyl-phenylcarbamoyl]-methyl}-phenylethynyl) -2-methylamino-pyridin-4-yl]-1-methyl-4-oxo-1,4,6,7-tetrahydro-pyrrolo[3,2-c]pyridine-5-carboxylic acid tert-butyl ester

A solution of 2-(5-ethynyl-2-methylamino-pyridin-4-yl)-1-methyl-4-oxo-1,4,6,7-tetrahydro-pyrrolo[3,2-c]pyridine-5-carboxylic acid tert-butyl ester (25 mg, 0.0644 mmol), 2-(3-iodo-phenyl)-N-[4-(4-ethyl-piperazin-1-ylmethyl)-3-trifluoromethyl-phenyl]-acetamide (68 mg, 0.1288 mmol), Cul (2.7 mg, 0.014 mmol), PdCl₂(PPh₃)₂ (10 mg, 0.014 mmol) and TEA (0.2 mL, 1,43 mmol) in DMF (6 mL) was degassed with argon and stirred at 50 °C for 3 h. The reaction was poured in water (15 mL) and extracted with EtOAc (2 x 15 mL). The organic phase was washed with brine, dried over anhydrous Na₂SO₄ and evaporated under vacuum to give the crude mixture that was purified by flash column chromatography (EtOAc/MeOH/NH₃ 95/15/0.5). The title product was obtained as yellowish solid (38 mg, 76%).
¹H NMR (401 MHz, DMSO-d₆) δ ppm 0.97 (t, J=7.14 Hz, 3 H) 1.45 (s, 9 H) 2.22 - 2.44 (m, 10 H) 2.84 (d, J=4.88 Hz, 3 H) 2.96 (t, J=6.35 Hz, 2 H) 3.51 (s, 3 H) 3.52 (s, 2 H) 3.64 (s, 2 H) 3.99 (t, J=6.23 Hz, 2 H) 6.47 (s, 1 H) 6.64 (s, 1 H) 7.07 - 7.14 (m, 1 H) 7.19 (dt, J=7.05, 1.66 Hz, 1 H) 7.25 - 7.38 (m, 3 H) 7.65 (d, J=8.54 Hz, 1 H) 7.77 (dd, J=8.54, 1.95 Hz, 1 H) 8.03 (d, J=2.20 Hz, 1 H) 8.28 (s, 1 H) 10.42 (br. s., 1 H).

According to this same methodology, but employing suitable substituted derivatives, the following intermediates were prepared:

### 2-[2-Amino-5-(3-{[4-(4-ethyl-piperazin-1-ylmethyl)-3-trifluoromethyl-phenylcarbamoyl]-methyl}-phenylethynyl) -pyridin-4-yl]-1-methyl-4-oxo-1,4,6,7-tetrahydro-pyrrolo[3,2-c]pyridine-5-carboxylic acid tert-butyl ester

(ESI) *mlz* 770 [(M + H)⁺]. HRMS (ESI) calculated for C₄₂H₄₇F₃N₇O₄⁺ [(M + H)⁺] 770.3636, found 770.3615.

### Step 12. N-[4-(4-Ethyl-piperazin-1-ylmethyl)-3-trifluoromethyl-phenyl]-2-{3-[6-methylamino-4-(1-methyl-4-oxo-4,5,6,7-tetrahydro-1H-pyrrolo[3,2-c]pyridin-2-yl)-pyridin-3-ylethynyl]-phenyl}-acetamide (cmpd 209)

2-[5-(3-{[4-(4-Ethyl-piperazin-1-ylmethyl)-3-trifluoromethyl-phenylcarbamoyl]-methyl}-phenylethynyl)-2-methylaminopyridin-4-yl]-1-methyl-4-oxo-1,4,6,7-tetrahydro-pyrrolo[3,2-c]pyridine-5-carboxylic acid tert-butyl ester (35 mg, 0.0457 mmol) was treated with TFA (0.5 mL) in DCM (3 mL) for 1 h. The solvents were removed under vacuum. The resulting crude was dissolved in water, treated with a saturated solution of NaHCO₃ and extracted with DCM (3 x 4 mL). The combined organic layers were dried over Na₂SO₄ and taken to dryness under reduced pressure. The title compound was purified by column chromatography (DCM/EtOH/NH₃ 95/5/0.5) and isolated as pale yellow solid (25 mg, 81%).
¹H NMR (401 MHz, DMSO-d₆) δ ppm 0.97 (t, J=7.14 Hz, 3 H) 2.23 - 2.45 (m, 10 H) 2.79 - 2.89 (m, 5 H) 3.44 (td, J=6.96, 2.44 Hz, 2 H) 3.49 - 3.55 (m, 5 H) 3.64 (s, 2 H) 6.45 (s, 1 H) 6.56 (s, 1 H) 7.01 - 7.09 (m, 2 H) 7.19 (dt, J=7.11, 1.69 Hz, 1 H) 7.26 - 7.36 (m, 3 H) 7.65 (d, J=8.54 Hz, 1 H) 7.77 (dd, J=8.67, 1.95 Hz, 1 H) 8.04 (d, J=2.20 Hz, 1 H) 8.27 (s, 1 H) 10.45 (s, 1 H).
(ESI) *m*/*z* 684 [(M + H)⁺]. HRMS (ESI) calculated for C₃₈H₄₁F₃N₇O₂+ [(M + H)⁺] 684.3269, found 684.3280.

According to this same methodology, but employing suitable substituted derivatives, the following intermediates were prepared:

### 2-{3-[6-Amino-4-(1-methyl-4-oxo-4,5,6,7-tetrahydro-1H-pyrrolo[3,2-c]pyridin-2-yl)-pyridin-3-ylethynyl]-phenyl)-N-[4-(4-ethyl-piperazin-1-ylmethyl)-3-trifluoromethyl-phenyl]-acetamide (cmpd 208)

¹H NMR (401 MHz, DMSO-d₆) δ ppm 1.00 (t, J=6.65 Hz, 3 H) 2.20 - 2.47 (m, 10 H) 2.86 (t, J=6.96 Hz, 2 H) 3.41 - 3.46 (m, 2 H) 3.51 (s, 3 H) 3.54 (s, 2 H) 3.64 (s, 2 H) 6.46 (d, J=0.61 Hz, 1 H) 6.51 (s, 2 H) 6.56 (s, 1 H) 7.06 (t, J=2.20 Hz, 1 H) 7.19 (dt, J=7.02, 1.68 Hz, 1 H) 7.26 - 7.35 (m, 2 H) 7.65 (d, J=8.54 Hz, 1 H) 7.78 (dd, J=8.42, 1.71 Hz, 1 H) 8.04 (d, J=2.20 Hz, 1 H) 8.19 (d, J=0.61 Hz, 1 H) 10.46 (s, 1 H).
(ESI) *m*/*z* 670 [(M + H)⁺]. HRMS (ESI) calculated for C₃₇H₃₉F₃N₇O₂⁺ [(M + H)⁺] 670.3112, found 670.3091.

### Example 9

### {3-[2-Amino-4-(1-methyl-4-oxo-4,5,6,7-tetrahydro-1H-pyrrolo[3,2-c]pyridin-2-yl)-pyrimidin-5-ylethynyl]-phenyl}-carbamic acid phenyl ester (cmpd 203)

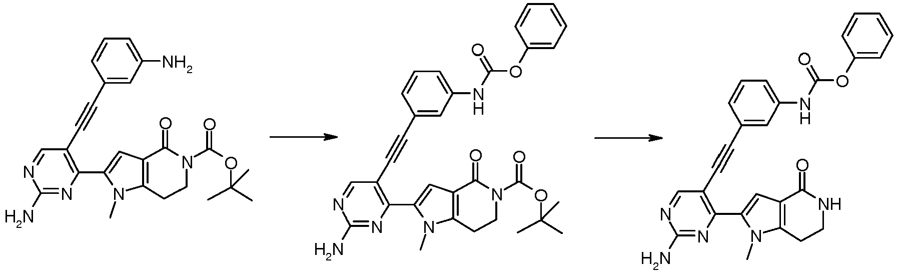

### Step 1. 2-[2-Amino-5-(3-phenoxycarbonylamino-phenylethynyl)-pyrimidin-4-yl]-1-methyl-4-oxo-1,4,6,7-tetrahydro-pyrrolo[3,2-c]pyridine-5-carboxylic acid tert-butyl ester

A suspension of 2-[2-amino-5-(3-amino-phenylethynyl)-pyrimidin-4-yl]-1-methyl-4-oxo-1,4,6,7-tetrahydro-pyrrolo[3,2-c]pyridine-5-carboxylic acid tert-butyl ester (100 mg, 0.218 mmol) in DCM (3 mL) and pyridine (1.5 mL) was treated with phenylchloroformate (0.027 mL, 0.218 mmol) for 4 h at room temperature. The mixture was then diluted with DCM and washed with water, 0.5N HCl, water and brine. The organic layer was dried over Na₂SO₄ and evaporated to dryness under reduced pressure affording the title compound as yellow solid that was used in the subsequent step without any further purification.

### Step 2. {3-[2-Amino-4-(1-methyl-4-oxo-4,5,6,7-tetrahydro-1H-pyrrolo[3,2-c]pyridin-2-yl)-pyrimidin-5-y)ethynyl]-phenyl}-carbamic acid phenyl ester (cmpd 203)

2-[2-amino-5-(3-amino-phenylethynyl)-pyrimidin-4-yl]-1-methyl-4-oxo-1,4,6,7-tetrahydro-pyrrolo[3,2-c]pyridine-5-carboxylic acid tert-butyl ester was dissolved in dioxane (3 mL) and treated with 4M HCL in dioxane (2 mL) for 1 h. The solvent was removed under vacuum and the crude was purified by column chromatography (EtOAc/MeOH/NH₃ 97/3/0.5) affording the title compound as white solid (48.6 mg, 46%).
¹H NMR (401 MHz, DMSO-d₆) δ ppm 2.87 (t, J = 6.8 Hz, 2 H), 3.43 (m, 2 H), 3.82 (s, 3 H), 7.04 (s, 2 H), 7.09-7.27 (m, 6H), 7.35 (t, J = 7.9 Hz, 1 H), 7.40-7.45 (m, 2H), 7.50(m, 1 H), 7.59(m, 1 H), 8.43 (s, 1 H), 10.30 (br. s., 1 H).

## Claims

1. A compound of formula (I), 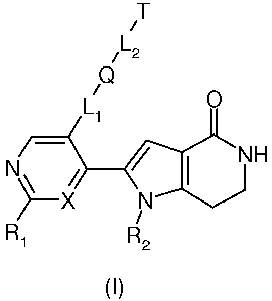 wherein
X is CH or N;
R₁ is H or NHR₃, wherein R₃ is H, an optionally substituted group selected from straight or branched C₁-C₃ alkyl, heterocyclyl and COR', wherein R' is an optionally substituted group selected from straight or branched C₁-C₆ alkyl, a C₃-C₆ carbocyclyl, aryl and heteroaryl;
L₁ is C ≡ C;
Q is an optionally substituted aryl or heteroaryl selected from: 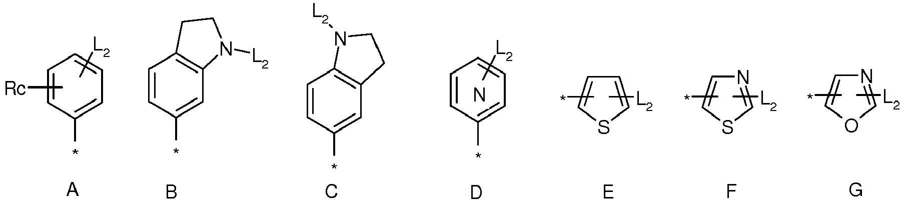
wherein Rc is selected from methyl and halogen, preferably fluorine;
L₂ is CONRa, C(RaRb)CONRa, C(RaRb)C(RaRb)CONRa, CONRaC(RaRb), OC(RaRb)CONRa, wherein Ra and Rb are both hydrogen;
T is a substituted aryl of formula J: 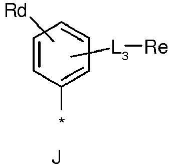 wherein
Rd is halogen, optionally substituted straight or branched C₁-C₄ alkyl, C₃-C₆ cycloalkyl or trifluoromethyl;
L₃ is direct linkage, O, NH, NCH₃, CH₂, CH₂NH, CH₂NCH₃ or C=O;
**Re** is NRfRg, whereinRf and Rg are each independently an optionally substituted straight or branched C₁-C₆ alkyl chain, wherein from 1 to 3 carbon atoms of said alkyl may be substituted independently by N or O, or Rf and Rg joined together with the nitrogen atom, represent a heterocyclic ring;
**R₂** is H or an optionally substituted group selected from straight or branched C₁-C₆ alkyl, C₃-C₆ carbocyclyl, heterocyclyl, aryl and heteroaryl;
or pharmaceutically acceptable salts thereof.

2. A compound of formula (I), as defined in claim 1, wherein
**Q** is an optionally substituted aryl of structure A: 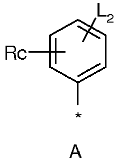 wherein **Rc** and **L₂** are as defined in claim 1.

3. A compound or a pharmaceutically acceptable salt thereof according to claim 1 which is selected from the group consisting of:
6-[2-Amino-4-(1-methyl-4-oxo-4,5,6,7-tetrahydro-1H-pyrrolo[3,2-c]pyridin-2-yl)-pyrimidin-5-ylethynyl]-2,3-dihydro-indole-1-carboxylic acid [4-(4-ethyl-piperazin-1-ylmethyl)-3-trifluoromethyl-phenyl]-amide (Cmpd 44),
5-[2-Amino-4-(1-methyl-4-oxo-4,5,6,7-tetrahydro-1H-pyrrolo[3,2-c]pyridin-2-yl)-pyrimidin-5-ylethynyl]-2,3-dihydro-indole-1-carboxylic acid [4-(4-ethyl-piperazin-1-ylmethyl)-3-trifluoromethyl-phenyl]-amide (Cmpd 45),
3-[2-Amino-4-(1-methyl-4-oxo-4,5,6,7-tetrahydro-1H-pyrrolo[3,2-c]pyridin-2-yl)-pyrimidin-5-ylethynyl]-N-[4-(4-ethyl-piperazin-1-ylmethyl)-3-trifluoromethyl-phenyl]-benzamide (Cmpd 48),
N-[4-(4-Ethyl-piperazin-1-ylmethyl)-3-trifluoromethyl-phenyl]-3-[2-methylamino-4-(1-methyl-4-oxo-4,5,6,7-tetrahydro-1H-pyrrolo[3,2-c]pyridin-2-yl)-pyrimidin-5-ylethynyl]-benzamide (Cmpd 49),
3-[2-Amino-4-(1-methyl-4-oxo-4,5,6,7-tetrahydro-1H-pyrrolo[3,2-c]pyridin-2-yl)-pyrimidin-5-ylethynyl]-N-[4-(4-ethyl-piperazin-1-ylmethyl)-3-trifluoromethyl-phenyl]-4-methyl-benzamide (Cmpd 50),
5-[2-Amino-4-(1-methyl-4-oxo-4,5,6,7-tetrahydro-1H-pyrrolo[3,2-c]pyridin-2-yl)-pyrimidin-5-ylethynyl]-thiophene-2-carboxylic acid [4-(4-ethyl-piperazin-1-ylmethyl)-3-trifluoromethyl-phenyl]-amide (Cmpd 51),
2-[2-Amino-4-(1-methyl-4-oxo-4,5,6,7-tetrahydro-1H-pyrrolo[3,2-c]pyridin-2-yl)-pyrimidin-5-ylethynyl]-thiazole-5-carboxylic acid [4-(4-ethyl-piperazin-1-ylmethyl)-3-trifluoromethyl-phenyl]-amide (Cmpd 52),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-ethyi-piperazin-1-ylmethyl)-3-trifluoromethyi-phenyl]-acetamide (Cmpd 77),
N-[4-(4-Ethyl-piperazin-1-ylmethyl)-3-trifluoromethyl-phenyl]-2-{3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-acetamide (Cmpd 78),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[3-(4-ethyl-piperazin-1-ylmethyl)-4-trifluoromethyl-phenyl]-acetamide (Cmpd 82),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-ethyl-piperazin-1-ylmethyl)-3-fluoro-phenyl]-acetamide (Cmpd 120),
2-{5-[2-Amino-4-(1-methyl-4-oxo-4,5,6,7-tetrahydro-1H-pyrrolo[3,2-c]pyridin-2-yl)-pyrimidin-5-ylethynyl]-pyridin-3-yl}-N-[4-(4-ethyl-piperazin-1-ylmethyl)-3-trifluoromethyl-phenyl]-acetamide (Cmpd 122),
2-{4-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-ethyl-piperazin-1-ylmethyl)-3-trifluoromethyl-phenyl]-acetamide (Cmpd 123),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[3-(4-ethyl-piperazine-1-carbonyl)-4-trifluoromethyl-phenyl]-acetamide (Cmpd 127),
2-{5-[2-Amino-4-(1-methyl-4-oxo-4,5,6,7-tetrahydro-1H-pyrrolo[3,2-c]pyridin-2-yl)-pyrimidin-5-ylethynyl]-thiophen-2-yl}-N-[4-(4-ethyl-piperazin-1-ylmethyl)-3-trifluoromethyl-phenyl]-acetamide (Cmpd 129),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-chloro-3-(4-ethyl-piperazin-1-ylmethyl)-phenyl]-acetamide (Cmpd 138),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-[1,4]diazepan-1-ylmethyl)-3-trifluoromethyl-phenyl]-acetamide (Cmpd 149),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-((S)-3-dimethylamino-pyrrolidin-1-ylmethyl)-3-trifluoromethyl-phenyl]-acetamide (Cmpd 150)
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[3-cyclopropyl-4-(4-methyl-piperazin-1-ylmethyl)-phenyl]-acetamide (Cmpd 154),
2-{3-[2-Amino-4-(1-methyl-4-oxo-4,5,6,7-tetrahydro-1H-pyrrolo[3,2-c]pyridin-2-yl)-pyrimidin-5-ylethynyl]-4-fluoro-phenyl}-N-[4-(4-ethyl-piperazin-1-ylmethyl)-3-trifluoromethyl-phenyl]-acetamide (Cmpd 155),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-isopropyl-piperazin-1-ylmethyl)-3-trifluoromethyl-phenyl]-acetamide (Cmpd 158),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide (Cmpd 161),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-propyl-piperazin-1-ylmethyl)-3-trifluoromethyl-phenyl]-acetamide (Cmpd 164),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-dimethylamino-piperidin-1-ylmethyl)-3-trifluoromethyl-phenyl]-acetamide (Cmpd 167)
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-cyclopropylmethyl-piperazin-1-ylmethyl)-3-trifluoromethyl-phenyl]-acetamide (Cmpd 171),
2-{3-[2-Methylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide (Cmpd 174),
N-[4-(4-lsopropyl-piperazin-1-ylmethyl)-3-trifluoromethyl-phenyl]-2-{3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-acetamide (Cmpd 176),
2-{3-[2-Methylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-propyl-piperazin-1-ylmethyl)-3-trifluoromethyl-phenyl]-acetamide (Cmpd 179),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-piperazin-1-ylmethyl-3-trifluoromethyl-phenyl)-acetamide (Cmpd 183),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(4-{[(2-hydroxy-ethyl)-methyl-amino]-methyl}-3-trifluoromethyl-phenyl)-acetamide (Cmpd 189),
2-{4-Fluoro-3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide (Cmpd 190),
2-(3-{2-Amino-4-[1-(2-hydroxy-ethyl)-4-oxo-4,5,6,7-tetrahydro-1H-pyrrolo[3,2-c]pyridin-2-yl]-pyrimidin-5-ylethynyl}-phenyl)-N-[4-(4-ethyl-piperazin-1-ylmethyl)-3-trifluoromethyl-phenyl]-acetamide (Cmpd 191),
2-{3-[2-Ethylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide (cmpd 193),
N-[4-((S)-3-Dimethylamino-pyrrolidin-1-ylmethyl)-3-trifluoromethyl-phenyl]-2-{3-[2-methylamino-4-(1-methyl-4-oxo-4,5,6,7-tetrahydro-1H-pyrrolo[3,2-c]pyridin-2-yl)-pyrimidin-5-ylethynyl]-phenyl}-acetamide (Cmpd 198),
2-{3-[2-Acetylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide (Cmpd 200),
2-{3-[6-Amino-4-(1-methyl-4-oxo-4,5,6,7-tetrahydro-1H-pyrrolo[3,2-c]pyridin-2-yl)-pyridin-3-ylethynyl]-phenyl}-N-[4-(4-methyl-piperazin-1-ylmethyl)-3-trifluoromethyl-phenyl]-acetamide (Cmpd 201), and
2-{3-[2-lsopropylamino-4-(1-methyl-4-oxo-4,5,6,7-tetrahydro-1H-pyrrolo[3,2-c]pyridin-2-yl)-pyrimidin-5-ylethynyl]-phenyl}-N-[4-(4-methyl-piperazin-1-ylmethyl)-3-trifluoromethyl-phenyl]-acetamide (Cmpd 202).

4. A compound or a pharmaceutically acceptable salt thereof selected from the group consisting of:
1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[4-(4-ethyl-piperazin-1-ylmethyl)-3-trifluoromethyl-phenyl]-urea (Cmpd 8),
1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[3-(4-ethyl-piperazin-1-ylmethyl)-4-trifluoromethyl-phenyl]-urea (Cmpd 22),
1-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[3-(4-ethyl-piperazin-1-ylmethyl)-5-trifluoromethyl-phenyl]-urea (Cmpd 29),
1-{4-[2-Amino-4-(1-methyl-4-oxo-4,5,6,7-tetrahydro-1H-pyrrolo[3,2-c]pyridin-2-yl)-pyrimidin-5-ylethynyl]-phenyl}-3-[4-(4-ethyl-piperazin-1-ylmethyl)-3-trifluoromethyl-phenyl]-urea (Cmpd 30),
1-{5-[2-Amino-4-(1-methyl-4-oxo-4,5,6,7-tetrahydro-1H-pyrrolo[3,2-c]pyridin-2-yl)-pyrimidin-5-ylethynyl]-2-fluoro-phenyl}-3-[4-(4-ethyl-piperazin-1-ylmethyl)-3-trifluoromethyl-phenyl]-urea (Cmpd 36),
1-[4-(4-Ethyl-piperazin-1-ylmethyl)-3-trifluoromethyl-phenyl]-3-{3-[4-(1-methyl-4-oxo-4,5,6,7-tetrahydro-1H-pyrrolo[3,2-c]pyridin-2-yl)-pyridin-3-ylethynyl]-phenyl}-urea (Cmpd 38),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-(5-tert-butyl-2-methyl-2H-pyrazol-3-yl)-acetamide (Cmpd 105),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(1-methyl-piperidin-4-yloxy)-3-trifluoromethyl-phenyl]-acetamide (Cmpd 172),
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-[4-(1-methyl-piperidin-4-ylamino)-3-trifluoromethyl-phenyl]-acetamide (Cmpd 177), and
2-{3-[2-Amino-4-(1-methyl-4-oxo-4,5,6,7-tetrahydro-1H-pyrrolo[3,2-c]pyridin-2-yl)-pyrimidin-5-ylethynyl]-phenyl}-N-{4-[methyl-(1-methyl-piperidin-4-yl)-amino]-3-trifluoromethyl-phenyl}-acetamide (Cmpd 178).

5. A process for preparing a compound of formula (I) as defined in claim 1 or for preparing a compound of claim 4, or the pharmaceutically acceptable salts thereof, **characterized in that** the process comprises the following steps:
Method A:
step a: coupling of a pyrrolopyridinone derivative of formula (III) 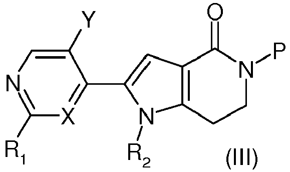 wherein X, R₁ and R₂ are as defined in claim 1, Y is halogen and P is a suitable protecting group such as the *tert*-butoxycarbonyl group, with an intermediate of formula (1a)-(1h) 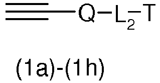 wherein L₂ is CONRa, C(RaRb)CONRa, C(RaRb)C(RaRb)CONRa, CONRaC(RaRb), OC(RaRb)CONRa , NRaCONRa and Ra, Rb, Q, and Tare as defined in claim 1, under Sonogashira reaction conditions;
step b: deprotection of the resultant intermediate of formula (II) 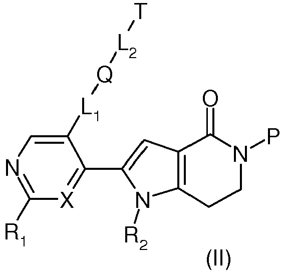 wherein L₁ is C ≡ C and X, R₁, R₂, L₂, Q, T and P are as defined above, under acid conditions to yield a compound of formula (I) as defined in claim 1 or a compound of claim 4;
alternatively, Method B:
step c: coupling of a pyrrolopyridinone derivative of formula (III), as defined above, with ethynyl-trimethylsilane under Sonogashira reaction conditions, followed by removal of the trimethylsilyl group under basic conditions;
step d: reaction of the resultant intermediate of formula (IV) 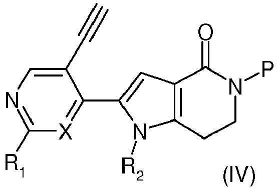 wherein X, R₁ and R₂ are as defined in claim 1 and P is as defined above, with an intermediate of formula (3a)-(3h) 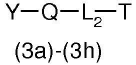 wherein Y is halogen, L₂ is CONRa, C(RaRb)CONRa, C(RaRb)C(RaRb)CONRa, CONRaC(RaRb), OC(RaRb)CONRa , NRaCONRa and Ra, Rb, Q, and T are as defined in claim 1, under Sonogashira reaction conditions;
step b: deprotection of the resultant intermediate of formula (II) as defined above;
alternatively, Method C:
step c: coupling of a pyrrolopyridinone derivative of formula (III), as defined above, with ethynyl-trimethylsilane under Sonogashira reaction conditions, followed by removal of the trimethylsilyl group under basic conditions;
step e: reaction of the resultant intermediate of formula (IV) as defined above, with an intermediate of formula Y-Q-L₂'-CO-Y' wherein L₂' is direct linkage, -C(RaRb)-, -C(RaRb)C(RaRb)- or -OC(RaRb)-, Y is halogen, Y' is OH and Q, Ra and Rb are as defined in claim 1, or with an intermediate of formula Y-Q-L₂'-NH-Ra wherein Y, Q, L₂' and Ra are as defined above under Sonogashira reaction conditions;
step f: coupling the resultant intermediate of formula (V) 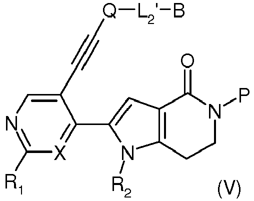 wherein B is COOH or NHRa and X, Q, Ra, R₁, R₂, L₂' and P are as defined above, with a suitable intermediate in the presence of a coupling agent to give an intermediate of formula (II) as defined above;
step b: deprotection of the intermediate of formula (II) as defined above;
alternatively, Method D:
step g: reaction of an intermediate of formula (III) as defined above with an intermediate of formula ≡-Q-L₂'-CO-Y' wherein L₂', Y, Y' and Q are as defined above, or with an intermediate of formula ≡-Q-L₂'-NH-Ra wherein Y, Q, L₂' and Ra are as defined above under Sonogashira reaction conditions to yield an intermediate of formula (V), as defined above;
step f: coupling the resultant intermediate of formula (V) with a suitable intermediate in the presence of a coupling agent to give an intermediate of formula (II) as defined above;
step b: deprotection of the intermediate of formula (II) as defined above;
optionally converting a compound of formula (I) into a different compound of formula (I) by known chemical reactions; and/or, if desired, converting a compound of formula (I) into a pharmaceutically acceptable salt thereof or converting a salt into a free compound of formula (I).

6. An *in vitro* method for inhibiting the RET family protein activity which comprises contacting the said protein with an effective amount of a compound of formula (I) as defined in claim 1, or of a compound of claim 4.

7. A pharmaceutical composition comprising a therapeutically effective amount of a compound of formula (I) as defined in claim 1, or of a compound of claim 4, or the pharmaceutically acceptable salts thereof, and at least one pharmaceutically acceptable excipient, carrier and/or diluent.

8. A pharmaceutical composition, according to claim7, further comprising one or more chemotherapeutic agents.

9. A product comprising a compound of formula (I) as defined in claim 1, or comprising a compound of claim 4, or the pharmaceutically acceptable salts thereof, and one or more chemotherapeutic agents, as a combined preparation for simultaneous, separate or sequential use in anticancer therapy.

10. A compound of formula (I) as defined in claim 1, or a compound of claim 4, or the pharmaceutically acceptable salts thereof, for use as a medicament.

11. A compound of formula (I) as defined in claim 1, or a compound of claim 4, or the pharmaceutically acceptable salts thereof, for use in a method of treating cancer.

## Patentansprüche

1. Verbindung der Formel (I), 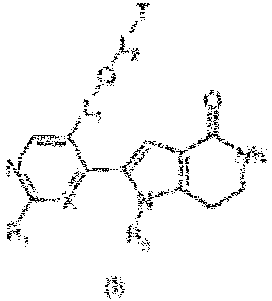 worin
X CH oder N ist;
R₁ H oder NHR₃ ist, wobei R₃ H ist, eine wahlweise substituierte Gruppe, ausgewählt aus geradkettigem oder verzweigtem C₁-C₃ Alkyl, Heterocyclyl und COR', wobei R' eine wahlweise substituierte Gruppe ist, ausgewählt aus geradkettigem oder verzweigtem C₁-C₆ Alkyl, einem C₃-C₆ Carbocyclyl, Aryl und Heteroaryl;
**L₁** C ≡ C ist;
**Q** ein wahlweise substituiertes Aryl oder Heteroaryl ist, ausgewählt aus: 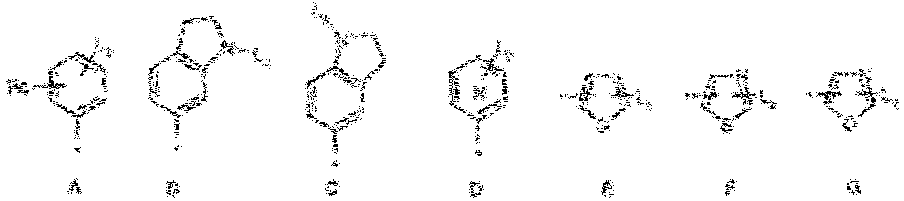
worin **Rc** ausgewählt ist aus Methyl und Halogen, vorzugsweise Fluor;
**L₂** CONRa, C(RaRb)CONRa, C(RaRb)C(RaRb)CONRa, CONRaC(RaRb), OC(RaRb)CONRa ist, worin Ra und Rb beide Wasserstoff sind;
**T** ein substituiertes Aryl der Formel J ist: 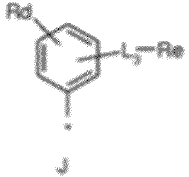 worin
**Rd** Halogen, wahlweise substituiertes geradkettiges oder verzweigtes C₁-C₄ Alkyl, C₃-C₆ Cycloalkyl oder Trifluormethyl ist;
**L₃** eine direkte Bindung ist, 0, NH, NCH₃, CH₂, CH₂NH, CH₂NCH₃ oder C = 0;
**Re** NRfRg ist, worin Rf und Rg jeweils unabhängig voneinander eine wahlweise substituierte geradkettige oder verzweigte C₁-C₆ Alkylkette sind, worin 1 bis 3 Kohlenstoffatome des Alkyls unabhängig voneinander durch N oder O substituiert sein können, oder Rf und Rg, miteinander mit dem Stickstoffatom verbunden, einen heterocyclischen Ring darstellen;
**R₂** H oder eine wahlweise substituierte Gruppe ist, ausgewählt aus geradkettigem oder verzweigtem C₁-C₆ Alkyl, C₃-C₆ Carbocyclyl, Heterocyclyl, Aryl und Heteroaryl; oder pharmazeutisch verträgliche Salze davon.

2. Verbindung der Formel (I), wie in Anspruch 1 definiert, worin
**Q** ein wahlweise substituiertes Aryl der Struktur A ist: 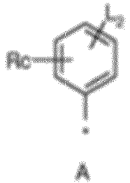 worin **Rc** und **L₂** wie in Anspruch 1 definiert sind.

3. Verbindung oder ein pharmazeutisch verträgliches Salz davon nach Anspruch 1, die ausgewählt ist aus der Gruppe, bestehend aus:
6-[2-Amino-4-(1-methyl-4-oxo-4,5,6,7-tetrahydro-1H-pyrrolo [3,2-c]pyridin-2-yl)pyrimidin-5-ylethinyl]-2,3-dihydro indol-1-carbonsäure[4-(4-ethylpiperazin-1-yl methyl)-3-tri fluor methylphenyl]-amid (Vbdg. 44),
5-[2-Amino-4-(1-methyl-4-oxo-4,5,6,7-tetrahydro-1H-pyrrolo [3,2-c]pyridin-2-yl)pyrimidin-5-ylethinyl]-2,3-dihydro indol-1-carbonsäure[4-(4-ethylpiperazin-1-yl methyl)-3-tri fluor methylphenyl]-amid (Vbdg. 45),
3-[2-Amino-4-(1-methyl-4-oxo-4,5,6,7-tetrahydro-1H-pyr rolo [3,2-c]pyridin-2-yl)pyrimidin-5-ylethinyl]-N-[4-(4-ethylpi perazin-1-ylmethyl)-3-trifluormethylphenyl]-benzamid (Vbdg. 48),
N-[4-(4-Ethylpiperazin-1-ylmethyl)-3-trifluormethyl phenyl]-3-[2-methylamino-4-(1-methyl-4-oxo-4,5,6,7-tetra hydro-1H-pyrrolo[3,2-c]pyridin-2-yl)pyrimidin-5-yl ethinyl]-benzamid (Vbdg. 49),
3-[2-Amino-4-(1-methyl-4-oxo-4,5,6,7-tetrahydro-1H-pyrrolo [3,2-c]pyridin-2-yl)pyrimidin-5-ylethinyl]-N-[4-(4-ethylpi perazin-1-ylmethyl)-3-trifluormethylphenyl]-4-methyl benzamid (Vbdg. 50),
5-[2-Amino-4-(1-methyl-4-oxo-4,5,6,7-tetrahydro-1H-pyr rolo [3,2-c]pyridin-2-yl)pyrimidin-5-ylethinyl]-thiophen-2-carbonsäure[4-(4-ethylpiperazin-1-ylmethyl)-3-trifluor methylphenyl]-amid (Vbdg. 51),
2-[2-Amino-4-(1-methyl-4-oxo-4,5,6,7-tetrahydro-1H-pyr rolo [3,2-c]pyridin-2-yl)pyrimidin-5-ylethinyl]-thiazol-5-carbonsäure[4-(4-ethylpiperazin-1-ylmethyl)-3-trifluor methylphenyl]-amid (Vbdg. 52),
2-{3-[2-Amino-4-(1-methyl-4-oxo-4,5,6,7-tetrahydro-1H-pyr rolo[3,2-c]pyridin-2-yl)pyrimidin-5-ylethinyl]-phenyl}-N-[4-(4-ethylpiperazin-1-ylmethyl)-3-trifluormethylphenyl]-acetamid (Vbdg. 77),
N-[4-(4-Ethylpiperazin-1-ylmethyl)-3-trifluormethyl phenyl]-2-{3-[2-methylamino-4-(1-methyl-4-oxo-4,5,6,7-tetrahydro-1H-pyrrolo[3,2-c]pyridin-2-yl)pyrimidin-5-ylethinyl]phenyl}-acetamid (Vbdg. 78),
2-13-[2-Amino-4-(1-methyl-4-oxo-4,5,6,7-tetrahydro-1H-pyr rolo[3,2-c]pyridin-2-yl)pyrimidin-5-ylethinyl]-phenyl}-N-[3-(4-ethylpiperazin-1-ylmethyl)-4-trifluormethylphenyl]-acetamid (Vbdg. 82),
2-13-[2-Amino-4-(1-methyl-4-oxo-4,5,6,7-tetrahydro-1H-pyr rolo[3,2-c]pyridin-2-yl)pyrimidin-5-ylethinyl]-phenyl}-N-[4-(4-ethylpiperazin-1-ylmethyl)-3-fluorphenyl]-acetamid (Vbdg. 120),
2-15-[2-Amino-4-(1-methyl-4-oxo-4,5,6,7-tetrahydro-1H-pyr rolo[3,2-c]pyridin-2-yl)pyrimidin-5-ylethinyl]-pyridin-3-yl]-N-[4-(4-ethylpiperazin-1-ylmethyl)-3-trifluormethyl phenyl]-acetamid (Vbdg. 122),
2-14-[2-Amino-4-(1-methyl-4-oxo-4,5,6,7-tetrahydro-1H-pyr rolo[3,2-c]pyridin-2-yl)pyrimidin-5-ylethinyl]-phenyl}-N-[4-(4-ethylpiperazin-1-ylmethyl)-3-trifluormethylphenyl]-acetamid (Vbdg. 123),
2-13-[2-Amino-4-(1-methyl-4-oxo-4,5,6,7-tetrahydro-1H-pyr rolo[3,2-c]pyridin-2-yl)pyrimidin-5-ylethinyl]-phenyl}-N-[3-(4-ethylpiperazin-1-carbonyl)-4-trifluormethylphenyl]-acetamid (Vbdg. 127),
2-15-[2-Amino-4-(1-methyl-4-oxo-4,5,6,7-tetrahydro-1H-pyr rolo[3,2-c]pyridin-2-yl)pyrimidin-5-ylethinyl]-thiophen-2-yl}-N-[4-(4-ethylpiperazin-1-ylmethyl)-3-trifluormethyl phenyl]-acetamid (Vbdg. 129),
2-13-[2-Amino-4-(1-methyl-4-oxo-4,5,6,7-tetrahydro-1H-pyr rolo[3,2-c]pyridin-2-yl)pyrimidin-5-ylethinyl]-phenyl}-N-[4-chlor-3-(4-ethylpiperazin-1-ylmethyl)phenyllacetamid Vbdg. 138),
2-13-[2-Amino-4-(1-methyl-4-oxo-4,5,6,7-tetrahydro-1H-pyr rolo[3,2-c]pyridin-2-yl)pyrimidin-5-ylethinyl]-phenyl}-N-[4-(4-methyl-[1,4]diazepan-1-ylmethyl)-3-trifluormethyl phenyl]-acetamid (Vbdg. 149),
2-13-[2-Amino-4-(1-methyl-4-oxo-4,5,6,7-tetrahydro-1H-pyr rolo[3,2-c]pyridin-2-yl)pyrimidin-5-ylethinyl]-phenyl}-N-[4-((S)-3-dimethylamino-pyrrolidin-1-ylmethyl)-3trifluor methylphenyl]-acetamid (Vbdg. 150),
2-13-[2-Amino-4-(1-methyl-4-oxo-4,5,6,7-tetrahydro-1H-pyr rolo[3,2-c]pyridin-2-yl)pyrimidin-5-ylethinyl]-phenyl}-N-[3-cyclopropyl-4-(4-methylpiperazin-1-ylmethyl)phenyl]-acetamid (Vbdg. 154),
2-13-[2-Amino-4-(1-methyl-4-oxo-4,5,6,7-tetrahydro-1H-pyr rolo[3,2-c]pyridin-2-yl)pyrimidin-5-ylethinyl]-4-fluor phenyl}-N-[4-(4-ethylpiperazin-1-ylmethyl)-3-trifluor methyl phenyl]-acetamid (Vbdg. 155),
2-13-[2-Amino-4-(1-methyl-4-oxo-4,5,6,7-tetrahydro-1H-pyr rolo[3,2-c]pyridin-2-yl)pyrimidin-5-ylethinyl]-phenyl}-N-[4-(4-isopropylpiperazin-1-ylmethyl)-3-trifluormethyl phenyl]-acetamid (Vbdg. 158),
2-13-[2-Amino-4-(1-methyl-4-oxo-4,5,6,7-tetrahydro-1H-pyr rolo[3,2-c]pyridin-2-yl)pyrimidin-5-ylethinyl]-phenyl}-N-[4-(4-methylpiperazin-1-ylmethyl)-3-trifluormethylphenyl]-acetamid (Vbdg. 161),
2-13-[2-Amino-4-(1-methyl-4-oxo-4,5,6,7-tetrahydro-1H-pyr rolo[3,2-c]pyridin-2-yl)pyrimidin-5-ylethinyl]-phenyl}-N-[4-(4-propylpiperazin-1-ylmethyl)-3-trifluormethylphenyl]-acet amid (Vbdg. 164),
2-13-[2-Amino-4-(1-methyl-4-oxo-4,5,6,7-tetrahydro-1H-pyr rolo[3,2-c]pyridin-2-yl)pyrimidin-5-ylethinyl]-phenyl}-N-[4-(4-dimethylaminopiperidin-1-ylmethyl)-3-trifluormethyl phenyl]-acetamid (Vbdg. 167),
2-13-[2-Amino-4-(1-methyl-4-oxo-4,5,6,7-tetrahydro-1H-pyr rolo[3,2-c]pyridin-2-yl)pyrimidin-5-ylethinyl]-phenyl}-N-[4-(4-cyclopropylmethylpiperazin-1-ylmethyl)-3-trifluor methylphenyl] -acetamid (Vbdg. 171),
2-{3-[2-Methylamino-4-(1-methyl-4-oxo-4,5,6,7-tetrahydro-1H-pyrrolo[3,2-c]pyridin-2-yl)-pyrimidin-5-ylethinyl]-phenyl}-N-[4-(4-methyl-piperazin-1-ylmethyl)-3-trifluor omethyl-phenyl]-acetamid (Vbdg.174),
N-[4-(4-Isopropylpiperazin-1-ylmethyl)-3-trifluormethyl phenyl]-2-{3-[2-methylamino-4-(1-methyl-4-oxo-4,5,6,7-te trahydro-1H-pyrrolo[3,2-c]pyridin-2-yl)pyrimidin-5-yl ethinyl]phenyl} acetamid (Vbdg. 176),
2-{3-[2-Methylamino-4-(1-methyl-4-oxo-4,5,6,7-tetrahydro-1H-pyrrolo[3,2-c]pyridin-2-yl)pyrimidin-5-ylethinyl] phenyl}-N-[4-(4-propylpiperazin-1-ylmethyl)-3-trifluor methylphenyl]-acetamid (Vbdg. 179),
2-{3-[2-Amino-4-(1-methyl-4-oxo-4,5,6,7-tetrahydro-1H-pyr rolo[3,2-c]pyridin-2-yl)pyrimidin-5-ylethinyl]-phenyl}-N-(4-piperazin-1-ylmethyl-3-trifluormethylphenyl)acetamid (Vbdg. 183),
2-{3-[2-Amino-4-(1-methyl-4-oxo-4,5,6,7-tetrahydro-1H-pyr rolo[3,2-c]pyridin-2-yl)pyrimidin-5-ylethinyl]-phenyl}-N-(4-{[(2-hydroxyethyl)methylamino]methyl}-3-trifluormethyl phenyl)-acetamid (Vbdg. 189),
2-{4-Fluor-3-[2-methylamino-4-(1-methyl-4-oxo-4,5,6,7-te trahydro-1H-pyrrolo[3,2-c]pyridin-2-yl)-pyrimidin-5-yl ethinyl]phenyl}-N-[4-(4-methylpiperazin-1-ylmethyl)-3-tri fluormethylphenyl]acetamid (Vbdg. 190),
2-(3-{2-Amino-4-[1-(2-hydroxyethyl)-4-oxo-4,5,6,7-tetra hydro-1H-pyrrolo[3,2-c]pyridin-2-yl]-pyrimidin-5-yl ethinyl}-phenyl)-N-[4-(4-ethylpiperazin-1-ylmethyl)-3-trifluor methyl phenyl]-acetamid (Vbdg. 191),
2-{3-[2-Ethylamino-4-(1-methyl-4-oxo-4,5,6,7-tetrahydro-1H-pyrrolo[3,2-c]pyridin-2-yl)pyrimidin-5-ylethinyl]-phenyl}-N-[4-(4-methylpiperazin-1-ylmethyl)-3-trifluor methylphenyl]-acetamid (Vbdg. 193),
N-[4-((S)-3-Dimethylamino-pyrrolidin-1-ylmethyl)-3-tri fluormethylphenyl]-2-{3-[2-methylamino-4-(1-methyl-4-oxo-4,5,6,7-tetrahydro-1H-pyrrolo[3,2-c]pyridin-2-yl) pyrimidin-5-ylethinyl]phenyl}-acetamid (Vbdg. 198),
2{3-[2-Acetylamino-4-(1-methyl-4-oxo-4,5,6,7-tetrahydro-1H-pyrrolo[3,2-c]pyridin-2-yl)pyrimidin-5-ylethinyl] phenyl}-N-[4-(4-methylpiperazin-1-ylmethyl)-3-trifluor methylphenyl]-acetamid (Vbdg. 200),
2-{3-[6-Amino-4-(1-methyl-4-oxo-4,5,6,7-tetrahydro-1H-pyr rolo[3,2-c]pyridin-2-yl)pyridin-3-ylethinyl]-phenyl}-N-[4-(4-methylpiperazin-1-ylmethyl)-3-trifluormethylphenyl]-ace tamid (Vbdg. 201) und
2-{3-[2-1sopropylamino-4-(1-methyl-4-oxo-4,5,6,7-tetra hydro-1H-pyrrolo[3,2-c]pyridin-2-yl)pyrimidin-5-ylethinyl]phenyl}-N-[4-(4-methylpiperazin-1-ylmethyl)-3-trifluor methylphenyl] -acetamid (Vbdg. 202).

4. Verbindung oder ein pharmazeutisch verträgliches Salz davon, ausgewählt aus der Gruppe, bestehend aus:
1-{3-[2-Amino-4-(1-methyl-4-oxo-4,5,6,7-tetrahydro-1H-pyr rolo[3,2-c]pyridin-2-yl)pyrimidin-5-ylethinyl]-phenyl}-3-[4-(4-ethylpiperazin-1-ylmethyl)-3-trifluormethylphenyl]-harn stoff (Vbdg. 8),
1-{3-[2-Amino-4-(1-methyl-4-oxo-4,5,6,7-tetrahydro-1H-pyr rolo[3,2-c]pyridin-2-yl)pyrimidin-5-ylethinyl]-phenyl}-3-[3-(4-ethylpiperazin-1-ylmethyl)-4-trifluormethylphenyl]-harn stoff (Vbdg. 22),
1-{3-[2-Amino-4-(1-methyl-4-oxo-4,5,6,7-tetrahydro-1H-pyr rolo[3,2-c]pyridin-2-yl)pyrimidin-5-ylethinyl]-phenyl}-3-[3-(4-ethylpiperazin-1-ylmethyl)-5-trifluormethylphenyl]-harn stoff (Vbdg. 29),
1-{4-[2-Amino-4-(1-methyl-4-oxo-4,5,6,7-tetrahydro-1H-pyr rolo[3,2-c]pyridin-2-yl)pyrimidin-5-ylethinyl]-phenyl}-3-[4-(4-ethylpiperazin-1-ylmethyl)-3-trifluormethylphenyl]-harn stoff (Vbdg. 30),
1-{5-[2-Amino-4-(1-methyl-4-oxo-4,5,6,7-tetrahydro-1H-pyr rolo[3,2-c]pyridin-2-yl)pyrimidin-5-ylethinyl]-2-fluor phenyl}-3-[4-(4-ethylpiperazin-1-ylmethyl)-3-trifluor methylphenyl]-harnstoff (Vbdg. 36),
1-[4-(4-Ethylpiperazin-1-ylmethyl)-3-trifluormethyl phenyl]-3-{3-[4-(1-methyl-4-oxo-4,5,6,7-tetrahydro-1H-pyrrolo[3,2-c]pyridin-2-yl)pyridin-3-ylethinyl]phenyl}-harnstoff (Vbdg. 38),
2-{3-[2-Amino-4-(1-methyl-4-oxo-4,5,6,7-tetrahydro-1H-pyr rolo[3,2-c]pyridin-2-yl)pyrimidin-5-ylethinyl]-phenyl}-N-(5-tert-butyl-2-methyl-2H-pyrazol-3-yl)-acetamid (Vbdg. 105),
2-{3-[2-Amino-4-(1-methyl-4-oxo-4,5,6,7-tetrahydro-1H-pyr rolo[3,2-c]pyridin-2-yl)pyrimidin-5-ylethinyl]-phenyl}-N-[4-(1-methylpiperidin-4-yloxy)-3-trifluormethylphenyl]-acetamid (Vbdg. 172),
2-{3-[2-Amino-4-(1-methyl-4-oxo-4,5,6,7-tetrahydro-1H-pyr rolo[3,2-c]pyridin-2-yl)pyrimidin-5-ylethinyl]-phenyl}-N-[4-(1-methylpiperidin-4-ylamino)-3-trifluormethylphenyl]-acetamid (Vbdg. 177),
2-{3-[2-Amino-4-(1-methyl-4-oxo-4,5,6,7-tetrahydro-1H-pyr rolo[3,2-c]pyridin-2-yl)pyrimidin-5-ylethinyl]-phenyl}-N-{4-[methyl-(1-methylpiperidin-4-yl)amino]-3-trifluormethyl phenyl}-acetamid (Vbdg. 178).

5. Verfahren zur Herstellung einer Verbindung der Formel (I), wie in Anspruch 1 definiert oder zur Herstellung einer Verbindung nach Anspruch 4 oder der pharmazeutisch verträglichen Salze davon, **dadurch gekennzeichnet, dass** das Verfahren die folgenden Schritte umfasst:
Methode A:
Schritt a: Kupplung eines Pyrrolopyridinonderivats der Formel (III) 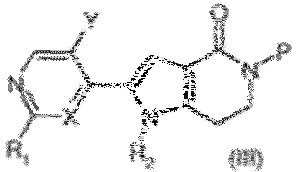 worin X₁, R₁ und R₂ wie in Anspruch 1 definiert sind, Y Halogen ist und P eine geeignete Schutzgruppe wie die tert-Butoxycarbonylgruppe ist, mit einem Zwischenprodukt der Formel (1a) - (1h) 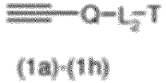 worin L₂ CONRa ist, C(RaRb)CONRa, C(RaRb)C(RaRb)CONRa, CONRaC(RaRb), OC(RaRb)CONRa, NRaCONRa und Ra, Rb, Q und T wie in Anspruch 1 definiert sind, unter Sonogashira-Reaktionsbedingungen;
Schritt b: Entschützen des resultierenden Zwischenprodukts der Formel (II) 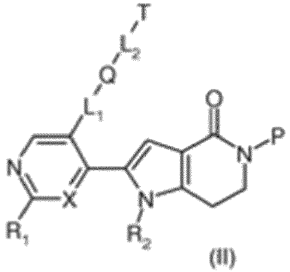 worin L₁ C ≡ C ist und X, R₁, R₂, L₂, Q, T und P wie oben definiert sind, unter sauren Bedingungen, um eine Verbindung der Formel (I) wie in Anspruch 1 definiert oder eine Verbindung nach Anspruch 4 zu ergeben;
alternativ Methode B:
Schritt c: Kuppeln eines Pyrrolopyridinonderivats der Formel (III), wie oben definiert, mit Ethinyltrimethylsilan unter Sonogashira-Reaktionsbedingungen, gefolgt von der Entfernung der Trimethylsilylgruppe unter basischen Bedingungen;
Schritt d: Reaktion des resultierenden Zwischenprodukts der Formel (IV) 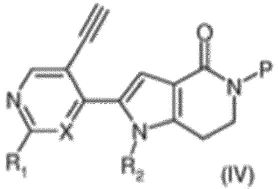 worin X, R₁ und R₂ wie in Anspruch 1 definiert sind und P wie oben definiert ist, mit einem Zwischenprodukt der Formel (3a) - (3h) 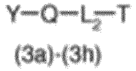 worin Y Halogen ist, L₂ CONRa, C(RaRb)CONRa, C(RaRb)C (RaRb)CONRa, CONRaC(RaRb), OC(RaRb)CONRa, NRaCONRa ist und Ra, Rb, Q und T wie in Anspruch 1 definiert sind unter Sonogashira-Reaktionsbedingungen;
Schritt b: Entschützen des resultierenden Zwischenprodukts der Formel (II) wie oben definiert;
alternativ Methode C:
Schritt c: Kuppeln eines Pyrrolopyridinonderivats der Formel (III), wie oben definiert, mit Ethinyltrimethylsilan unter Sonogashira-Reaktionsbedingungen, gefolgt von der Entfernung der Trimethylsilylgruppe unter basischen Bedingungen;
Schritt e: Reaktion des resultierenden Zwischenprodukts der Formel (IV) wie oben definiert mit einem Zwischenprodukt der Formel YQ-L₂'-CO-Y', worin L₂' eine direkte Bindung, -C(RaRb)-, -C(RaRb)C(RaRb)- oder-OC(RaRb) - ist, Y Halogen ist, Y' OH ist und Q, Ra und Rb wie in Anspruch 1 definiert sind, oder mit einem Zwischenprodukt der Formel YQ-L₂'-NH-Ra, worin Y, Q, L₂' und Ra wie oben definiert sind unter Sonogashira-Reaktionsbedingungen;
Schritt f: Kuppeln des resultierenden Zwischenprodukts der Formel (V) 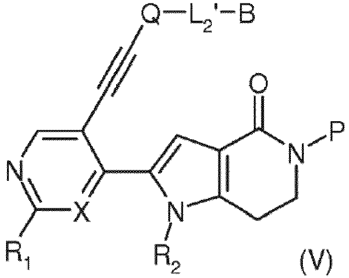 worin B COOH oder NHRa ist und X, Q, Ra, R₁, R₂, L₂' und P wie oben definiert sind, mit einem geeigneten Zwischenprodukt in Gegenwart eines Kupplungsmittels, um ein Zwischenprodukt der Formel (II) wie oben definiert zu ergeben;
Schritt b: Entschützen des Zwischenprodukts der Formel (II) wie oben definiert;
alternativ Methode D:
Schritt g: Reaktion eines Zwischenprodukts der Formel (III) wie oben definiert mit einem Zwischenprodukt der Formel ≡-Q-L₂'-CO-Y', worin L₂', Y, Y' und Q wie oben definiert sind, oder mit einem Zwischenprodukt der Formel ≡-Q-L₂'-NH-Ra, worin Y, Q, L₂' und Ra wie oben definiert sind unter Sonogashira-Reaktionsbedingungen, um ein Zwischenprodukt der Formel (V) wie oben definiert zu ergeben;
Schritt f: Kuppeln des resultierenden Zwischenprodukts der Formel (V) mit einem geeigneten Zwischenprodukt in Gegenwart eines Kupplungsmittels, um ein Zwischenprodukt der Formel (II) wie oben definiert zu ergeben;
Schritt b: Entschützen des Zwischenprodukts der Formel (II) wie oben definiert;
wahlweise Umwandeln einer Verbindung der Formel (I) in eine andere Verbindung der Formel (I) durch bekannte chemische Reaktionen; und/oder wahlweise Umwandeln einer Verbindung der Formel (I) in ein pharmazeutisch verträgliches Salz davon oder Umwandeln eines Salzes in eine freie Verbindung der Formel (I).

6. *In-vitro* Verfahren zur Hemmung der Proteinaktivität der RET-Familie, welches das Inkontaktbringen des Proteins mit einer wirksamen Menge einer Verbindung der Formel (I) wie in Anspruch 1 definiert oder einer Verbindung nach Anspruch 4 umfasst.

7. Pharmazeutische Zusammensetzung, die eine therapeutisch wirksame Menge einer Verbindung der Formel (I) wie in Anspruch 1 definiert oder einer Verbindung nach Anspruch 4 oder der pharmazeutisch verträglichen Salze davon umfasst, sowie mindestens ein pharmazeutisch verträgliches Bindemittel, einen Träger und/oder ein Verdünnungsmittel.

8. Pharmazeutische Zusammensetzung nach Anspruch 7, die ferner ein oder mehrere chemotherapeutische Mittel umfasst.

9. Produkt, das eine Verbindung der Formel (I) wie in Anspruch 1 definiert umfasst oder eine Verbindung nach Anspruch 4 oder die pharmazeutisch verträglichen Salze davon umfasst sowie ein oder mehrere Chemotherapeutika, als kombiniertes Präparat zur gleichzeitigen, getrennten oder aufeinanderfolgenden Verwendung in der Krebstherapie.

10. Verbindung der Formel (I) wie in Anspruch 1 definiert oder Verbindung nach Anspruch 4 oder pharmazeutisch verträgliche Salze davon zur Verwendung als Arzneimittel.

11. Verbindung der Formel (I) wie in Anspruch 1 definiert oder Verbindung nach Anspruch 4 oder pharmazeutisch verträgliche Salze davon zur Verwendung in einem Verfahren zur Behandlung von Krebs.

## Revendications

1. Composé de formule (I), 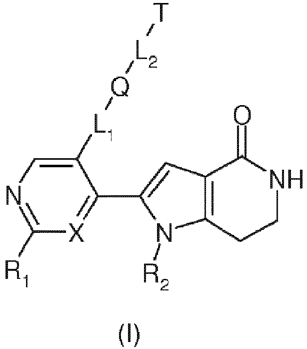 dans lequel
X est CH ou N ;
R₁ est H ou NHR₃, dans lequel R₃ est H, un groupe facultativement substitué sélectionné parmi un alkyle en C₁-C₃ linéaire ou ramifié, un hétérocyclyle et COR', dans lequel R' un groupe facultativement substitué sélectionné parmi un alkyle en C₁-C₆ linéaire ou ramifié, un carbocyclyle en C₃-C₆, un aryle et un hétéroaryle ;
L₁ est C≡C ;
Q est un aryle ou un hétéroaryle facultativement substitué sélectionné parmi : 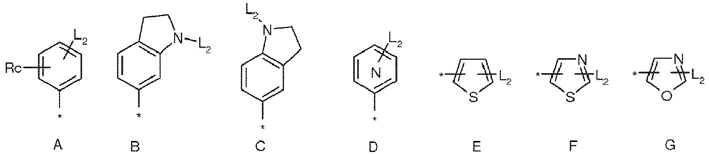
dans lequel Rc est sélectionné parmi un méthyle et un halogène, de préférence un fluor ;
L₂ est CONRa, C(RaRb)CONRa, C(RaRb)C(RaRb)CONRa, CONRaC(RaRb), OC(RaRb)CONRa, dans lequel Ra et Rb sont tous les deux un hydrogène ;
T est un aryle substitué de formule J : 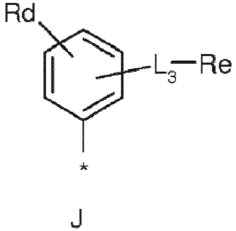 dans lequel
Rd est un halogène, un alkyle en C₁-C₄ linéaire ou ramifié facultativement substitué, un cycloalkyle en C₃-C₆ ou un trifluorométhyle ;
L₃ est une liaison directe, O, NH, NCH₃, CH₂, CH₂NH, CH₂NCH₃ ou C=O ;
Re est NRfRg, dans lequel Rf et Rg sont chacun indépendamment une chaîne alkyle en C₁-C₆ linéaire ou ramifiée facultativement substituée, dans lequel de 1 à 3 atomes de carbone dudit alkyle peuvent être indépendamment substitués par N ou 0, ou Rf et Rg joints l'un à l'autre avec l'atome d'azote représentent un cycle hétérocyclique ;
R₂ est H ou un groupe facultativement substitué sélectionné parmi un alkyle en C₁-C₆ linéaire ou ramifié, un carbocyclyle en C₃-C₆, un hétérocyclyle, un aryle et un hétéroaryle ;
ou des sels pharmaceutiquement acceptables de ceux-ci.

2. Composé de formule (I), selon la revendication 1, dans lequel
Q est un aryle facultativement substitué de formule A : 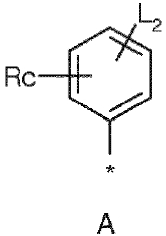 dans lequel Rc et L₂ sont tels que définis dans la revendication 1.

3. Composé ou sel pharmaceutiquement acceptable de celui-ci selon la revendication 1, qui est sélectionné dans le groupe constitué par les :
[4-(4-éthyl-pipérazin-1-ylméthyl)-3-trifluorométhylphényl]-amide de l'acide 6-[2-amino-4-(1-méthyl-4-oxo-4,5,6,7-tétrahydro-1H-pyrrolo[3,2-c]pyridin-2-yl)-pyrimi din-5-yléthynyl]-2,3-dihydro-indole-1-carboxylique (Cp 44),
[4-(4-éthyl-pipérazin-1-ylméthyl)-3-trifluorométhylphényl]-amide de l'acide 5-[2-amino-4-(1-méthyl-4-oxo-4,5,6,7-tétrahydro-1H-pyrrolo[3,2-c]pyr-idin-2-yl)-pyri midin-5-yléthynyl]-2,3-dihydro-indole-1-carboxylique (Cp 45),
3-[2-amino-4-(1-méthyl-4-oxo-4,5,6,7-tétrahydro-1H-pyr rolo[3,2-c]pyridin-2-yl)-pyrimidin-5-yléthynyl]-N-[4-(4-éthyl-pipérazin-1-ylméthyl)-3-trifluorométhyl-phényl]-benzamide (Cp 48),
N-[4-(4-éthyl-pipérazin-1-ylméthyl)-3-trifluorométhylphényl]-3-[2-méthyl-amino-4-(1-méthyl-4-oxo-4,5,6,7-té trahydro-1H-pyrrolo[3,2-c]pyridin-2-yl)-pyrimidin-5-yl éthynyl]-benzamide (Cp 49),
3-[2-amino-4-(1-méthyl-4-oxo-4,5,6,7-tétrahydro-1H-pyr rolo[3,2-c]pyridin-2-yl)-pyrimidin-5-yléthynyl]-N-[4-(4-éthyl-pipérazin-1-ylméthyl)-3-trifluorométhyl-phényl]-4-méthyl-benzamide (Cp 50),
[4-(4-éthyl-pipérazin-1-ylméthyl)-3-trifluorométhylphényl]-amide de l'acide 5-[2-amino-4-(1-méthyl-4-oxo-4,5,6,7-tétrahydro-1H-pyrrolo[3,2-c]pyridin-2-yl)-py rimidin-5-yléthynyl]-thiophène-2-carboxylique (Cp 51),
[4-(4-éthyl-pipérazin-1-ylméthyl)-3-trifluorométhylphényl]-amide de l'acide 2-[2-amino-4-(1-méthyl-4-oxo-4,5,6,7-tétrahydro-1H-pyrrolo[3,2-c]pyridin-2-yl)-py rimidin-5-yléthynyl]-thiazole-5-carboxylique (Cp 52),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl} -N-[4-(4-éthyl-pipérazin-1-ylméthyl)-3-trifluorométhylphényl]-acétamide (Cp 77),
N-[4-(4-éthyl-pipérazin-1-ylméthyl)-3-trifluorométhylphényl]-2-{3-[2-methylamino-4-(1-méthyl-4-oxo-4,5,6,7-tétrahydro-1H-pyrrolo[3,2-c]pyridin-2-yl)-pyrimidin-5-yléthynyl]-phényl}-acétamide (Cp 78),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl}-N-[3-(4-éthyl-pipérazin-1-ylméthyl)-4-trifluoro méthyl-phényl]-acétamide (Cp 82),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl}-N-[4-(4-éthyl-pipérazin-1-ylméthyl)-3-fluorophényl]-acétamide (Cp 120),
2-{5-[2-amino-4-(1-méthyl-4-oxo-4,5,6,7-tétrahydro-1H-pyrrolo[3,2-c]pyridin-2-yl)-pyrimidin-5-yléthynyl]-pyridin-3-yl}-N-[4-(4-éthyl-pipérazin-1-yl-méthyl)-3-trifluorométhyl-phényl]-acétamide (Cp 122),
2-{4-[2-amino-4-(1-méthyl-4-oxo-4,5,6,7-tétrahydro-1H-pyrrolo[3,2-c]pyridin-2-yl)-pyrimidin-5-yléthynyl]-phényl}-N-[4-(4-éthyl-pipérazin-1-ylméthyl)-3-trifluorométhyl-phényl]-acétamide (Cp 123),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl}-N-[3-(4-éthyl-pipérazine-1-carbony-l)-4-trifluorométhyl-phényl]-acétamide (Cp 127),
2-{5-[2-amino-4-(1-méthyl-4-oxo-4,5,6,7-tétrahydro-1H-pyrrolo[3,2-c]pyridin-2-yl)-pyrimidin-5-yléthynyl]-thiophén-2-yl}-N-[4-(4-éthyl-pipérazin-1-ylméthyl)-3-trifluorométhyl-phényl]-acétamide (Cp 129),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl}-N-[4-chloro-3-(4-éthyl-pipérazin-1-ylméthyl)-phényl]-acétamide (Cp 138),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl}-N-[4-(4-méthyl-[1,4]diazépan-1-ylméthyl)-3-trifluorométhyl-phényl]-acétamide (Cp 149),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl}-N-[4-((S)-3-diméthylamino-pyrrolidin-1-ylméthyl)-3-trifluorométhyl-phényl]-acetamide (Cp 150),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl}-N-[3-cyclopropyl-4-(4-méthyl-pipérazin-1-ylméthyl)-phényl]-acétamide (Cp 154),
2-{3-[2-amino-4-(1-méthyl-4-oxo-4,5,6,7-tétrahydro-1H-pyrrolo[3,2-c]pyridin-2-yl)-pyrimidin-5-yléthynyl]-4-fluoro-phényl}-N-[4-(4-éthyl-pipérazin-1-ylméthyl)-3-trifluorométhyl-phényl]-acétamide (Cp 155),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl}-N-[4-(4-isopropyl-pipérazin-1-ylméthyl)-3-trifluorométhyl-phényl]-acétamide (Cp 158),
2-{3-[2-amino-4-(1-methyl-4-oxo-4,5,6,7-tétrahydro-1H-pyrrolo[3,2-c]pyridin-2-yl)-pyrimidin-5-yléthynyl]-phényl}-N-[4-(4-méthyl-pipérazin-1-ylméthyl)-3-trifluorométhyl-phényl]-acétamide (Cp 161),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl}-N-[4-(4-propyl-pipérazin-1-ylméthyl)-3-trifluorométhyl-phényl]-acétamide (Cp 164),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl}-N-[4-(4-diméthylamino-pipéridin-1-ylméthyl)-3-trifluorométhyl-phényl]-acétamide (Cp 167),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl}-N-[4-(4-cyclopropylmethyl-pipérazin-1-ylméthyl)-3-trifluorométhyl-phényl]-acétamide (Cp 171),
2-{3-[2-méthylamino-4-(1-méthyl-4-oxo-4,5,6,7-tétrahydro-1H-pyrrolo[3,2-c-]pyridin-2-yl)-pyrimidin-5-yléthynyl]-phényl}-N-[4-(4-méthyl-pipérazin-1-ylméthyl)-3-trifluor ométhyl-phényl]-acétamide (Cp 174),
N-[4-(4-isopropyl-pipérazin-1-ylméthyl)-3-trifluor ométhyl-phényl]-2-{3-[2-méthylamino-4-(1-méthyl-4-oxo-4,5,6,7-tétrahydro-1H-pyrrolo[3,2-c]pyridin-2-yl)-pyrimidin-5-yléthynyl]-phényl}-acétamide (Cp 176),
2-{3-[2-méthylamino-4-(1-méthyl-4-oxo-4,5,6,7-tétrahydro-1H-pyrrolo[3,2-c-]pyridin-2-yl)-pyrimidin-5-yléthynyl]-phényl}-N-[4-(4-propyl-pipérazin-1-ylméthyl)-3-trifluor ométhyl-phényl]-acétamide (Cp 179),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl}-N-(4-pipérazin-1-ylméthyl-3-trifluorométhylphéényl)-acétamide (Cp 183),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl}-N-(4-{[(2-hydroxy-éthyl)-méthyl-amino]-méthyl}-3-trifluorométhyl-phényl)-acétamide (Cp 189),
2-{4-fluoro-3-[2-méthylamino-4-(1-méthyl-4-oxo-4,5,6,7-tétrahydro-1H-pyrrolo[3,2-c]pyridin-2-yl)-pyrimidin-5-yléthynyl]-phényl}-N-[4-(4-méthyl-pipérazin-1-ylméthyl)-3-trifluorométhyl-phényl]-acétamide (Cp 190),
2-(3-{2-amino-4-[1-(2-hydroxy-éthyl)-4-oxo-4,5,6,7-tétrahydro-1H-pyrrolo[3,2-c]pyridin-2-yl]-pyrimidin-5-yléthynyl}-phényl)-N-[4-(4-éthyl-pipérazin-1-ylméthyl)-3-trifluorométhyl-phényl]-acétamide (Cp 191),
2-{3-[2-éthylamino-4-(1-méthyl-4-oxo-4,5,6,7-tétrahydro-1H-pyrrolo[3,2-c]-pyridin-2-yl)-pyrimidin-5-yléthynyl]-phényl}-N-[4-(4-méthyl-pipérazin-1-ylméthyl)-3-trifluorométhyl-phényl]-acétamide (Cp 193),
N-[4-((S)-3-diméthylamino-pyrrolidin-1-ylméthyl)-3-tri fluorométhyl-phényl-]-2-{3-[2-méthylamino-4-(1-méthyl-4-oxo-4,5,6,7-tétrahydro-1H-pyrrolo[3,2-c]pyridin-2-yl)-pyrimidin-5-yléthynyl]-phényl}-acétamide (Cp 198),
2-{3-[2-acétylamino-4-(1-méthyl-4-oxo-4,5,6,7-tétrahydro-1H-pyrrolo[3,2-c-]pyridin-2-yl)-pyrimidin-5-yléthynyl]-phényl}-N-[4-(4-méthyl-pipérazin-1-ylméthyl)-3-trifluoro méthyl-phényl]-acétamide (Cp 200),
2-{3-[6-amino-4-(1-méthyl-4-oxo-4,5,6,7-tétrahydro-1H-pyrrolo[3,2-c]pyridin-2-yl)-pyridin-3-yléthynyl]-phényl}-N-[4-(4-méthyl-pipérazin-1-ylméthyl)-3-trifluorométhylphényl]-acétamide (Cp 201), et
2-{3-[2-isopropylamino-4-(1-méthyl-4-oxo-4,5,6,7-tétra hydro-1H-pyrrolo[3,-2-c]pyridin-2-yl)-pyrimidin-5-yl éthynyl]-phényl}-N-[4-(4-méthyl-pipérazin-1-ylméthyl)-3-trifluorométhyl-phényl]-acétamide (Cp 202).

4. Composé ou un sel pharmaceutiquement acceptable de celui-ci sélectionné dans le groupe constitué par les :
1-{3-[2-amino-4-(1-méthyl-4-oxo-4,5,6,7-tétrahydro-1H-pyrrolo[3,2-c]pyridin-2-yl)-pyrimidin-5-yléthynyl]-phé nyl}-3-[4-(4-éthyl-pipérazin-1-ylméthyl)-3-trifluoro méthyl-phényl]-urée (Cp 8),
1-{3-[2-amino-4-(1-méthyl-4-oxo-4,5,6,7-tétrahydro-1H-pyrrolo[3,2-c]pyridin-2-yl)-pyrimidin-5-yléthynyl]-phényl} -3-[3-(4-éthyl-pipérazin-1-ylméthyl)-4-trifluorométhylphényl]-urée (Cp 22),
1-{3-[2-amino-4-(1-méthyl-4-oxo-4,5,6,7-tétrahydro-1H-pyrrolo[3,2-c]pyridin-2-yl)-pyrimidin-5-yléthynyl]-phényl} -3-[3-(4-éthyl-pipérazin-1-ylméthyl)-5-trifluorométhylphényl]-urée (Cp 29),
1-{4-[2-amino-4-(1-méthyl-4-oxo-4,5,6,7-tétrahydro-1H-pyrrolo[3,2-c]pyridin-2-yl)-pyrimidin-5-yléthynyl]-phényl} -3-[4-(4-éthyl-pipérazin-1-ylméthyl)-3-trifluorométhylphényl]-urée (Cp 30),
1-{5-[2-amino-4-(1-méthyl-4-oxo-4,5,6,7-tétrahydro-1H-pyrrolo[3,2-c]pyridin-2-yl)-pyrimidin-5-yléthynyl]-2-fluoro-phényl}-3-[4-(4-éthyl-pipérazin-l-ylméthyl)-3-trifluorométhyl-phényl]-urée (Cp 36),
1-[4-(4-éthyl-pipérazin-1-ylméthyl)-3-trifluorométhylphényl]-3-{3-[4-(1-méthyl-4-oxo-4,5,6,7-tétrahydro-1H-pyrrolo[3,2-c]pyridin-2-yl)-pyridin-3-y-léthynyl]-phényl}-urée (Cp 38),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl}-N-(5-tert-butyl-2-méthyl-2H-pyrazol-3-yl)-acétamide (Cp 105),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl}-N-[4-(1-méthyl-pipéridin-4-yloxy)-3-trifluoro méthyl-phényl]-acétamide (Cp 172),
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl}-N-[4-(1-méthyl-pipéridin-4-ylamino)-3-trifluoro méthyl-phényl]-acétamide (Cp 177) et
2-{3-[2-amino-4-(1-méthyl-4-oxo-4,5,6,7-tétrahydro-1H-pyrrolo[3,2-c]pyridin-2-yl)-pyrimidin-5-yléthynyl]-phényl} -N-{4-[méthyl-(1-méthyl-pipéridin-4-yl)-amino]-3-tri fluorométhyl-phényl}-acétamide (Cp 178).

5. Procédé de préparation d'un composé de formule (I) selon la revendication 1 ou de préparation d'un composé selon la revendication 4, ou des sels pharmaceutiquement acceptables de ceux-ci, **caractérisé en ce que** le procédé comprend les étapes suivantes :
méthode A :
étape a :
couplage d'un dérivé de pyrrolopyridinone de formule (III) 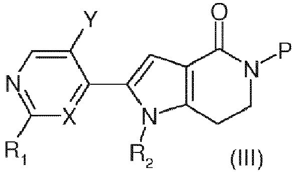 dans lequel X, R₁ et R₂ sont tels que définis dans la revendication 1, Y est un halogène et P est un groupe protecteur adapté tel que le groupe tert-butoxycarbonyle, avec un intermédiaire de formule (la)-(lh) 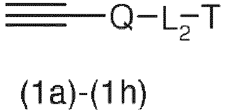 dans lequel L₂ est CONRa, C(RaRb)CONRa, C(RaRb)C(RaRb)CONRa, CONRaC(RaRb), OC(RaRb)CONRa, NRaCONRa et Ra, Rb, Q et T sont tels que définis dans la revendication 1, dans des conditions de réaction de Sonogashira ;
étape b : déprotection de l'intermédiaire résultant de formule (II) 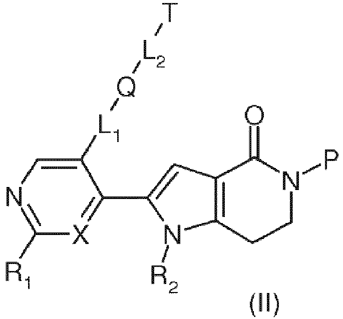 dans lequel L₁ est C≡C et X, R₁, R₂, L₂, Q, T et P sont tels que définis ci-dessus, dans des conditions acides pour donner un composé de formule (I) tel que défini dans la revendication 1 ou un composé selon la revendication 4 ;
en variante, méthode B :
étape c : couplage d'un dérivé de pyrrolopyridinone de formule (III), tel que défini ci-dessus, avec un éthynyltriméthyl-silane dans des conditions de réaction de Sonogashira, suivi par le retrait du groupe triméthylsilyle dans des conditions basiques ;
étape d : réaction de l'intermédiaire résultant de formule (IV) 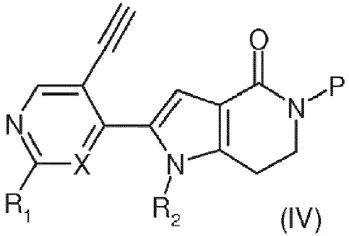 dans lequel X, R₁ et R₂ sont tels que définis dans la revendication 1 et P est tel que défini ci-dessus, avec un intermédiaire de formule (3a)-(3h) 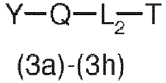 dans lequel Y est un halogène, L₂ est CONRa, C(RaRb)CONRa, C(RaRb)C(RaRb)CONRa, CONRaC(RaRb), OC(RaRb)CONRa, NRaCONRa et Ra, Rb, Q et T sont tels que définis dans la revendication 1, dans des conditions de réaction de Sonogashira ;
étape b : déprotection de l'intermédiaire résultant de formule (II) tel que défini ci-dessus ;
en variante, méthode C :
étape c : couplage d'un dérivé de pyrrolopyridinone de formule (III), tel que défini ci-dessus, avec un éthynyltriméthyl-silane dans des conditions de réaction de Sonogashira, suivi par le retrait du groupe triméthylsilyle dans des conditions basiques ;
étape e : mise en réaction de l'intermédiaire résultant de formule (IV) tel que défini ci-dessus, avec un intermédiaire de formule Y-Q-L₂'-CO-Y' dans lequel L₂' est une liaison directe, -C(RaRb)-, -C(RaRb)C(RaRb)- ou - OC(RaRb)-, Y est un halogène, Y' est OH et Q, Ra et Rb sont tels que définis dans la revendication 1, ou avec un intermédiaire de formule Y-Q-L₂'-NH-Ra dans lequel Y, Q, L₂' et Ra sont tels que définis ci-dessus dans des conditions de réaction de Sonogashira ;
étape f : couplage de l'intermédiaire résultant de formule (V) 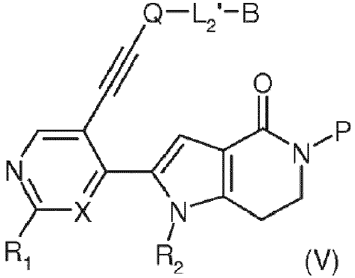 dans lequel B est COOH ou NHRa et X, Q, Ra, R₁, R₂, L₂' et P sont tels que définis ci-dessus, avec un intermédiaire adapté en présence d'un agent de couplage pour donner un intermédiaire de formule (II) tel que défini ci-dessus ;
étape b : déprotection de l'intermédiaire de formule (II) tel que défini ci-dessus ;
en variante, méthode D :
étape g : mise en réaction d'un intermédiaire de formule (III) tel que défini ci-dessus avec un intermédiaire de formule ≡-Q-L₂'-CO-Y' dans lequel L₂', Y, Y' et Q sont tels que définis ci-dessus, ou avec un intermédiaire de formule ≡-Q-L₂'-NH-Ra dans lequel Y, Q, L₂' et Ra sont tels que définis ci-dessus dans des conditions de réaction de Sonogashira pour donner un intermédiaire de formule (V), tel que défini ci-dessus ;
étape f : couplage de l'intermédiaire résultant de formule (V) avec un intermédiaire adapté en présence d'un agent de couplage pour donner un intermédiaire de formule (II) tel que défini ci-dessus ;
étape b : déprotection de l'intermédiaire de formule (II) tel que défini ci-dessus ;
conversion facultative d'un composé de formule (I) en un composé de formule (I) différent par des réactions chimiques connues ; et/ou, si souhaité, conversion d'un composé de formule (I) en un sel pharmaceutiquement acceptable de celui-ci ou conversion d'un sel en un composé libre de formule (I).

6. Procédé *in vitro* d'inhibition de l'activité des protéines de la famille RET qui comprend la mise en contact de ladite protéine avec une quantité efficace d'un composé de formule (I) selon la revendication 1, ou d'un composé selon la revendication 4.

7. Composition pharmaceutique comprenant une quantité thérapeutiquement efficace d'un composé de formule (I) selon la revendication 1, ou d'un composé selon la revendication 4, ou de leurs sels pharmaceutiquement acceptables, et au moins un excipient, un vecteur et/ou un diluant pharmaceutiquement acceptables.

8. Composition pharmaceutique, selon la revendication 7, comprenant en outre un ou plusieurs agents chimiothérapeutiques.

9. Produit comprenant un composé de formule (I) selon la revendication 1, ou comprenant un composé selon la revendication 4, ou leurs sels pharmaceutiquement acceptables, et un ou plusieurs agents chimiothérapeutiques, sous la forme d'une préparation combinée pour une utilisation simultanée, séparée ou séquentielle dans une thérapie anticancéreuse.

10. Composé de formule (I) selon la revendication 1, ou un composé selon la revendication 4, ou leurs sels pharmaceutiquement acceptables, pour leur utilisation en tant que médicament.

11. Composé de formule (I) selon la revendication 1, ou un composé selon la revendication 4, ou leurs sels pharmaceutiquement acceptables, pour leur utilisation dans un procédé de traitement du cancer.
